# EUROPÄISCHE PATENTSCHRIFT

(11) **EP 1 347 957 B1**
(45) Veröffentlichungstag und Bekanntmachung des Hinweises auf die Patenterteilung: **07.09.2005**
(21) Anmeldenummer: 01999212.2
(22) Anmeldetag: 22.11.2001
(51) Int. Cl.: C07D 207/20, C07D 401/10, C07D 403/10, A01N 43/36, A01N 43/40, A01N 43/54, A01N 43/58, A01N 43/60

(54) **DELTA 1-PYRROLINE ALS SCHÄDLINGSBEKÄMPFUNGSMITTEL**
DELTA 1-PYRROLINES USED AS PESTICIDES
DELTA 1-PYRROLINES UTILISEES COMME AGENTS DE LUTTE CONTRE LES PARASITES

(30) Priorität: 05.12.2000 DE 10060412
(43) Veröffentlichungstag der Anmeldung: 01.10.2003
(73) Patentinhaber: Bayer CropScience AG, 40789 Monheim (DE)
(72) Erfinder: PLANT, Andrew, Winnersh, Berkshire RG 41 5PD (GB); SEITZ, Thomas, 40764 Langenfeld (DE); JANSEN, Johannes, Rudolf, 40789 Monheim (DE); ERDELEN, Christoph, 42799 Leichlingen (DE); TURBERG, Andreas, 42781 Haan (DE); HANSEN, Olaf, 42799 Leichlingen (DE)
(86) Internationale Anmeldenummer: PCT/EP2001/013585
(87) Internationale Veröffentlichungsnummer: WO 2002/046151

(56) Entgegenhaltungen:
- WO-A-98/22438
- DE-A- 19 822 245
- DE-A- 19 822 247
- DE-A- 19 847 076

## Beschreibung

Die vorliegende Erfindung betrifft neue Δ¹-Pyroline, mehrere Verfahren zu ihrer Herstellung und ihre Verwendung als Schädlingsbekämpfungsmittel.

Es ist bereits bekannt, dass zahlreiche Δ¹-Pyrroline insektizide Eigenschaften besitzen (vgl. WO 00/21958, WO 99/59968, WO 99/59967 und WO 98/22438). Die Wirksamkeit dieser Stoffe ist gut, lässt aber in manchen Fällen zu wünschen übrig.

Es wurden nun neue Δ¹-Pyrroline der Formel (I) 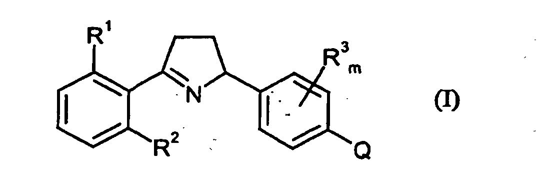 gefunden, in welcher
- R¹: für Halogen oder Methyl steht,
- R²: für Wasserstoff oder Halogen steht,
- R³: für Halogen, für jeweils gegebenenfalls durch Halogen substituiertes Alkyl, Alkoxy oder Alkylthio steht,
- m: für 0, 1, 2, 3 oder 4 steht,
- Q: für eine der folgenden Gruppierungen 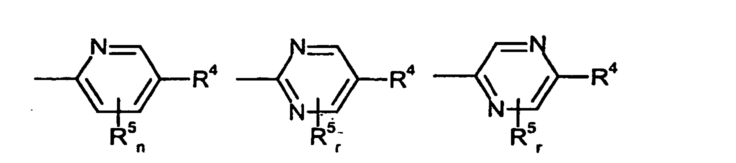 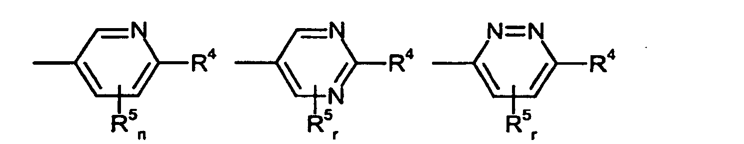
- R⁴: steht, für Wasserstoff, Halogen, Cyano, Formyl, Nitro, Trialkylsilyl; für jeweils gegebenenfalls einfach oder mehrfach, gleich oder verschieden durch Halogen, Cyano und/oder -NR⁷R⁸ substituiertes Alkyl, Alkenyl, Alkoxy, Alkenyloxy; für Pentafluorthio, -S(O)ₚR⁶, -NR⁷R⁸, -COR⁶, -CO₂R⁶, -CONR⁹R¹⁰, -N(R¹¹)COR¹² oder -C(R¹³)=N-OR¹⁴; oder für jeweils gegebenenfalls einfach oder mehrfach, gleich oder verschieden durch Halogen, Cyano, Nitro, Alkyl, Halogenalkyl, Alkenyl, Halogenalkenyl, Alkoxy, Halogenalkoxy, Alkylthio und/oder Halogenalkylthio substituiertes Cycloalkyl, Cycloalkylalkyl, Aryl, Arylalkyl, gesättigtes oder ungesättigtes, 5- bis 10-gliedriges Heterocyclyl oder Heterocyclylalkyl steht,
- R⁴: außerdem für -CH=NOH, Formyl; für jeweils gegebenenfalls einfach oder mehrfach, gleich oder verschieden durch Halogen und/oder Alkyl substituiertes Cycloalkyloxy oder Cycloalkylalkoxy steht,
- R⁵: für Halogen, Cyano, Formyl, Nitro, Trialkylsilyl; für jeweils gegebenenfalls einfach oder mehrfach, gleich oder verschieden durch Halogen, Cyano und/oder -NR⁷R⁸ substituiertes Alkyl, Alkenyl, Alkoxy, Alkenyloxy; für Pentafluorthio, -S(O)ₚR⁶, -NR⁷R⁸, -COR⁶, -CO₂R⁶, -CONR⁹R¹⁰, -N(R¹¹)COR¹² oder -C(R¹³)=N-OR¹⁴; oder für jeweils gegebenenfalls einfach oder mehrfach, gleich oder verschieden durch Halogen, Alkyl, Halogenalkyl, Alkenyl, Halogenalkenyl, Alkoxy, Halogenalkoxy, Alkylthio und/oder Halogenalkylthio substituiertes Cycloalkyl, Cycloalkylalkyl, Aryl, Arylalkyl, gesättigtes oder ungesättigtes, 5- bis 10-gliedriges Heterocyclyl oder Heterocyclylalkyl steht,
- p: für 0, 1 oder 2 steht,
- n: für 0, 1, 2 oder 3 steht, wobei Substituenten R⁵ gleich oder verschieden sein können, wenn n für 2 oder 3 steht,
- r: für 0, 1 oder 2 steht, wobei Substituenten R⁵ gleich oder verschieden sein können, wenn r für 2 steht,
- R⁶: für gegebenenfalls einfach oder mehrfach, gleich oder verschieden durch Halogen und/oder -NR⁷R⁸ substituiertes Alkyl, für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Aryl oder Arylalkyl steht,
- R⁶: außerdem für einfach oder mehrfach, gleich oder verschieden substituiertes Alkyl steht, wobei die Substituenten zusätzlich aus Alkoxy, Alkylthio, Halogenalkoxy und/oder Halogenalkylthio ausgewählt sein können,
- R⁷ und R⁸: unabhängig voneinander für Wasserstoff, -SO₂R⁶, -CO_{R}⁶, -CO₂R⁶, für jeweils gegebenenfalls einfach oder mehrfach, gleich oder verschieden durch Halogen, Alkylcarbonyl, Alkylcarbonyloxy, Alkylamino, Dialkylamino, Alkoxy, Halogenalkoxy, Alkylthio und/oder Halogenalkylthio substituiertes Alkyl oder Alkenyl; für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Cycloalkylalkyl, Aryl, Arylalkyl, gesättigtes oder ungesättigtes, 5- bis 10-gliedriges Heterocyclyl oder Heterocyclylalkyl stehen,
- R⁷ und R⁸: außerdem gemeinsam für gegebenenfalls einfach oder mehrfach, gleich oder verschieden durch Halogen, Cyano, Alkoxy, Halogenalkoxy, Alkylthio und/oder Halogenalkylthio substituiertes Alkenylen oder für gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Alkylen, wobei die Alkylenkette jeweils durch -O-, -S- oder -NR¹⁵- unterbrochen sein kann, stehen,
- R⁷ und R⁸: außerdem gemeinsam für einfach oder mehrfach, gleich oder verschieden substituiertes Alkylen, wobei die Substituenten zusätzlich aus Alkoxycarbonyl und/oder Oxyalkylenoxy ausgewählt sein können, stehen,
- R⁷ und R⁸: außerdem gemeinsam für Alkylen, wobei die Alkylenkette entweder durch C=O oder C=NO-Alkyl unterbrochen ist, stehen,
- R⁹ und R¹⁰: unabhängig voneinander für Wasserstoff, -SO₂R⁶, für jeweils gegebenenfalls einfach oder mehrfach, gleich oder verschieden durch Halogen, Alkylamino, Dialkylamino, Alkoxy und/oder Alkylthio substituiertes Alkyl oder Alkenyl; für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Cycloalkylalkyl, Aryl, Arylalkyl, gesättigtes oder ungesättigtes, 5- bis 10-gliedriges Heterocyclyl oder Heterocyclylalkyl stehen,
- R⁹ und R¹⁰: außerdem gemeinsam für gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Alkylen, wobei die Alkylenkette jeweils durch -O-, -S- oder -NR¹⁵- unterbrochen sein kann, stehen,
- R¹¹ und R¹²: unabhängig voneinander für Wasserstoff, für gegebenenfalls einfach oder mehrfach, gleich oder verschieden durch Halogen, Cyano, Alkoxy und/oder Alkylthio substituiertes Alkyl, für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Cycloalkylalkyl, Aryl oder Arylalkyl stehen,
- R¹¹ und R¹²: außerdem gemeinsam für jeweils gegebenenfalls einfach oder mehrfach, gleich oder verschieden durch Halogen, Alkyl, Halogenalkyl, Alkoxy, Halogenalkoxy, Alkylthio und/oder Halogenalkylthio substituiertes Alkylen oder Alkenylen stehen,
- R¹³ und R¹⁴: unabhängig voneinander für Wasserstoff, für jeweils gegebenenfalls einfach oder mehrfach durch Halogen substituiertes Alkyl oder Alkenyl stehen,
- R¹⁵: für Wasserstoff, -SO₂R⁶, -COR⁶ oder -CO₂R⁶; für jeweils gegebenenfalls einfach oder mehrfach, gleich oder verschieden durch Halogen, Cyano, Alkylamino, Dialkylamino, Alkoxy, Halogenalkoxy, Alkylthio und/oder Halogenalkylthio substituiertes Alkyl oder Alkenyl; für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Cycloalkylalkyl, Aryl, Arylalkyl, gesättigtes oder ungesättigtes, 5- bis 10-gliedriges Heterocyclyl oder Heterocyclylalkyl steht.

Die Verbindungen der Formel (I) können gegebenenfalls in Abhängigkeit von der Art und Anzahl der Substituenten als geometrische und/oder optische Isomere bzw. Regioisomere oder deren Isomerengemische in unterschiedlicher Zusammensetzung vorliegen. Sowohl die reinen Isomere als auch die Isomerengemische werden erfindungsgemäß beansprucht.

Weiterhin wurde gefunden, dass sich Δ¹-Pyrroline der Formel (I) herstellen lassen, indem man
A) Δ¹-Pyrroline der Formel (II) 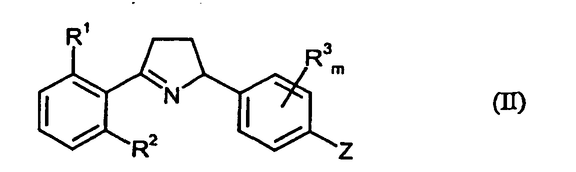 in welcher
   R¹, R², R³ und m die oben angegebenen Bedeutungen haben, und
   - Z: für Chlor, Brom, Iod, -OSO₂CF₃ oder -OSO₂(CF₂)₃CF₃ steht,
   mit Heterocyclen der Formel (III)

   Q-X (III)

   in welcher
   Q die oben angegebene Bedeutung hat, und
   - X: für Chlor, Brom, Iod, -OSO₂CF₃ oder -OSO₂(CF₂)₃CF₃ steht,
   in Gegenwart eines Katalysators, in Gegenwart eines Diboronsäureesters und gegebenenfalls in Gegenwart eines Säurebindemittels und gegebenenfalls in Gegenwart eines Verdünnungsmittels in einer Tandem-Reaktion umsetzt,
   oder
B) Δ¹-Pyrroline der Formel (IV) 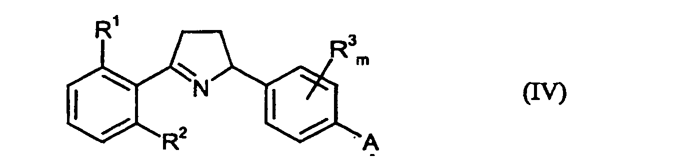 in welcher
   R¹, R², R³ und m die oben angegebenen Bedeutungen haben,
   und
   - A: für -B(OH)₂, (4,4,5,5-Tetramethyl-1,3,2-dioxaborolan)-2-yl, (5,5-Dimethyl-1,3,2-dioxaborinan)-2-yl, (4,4,6-Trimethyl-1,3,2-dioxaborinan)-2-yl oder 1,3,2-Benzodioxaborol-2-yl steht,
   mit Heterocyclen der Formel (III)

   Q-X (III)

   in welcher
   Q und X die oben angegebenen Bedeutungen haben,
   in Gegenwart eines Katalysators, gegebenenfalls in Gegenwart eines Säurebindemittels und gegebenenfalls in Gegenwart eines Verdünnungsmittels umsetzt,
   oder
C) Δ¹-Pyrroline der Formel (II) 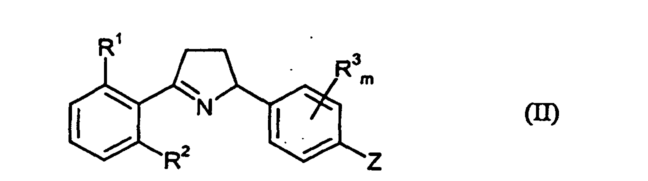 in welcher
   R¹, R², R³, m und Z die oben angegebenen Bedeutungen haben,
   mit Boronsäure-Derivaten der Formel (V)

   Q-A (V)

   in welcher
   Q und A die oben angegebenen Bedeutungen haben,
   in Gegenwart eines Katalysators, gegebenenfalls in Gegenwart eines Säurebindemittels und gegebenenfalls in Gegenwart eines Verdünnungsmittels umsetzt,
   oder
D) Δ¹-Pyrroline der Formel (II-a) 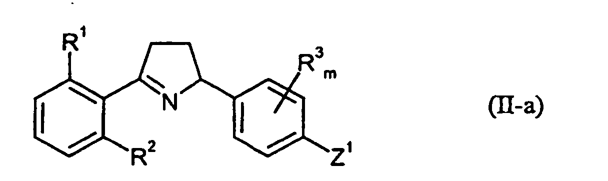
in welcher
R¹, R², R³, m die oben angegebenen Bedeutungen haben,
- Z¹: für Brom oder Iod steht,
mit organometallischen Verbindungen der Formel (VI)

Q-M (VI)

in welcher
Q die oben angegebenen Bedeutungen hat,
und
- M: für ZnCl, Sn(Me)₃ oder Sn(n-Bu)₃ steht,
in Gegenwart eines Katalysators, gegebenenfalls in Gegenwart eines Säurebindemittels und gegebenenfalls in Gegenwart eines Verdünnungsmittels umsetzt.

Schließlich wurde gefunden, dass die erfindungsgemäßen Verbindungen der Formel (I) sehr gute insektizide Eigenschaften besitzen und sich sowohl im Pflanzenschutz als auch im Materialschutz zur Bekämpfung unerwünschter Schädlinge, wie Insekten, verwenden lassen.

Die erfindungsgemäßen Δ¹-Pyrroline sind durch die Formel (I) allgemein definiert.
- R¹: steht bevorzugt für Fluor, Chlor, Brom oder Methyl.
- R²: steht bevorzugt für Wasserstoff, Fluor, Chlor oder Brom.
- R³: steht bevorzugt für Fluor, Chlor, Brom, für jeweils gegebenenfalls einfach bis achtfach durch Halogen substituiertes C₁-C₆-Alkyl, C₁-C₆-Alkoxy oder C₁-C₆-Alkylthio.
- m: steht bevorzugt für 0, 1, 2 oder 3.
- Q: steht bevorzugt für eine der folgenden Gruppierungen

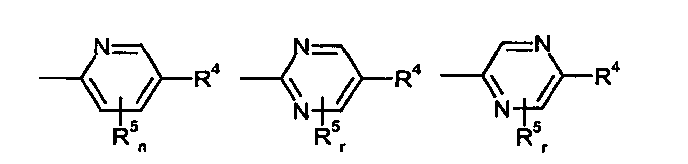

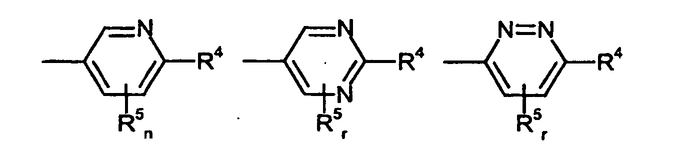

- R⁴: steht bevorzugt für Wasserstoff, Fluor, Chlor, Brom, Cyano, Formyl, Nitro, Tri-(C₁-C₆-alkyl)silyl; für jeweils gegebenenfalls einfach oder mehrfach, gleich oder verschieden durch Halogen, Cyano, -NR⁷R⁸ substituiertes C₁-C₂₀-Alkyl, C₂-C₂₀-Alkenyl, C₁-C₂₀-Alkoxy, C₂-C₂₀-Alkenyloxy; für Pentafluorthio, -S(O)ₚR⁶, -NR⁷R⁸, -COR⁶, -CO₂R⁶, -CONR⁹R¹⁰, -N(R¹¹)COR¹² oder -C(R¹³)=N-OR¹⁴; oder für jeweils gegebenenfalls einfach bis vierfach, gleich oder verschieden, durch Halogen, Cyano, Nitro, C₁-C₆-Alkyl, C₁-C₆-Halogenalkyl, C₂-C₆-Alkenyl, C₂-C₆-Halogenalkenyl, C₁-C₆-Alkoxy, C₁-C₆-Halogenalkoxy, C₁-C₆-Alkylthio, C₁-C₆-Halogenalkylthio substituiertes C₃-C₁₂-Cycloalkyl, C₃-C₇-Cycloalkyl-C₁-C₄-alkyl, Aryl, Aryl-C₁-C₄-alkyl, gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidino, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Piperidino, Morpholinyl, Thiomorpholinyl, Morpholino, Thiomorpholino, Triazinyl, Triazolyl, Chinolinyl oder Isochinolinyl).
- R⁴: steht außerdem bevorzugt für -CH=NOH, Formyl; für jeweils gegebenenfalls einfach bis vierfach, gleich oder verschieden durch Fluor, Chlor, Brom und/oder C₁-C₂-Alkyl substituiertes C₃-C₆-Cycloalkyloxy oder C₃-C₆-Cycloalkyl-C₁-C₄-alkoxy.
- R⁵: steht bevorzugt für Fluor, Chlor, Brom, Cyano, Formyl, Nitro, Tri-(C₁-C₆-alkyl)silyl; für jeweils gegebenenfalls einfach bis dreizehnfach durch Halogen substituiertes C₁-C₆-Alkyl, C₂-C₆-Alkenyl, C₁-C₆-Alkoxy, C₂-C₆-Alkenyloxy; für Pentafluorthio, -S(O)ₚR⁶, -NR⁷R⁸, -COR⁶, -CO₂R⁶, -CONR⁹R¹⁰ oder -N(R¹¹)COR¹².
- p: steht bevorzugt für 0, 1 oder 2.
- n: steht bevorzugt für 0, 1, 2 oder 3, wobei Substituenten R⁵ gleich oder verschieden sein können, wenn n für 2 oder 3 steht,
- r: steht bevorzugt für 0, 1 oder 2, wobei Substituenten R⁵ gleich oder verschieden sein können, wenn r für 2 steht,
- R⁶: steht bevorzugt für gegebenenfalls einfach oder mehrfach, gleich oder verschieden durch Halogen oder -NR⁷R⁸ substituiertes C₁-C₂₀-Alkyl, für jeweils gegebenenfalls einfach bis achtfach, gleich oder verschieden durch-Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₆-Cycloalkyl, Aryl oder Aryl-C₁-C₄-alkyl.
- R⁶: steht außerdem bevorzugt für einfach oder mehrfach, gleich oder verschieden substituiertes C₁-C₂₀-Alkyl, wobei die Substituenten zusätzlich aus C₁-C₆-Alkoxy, C₁-C₆-Alkylthio, C₁-C₆-Halogenalkoxy und/oder C₁-C₆-Halogenalkylthio ausgewählt sein können.
- R⁷ und R⁸: stehen unabhängig voneinander bevorzugt für Wasserstoff, -SO₂R⁶, -COR⁶, -CO₂R⁶, für jeweils gegebenenfalls einfach oder mehrfach, gleich oder verschieden durch Halogen, C₁-C₆-Alkylcarbonyl, C₁-C₆-Alkyl-carbonyloxy, C₁-C₆-Alkylamino, Di-(C₁-C₆-alkyl)amino, C₁-C₆-Alkoxy, C₁-C₆-Halogenalkoxy, C₁-C₆-Alkylthio, C₁-C₆-Halogenalkylthio substituiertes C₁-C₂₀-Alkyl oder C₂-C₂₀-Alkenyl; für jeweils gegebenenfalls einfach bis vierfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₁₂-Cycloalkyl, C₃-C₇-Cycloalkyl-C₁-C₄-alkyl, Aryl, Aryl-C₁-C₄-alkyl, gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl).
- R⁷ und R⁸: stehen außerdem unabhängig voneinander bevorzugt für jeweils gegebenenfalls einfach bis vierfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Pyridinylmethyl, Pyridinylethyl, Pyrimidinylmethyl, Pyrimidinylethyl, Pyridazylmethyl, Pyridazylethyl, Pyrazinylmethyl oder Pyrazinylethyl).
- R⁷ und R⁸: stehen außerdem gemeinsam bevorzugt für gegebenenfalls einfach oder mehrfach, gleich oder verschieden durch Halogen, Cyano, C₁-C₆-Alkoxy, C₁-C₆-Halogenalkoxy, C₁-C₆-Alkylthio, C₁-C₆-Halogenalkylthio substituiertes C₂-C₁₂-Alkenylen oder für gegebenenfalls im Alkylenteil einfach oder mehrfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₁₂-Alkylen, wobei die Alkylenkette jeweils durch -O-, -S- oder -NR¹⁵- unterbrochen sein kann.
- R⁷ und R⁸: stehen außerdem gemeinsam bevorzugt für gegebenenfalls einfach oder mehrfach, gleich oder verschieden substituiertes C₃-C₁₂-Alkylen, wobei die Substituenten zusätzlich aus C₁-C₄-Alkoxycarbonyl und/oder Oxy-(C₁-C₄-alkylen)-oxy ausgewählt sein können.
- R⁷ und R⁸: stehen außerdem gemeinsam bevorzugt für C₃-C₈-Alkylen, wobei die Alkylenkette entweder durch C=O oder C=NO-(C₁-C₆-Alkyl) unterbrochen ist.
- R⁹ und R¹⁰: stehen unabhängig voneinander bevorzugt für Wasserstoff, -SO₂R⁶, für jeweils gegebenenfalls einfach bis dreizehnfach, gleich oder verschieden durch Halogen, C₁-C₆-Alkylamino, Di-(C₁-C₆-alkyl)amino, C₁-C₆-Alkoxy, C₁-C₆-Halogenalkoxy, C₁-C₆-Alkylthio, C₁-C₆-Halogenalkylthio substituiertes C₁-C₆-Alkyl oder C₂-C₆-Alkenyl; für jeweils gegebenenfalls einfach bis vierfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₇-Cycloalkyl, C₃-C₇-Cycloalkyl-C₁-C₄-alkyl, Aryl, Aryl-C₁-C₄-alkyl, gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl).
- R⁹ und R¹⁰: stehen außerdem gemeinsam bevorzugt für jeweils gegebenenfalls im Alkylenteil einfach bis vierfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₆-Alkylen, -(CH₂)₂-O-(CH₂)₂-, -(CH₂)₂-S-(CH₂)₂- oder -(CH₂)₂-N(R¹⁵)-(CH₂)₂-.
- R¹¹ und R¹²: stehen unabhängig voneinander bevorzugt für Wasserstoff, für gegebenenfalls einfach bis dreizehnfach, gleich oder verschieden durch Halogen, Cyano, C₁-C₆-Alkoxy, C₁-C₆-Alkylthio substituiertes C₁-C₆-Alkyl, für jeweils gegebenenfalls einfach bis achtfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₇-Cycloalkyl, C₃-C₇-Cycloalkyl-C₁-C₄-alkyl, Aryl oder Aryl-C₁-C₄-alkyl.
- R¹¹ und R¹²: stehen außerdem gemeinsam bevorzugt für jeweils gegebenenfalls einfach bis achtfach, gleich oder verschieden, durch Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₁₀-Alkylen oder C₃-C₁₀-Alkenylen.
- R¹³ und R¹⁴: stehen unabhängig voneinander bevorzugt für Wasserstoff, für jeweils gegebenenfalls einfach oder mehrfach durch Halogen substituiertes C₁-C₆-Alkyl oder C₂-C₆-Alkenyl.
- R¹⁵: steht bevorzugt für Wasserstoff, -SO₂R⁶, -COR⁶ oder -CO₂R⁶; für jeweils gegebenenfalls einfach oder mehrfach, gleich oder verschieden durch Halogen, Cyano, C₁-C₆-Alkylamino, Di-(C₁-C₆-alkyl)amino, C₁-C₆-Alkoxy, C₁-C₆-Halogenalkoxy, C₁-C₆-Alkylthio, C₁-C₆-Halogenalkylthio substituiertes C₁-C₂₀-Alkyl oder C₂-C₂₀-Alkenyl; für jeweils gegebenenfalls einfach bis vierfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₁₂-Cycloalkyl, C₃-C₇-Cycloalkyl-C₁-C₄-alkyl, Aryl, Aryl-C₁-C₄-alkyl, gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl).
- R¹: steht besonders bevorzugt für Fluor, Chlor oder Methyl.
- R²: steht besonders bevorzugt für Wasserstoff, Fluor oder Chlor.
- R³: steht besonders bevorzugt für Fluor, Chlor, C₁-C₄-Alkyl, C₁-C₄-Alkoxy, C₁-C₄-Alkylthio; C₁-C₄-Halogenalkyl, C₁-C₄-Halogenalkoxy, C₁-C₄-Halogenalkylthio mit jeweils 1 bis 9 Fluor, Chlor und/oder Bromatomen.
- m: steht besonders bevorzugt für 0, 1 oder 2.
- Q: steht besonders bevorzugt für eine der folgenden Gruppierungen

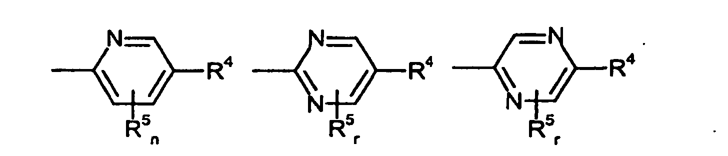

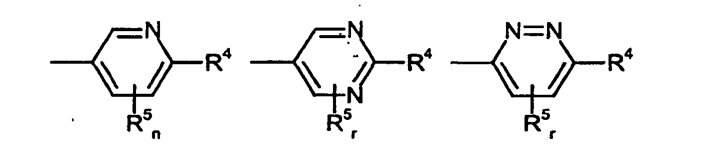

- R⁴: steht besonders bevorzugt für Wasserstoff, Fluor, Chlor, Brom, Cyano, Formyl, Nitro, Tri-(C₁-C₄-alkyl)silyl; für jeweils gegebenenfalls einfach oder mehrfach, gleich oder verschieden durch Fluor, Chlor, Brom, Cyano, -NR⁷R⁸ substituiertes C₁-C₁₆-Alkyl, C₂-C₁₆-Alkenyl, C₁-C₁₆-Alkoxy, C₂-C₁₆-Alkenyloxy; für -S(O)ₚR⁶, -NR⁷R⁸, -COR⁶, -CO₂R⁶, -CONR⁹R¹⁰ oder -N(R¹¹)COR¹²; oder für jeweils gegebenenfalls einfach bis dreifach, gleich oder verschieden, durch Fluor, Chlor, Brom, Cyano, C₁-C₄-Alkyl, C₁-C₄-Halogenalkyl, C₂-C₆-Alkenyl, C₂-C₆-Halogenalkenyl, C₁-C₄-Alkoxy, C₁-C₄-Halogenalkoxy, C₁-C₄-Alkylthio, C₁-C₄-Halogenalkylthio substituiertes C₃-C₁₀-Cycloalkyl, C₃-C₆-Cycloalkyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidino, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Piperidino, Morpholinyl, Thiomorpholinyl, Morpholino, Thiomorpholino, Triazinyl, Triazolyl, Chinolinyl oder Isochinolinyl.
- R⁴: steht außerdem besonders bevorzugt für -CH=NOH, Formyl; für jeweils gegebenenfalls einfach bis dreifach, gleich oder verschieden durch Fluor, Chlor und/oder Methyl substituiertes Cyclopropyloxy, Cyclobutyloxy, Cyclopentyloxy, Cyclohexyloxy, Cyclopropylmethoxy, Cyclobutylmethoxy, Cyclopentylmethoxy oder Cyclohexylmethoxy.
- R⁵: steht besonders bevorzugt für Fluor, Chlor, Trimethylsilyl, für jeweils gegebenenfalls einfach bis neunfach, gleich oder verschieden durch Fluor, Chlor und/oder Brom substituiertes C₁-C₄-Alkyl, C₂-C₆-Alkenyl, C₁-C₄-Alkoxy, C₂-C₆-Alkenyloxy, für -S(O)ₚR⁶, -NR⁷R⁸, -COR⁶, -CO₂R⁶ oder -CONR⁹R¹⁰.
- p: steht besonders bevorzugt für 0, 1 oder 2.
- n: steht besonders bevorzugt für 0, 1 oder 2, wobei Substituenten R⁵ gleich oder verschieden sein können, wenn n für 2 steht,
- r: steht besonders bevorzugt für 0, 1 oder 2, wobei Substituenten R⁵ gleich oder verschieden sein können, wenn r für 2 steht,
- R⁶: steht besonders bevorzugt für gegebenenfalls einfach oder mehrfach, gleich oder verschieden durch Fluor, Chlor, Brom und/oder -NR⁷R⁸ substituiertes C₁-C₁₀-Alkyl, für jeweils gegebenenfalls einfach bis vierfach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes Cyclopropyl, Cyclopentyl, Cyclohexyl, Phenyl oder Benzyl.
- R⁶: steht außerdem besonders bevorzugt für einfach bis dreifach, gleich oder verschieden substituiertes C₁-C₁₀-Alkyl, wobei die Substituenten zusätzlich aus C₁-C₄-Alkoxy, C₁-C₄-Alkylthio, C₁-C₄-Halogenalkoxy und/oder C₁-C₄-Halogenalkylthio ausgewählt sein können.
- R⁷ und R⁸: stehen unabhängig voneinander besonders bevorzugt für Wasserstoff, -SO₂R⁶, -COR⁶, -CO₂R⁶, für jeweils gegebenenfalls einfach oder mehrfach, gleich oder verschieden, durch Fluor, Chlor, Brom, C₁-C₄-Alkylcarbonyl, C₁-C₄-Alkylcarbonyloxy, C₁-C₄-Alkylamino, Di-(C₁-C₄-alkyl)amino, C₁-C₄-Alkoxy, C₁-C₄-Halogenalkoxy, C₁-C₄-Alkylthio und/oder C₁-C₄-Halogenalkylthio substituiertes C₁-C₁₆-Alkyl oder C₂-C₁₆-Alkenyl; für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes C₃-C₁₀-Cycloalkyl, C₃-C₆-Cycloalkyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl.
- R⁷ und R⁸: stehen außerdem unabhängig voneinander besonders bevorzugt für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy C₁-C₄-Alkylthio und/oder C₁-C₄-Halogenalkylthio substituiertes Pyridinylinethyl, Pyridinylethyl, Pyrimidinylmethyl, Pyrimidinylethyl, Pyridazylmethyl, Pyridazylethyl, Pyrazinylmethyl oder Pyrazinylethyl.
- R⁷ und R⁸: stehen außerdem gemeinsam besonders bevorzugt für gegebenenfalls einfach oder mehrfach, gleich oder verschieden durch Fluor, Chlor, Brom, C₁-C₄-Alkoxy, C₁-C₄-Halogenalkoxy, C₁-C₄-Alkylthio und/oder C₁-C₄-Halogenalkylthio substituiertes C₂-C₁₀-Alkenylen oder für gegebenenfalls im Alkylenteil einfach oder mehrfach, gleich oder verschieden durch Fluor, Chlor, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₁₀-Alkylen, wobei die Alkylenkette jeweils durch -O-, -S- oder -NR¹⁵- unterbrochen sein kann.
- R⁷ und R⁸: stehen außerdem gemeinsam besonders bevorzugt für einfach bis dreifach, gleich oder verschieden substituiertes C₃-C₁₀-Alkylen, wobei die Substituenten zusätzlich aus n-Propoxycarbonyl, i-Propoxycarbonyl, Ethoxycarbonyl, Methoxycarbonyl, Oxypropylenoxy, Oxyethylenoxy und/oder Oxymethylenoxy ausgewählt sein können.
- R⁷ und R⁸: stehen außerdem gemeinsam besonders bevorzugt für C₃-C₆-Alkylen, wobei die Alkylenkette entweder durch C=O oder C=NO-(C₁-C₄-Alkyl) unterbrochen ist.
- R⁹ und R¹⁰: stehen unabhängig voneinander besonders bevorzugt für Wasserstoff, -SO₂R⁶, für jeweils gegebenenfalls einfach bis neunfach, gleich oder verschieden durch Fluor, Chlor und/oder Brom, C₁-C₄-Alkylamino, Di-(C₁-C₄-alkyl)amino, C₁-C₄-Alkoxy, C₁-C₄-Halogenalkoxy, C₁-C₄-Alkylthio, C₁-C₄-Halogenalkylthio substituiertes C₁-C₄-Alkyl oder C₂-C₆-Alkenyl; für jeweils gegebenenfalls einfach bis dreifach, gleich oder verschieden durch Fluor, Chlor und/oder Brom, C₁-C₄-Alkyl, Cyano, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₆-Cycloalkyl, C₃-C₆-Cycloalkyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl.
- R⁹ und R¹⁰: stehen außerdem gemeinsam besonders bevorzugt für jeweils gegebenenfalls im Alkylenteil einfach bis vierfach, gleich oder verschieden durch Fluor, Chlor, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₄-C₅-Alkylen, -(CH₂)₂-O-(CH₂)₂-, -(CH₂)₂-S-(CH₂)₂-, -(CH₂)₂-N(R¹⁵)-(CH₂)₂-.
- R¹¹: und R¹² stehen unabhängig voneinander besonders bevorzugt für Wasserstoff, für gegebenenfalls einfach bis neunfach, gleich oder verschieden durch Fluor, Chlor, Brom, C₁-C₄-Alkoxy, C₁-C₄-Alkylthio, substituiertes C₁-C₆-Alkyl, für gegebenenfalls einfach bis vierfach, gleich oder verschieden durch Fluor, Chlor, Brom, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₆-Cycloalkyl, C₃-C₆-Cycloalkyl-C₁-C₄-alkyl, Phenyl, Benzyl oder Phenylethyl.
- R¹¹ und R¹²: stehen außerdem gemeinsam besonders bevorzugt für jeweils gegebenenfalls einfach bis vierfach, gleich oder verschieden durch Fluor, Chlor, Brom, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₈-Alkylen oder C₃-C₈-Alkenylen.
- R¹⁵: steht besonders bevorzugt für Wasserstoff, -SO₂R⁶, für -COR⁶ oder -CO₂R⁶; für gegebenenfalls einfach oder mehrfach, gleich oder verschieden durch Fluor, Chlor, Brom, Cyano, Methylamino, Ethylamino, Di-(C₁-C₆-alkyl)amino, C₁-C₄-Alkoxy, C₁-C₄-Halogenalkoxy, C₁-C₄-Alkylthio, C₁-C₄-Halogenalkylthio substituiertes C₁-C₁₆-Alkyl oder C₂-C₁₆-Alkenyl; für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₁₀-Cycloalkyl, C₃-C₆-Cycloalkyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl.
- R¹: steht ganz besonders bevorzugt für Fluor oder Chlor.
- R²: steht ganz besonders bevorzugt für Wasserstoff oder Fluor.
- R³: steht ganz besonders bevorzugt für Fluor, Chlor, Methyl, Ethyl, n-Propyl, i-Propyl, n-Butyl, i-Butyl, s-Butyl, t-Butyl, Trifluormethyl, Trifluorethyl, Methoxy, Ethoxy, n-Propoxy, i-Propoxy, n-Butoxy, i-Butoxy, s-Butoxy, t-Butoxy, Trifluormethoxy, Trifluorethoxy, Methylthio, Ethylthio, n-Propylthio, i-Propylthio, n-Butylthio, i-Butylthio, s-Butylthio, t-Butylthio, Trifluormethylthio oder Trifluorethylthio.
- m: steht ganz besonders bevorzugt fiir 0 oder 1.
- Q: steht ganz besonders bevorzugt für eine der folgenden Gruppierungen 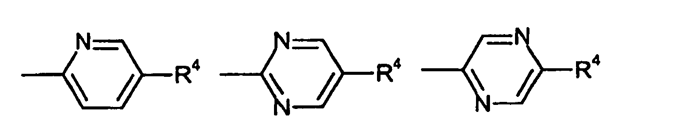 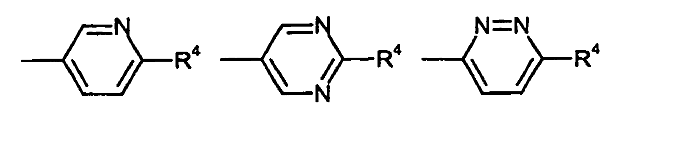
- R⁴: steht ganz besonders bevorzugt für Wasserstoff, Fluor, Chlor, Brom, Cyano, Formyl, für jeweils gegebenenfalls einfach oder mehrfach, gleich oder verschieden durch Fluor, Chlor, Brom, Cyano, -NR⁷R⁸ substituiertes C₁-C₁₆-Alkyl, C₂-C₁₆-Alkenyl, C₁-C₁₆-Alkoxy, C₂-C₁₆-Alkenyloxy; für -S(O)ₚR⁶, -NR⁷R⁸, -COR⁶, -CO₂R⁶, -CONR⁹R¹⁰, -N(R¹¹)COR¹²;
oder für jeweils gegebenenfalls einfach bis dreifach, gleich oder verschieden, durch Fluor, Chlor, Brom, Cyano, Methyl, Ethyl, n-Propyl, i-Propyl, -CF₃, -CHF₂, -CClF₂, -CF₂CHFCl, -CF₂CH₂F, -CF₂CCl₃, -CH₂CF₃, -CF₂CHFCF₃, -CH₂CF₂H, -CH₂CF₂CF₃, -CF₂CF₂H, -CF₂CHFCF₃, Vinyl, Allyl, 1-Propenyl, Butenyl, -CF=CHF, -CF=CH₂, -CF=CCl₂, -CH=CF₂, -CF₂CF=CF₂, -CH=CFH, -CH₂CF=CF₂, -CF=CF₂, -CF₂CH=CF₂, Methoxy, Ethoxy, n-Propoxy, i-Propoxy, n-Butoxy, i-Butoxy, s-Butoxy, t-Butoxy, Trifluormethoxy, Difluormethoxy, Chlordifluormethoxy, Trifluorethoxy, Methylthio, Ethylthio, n-Propylthio, i-Propylthio, n-Butylthio, i-Butylthio, s-Butylthio, t-Butylthio, Trifluormethylthio, Difluormethylthio, Chlordifluormethylthio, Trifluorethylthio substituiertes Cyclopropyl, Cyclopentyl, Cyclohexyl, Cyclopropylmethyl, Cyclopentylmethyl, Cyclohexylmethyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidino, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Piperidino, Morpholinyl, Thiomorpholinyl, Morpholino, Thiomorpholino, Triazinyl, Triazolyl, Chinolinyl oder Isochinolinyl.
- R⁴: steht außerdem ganz besonders bevorzugt für -CH=NOH, Formyl, Cyclopropyloxy, Cyclobutyloxy, Cyclopentyloxy, Cyclohexyloxy, Cyclopropylinethoxy, Cyclobutylmethoxy, Cyclopentylmethoxy oder Cyclohexylmethoxy.
- p: steht ganz besonders bevorzugt für 0, 1 oder 2.
- R⁶: steht ganz besonders bevorzugt für Methyl, Ethyl, n-Propyl, i-Propyl, n-Butyl, i-Butyl, s-Butyl, t-Butyl, Pentyl, Hexyl, -CF₃, -CHF₂, -CCl₃, -CCl₂F, Dimethylaminomethyl, Dimethylaminoethyl, Diethylaminomethyl, Diethylaminoethyl, Cyclopropyl, Cyclopentyl, Cyclohexyl, Phenyl oder Benzyl.
- R⁶: steht außerdem ganz besonders bevorzugt für Methoxymethyl, Trifluormethoxymethyl, Methylthiomethyl oder Trifluormethylthiomethyl.
- R⁷ und R⁸: stehen unabhängig voneinander ganz besonders bevorzugt für Wasserstoff, -SO₂R⁶, -COR⁶, -CO₂R⁶, für jeweils gegebenenfalls einfach oder mehrfach, gleich oder verschieden, durch Fluor, Chlor, Brom, C₁-C₄-Alkylcarbonyl, C₁-C₄-Alkylcarbonyloxy, C₁-C₄-Alkylamino, Di-(C₁-C₄-alkyl)amino, C₁-C₄-Alkoxy, C₁-C₄-Halogenalkoxy, C₁-C₄-Alkylthio und/oder C₁-C₄-Halogenalkylthio substituiertes C₁-C₁₆-Alkyl, C₂-C₁₆-Alkenyl; für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes C₃-C₈-Cycloalkyl, Cyclopropylmethyl, Cyclopentylmethyl, Cyclohexylmethyl, Cyclopropylethyl, Cyclopentylethyl, Cyclohexylethyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl.
- R⁷ und R⁸: stehen außerdem unabhängig voneinander ganz besonders bevorzugt für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes Pyridinylmethyl, Pyrimidinylmethyl, Pyridazylmethyl oder Pyrazinylmethyl.
- R⁷ und R⁸: stehen außerdem gemeinsam ganz besonders bevorzugt für gegebenenfalls einfach oder mehrfach, gleich oder verschieden durch Fluor, Chlor, Brom, C₁-C₄-Alkoxy, C₁-C₄-Halogenalkoxy, C₁-C₄-Alkylthio und/oder C₁-C₄-Halogenalkylthio substituiertes C₂-C₈-Alkenylen oder für gegebenenfalls im Alkylenteil einfach oder mehrfach, gleich oder verschieden durch Fluor, Chlor, Cyano, Methyl, Ethyl, n-Propyl, i-Propyl, n-Butyl, i-Butyl, s-Butyl, t-Butyl, C₁-C₄-Halogenalkyl, Methoxy, Ethoxy, n-Propoxy, i-Propoxy, n-Butoxy, i-Butoxy, s-Butoxy, t-Butoxy, C₁-C₄-Halogenalkoxy, Methylthio, Ethylthio, n-Propylthio, i-Propylthio, n-Butylthio, i-Butylthio, s-Butylthio, t-Butylthio, C₁-C₄-Halogenalkylthio substituiertes C₃-C₈-Alkylen, wobei die Alkylenkette jeweils durch -O-, -S- oder -NR¹⁵- unterbrochen sein kann.
- R⁷ und R⁸: stehen außerdem gemeinsam ganz besonders bevorzugt für einfach oder zweifach, gleich oder verschieden substituiertes C₃-C₈-Alkylen, wobei die Substituenten zusätzlich aus Ethoxycarbonyl, Methoxycarbonyl und/oder Oxyethylenoxy ausgewählt sein können.
- R⁷ und R⁸: stehen außerdem gemeinsam ganz besonders bevorzugt für -CH₂-CH₂-C(=O)-CH₂-CH₂-, -CH₂-CH₂-C(=NO-Me)-CH₂-CH₂-, -CH₂-CH₂-C(=NO-Et)-CH₂-CH₂- oder -CH₂-CH₂-C(=NO-iPr)-CH₂-CH₂-.
- R⁹ und R¹⁰: stehen unabhängig voneinander ganz besonders bevorzugt für Wasserstoff, -SO₂CF₃, Methyl, Ethyl, n-Propyl, i-Propyl, n-Butyl, i-Butyl, s-Butyl, t-Butyl, Pentyl, Hexyl, -CF₃, -CH₂CF₃, -(CF₂)₃CF₃, Cyclopropyl, Cyclopentyl, Cyclohexyl, Methoxymethyl, Methoxyethyl, oder für jeweils gegebenenfalls durch Fluor, Chlor, Brom, Methyl, Trifluormethyl, Methoxy, Trifluonnethoxy substituiertes Phenyl oder Benzyl.
- R⁹ und R¹⁰: stehen außerdem gemeinsam ganz besonders bevorzugt für -(CH₂)₄-, -(CH₂)₅-, -(CH₂)₆-, -CH₂-CH(CH₃)-CH₂-CH(CH₃)-CH₂-, -(CH₂)₂-O-(CH₂)₂-, -(CH₂)₂-S-(CH₂)₂- oder -(CH₂)₂-N(R¹⁵)-(CH₂)₂-.
- R¹¹ und R¹²: stehen unabhängig voneinander ganz besonders bevorzugt für Methyl, Ethyl, n-Propyl, i-Propyl, n-Butyl, i-Butyl, s-Butyl, t-Butyl, n-Hexyl, Trifluormethyl, Trifluorethyl, Cyclopropyl, Cyclopentyl, Cyclohexyl oder für jeweils gegebenenfalls durch Fluor, Chlor, Brom, Methyl, Trifluormethyl, Methoxy, Trifluormethoxy substituiertes Phenyl oder Benzyl.
- R¹¹ und R¹²: stehen außerdem gemeinsam ganz besonders bevorzugt für jeweils gegebenenfalls durch Fluor, Chlor, Brom, Methyl, Ethyl, Methoxy, Ethoxy, Methylthio, Ethylthio, Trifluormethyl, Trifluormethoxy, Trifluormethylthio substituiertes -(CH₂)₃-, -(CH₂)₄-, -(CH₂)₅-, -(CH₂)₆-.
- R¹⁵: steht ganz besonders bevorzugt für Wasserstoff, -SO₂R⁶, für -COR⁶ oder -CO₂R⁶; für jeweils gegebenenfalls einfach oder mehrfach, gleich oder verschieden durch Fluor, Chlor, Brom, Cyano, Methylamino, Ethylamino, Di-(C₁-C₆-alkyl)amino, C₁-C₄-Alkoxy, C₁-C₄-Halogenalkoxy, C₁-C₄-Alkylthio, C₁-C₄-Halogenalkylthio substituiertes C₁-C₁₆-Alkyl, C₂-C₁₆-Alkenyl;
für jeweils gegebenenfalls einfach bis dreifach, gleich oder verschieden durch Fluor, Chlor, Brom, Cyano, Methyl, Ethyl, n-Propyl, i-Propyl, n-Butyl, i-Butyl, s-Butyl, t-Butyl, C₁-C₄-Halogenalkyl, Methoxy, Ethoxy, n-Propoxy, i-Propoxy, n-Butoxy, i-Butoxy, s-Butoxy, t-Butoxy, C₁-C₄-Halogenalkoxy, Methylthio, Ethylthio, n-Propylthio, i-Propylthio, n-Butylthio, i-Butylthio, s-Butylthio, t-Butylthio, C₁-C₄-Halogenalkylthio substituiertes C₃-C₈-Cycloalkyl, Cyclopropylmethyl, Cyclopentylmethyl, Cyclohexylmethyl, Cyclopropylethyl, Cyclopentylethyl, Cyclohexylethyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl.

Weiterhin ganz besonders bevorzugt sind Verbindungen der Formeln (I-a) bis (I-e) 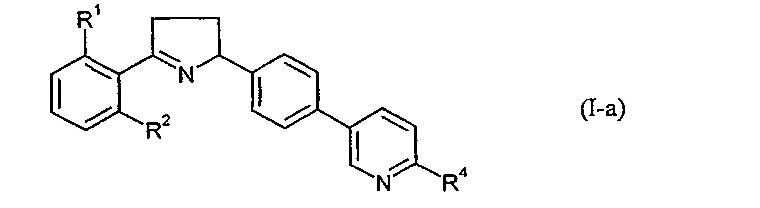 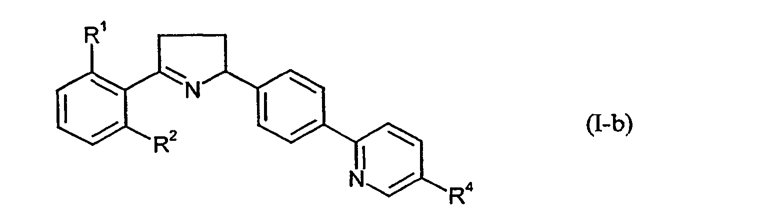 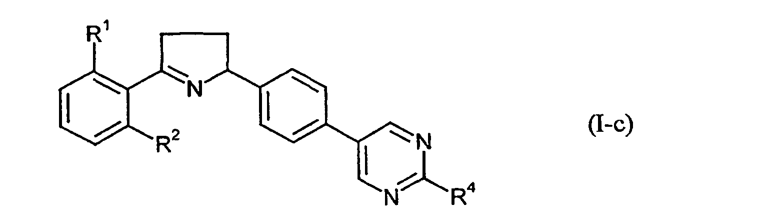 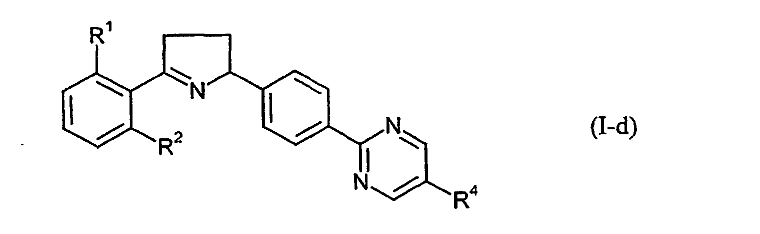 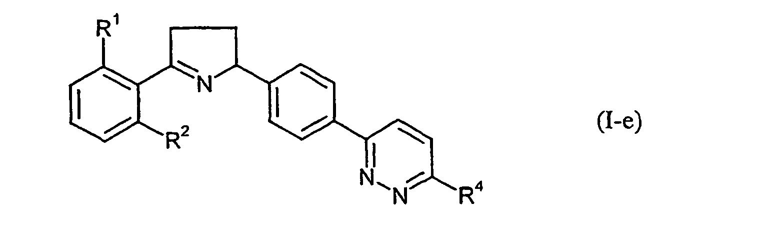 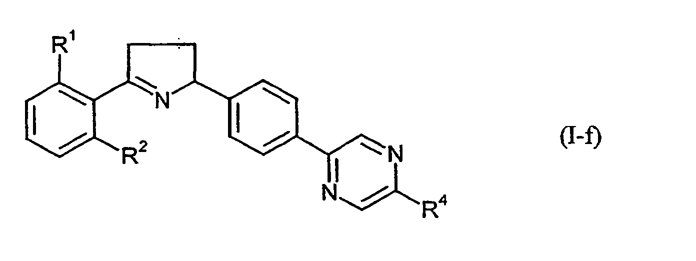 in welchen jeweils
- R¹: für Fluor oder Chlor steht,
- R²: für Wasserstoff oder Fluor steht und
- R⁴: die oben angegebenen Bedeutungen hat.

Weiterhin ganz besonders bevorzugt sind (R)-konfigurierte Verbindungen der Formeln (I-g) bis (I-1) 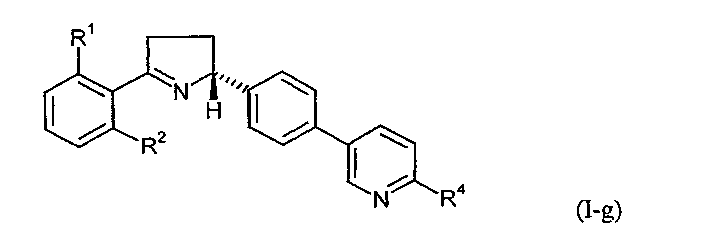 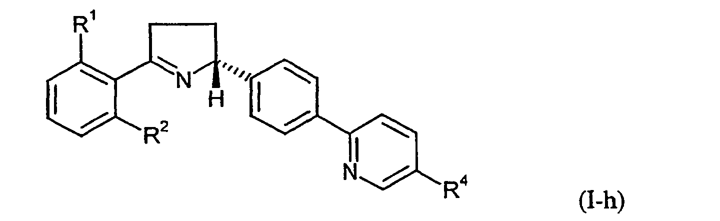 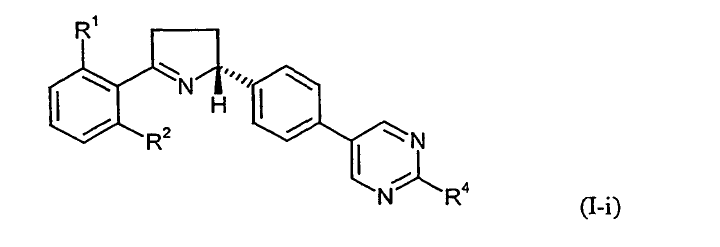 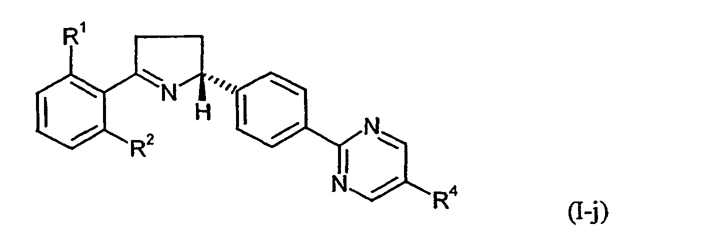 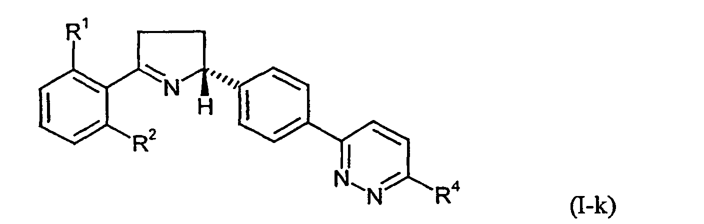 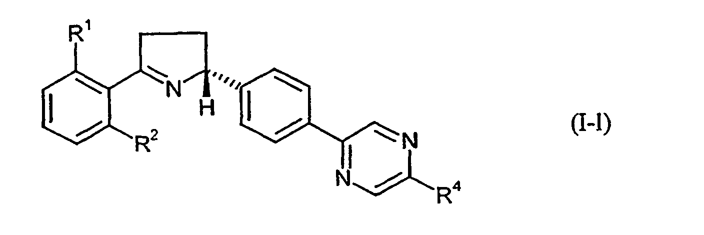 in welchen jeweils
- R¹: für Fluor oder Chlor steht,
- R²: für Wasserstoff oder Fluor steht und
- R⁴: die oben angegebenen Bedeutungen hat.

Verbindungen der Formeln (I-g) bis (I-l) erhält man durch übliche Verfahren zur Racematspaltung, wie zum Beispiel durch Chromatographie der entsprechenden Racemate an einer chiralen stationären Phase. Es ist möglich, sowohl die racemischen Endprodukte oder racemische Zwischenprodukte auf diese Weise in die beiden Enantiomere zu zerlegen.

Gesättigte Kohlenwasserstoffreste wie Alkyl können, auch in Verbindung mit Heteroatomen, wie z.B. in Alkoxy, soweit möglich, jeweils geradkettig oder verzweigt sein.

Durch Halogen substituierte Reste, z.B. Halogenalkyl, sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Verwendet man 5-(2,6-Difluorphenyl)-2-(4-bromphenyl)-3,4-dihydro-2H-pyrrol, N-(5-Brom-2-pyrimidinyl)-N-ethyl-N-propylamin und 4,4,4',4',5,5,5',5'-Octamethyl-2,2'-bis-1,3,2-dioxaborolan als Ausgangsstoffe sowie einen Palladiumkatalysator, so kann der Verlauf des erfindungsgemäßen Verfahrens (A) durch das folgende Formelschema veranschaulicht werden.

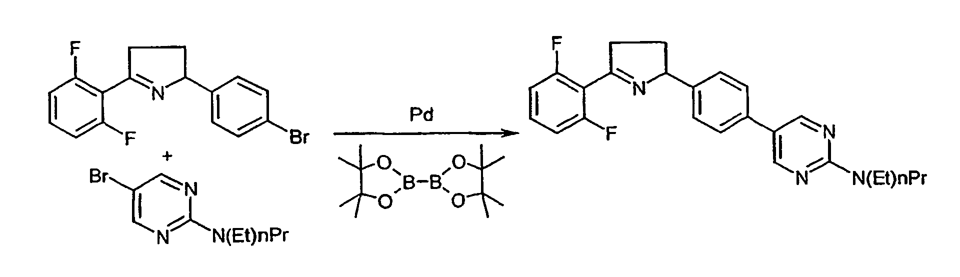

Verwendet man 5-(2,6-Difluorphenyl)-2-[4-(4,4,5,5-tetramethyl-1,3,2-dioxaborolan-2-yl)phenyl]-3,4-dihydro-2H-pyrrol und 5-Brom-2-(2,2,2-trifluorethoxy)pyrimidin als Ausgangsstoffe sowie einen Palladiumkatalysator, so kann der Verlauf des erfindungsgemäßen Verfahrens (B) durch das folgende Formelschema veranschaulicht werden.

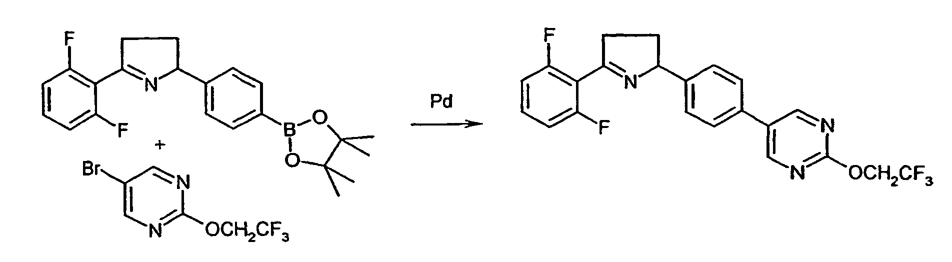

Verwendet man 5-(2,6-Difluorphenyl)-2-[4-(trifluormethylsulfonyloxy)phenyl]-3,4-dihydro-2H-pyrrol und 6-(Dimethylamino)-3-pyridazinylboronsäure als Ausgangsstoffe sowie einen Palladiumkatalysator, so kann der Verlauf des erfindungsgemäßen Verfahrens (C) durch das folgende Formelschema veranschaulicht werden.

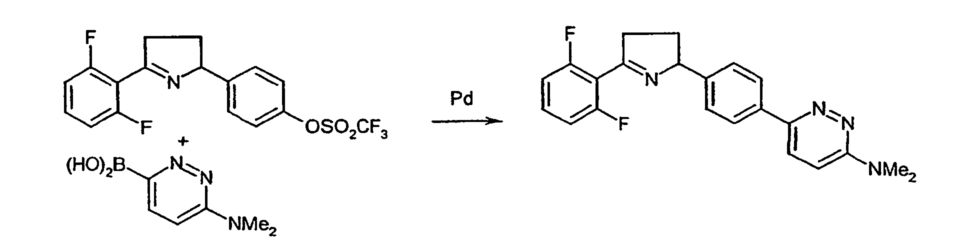

Verwendet man 5-(2,6-Difluorphenyl)-2-(4-bromphenyl)-3,4-dihydro-2H-pyrrol und 2-Trifluormethyl-5-(tributylstannyl)pyridin als Ausgangsstoffe sowie einen Palladiumkatalysator, so kann der Verlauf des erfindungsgemäßen Verfahrens (D) durch das folgende Formelschema veranschaulicht werden.

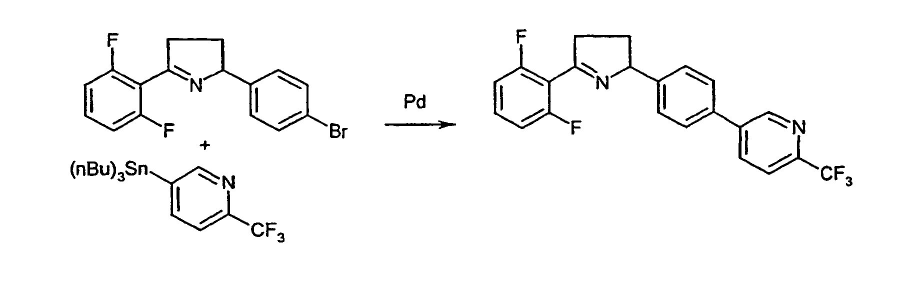

### Erläuterung der Verfahren und Zwischenprodukte

### Verfahren (A)

In einem ersten Reaktionsschritt wird eine Verbindung der Formel (II) mit einem Diboronsäureester in Gegenwart eines Palladium-Katalysators, gegebenenfalls in Gegenwart eines Säurebindemittels und gegebenenfalls in Gegenwart eines Lösungsmittels gekuppelt. Ohne Isolierung des Zwischenprodukts wird in demselben Reaktionsgefäß in einem zweiten Reaktionsschritt eine Verbindung der Formel (III) in Gegenwart eines Katalysators, gegebenenfalls in Gegenwart eines Säurebindemittels und gegebenenfalls in Gegenwart eines Lösungsmittels gekuppelt (vgl. z.B. Tetrahedron Lett. 1997, 38, 3841).

Das erfindungsgemäße Verfahren (A) kann in zwei Varianten durchgeführt werden. Es kann entweder eine Verbindung der Formel (II) oder eine Verbindung der Formel (III) vorgelegt werden. Verfahren (A) ist als Tandem-Reaktion der nachfolgend beschriebenen Verfahren (B) und (C) anzusehen.

Die bei der Durchführung des erfindungsgemäßen Verfahrens (A) als Ausgangsstoffe benötigten Δ¹-Pyrroline sind durch die Formel (II) allgemein definiert. In dieser Formel stehen R¹, R², R³ und m bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. Z steht bevorzugt für Brom, lod, -OSO₂CF₃ oder -OSO₂(CF₂)₃CF₃, besonders bevorzugt für Brom, -OSO₂CF₃ oder -OSO₂(CF₂)₃CF₃, ganz besonders bevorzugt für Brom oder -OSO₂CF₃.

Δ¹-Pyrroline der Formel (II) lassen sich nach bekannten Verfahren herstellen (vgl. WO 98/22438).

Die bei der Durchführung des erfindungsgemäßen Verfahrens (A) als Ausgangsstoffe benötigten Heterocyclen sind durch die Formel (III) allgemein definiert. In dieser Formel steht Q bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. X steht bevorzugt für Brom, Chlor, Iod oder -OSO₂CF₃, besonders bevorzugt für Brom, Chlor oder Iod, ganz besonders bevorzugt für Brom oder Chlor.

Die Heterocyclen der Formel (III) sind bekannt oder können nach bekannten Verfahren hergestellt werden (vgl. Aust. J. Chem. 1964, 17, 794; Chem. Ber. 1992, 125, 1169; Chem. Pharm. Bull. 1995, 43, 247; Eur. J. Med. Chem. 1989, 24, 249; J. Chem. Soc. C 1971, 1889; J. Chem. Soc. Perkin Trans. 1 1995, 2497; J. Med. Chem. 1991, 34, 315; J. Org. Chem. 1984, 49, 2240; J. Org. Chem. 1990, 55, 69; Org. Prep. Proced. Int. 1998, 30, 433; Synthesis 1999, 1163; Tetrahedron 1999, 40, 7975; Tetrahedron Lett. 1996, 37, 4447; Tetrahedron Lett. 2000, 41, 4335).

Als Diboronsäureester kommen bei der Durchführung des erfindungsgemäßen Verfahrens (A) 4,4,4',4',5,5,5',5'-Octamethyl-2,2'-bis-1,3,2-dioxaborolan, 5,5,5',5'-Tetramethyl-2,2'-bis-1,3,2-dioxaborinan, 4,4,4',4',6,6'-Hexamethyl-2,2'-bis-1,3,2-dioxaborinan oder 2,2'-Bis-1,3,2-benzodioxaborol in Frage. Bevorzugt verwendet man 4,4,4',4',5,5,5',5'-Octamethyl-2,2'-bis-1,3,2-dioxaborolan, 5,5,5',5'-Tetramethyl-2,2'-bis-1,3,2-dioxaborinan oder 4,4,4',4',6,6'-Hexamethyl-2,2'-bis-1,3,2-dioxaborinan, besonders bevorzugt 4,4,4',4',5,5,5',5'-Octamethyl-2,2'-bis-1,3,2-dioxaborolan oder 5,5,5',5'-Tetramethyl-2,2'-bis-1,3,2-dioxaborinan, ganz besonders bevorzugt 4,4,4',4',5,5,5',5'-Octamethyl-2,2'-bis-1,3,2-dioxaborolan.

Bei der Durchführung des erfindungsgemäßen Verfahrens (A) setzt man auf 1 Mol an Verbindung der Formel (II) im allgemeinen 1 Mol oder einen leichten Überschuss eines Diboronesters und 1 Mol oder einen leichten Überschuss einer Verbindung der Formel (III), sowie 3 % eines Palladiumkatalysators ein. Es ist jedoch auch möglich, die Reaktionskomponenten in anderen Verhältnissen einzusetzen. Es kann wahlweise die Verbindung der Formel (II) oder die Verbindung der Formel (III) zuerst vorgelegt werden. Die Aufarbeitung erfolgt nach üblichen Methoden. Im allgemeinen verfährt man in der Weise, dass man das Reaktionsgemisch mit Wasser verdünnt und mit Essigsäureethylester extrahiert. Die organische Phase wird gewaschen, getrocknet, filtriert und eingeengt. Der Rückstand wird gegebenenfalls nach üblichen Methoden, wie Chromatographie oder Umkristallisation, von eventuell noch vorhandenen Verunreinigungen befreit.

### Verfahren (B)

Die bei der Durchführung des erfindungsgemäßen Verfahrens (B) als Ausgangsstoffe benötigten Δ¹-Pyrroline sind durch die Formel (IV) allgemein definiert. In dieser Formel stehen R¹, R², R³ und m bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. A steht bevorzugt für (4,4,5,5-Tetramethyl-1,3,2-dioxaborolan)-2-yl, (5,5-Dimethyl-1,3,2-dioxaborinan)-2-yl, (4,4,6-Trimethyl-1,3,2-dioxaborinan)-2-yl oder 1,3,2-Benzodioxaborol-2-yl, besonders bevorzugt für (4,4,5,5-Tetramethyl-1,3,2-dioxaborolan)-2-yl, (5,5-Dimethyl-1,3,2-dioxaborinan)-2-yl oder (4,4,6-Trimethyl-1,3,2-dioxaborinan)-2-yl, ganz besonders bevorzugt für (4,4,5,5-Tetramethyl-1,3,2-dioxaborolan)-2-yl, (5,5-Dimethyl-1,3,2-dioxaborinan)-2-yl.

Δ¹-Pyrroline der Formel (IV) lassen sich herstellen, indem man
a) Verbindungen der Formel (II) 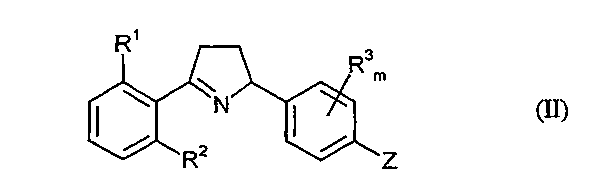
in welcher
R¹, R², R³, m und Z die oben angegebenen Bedeutungen haben, mit einem Diboronsäureester in Gegenwart eines Katalysators, gegebenenfalls in Gegenwart eines Säurebindemittels und gegebenenfalls in Gegenwart eines Verdünnungsmittels umsetzt (vgl. J. Org. Chem. 1995, 60, 7508; Tetrahedron Lett. 1997, 38, 3447).

Geeignete Diboronsäureester zur Durchführung des Verfahrens (a) sind bei der Beschreibung des erfindungsgemäßen Verfahrens (A) bereits genannt worden.

Die bei der Durchführung des erfindungsgemäßen Verfahrens (B) als Ausgangsstoffe benötigten Heterocyclen der Formel (III) wurden bereits oben bei der Beschreibung des Verfahrens (A) beschrieben.

Bei der Durchführung des erfindungsgemäßen Verfahrens (B) setzt man auf 1 Mol an Verbindung der Formel (IV) im allgemeinen 1 Mol oder einen leichten Überschuss an einer Verbindung der Formel (III) ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch in Essigsäureethylester aufnimmt und die organische Phase mit Wasser wäscht, über Natriumsulfat trocknet, filtriert und einengt. Der Rückstand wird gegebenenfalls nach üblichen Methoden, wie Chromatographie oder Umkristallisation, von eventuell noch vorhandenen Verunreinigungen befreit.

### Verfahren (C)

Die bei der Durchführung des erfindungsgemäßen Verfahrens (C) als Ausgangsstoffe benötigten Δ¹-Pyrroline der Formel (II) wurden bereits bei der Beschreibung des Verfahrens (A) beschrieben.

Die bei der Durchführung des erfindungsgemäßen Verfahrens (C) als Ausgangsstoffe benötigten Boronsäure-Derivate sind durch die Formel (V) allgemein definiert. In dieser Formel steht Q bevorzugt, besonders bevorzugt bzw. ganz besonders bevorzugt für diejenigen Bedeutungen, die bereits in Zusammenhang mit der Beschreibung der erfindungsgemäßen Stoffe der Formel (1) für diese Reste als bevorzugt, besonders bevorzugt etc. genannt wurden. A steht bevorzugt für (4,4,5,5-Tetramethyl-1,3,2-dioxaborolan)-2-yl, (5,5-Dimethyl-1,3,2-dioxaborinan)-2-yl, (4,4,6-Trimethyl-1,3,2-dioxaborinan)-2-yl oder 1,3,2-Benzodioxaborol-2-yl, besonders bevorzugt für (4,4,5,5-Tetramethyl-1,3,2-dioxaborolan)-2-yl, (5,5-Dimethyl-1,3,2-dioxaborinan)-2-yl oder (4,4,6-Trimethyl-1,3,2-dioxaborinan)-2-yl, ganz besonders bevorzugt für (4,4,5,5-Tetramethyl-1,3,2-dioxaborolan)-2-yl, (5,5-Dimethyl-1,3,2-dioxaborinan)-2-yl.

Die Verbindungen der Formel (V) sind bekannt oder lassen sich nach bekannten Verfahren herstellen (vgl. J. Org. Chem. 1995, 60, 7508, Tetrahedron Lett. 1997, 38, 3447).

Bei der Durchführung des erfindungsgemäßen Verfahrens (C) setzt man auf 1 Mol an Verbindung der Formel (II) im allgemeinen 1 Mol oder einen leichten Überschuss einer Verbindung der Formel (V) ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch in Essigsäureethylester aufnimmt und die organische Phase mit Wasser wäscht, über Natriumsulfat trocknet, filtriert und einengt. Der Rückstand wird gegebenenfalls nach üblichen Methoden, wie Chromatographie oder Umkristallisation, von eventuell noch vorhandenen Verunreinigungen befreit.

### Verfahren (D)

Die bei der Durchführung des erfindungsgemäßen Verfahrens (D) als Ausgangsstoffe benötigten Δ¹-Pyrroline sind durch die Formel (II-a) allgemein definiert. In dieser Formel stehen R¹, R², R³ und m bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. Z¹ steht bevorzugt für Brom oder Iod.

Δ¹-Pyrroline der Formel (II-a) lassen sich nach bekannten Verfahren herstellen (vgl. WO 98/22438).

Die bei der Durchführung des erfindungsgemäßen Verfahrens (D) als Ausgangsstoffe benötigten organometallischen Verbindungen sind durch die Formel (VI) allgemein definiert. In dieser Formel steht Q bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. M steht bevorzugt für ZnCl, Sn(Me)₃ oder Sn(n-Bu)₃.

Organometallische Verbindungen der Formel (VI) sind teilweise bekannt oder können nach bekannten Methoden hergestellt werden. Es ist z.B. möglich, Verbindungen der Formel (VI) aus den entsprechenden Verbindungen der Formel (III), in welchen X für -OSO₂CF₃ steht, in situ herzustellen (vgl. Tetrahedron Lett. 1995, 36, 9085).

Bei der Durchführung des erfindungsgemäßen Verfahrens (D) setzt man auf 1 Mol an Verbindung der Formel (II-a) im allgemeinen 1 Mol oder einen leichten Überschuss einer Verbindung der Formel (VI) ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch in Essigsäureethylester aufnimmt und die organische Phase mit Wasser wäscht, über Natriumsulfat trocknet, filtriert und einengt. Der Rückstand wird gegebenenfalls nach üblichen Methoden, wie Chromatographie oder Umkristallisation, von eventuell noch vorhandenen Verunreinigungen befreit.

### Chirale Verbindungen der Formeln (I-g) bis (I-1)

Zur Herstellung chiraler Verbindungen der Formeln (I-g) bis (I-1) können beispielsweise Δ¹-Pyrroline der Formel (II-b) 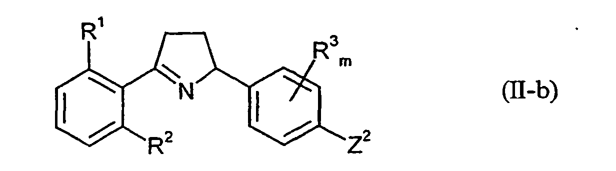 in welcher
R¹, R², R³ und m die oben angegebenen Bedeutungen haben,
- Z²: für Chlor, Brom oder Iod steht,
einer Racematspaltung unterzogen werden. Dabei arbeitet man beispielsweise nach Methoden der präparativen Chromatographie, vorzugsweise nach der Methode der High Performance Liquid Chromatography (HPLC). Dabei wird eine chirale stationäre Kieselgelphase verwendet. Als besonders geeignet für die Trennung der Verbindungen der Formel (II-b) in die beiden Enantiomere hat sich ein mit Tris(3,5-dimethylphenylcarbamat)-cellulose modifiziertes Kieselgel erwiesen. Dieses Trennmaterial ist kommerziell erhältlich. Es ist aber auch möglich, andere stationäre Phasen zu verwenden. Als Eluenten kommen alle üblichen inerten, organischen Solventien sowie Gemische von diesen in Frage. Vorzugsweise verwendbar sind gegebenenfalls halogenierte aliphatische, alicyclische oder aromatische Kohlenwasserstoffe, wie Petrolether, Hexan, Heptan, Cyclohexan; Dichlormethan, Chloroform; Alkohole, wie Methanol, Ethanol, Propanol; Nitrile, wie Acetonitril; Ester wie Essigsäuremethylester oder Essigsäureethylester. Besonders bevorzugt verwendet man aliphatische Kohlenwasserstoffe, wie Hexan oder Heptan, und Alkohole, wie Methanol oder Propanol, ganz besonders bevorzugt n-Heptan und Isopropanol bzw. Gemische von diesen. Im allgemeinen arbeitet man bei Temperaturen zwischen 10°C und 60°C, vorzugsweise zwischen 10°C und 40°C, besonders bevorzugt bei Raumtemperatur. Die auf diesem Wege erhaltenen (R)-konfigurierten Enantiomere werden dann als Ausgangsstoffe für die Verfahren (A), (C) oder (D) eingesetzt.

Bei der Durchführung der erfindungsgemäßen Verfahren (A), (B), (C) und (D) setzt man jeweils einen Palladium-Katalysator ein, der wiederum mit oder ohne Zusatz von weiteren Liganden verwendet werden kann. Vorzugsweise verwendet man als Katalysator PdCl₂(dppf) [dppf = 1,1'-Bis(diphenylphosphino)ferrocene], Pd(PPh₃)₄, PdCl₂(PPh₃)₂, PdCl₂(CH₃CN)₂, Pd₂(dba)₃ [dba = Dibenzylidenaceton] oder Pd(OAc)₂, besonders bevorzugt PdCl₂(dppf), Pd(PPh₃)₄, PdCl₂(PPh₃)₂, oder Pd(OAc)₂, ganz besonders bevorzugt PdCl₂(dppf) oder PdCl₂(PPh₃)₂.

Als Liganden kommen Triarylphosphine, Trialkylphosphine oder Arsine in Frage. Vorzugsweise verwendet man dppf, PPh₃, P(t-Bu)₃, Pcy₃ oder AsPh₃, besonders bevorzugt dppf.

Als Verdünnungsmittel kommen bei der Durchführung der erfindungsgemäßen Verfahren (A), (B) und (C) jeweils alle üblichen inerten, organischen Solventien in Frage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid oder Sulfone, wie Sulfolan. Besonders bevorzugt verwendet man Aceton, Dimethoxyethan, Dioxan, Tetrahydrofuran, Dimethylformamid, Dimethylacetamid, Dimethylsulfoxid, Ethanol, Toluol oder gegebenenfalls Gemische dieser genannten Verdünnungsmittel mit Wasser.

Als Verdünnungsmittel kommen bei der Durchführung des erfindungsgemäßen Verfahrens (D) jeweils alle üblichen inerten, organischen Solventien in Frage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol. Besonders bevorzugt verwendet man Dioxan, Tetrahydrofuran oder Toluol.

Als Säurebindemittel kommen bei der Durchführung der erfindungsgemäßen Verfahrens (A), (B), (C) und (D) jeweils alle für derartige Reaktionen üblichen anorganischen und organischen Basen in Betracht. Vorzugsweise verwendbar sind Erdalkali- oder Alkalimetallhydroxide, wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, Alkalimetallfluoride, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU). Es ist jedoch auch möglich, ohne zusätzliches Säurebindemittel zu arbeiten, oder die Aminkomponente in einem Überschuss einzusetzen, so dass sie gleichzeitig als Säurebindemittel fungiert. Besonders bevorzugt verwendet man Bariumhydroxid, Natriumhydroxid, Kaliumhydroxid, Trikaliumphosphat, Caesiumcarbonat, Kaliumcarbonat, Natriumcarbonat, Kaliumacetat, Triethylamin, Kalium-tert-butanolat, Caesiumfluorid oder Kaliumfluorid.

Die Reaktionstemperaturen können bei der Durchführung der erfindungsgemäßen Verfahren (A), (B) und (C) jeweils in einem größeren Bereich variiert werden. Im allgemeinen arbeitet man bei Temperaturen zwischen 0°C und 140°C, vorzugsweise zwischen 20°C und 120°C, besonders bevorzugt zwischen 60°C und 100°C.

Die Reaktionstemperaturen können bei der Durchführung des erfindungsgemäßen Verfahrens (D) jeweils in einem größeren Bereich variiert werden. Im allgemeinen arbeitet man bei Temperaturen zwischen 0°C und 140°C, vorzugsweise zwischen 20°C und 120°C.

Bei der Durchführung aller erfindungsgemäßen Verfahren arbeitet man im allgemeinen unter Atmosphärendruck. Es ist aber auch möglich, jeweils unter erhöhtem oder vermindertem Druck zu arbeiten.

Die erfindungsgemäßen Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica.
Aus der Ordnung der Dermaptera z.B. Forficula auricularia.
Aus der Ordnung der Isoptera z.B. Reticulitermes spp..
Aus der Ordnung der Phthiraptera z.B. Pediculus humanus corporis, Haematopinus spp., Linognathus spp., Trichodectes spp., Damalinia spp..
Aus der Ordnung der Thysanoptera z.B. Hercinothrips femoralis, Thrips tabaci, Thrips palmi, Frankliniella accidentalis.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..
Aus der Ordnung der Siphonaptera z.B. Xenopsylla cheopis, Ceratophyllus spp..
Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die erfindungsgemäßen Verbindungen der Formel (I) zeichnen sich insbesondere durch eine hervorragende Wirkung gegen Raupen, Käferlarven, Spinnmilben, Blattläuse und Minierfliegen aus.

Die erfindungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die erfindungsgemäßen Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der erfindungsgemäßen Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln;
als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate;
als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat, Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Edifenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox, Guazatin, Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G, OK-8705, OK-8801,
α-(1,1-Dimethylethyl)-β-(2-phenoxyethyl)-1H-1,2,4-triazol-1-ethanol,
α-(2,4-Dichlorphenyl)-β-fluor-β-propyl-1H-1,2,4-triazol-1-ethanol,
α-(2,4-Dichlorphenyl)-β-methoxy-α-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-α-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl)-2,6-dimethyl-morpholin-hydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioat,
S-Methyl-1,2,3-benzothiadiazol-7-carbothioat,
spiro[2H]-1-Benzopyran-2,1'(3'H)-isobenzofuran]-3'-on,

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Eprinomectin, Esfenvalerate, Ethiofencarb, Ethion, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Isazofos, Isofenphos, Isoxathion, Ivermectin,
Kernpolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Selamectin, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat 1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol 2-(Acetlyoxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid 3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N"-nitro-guanidin
N-Methyl-N'-(1-methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der erfindungsgemäßen Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt.

Je nach Pflanzenarten bzw. Pflanzensorten, deren Standort und Wachstumsbedingungen (Böden, Klima, Vegetationsperiode, Ernährung) können durch die erfindungsgemäße Behandlung auch über additive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Emteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Emteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus thuringiensis (z.B. durch die Gene CryIA(a), CrylA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylhamstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Stemostoma spp., Varroa spp..
Aus der Ordnung der Actinedida (Prostigmata) und Acaridida (Astigmata) z.B. Acarapis spp., Cheyletiella spp., Omithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp.; Sarcoptes spp., Notoedres spp., Knemidocoptes spp., Cytodites spp., Laminosioptes spp..

Beispielsweise zeigen sie eine hervorragende Wirksamkeit gegen die Entwicklungsstadien von Zecken wie zum Beispiel Amblyomma hebraeum, gegen parasitierende Fliegen wie zum Beispiel gegen Lucilia cuprina, gegen Flöhe wie zum Beispiel Ctenocephalides felis

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die erfindungsgemäßen Wirkstoffe der Formel (I) als Formulierungen (beispielsweise Pulver, Emulsionen, fließfähige Mittel), die die erfindungsgemäßen Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
   Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:
Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die erfindungsgemäßen Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der erfindungsgemäßen Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindelöl und/oder Monochlornaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer

Verflüchtigung der Wirkstoffe sowie einer Kristallisation bzw. Ausfällen vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyfenozid und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis-(trialkylzinn)-sulfiden, Tri-*n*-butylzinnlaurat, Tri-*n*-butylzinnchlorid, Kupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
   2-*tert*.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
   Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie Azaconazole, Cyproconazole, Epoxyconazole, Hexaconazole, Metconazole, Propiconazole und Tebuconazole;
Molluskizide wie
   Fentinacetat, Metaldehyd, Methiocarb, Niclosamid, Thiodicarb und Trimethacarb; oder herkömmliche Antifouling-Wirkstoffe wie 4,5-Dichlor-2-octyl-4-isothiazolin-3-on, Diiodmethylparatrylsulfon, 2-(N,N-Di-methylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten des weiteren die üblichen Bestandteile wie z.B. in Ungerer, *Chem. Ind*. **1985,** 37, 730-732 und Williams, Antifouling Marine Coatings, Noyes, Park Ridge, **1973** beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die erfindungsgemäßen Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Omithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködem oder Köderstationen.

Die Herstellung und die Verwendung der erfindungsgemäßen Stoffe geht aus den folgenden Beispielen hervor.

### Herstellungsbeispiele

### Beispiel 1

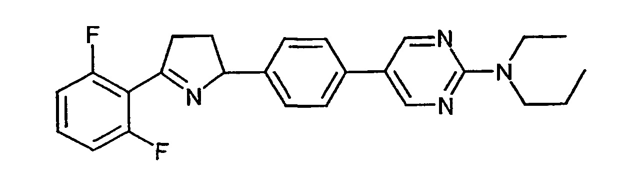

### Verfahren (A)

2-(4-Bromphenyl)-5-(2,6-difluorphenyl)-3,4-dihydro-2H-pyrrol (8.40 g, 0.025 mol), Bis(pinacolato)diboron (7.60 g, 0.03 mol), Kaliumacetat (7.40 g, 0.075 mol), PdCl₂[dppf] (0.56 g, 75 mmol) und Dimethylacetamid (150 ml) werden 3 h bei 80°C gerührt. Die Reaktionsmischung wird auf Raumtemperatur abgekühlt. Anschließend werden N-(5-Brom-2-pyrimidinyl)-N-ethyl-N-propylamin (6.30 g, 0.026 mol), PdCl₂[dppf] (0.56 g, 75 mmol) und 2 M wässrige Natriumcarbonatlösung (75 ml) zugegeben und 16 h bei 80°C nachgerührt. Die Reaktionsmischung wird auf Raumtemperatur abgekühlt, mit Wasser verdünnt und über Celite abgesaugt. Der Filterkuchen wird mit Essigsäureethylester nachgewaschen. Die organische Phase wird mit Wasser gewaschen, über Natriumsulfat getrocknet und filtriert. Florisil (60 g) wird zugegeben und das Lösungsmittel unter vermindertem Druck entfernt. Das Rohprodukt wird chromatographisch an Kieselgel (Laufmittel: n-Hexan/ Essigsäureethylester, 4:1, *v*/v) aufgereinigt.

Man erhält 5.10 g (57 % d. Th.) an N-(5-{4-[5-(2,6-Difluorphenyl)-3,4-dihydro-2H-pyrrol-2-yl]phenyl}-2-pyrimidinyl)-N-ethyl-N-propylamin.

| | |
|---|---|
| HPLC: | Log P (pH 2.3) = 3.57 (Reinheit: 99 %) |
| Fp. | 88-89°C |

### Beispiel 2

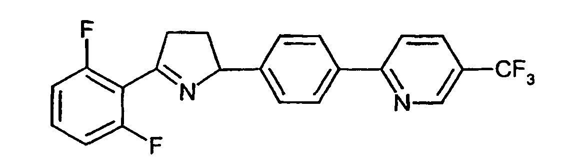

### Verfahren (A)

4-[5-(2,6-Difluorphenyl)-3,4-dihydro-2H-pyrrol-2-yl]phenyltrifluormethansulfonat (10.10 g, 0.025 mol), Bis(pinacolato)diboron (7.60 g, 0.03 mol), Kaliumacetat (7.40 g, 0.075 mol), PdCl₂[dppf] (0.56 g, 75 mmol) und Dimethylacetamid (150 ml) werden 3 h bei 80°C gerührt. Die Reaktionsmischung wird auf Raumtemperatur abgekühlt. Anschließend wird 2-Brom-5-trifluormethylpyridin (4.72 g, 0.026 mol), PdCl₂[dppf] (0.56, 75 mmol) und 2 M wässrige Natriumcarbonatlösung (75 ml) zugegeben und 16 h bei 80°C nachgerührt. Die Reaktionsmischung wird auf Raumtemperatur abgekühlt, mit Wasser verdünnt und der Niederschlag abgesaugt. Dieser feste Rückstand wird in Essigsäureethylester aufgenommen, mit Wasser gewaschen, über Natriumsulfat getrocknet und filtriert. Florisil (60 g) wird zugegeben und das Lösungsmittel unter vermindertem Druck entfernt. Das Rohprodukt wird chromatographisch an Kieselgel (Laufmittel: n-Hexan/Essigsäureethylester, 4:1, v/v) aufgereinigt.

Man erhält 7.10 g (71% d. Th.) an 2-{4-[5-(2,6-Difluorphenyl)-3,4-dihydro-2H-pyrrol-2-yl]phenyl}-5-(trifluoromethyl)pyridin.

| | |
|---|---|
| HPLC: | Log P (pH 2.3) = 3.25 (Reinheit: 97.90 %) |
| Fp. | 125°C |

### Beispiel 3

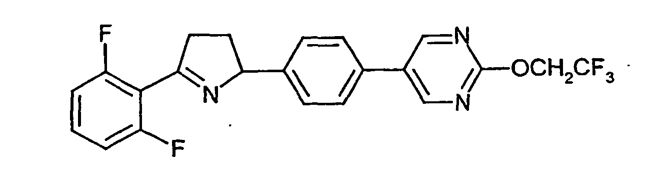

### Verfahren (B)

5-(2,6-Difluorphenyl)-2-[4-(4,4,5,5-tetramethyl-1,3,2-dioxaborolan-2-yl)phenyl]-3,4-dihydro-2H-pyrrol (0.96 g, 2.50 mmol) wird in Dimethoxyethan (15 ml) vorgelegt. Nacheinander werden 5-Brom-2-(2,2,2-trifluorethoxy)pyrimidin (0.71 g, 2.75 mmol), PdCl₂[dppf] (56 mg, 0.075 mmol) und wässrige gesättigte Natriumcarbonatlösung (3.75 ml) zugegeben und das Reaktionsgemisch 16 Stunden auf 80°C erhitzt. Nach Abkühlen auf Raumtemperatur wird das Reaktionsgemisch mit Wasser und Essigsäureethylester versetzt. Die organische Phase wird abgetrennt und über Natriumsulfat getrocknet, filtriert und unter vermindertem Druck eingeengt. Das Rohprodukt wird mittels Chromatographie an Kieselgel (Laufmittel: n-Hexan/ Essigsäureethylester 9:1→ 3:1, jeweils v/v) aufgereinigt.

Man erhält 0.71 g (64 % d. Th.) an 5-{4-[5-(2,6-Difluorphenyl)-3,4-dihydro-2H-pyrrol-2-yl]phenyl}-2-(2,2,2-trifluorethoxy)pyrimidin.

| | |
|---|---|
| HPLC: | Log P (pH 2.3) = 2.88 (Reinheit: 98 %) |
| Fp. | 112-114 °C |

Analog können nach einem der Verfahren (A), (B), (C) oder (D) die in der folgenden Tabelle genannten Verbindungen hergestellt werden.

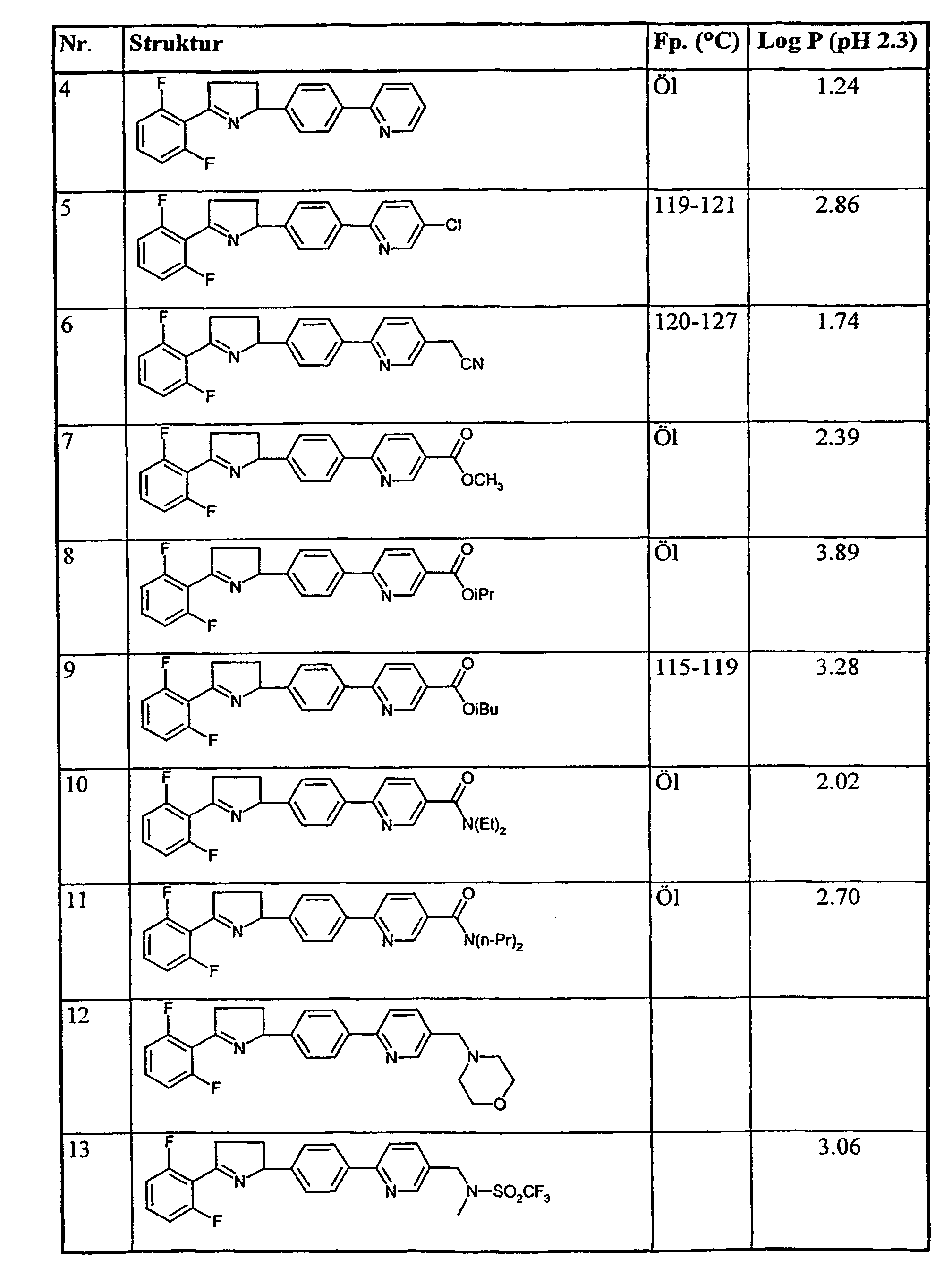

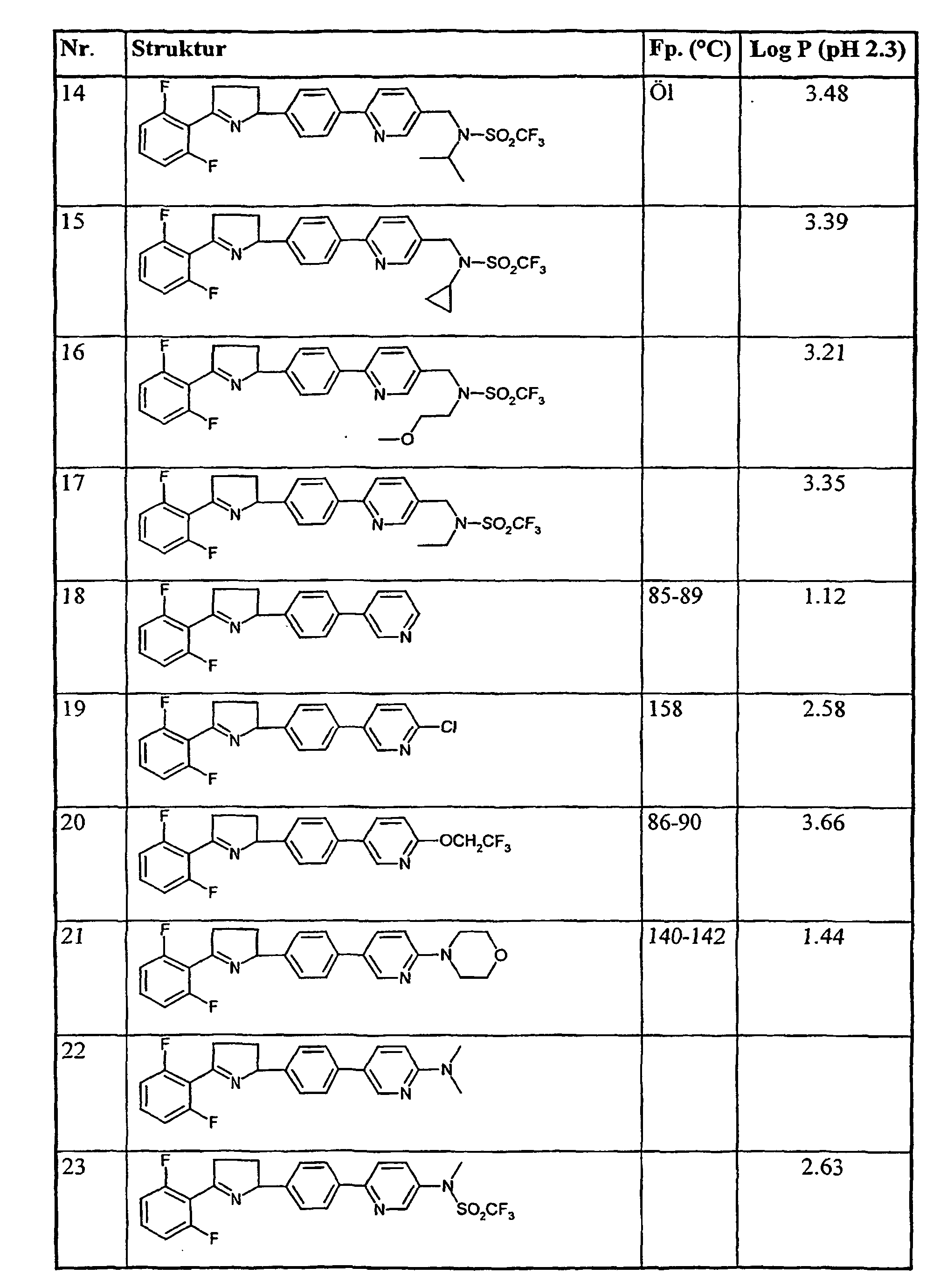

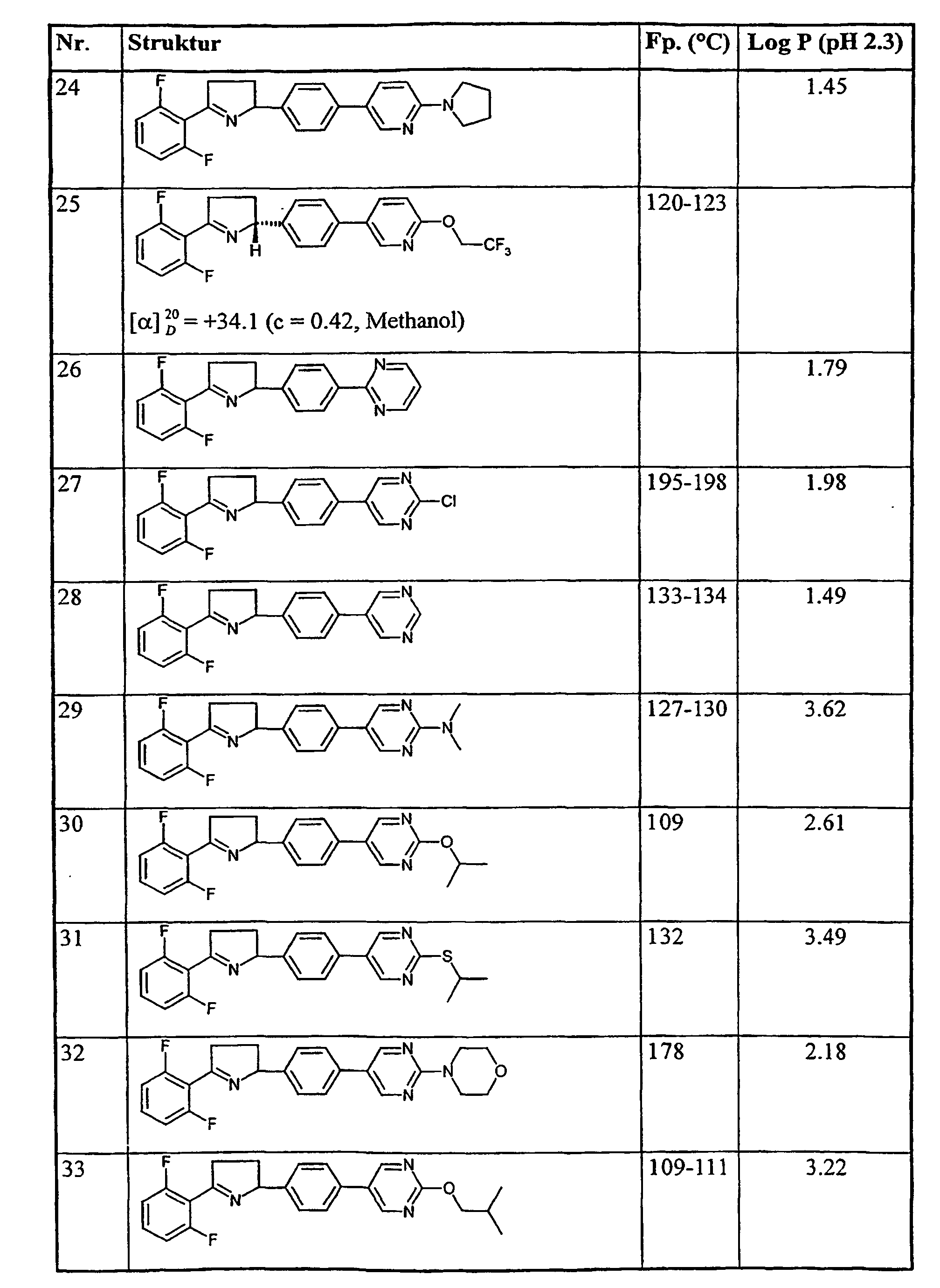

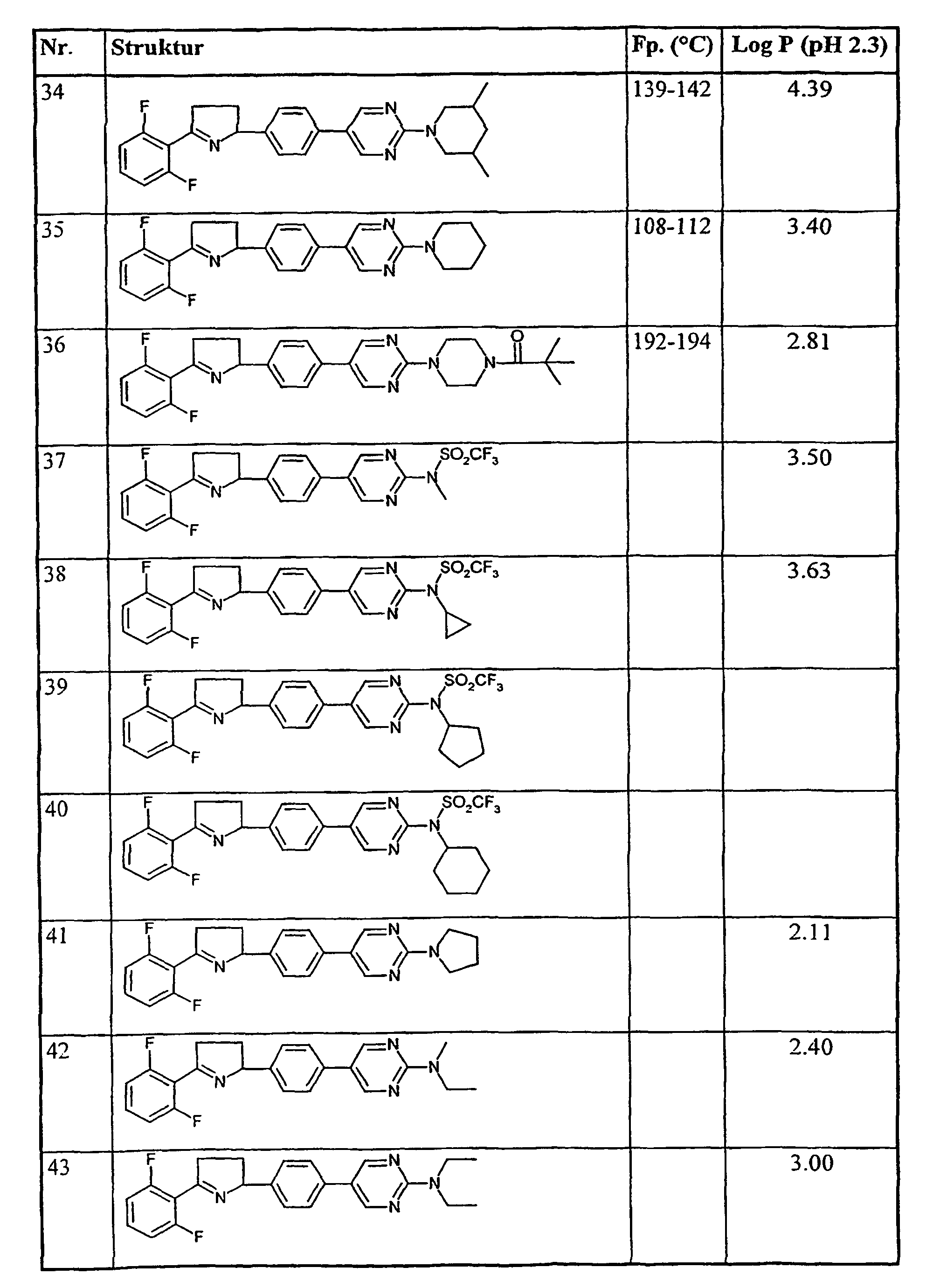

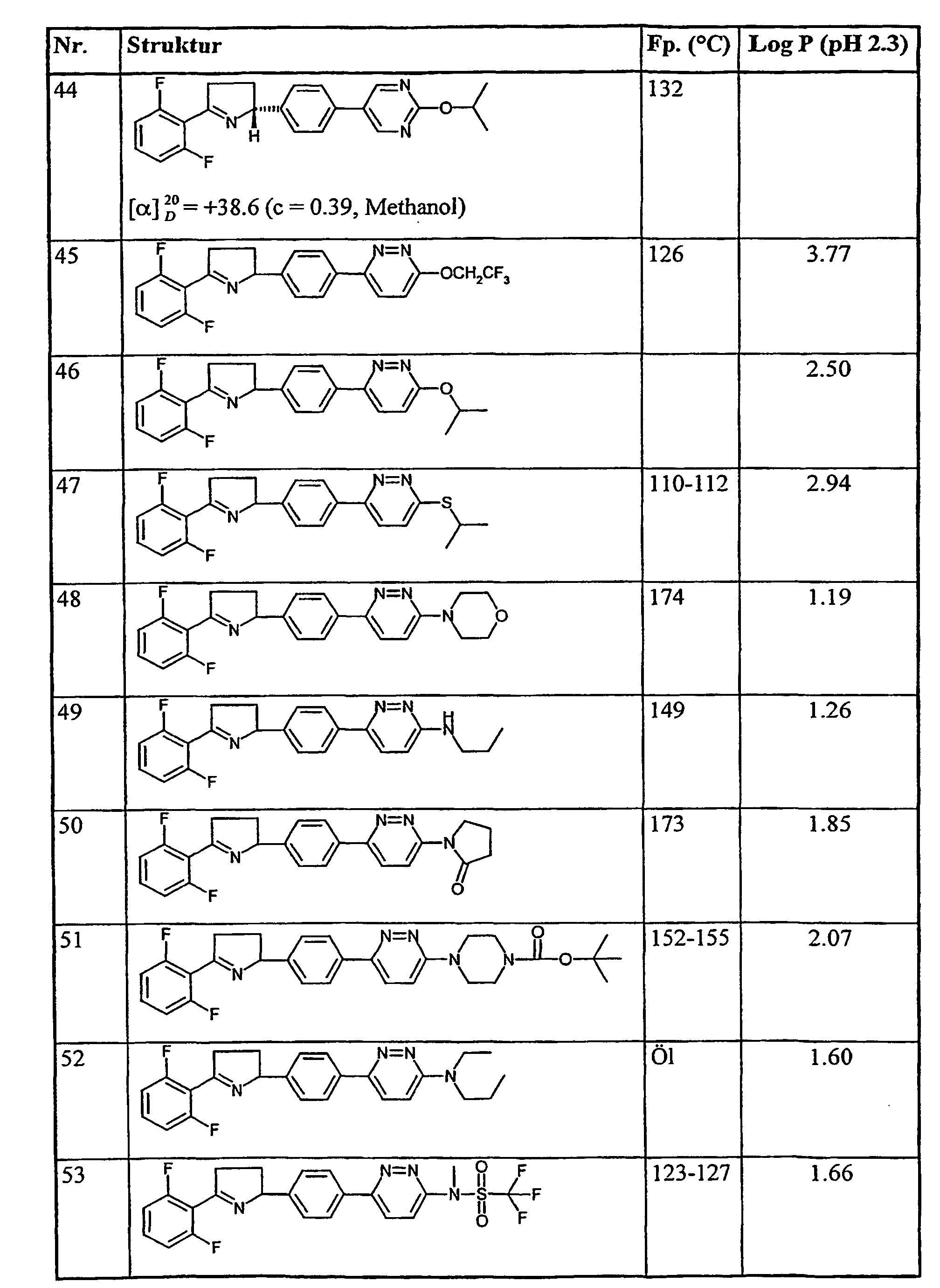

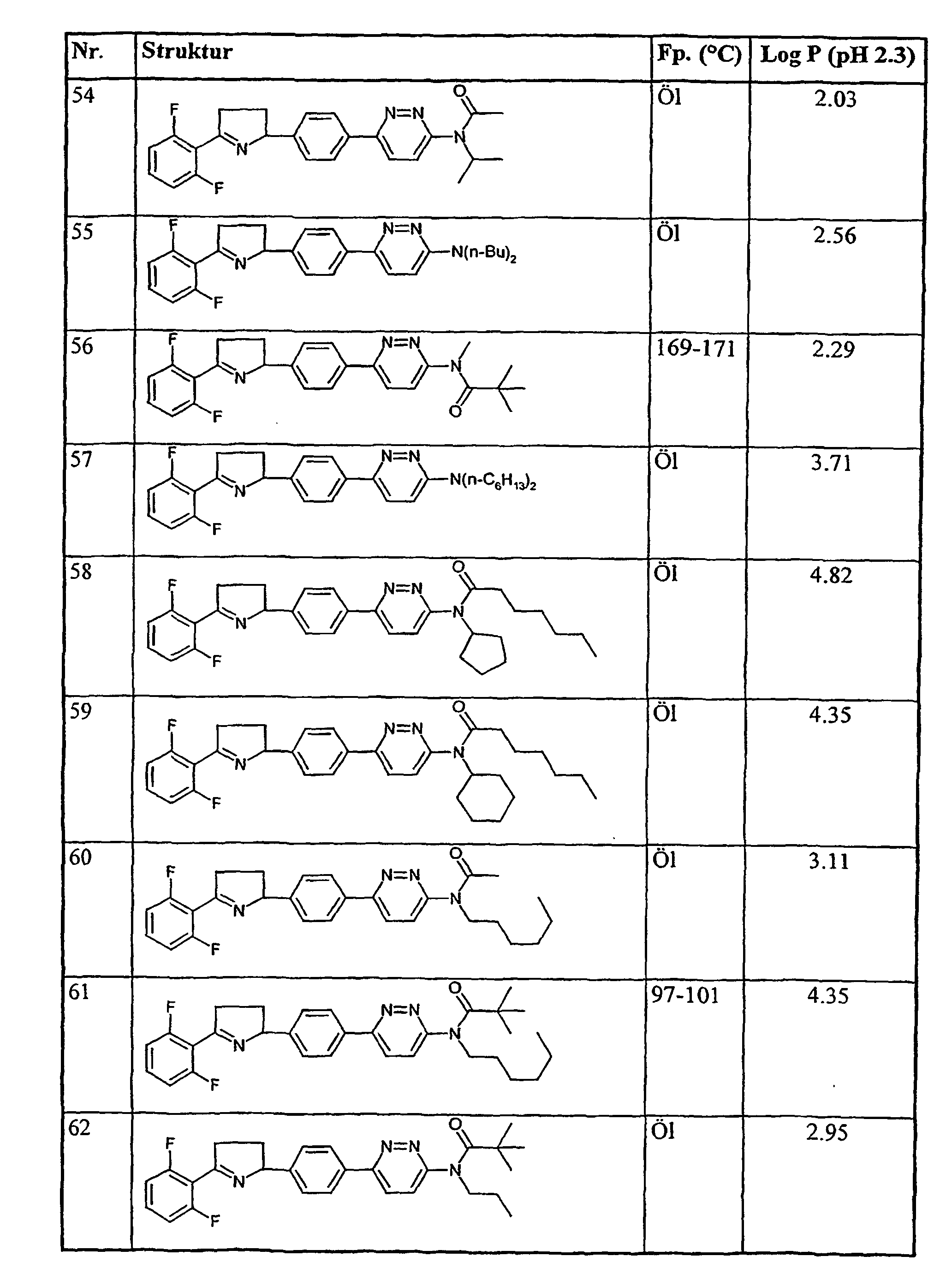

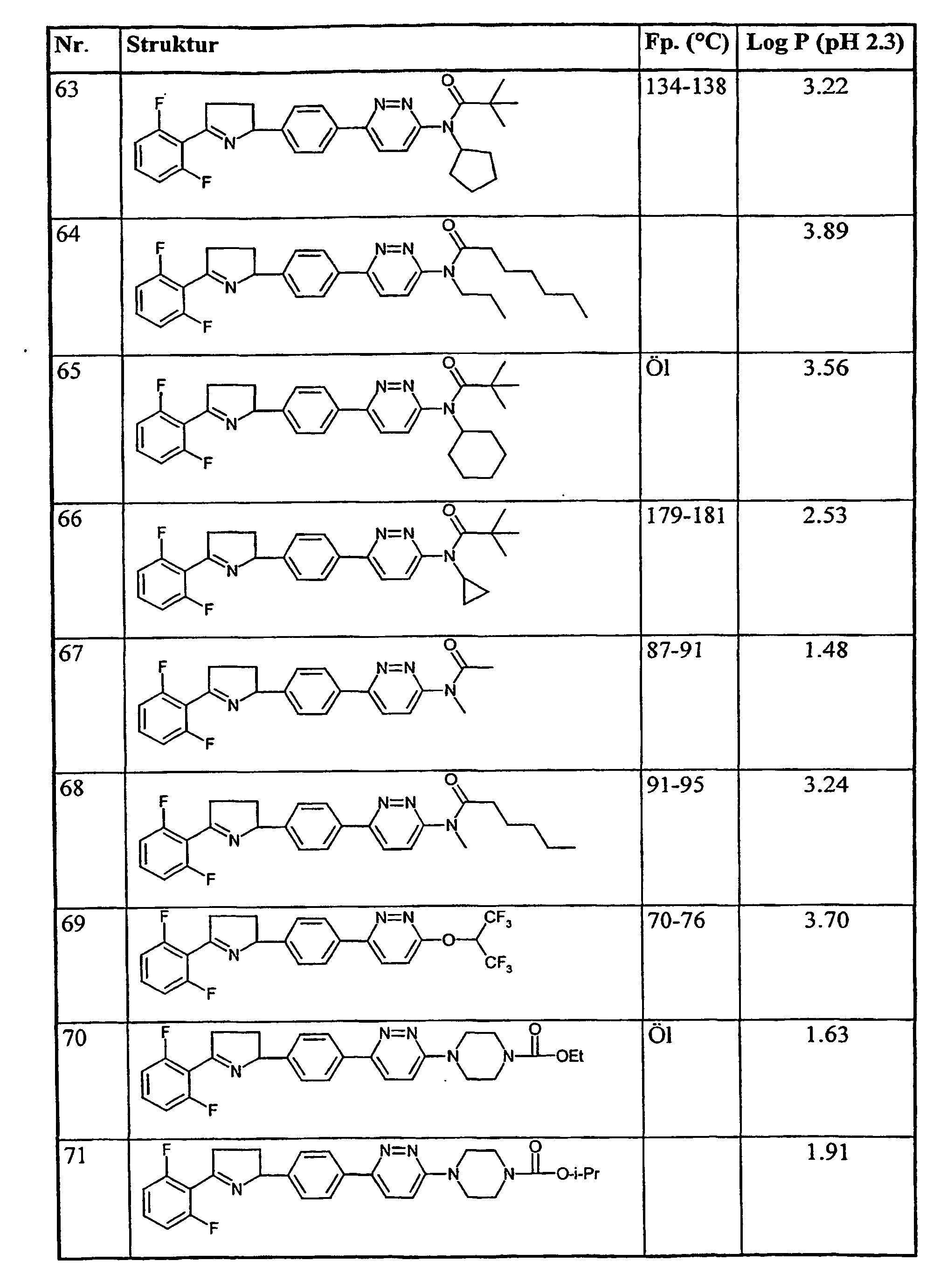

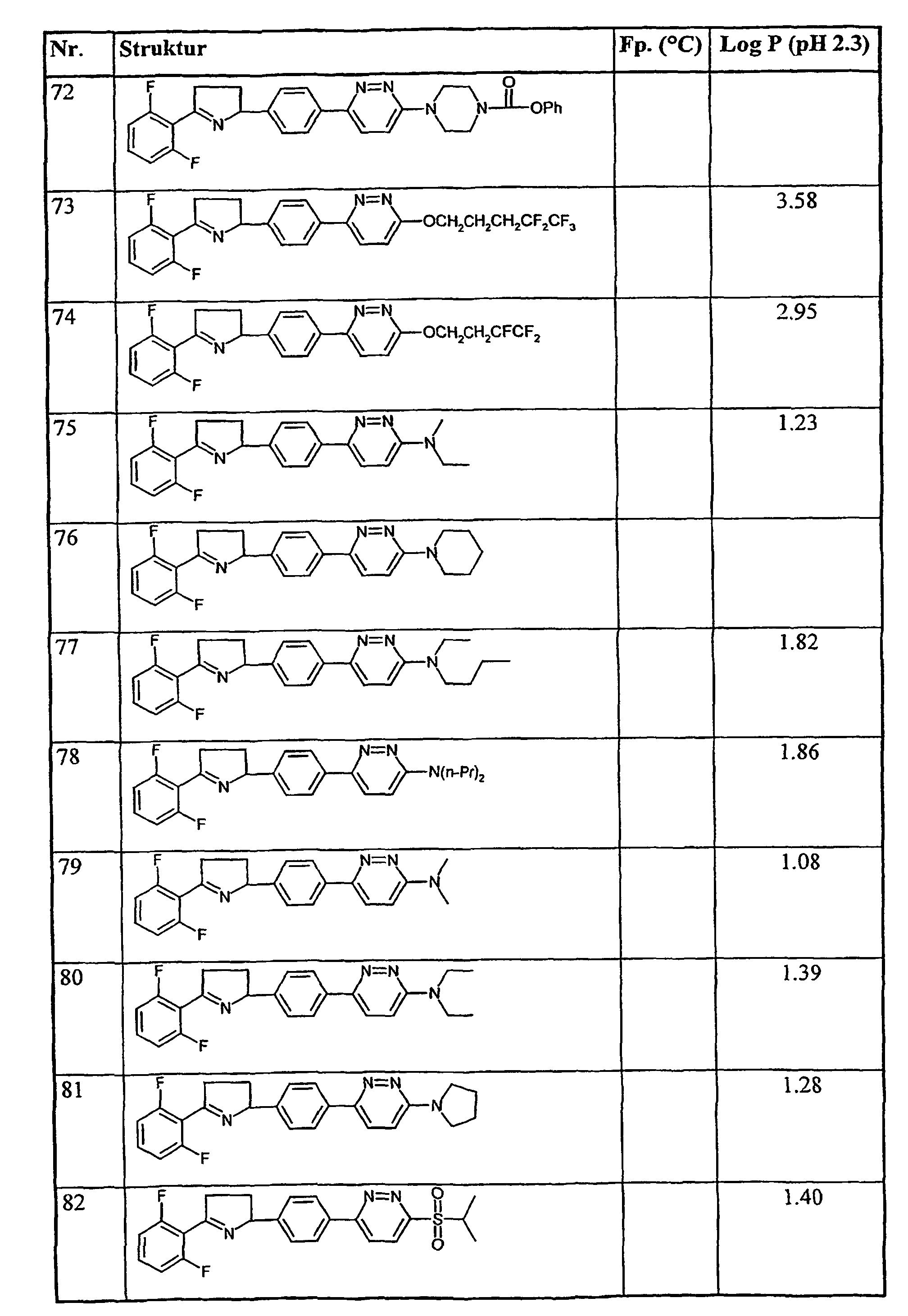

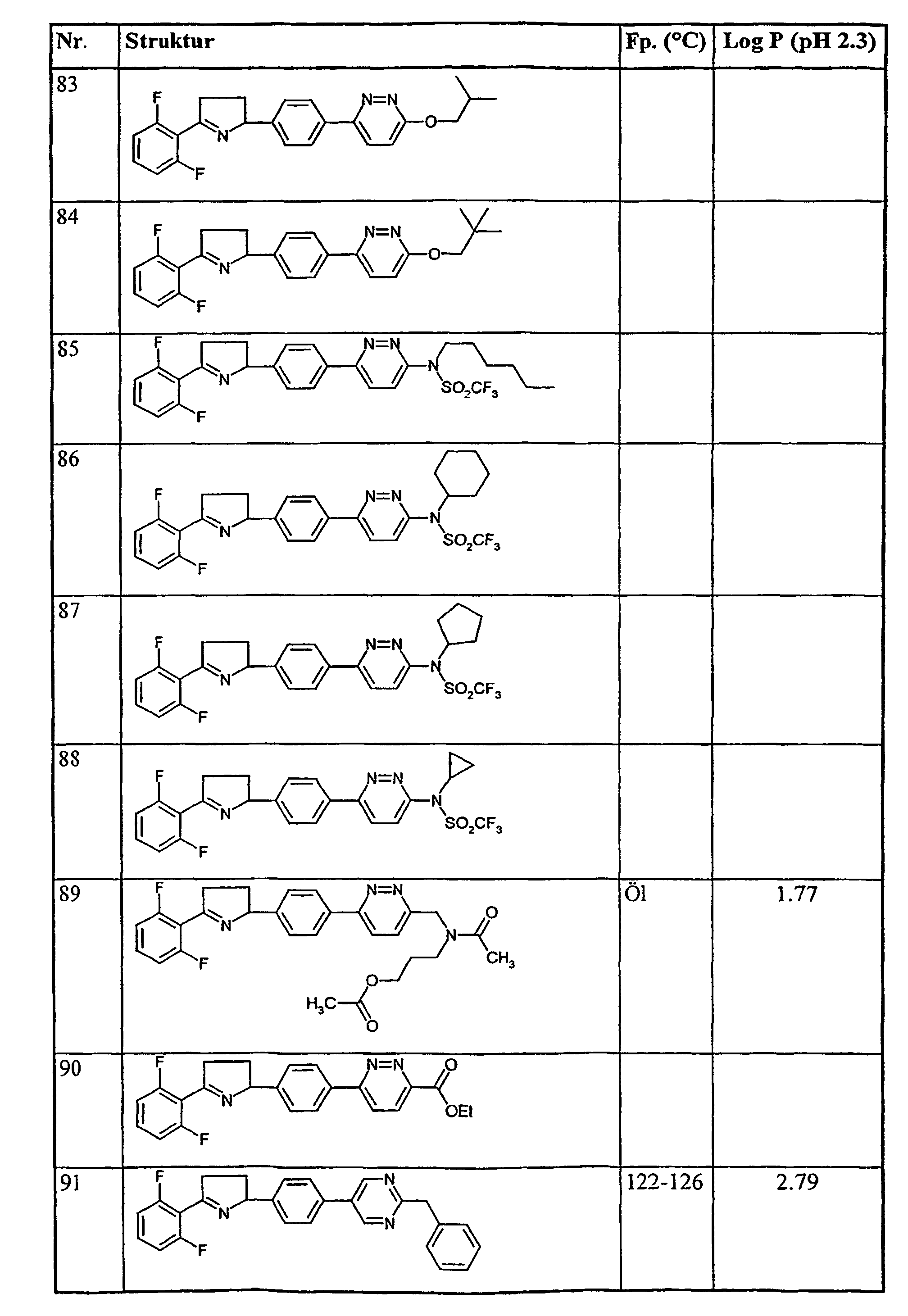

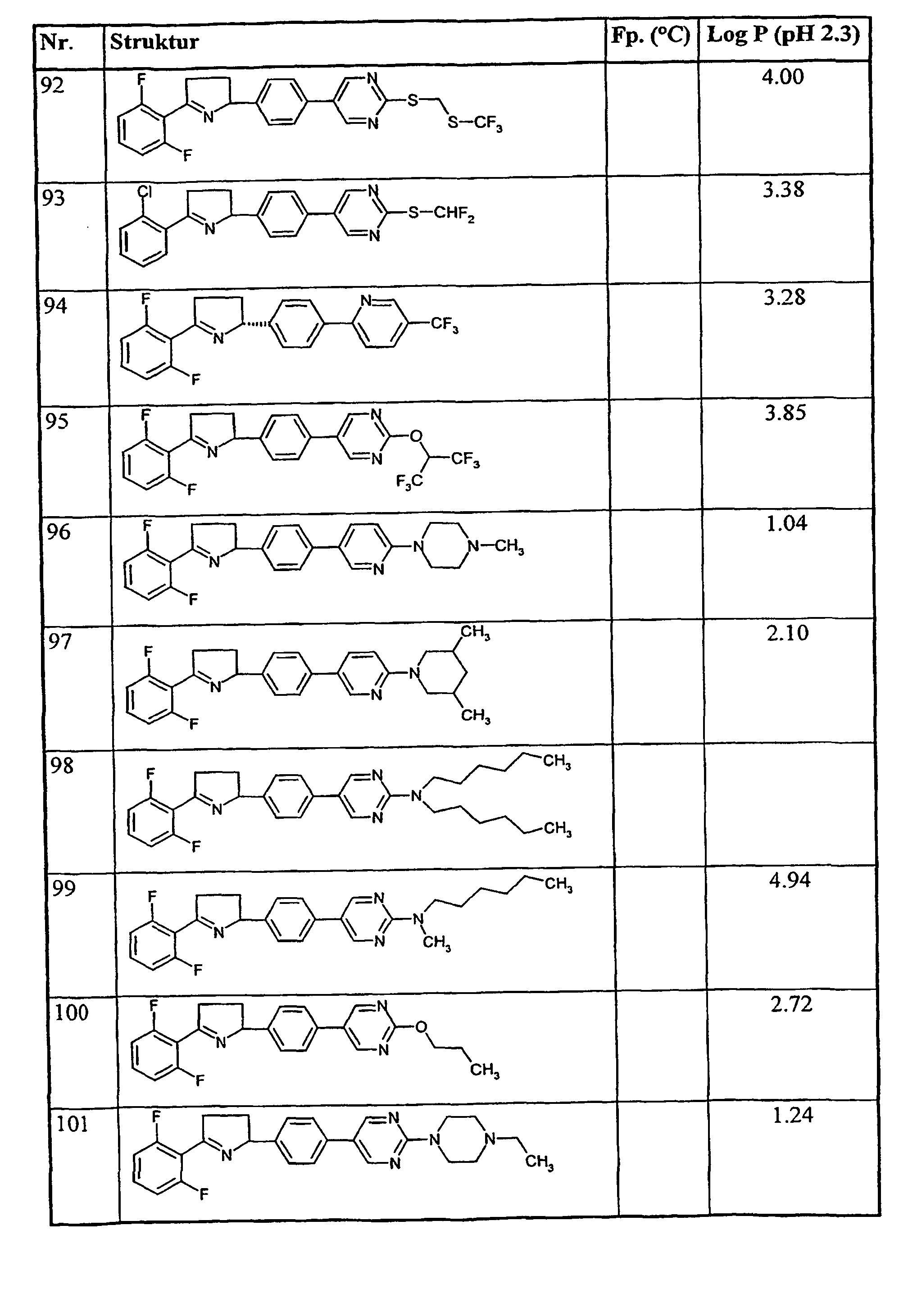

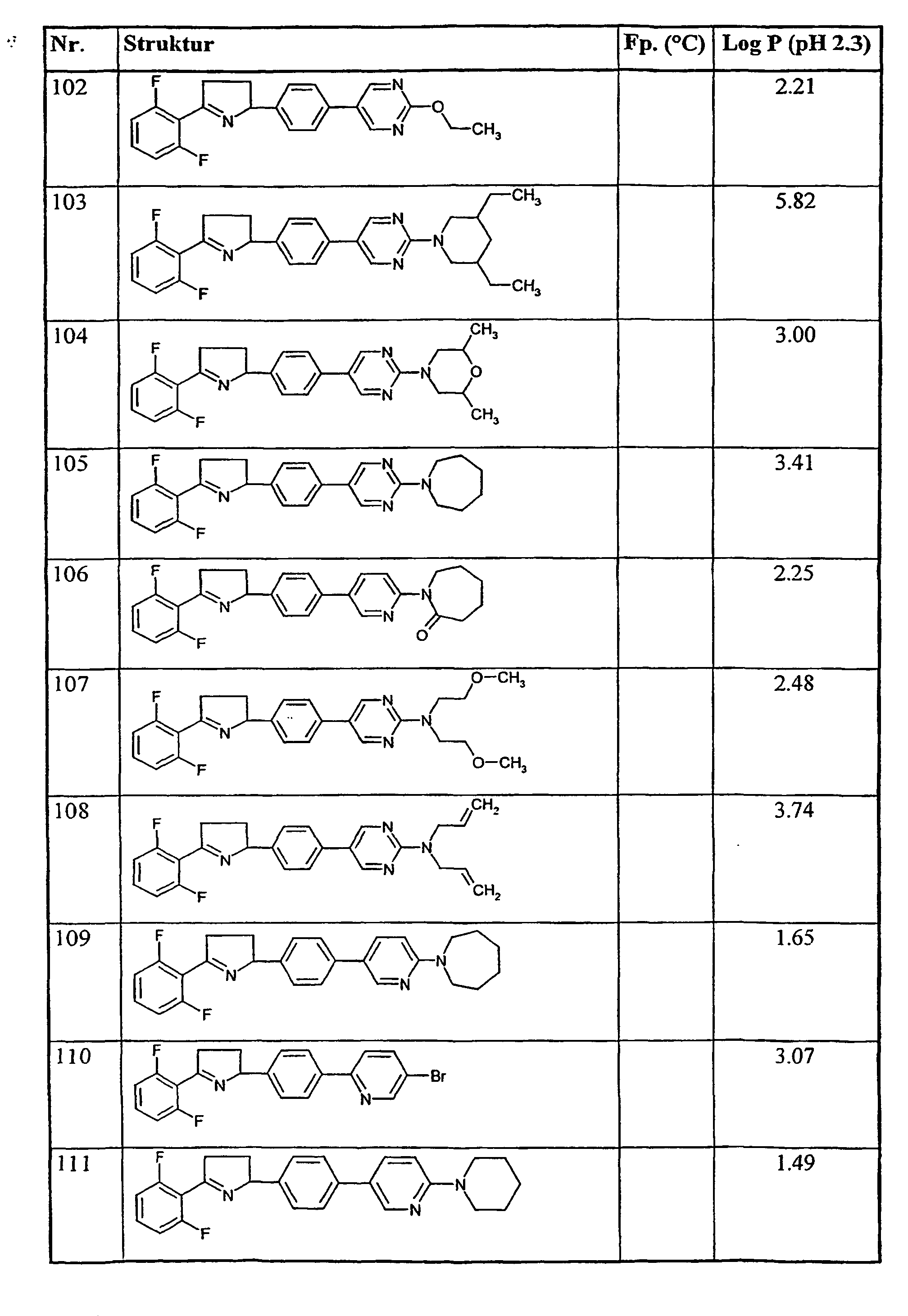

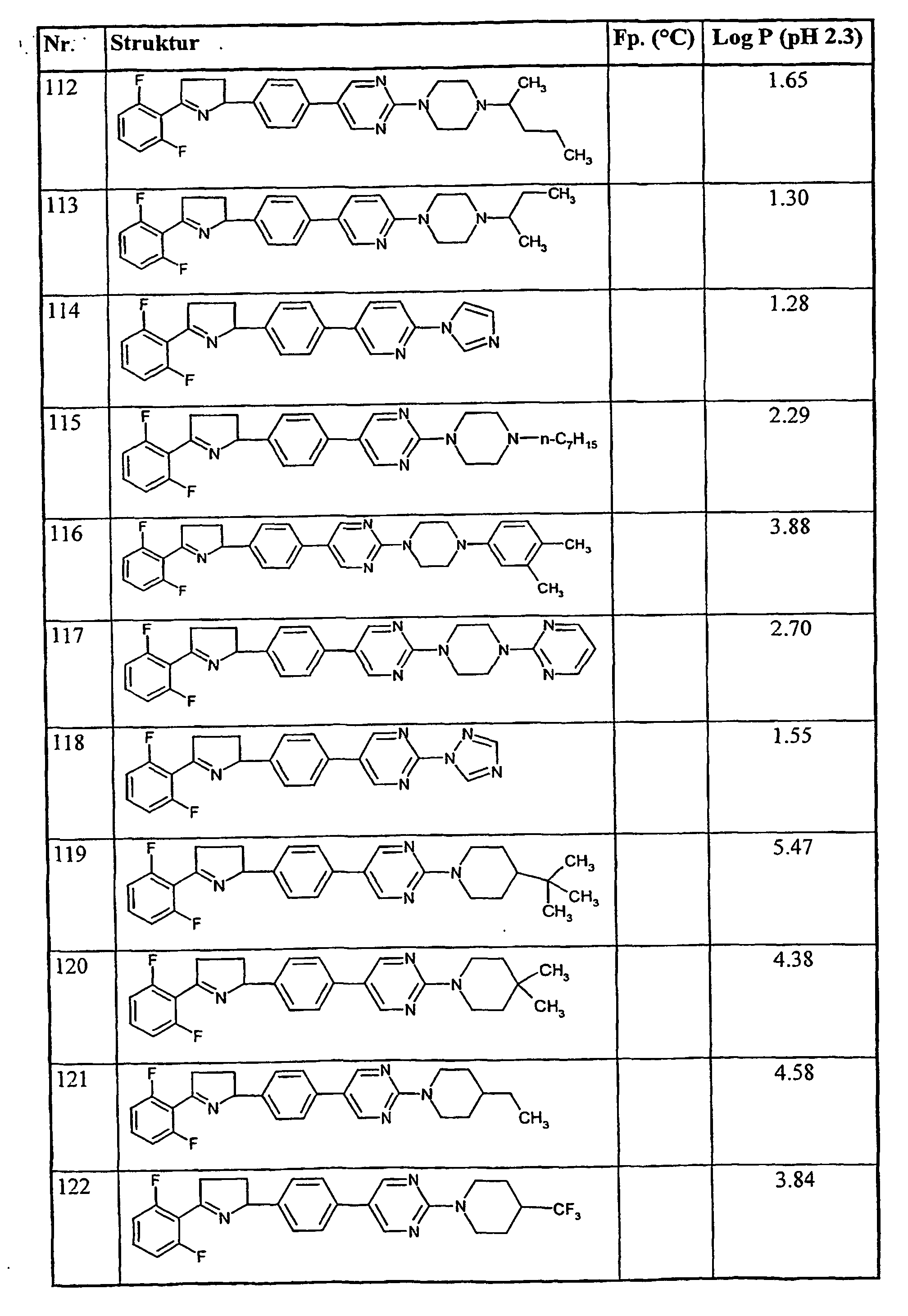

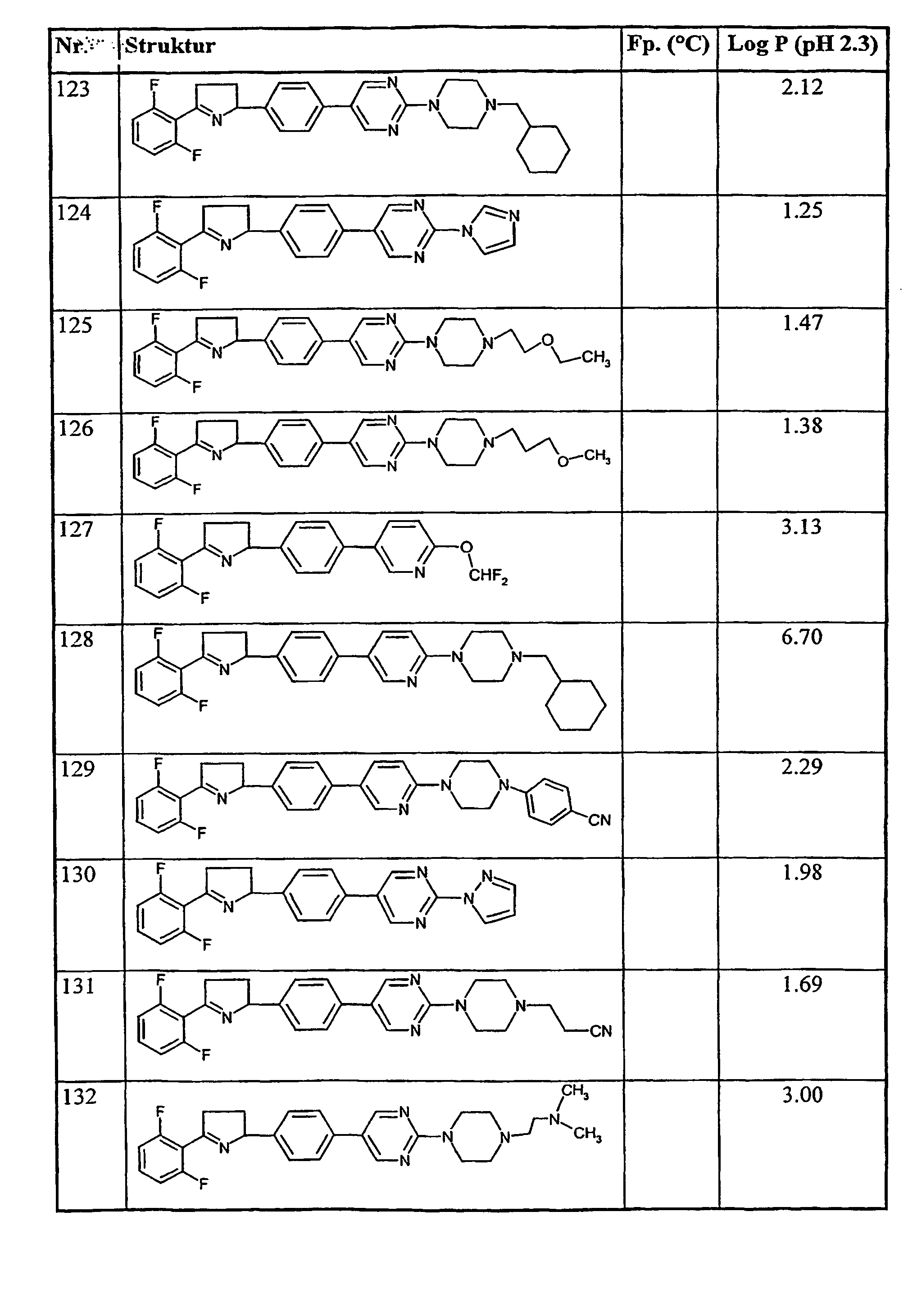

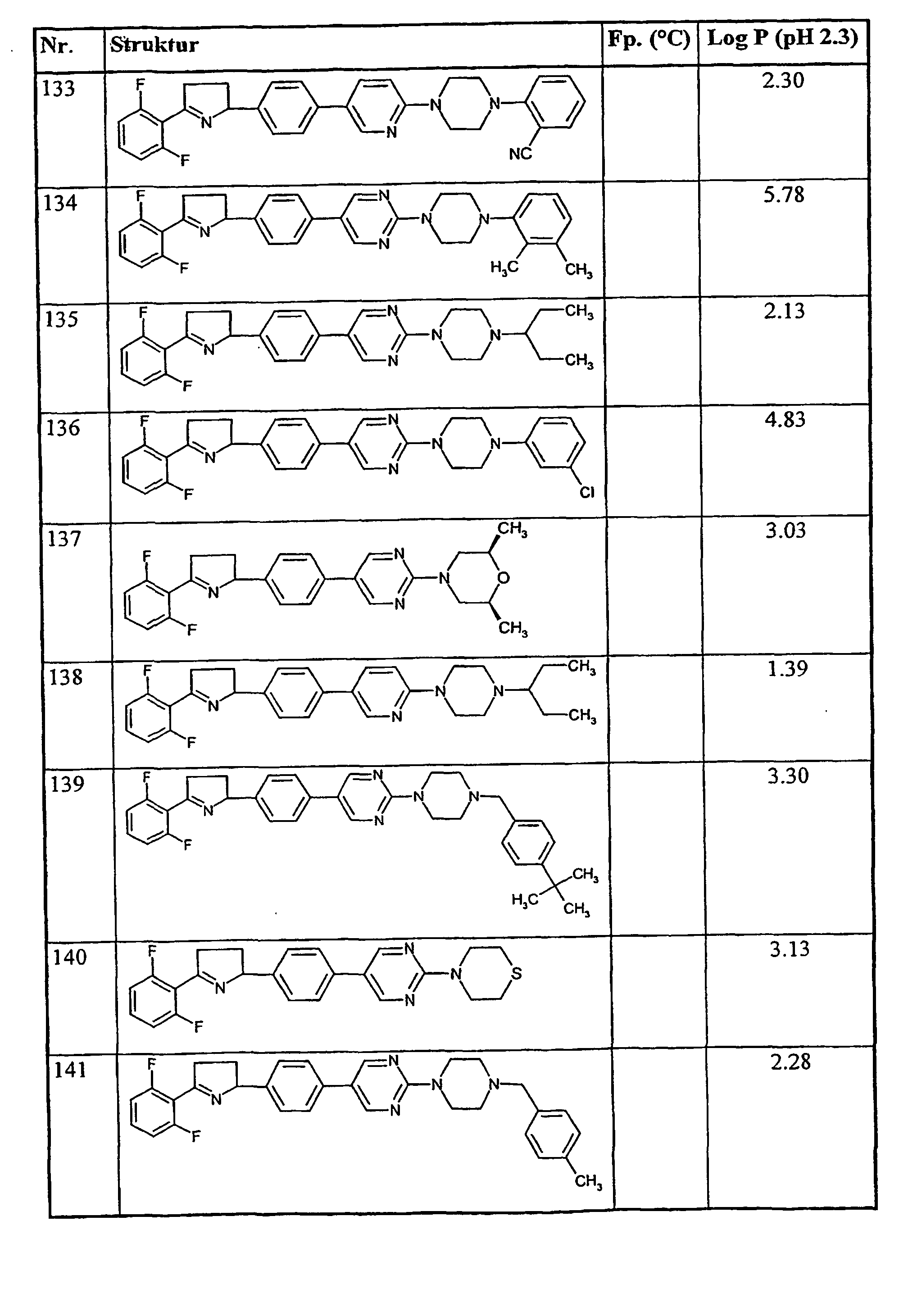

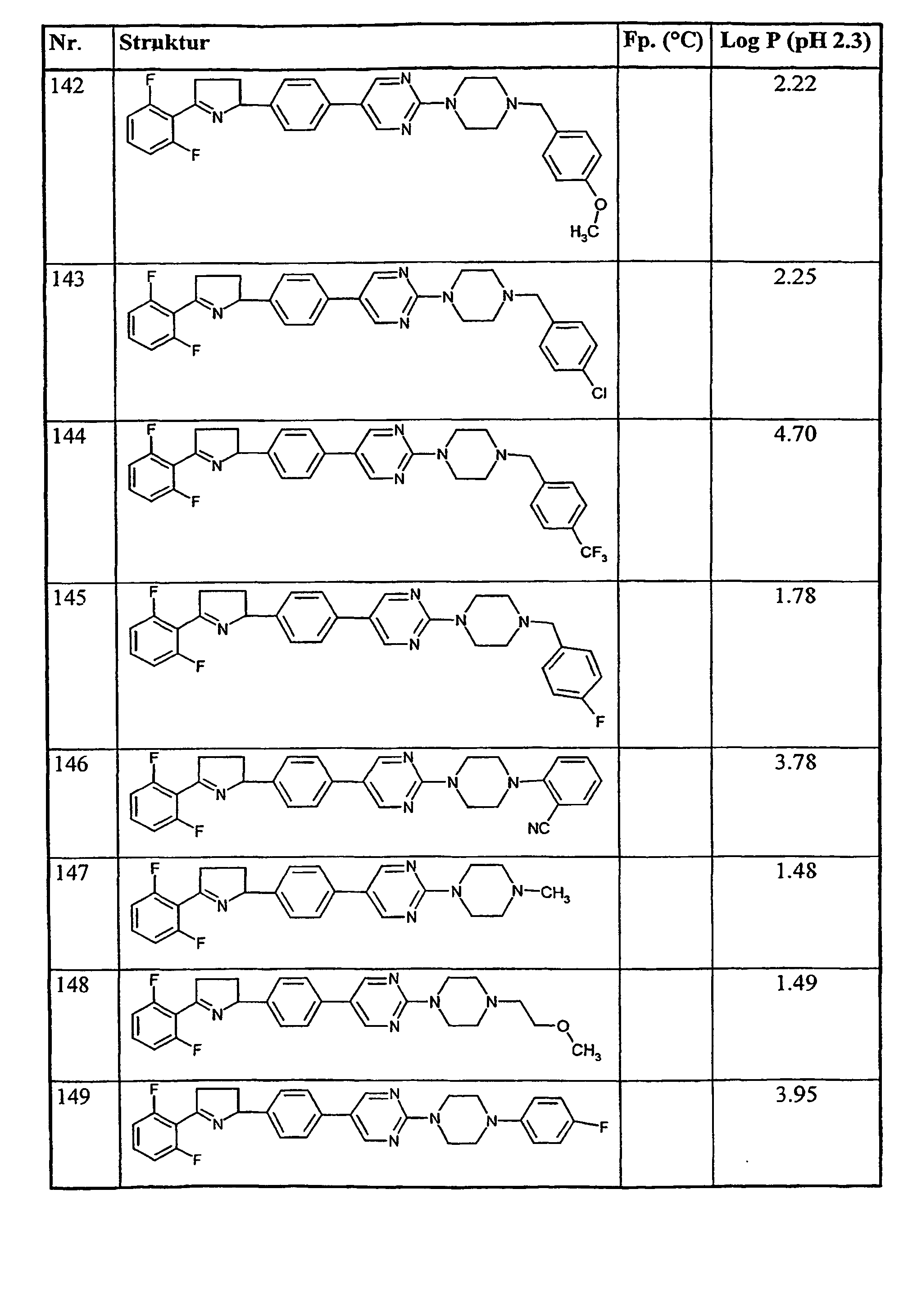

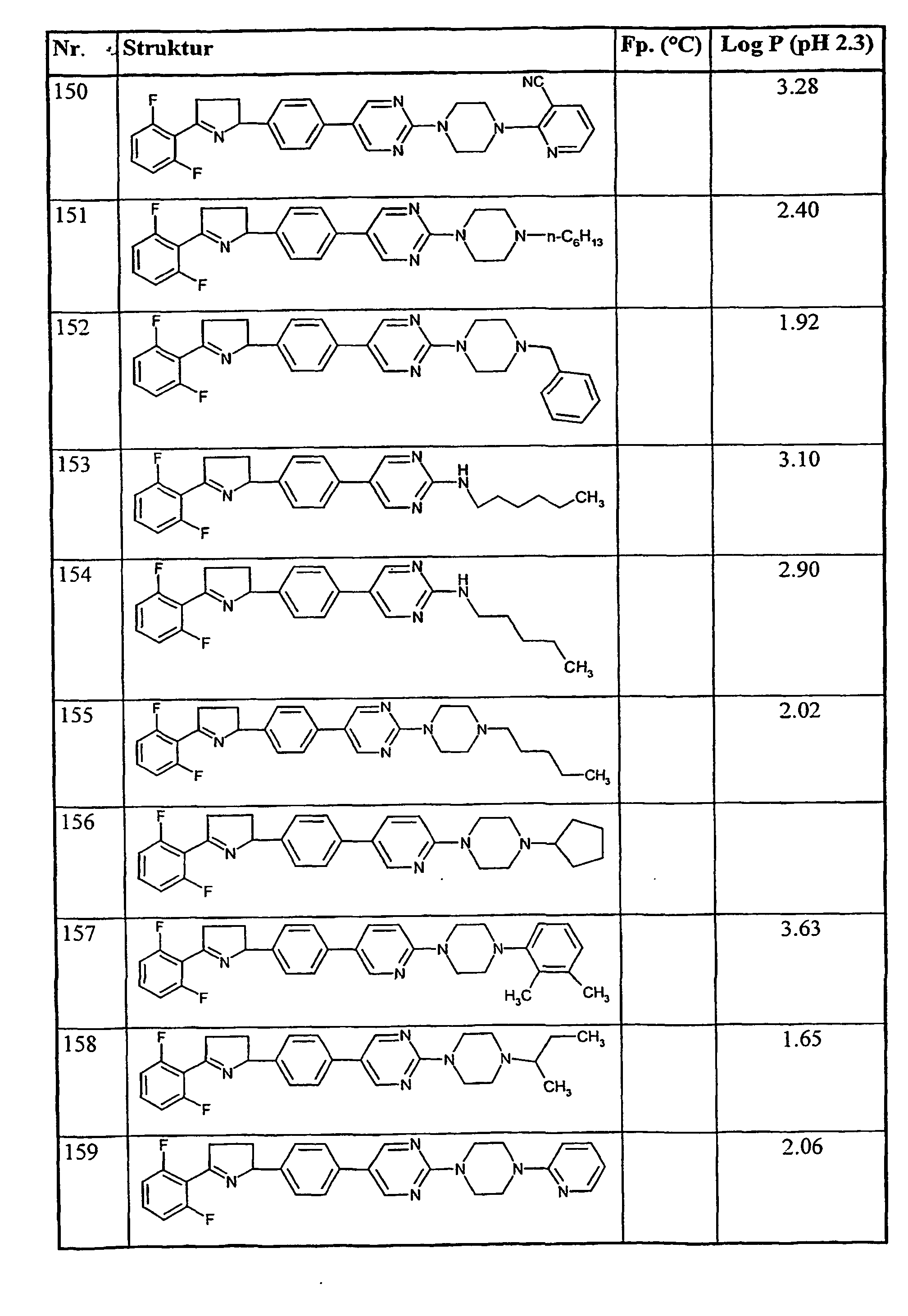

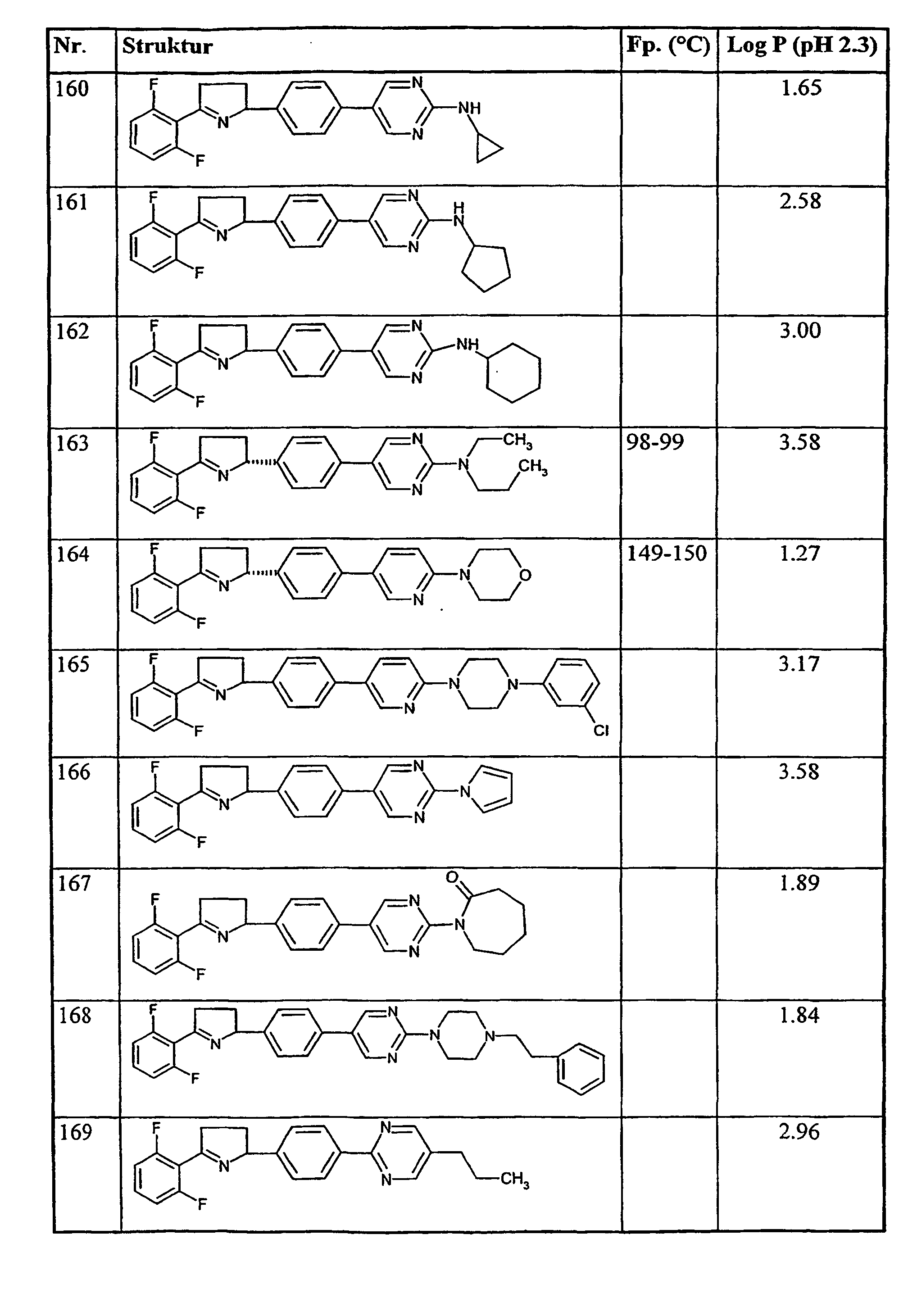

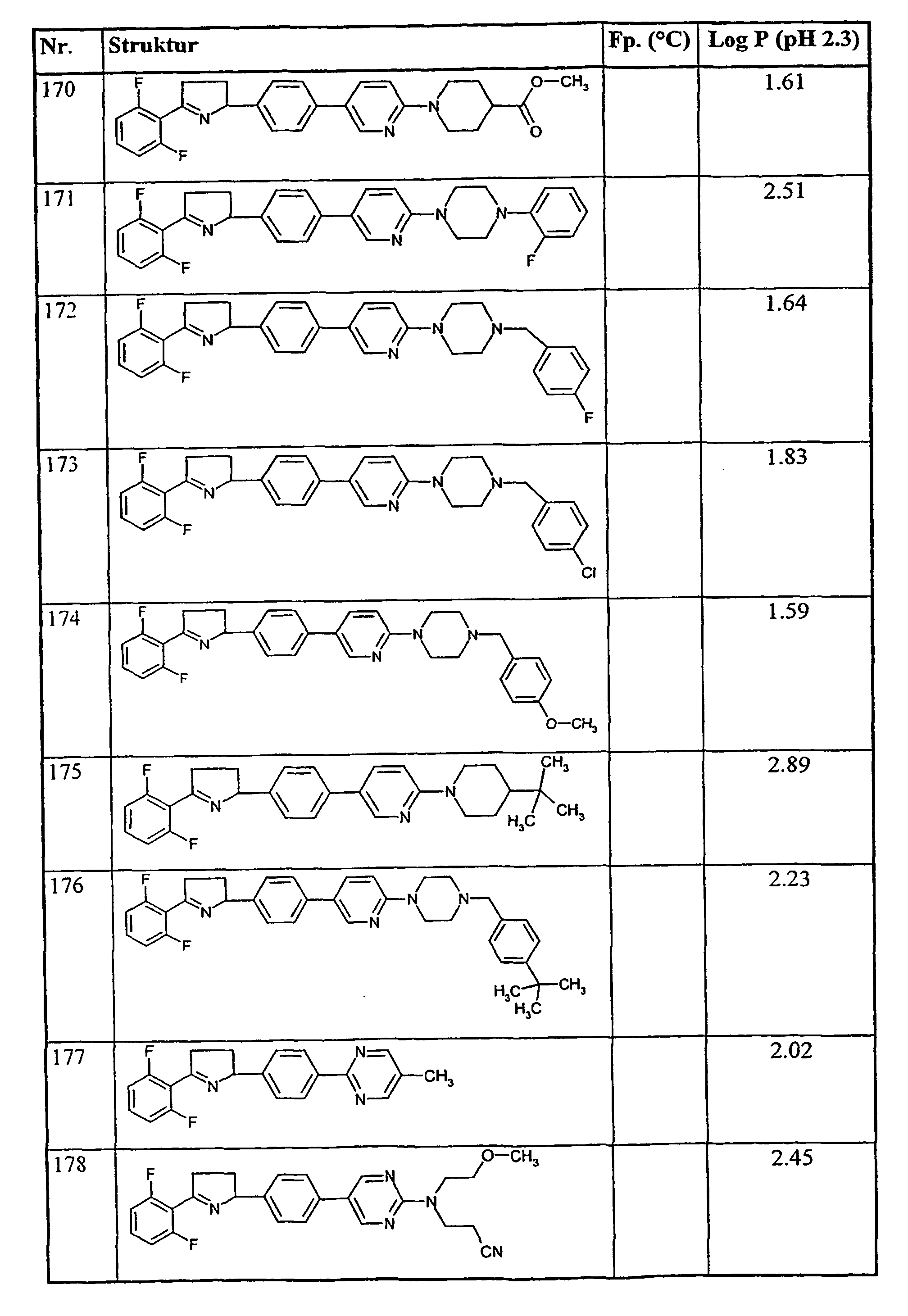

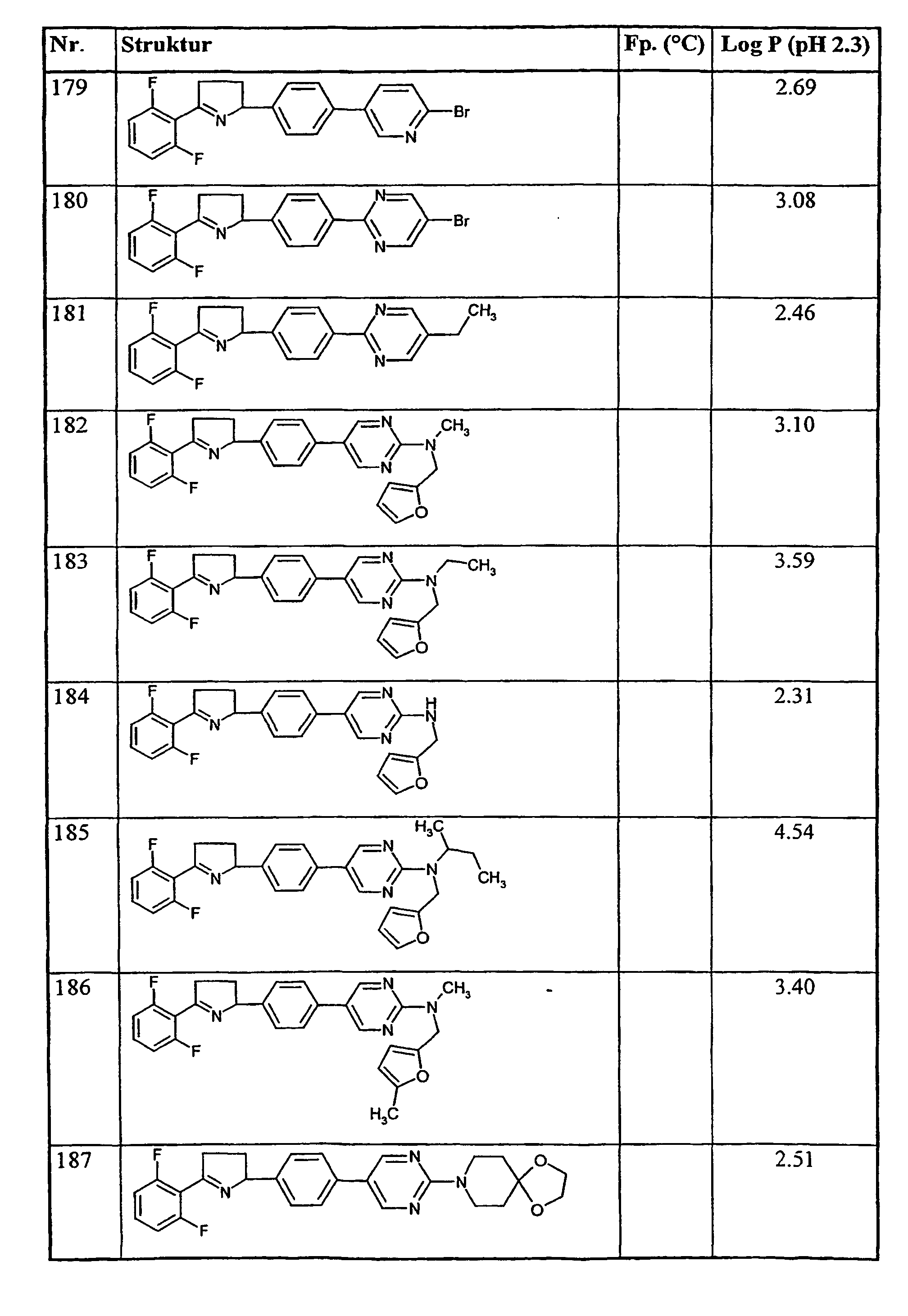

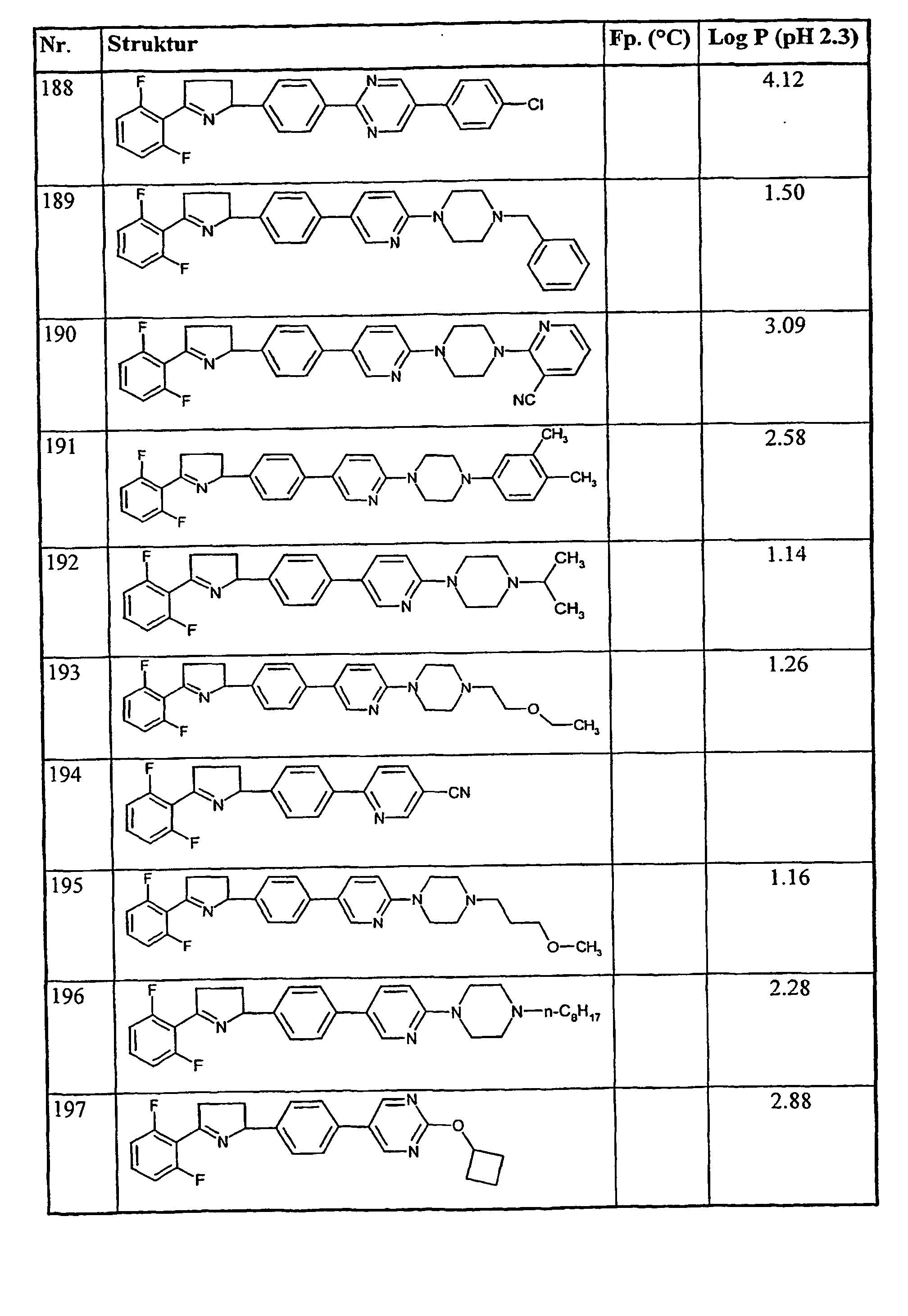

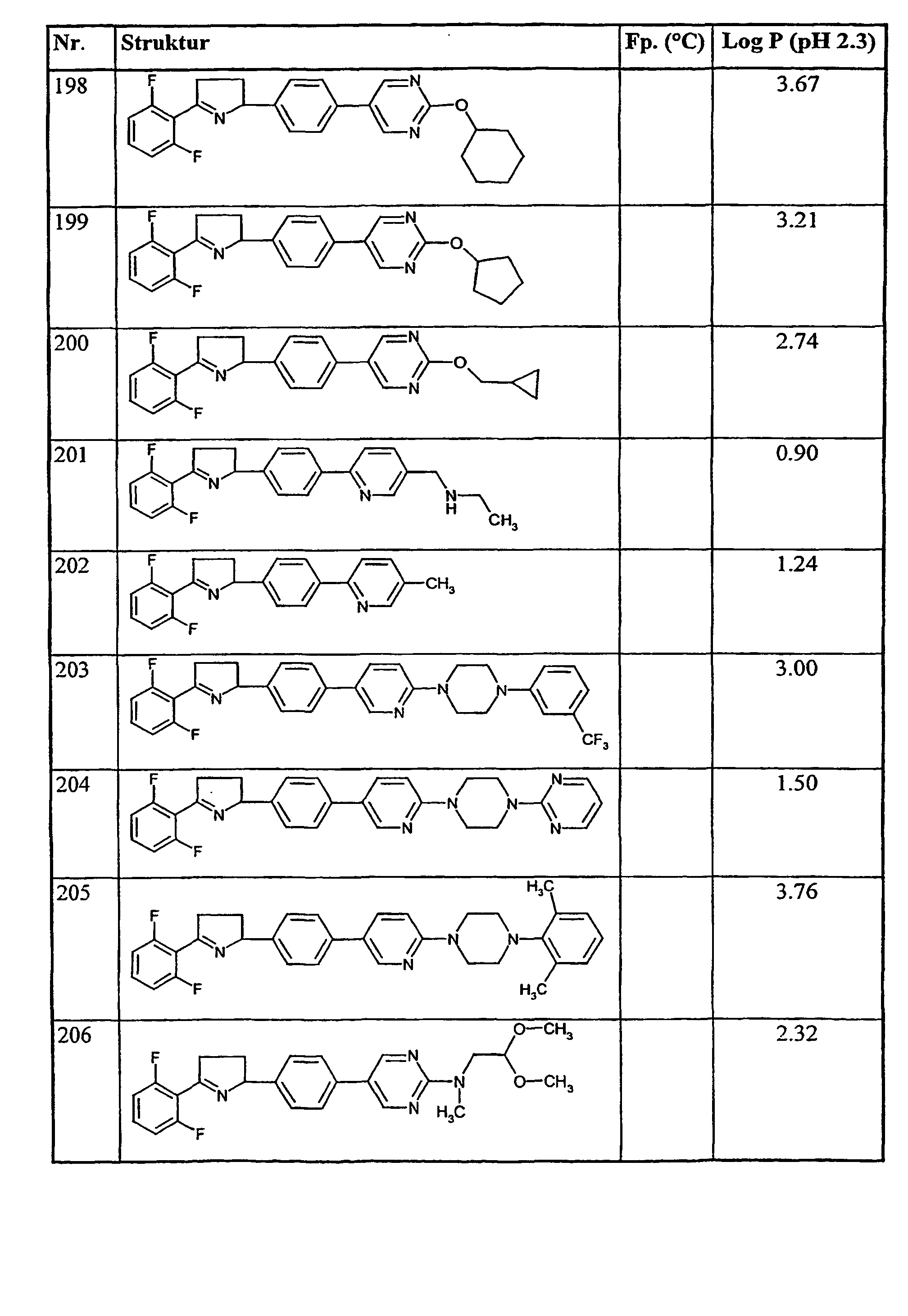

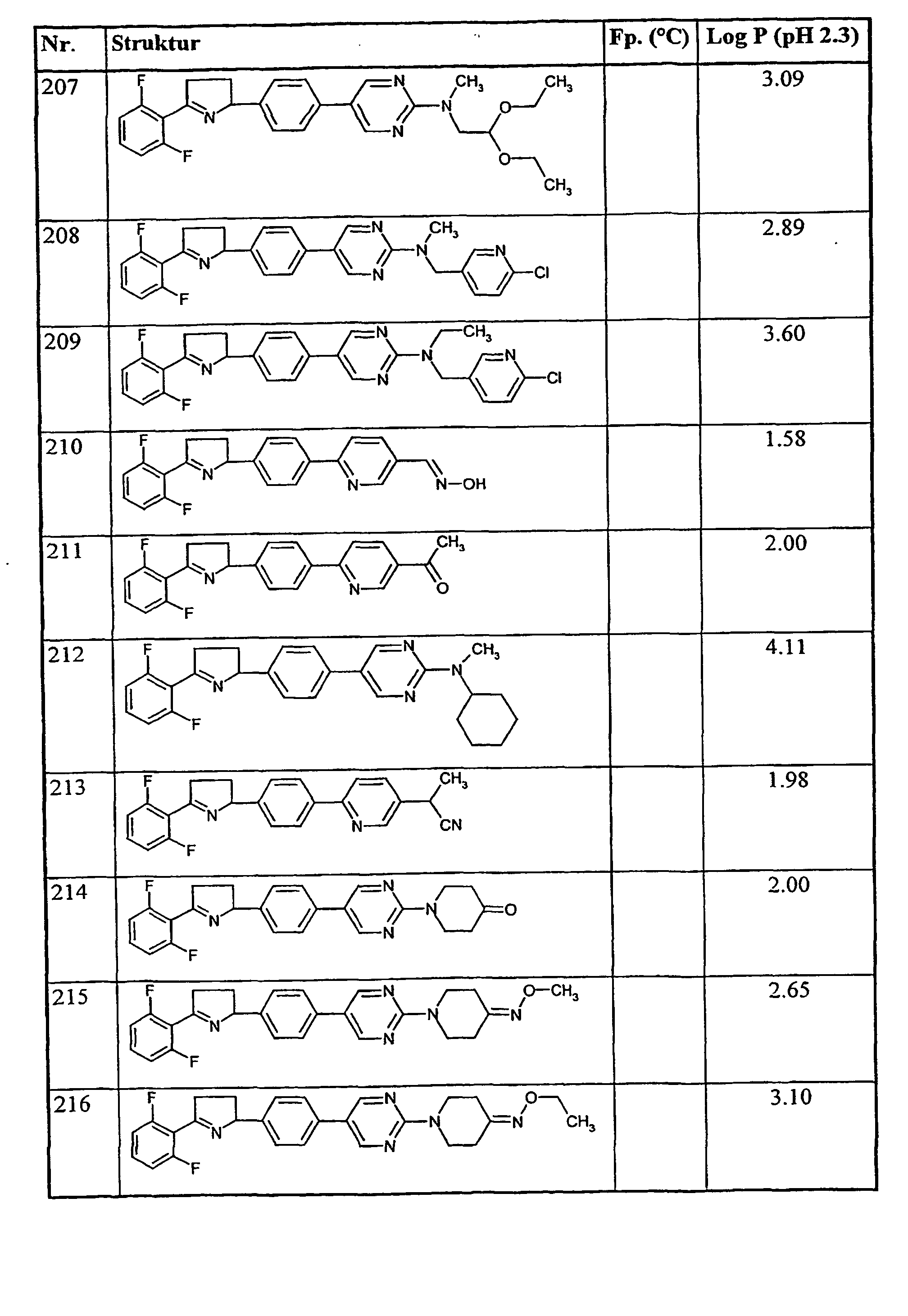

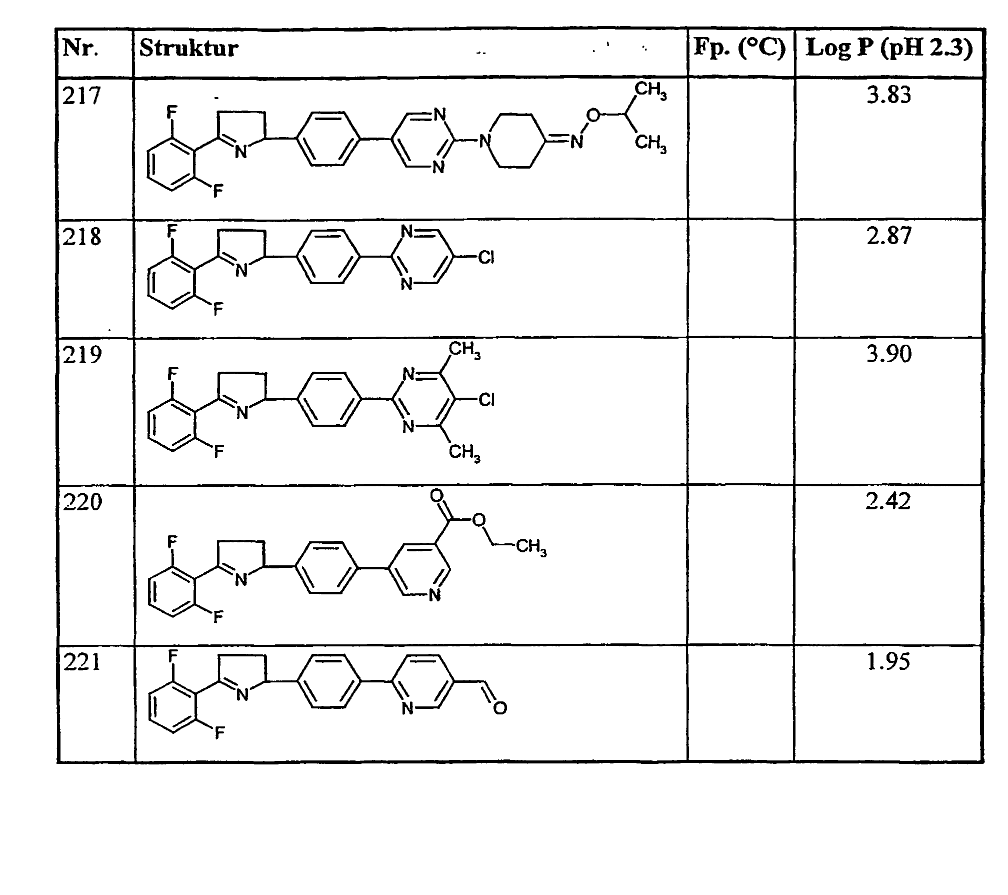

### Herstellung von Ausgangsstoffen der Formel (III)

### Beispiel III-1

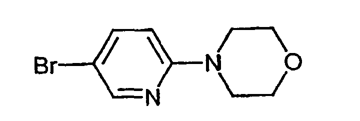

2,5-Dibrompyridin (50.00 g, 0.21 mol) und Morpholin (38.62 g, 0.44 mmol) werden in Toluol (300 ml) für 24 Stunden auf Rückfluss erhitzt. Nach Abkühlen wird das Reaktionsgemisch eingeengt und der Rückstand in Dichlormethan aufgenommen. Die organische Lösung wird mit Wasser gewaschen, über Magnesiumsulfat getrocknet, filtriert und eingeengt. Das Rohprodukt wird mittels Chromatographie an Kieselgel (Laufmittel: Cyclohexan/Essigsäureethylester 10:1, v/v) aufgereinigt.

Man erhält 23.65 g (46 % d. Th.) an 4-(5-Brom-2-pyridinyl)morpholin.

| | |
|---|---|
| HPLC: | Log P (pH 2.3) = 1.63 (Reinheit: 100 %) |
| Fp. | 65-68°C |

### Beispiel III-2

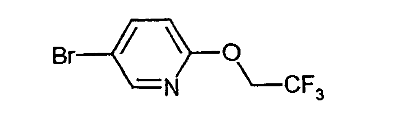

Natriumhydrid (0.75 g, 18.64 mmol) wird in DMF (50 ml) unter Argon bei 10°C vorgelegt und 2,2,2-Trifluorethanol (1.71 g, 17.09 mmol) wird zugetropft. Das Reaktionsgemisch wird anschließend bei 10°C für eine Stunde nachgerührt. Man gibt 2,5-Dibrompyridin (3.68 g, 15.53 mmol) portionsweise zu und rührt das Reaktionsgemisch für 16 Stunden bei Raumtemperatur nach. Wasser wird vorsichtig zugetropft und das Reaktionsgemisch mit Essigsäureethylester extrahiert. Die organische Phase wird über Magnesiumsulfat getrocknet, filtriert und eingeengt.

Man erhält 3.15 g (69 % d. Th.) an 5-Brom-2-(2,2,2-trifluorethoxy)pyridin.

| | |
|---|---|
| HPLC | Log P (pH 2.3) = 3.48 (Reinheit: 87 %) |

### Beispiel III-3

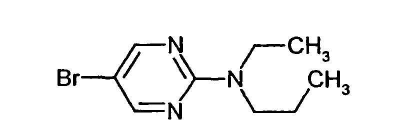

N-Ethyl-N-propylamin (4.80 g, 0.05 mol) wird in Acetonitril (200 ml) vorgelegt. Nach Zugabe von Kaliumcarbonat (7.60 g, 0.055 mol) wird das Reaktionsgemisch bei Raumtemperatur für eine Stunde nachgerührt. 5-Brom-2-chlorpyrimidin (9.67 g, 0.055 mol) wird zugegeben und das Reaktionsgemisch für 16 Stunden auf Rückfluss nachgerührt. Nach Abkühlen wird das Reaktionsgemisch in Wasser (500 ml) eingerührt und anschließend mit Essigsäureethylester (2 × 300 ml) extrahiert. Die organische Phase wird über Natriumsulfat getrocknet, filtriert und eingeengt.

Man erhält 12.20 g (99 % d. Th.) an N-(5-Brom-2-pyrimidinyl)-N-ethyl-N-propylamin.

| | |
|---|---|
| HPLC | Log P (pH 2.3) = 4.38 (Reinheit: 95 %) |

### Beispiel III-4

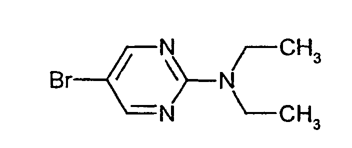

N,N-Dimethylaminhydrochlorid (2.04 g, 25 mmol), Kaliumcarbonat (6.91 g, 50 mmol), 5-Brom-2-chlorpyrimidin (4.35 g, 22.50 mmol) und Toluol (25 ml) werden in einem Autoklav 20 Stunden auf 110°C (Badtemperatur) erhitzt. Das Reaktionsgemisch wird mit Essigsäureethylester (50 ml) versetzt und die organische Phase anschließend mit Wasser gewaschen (2 × 50 ml), über Natriumsulfat getrocknet, filtriert und eingeengt.

Man erhält 4.01 g (72 % d. Th.) an N-(5-Brom-2-pyrimidinyl)-N,N-dimethylamin.

| | |
|---|---|
| HPLC: | Log P (pH 2.3) = 2.20 (Reinheit: 91 %) |
| Fp. | 68-69°C |

### Beispiel III-5

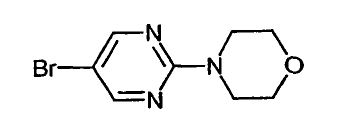

Morpholin (1.44 g, 16.5 mmol) wird in Acetonitril (70 ml) vorgelegt. Man gibt Kaliumcarbonat (2.30 g, 16.5 mmol) zu und rührt das Reaktionsgemisch bei Raumtemperatur für 1 Stunde nach. 5-Brom-2-chlorpyrimidin (2.90 g, 15.0 mmol) wird zugegeben und das Reaktionsgemisch für 16 Stunden auf Rückfluss nachgerührt. Nach Abkühlen wird das Reaktionsgemisch in Wasser (100 ml) eingerührt und anschließend mit Essigsäureethylester (2 x 50 ml) extrahiert. Die organische Phase wird über Natriumsulfat getrocknet, filtriert und eingeengt.

Man erhält 2.95 g (81 % d. Th.) an 4-(5-Brom-2-pyrimidinyl)morpholin.

| | |
|---|---|
| HPLC: | Log P (pH 2.3) = 2.15 (Reinheit: 92 %) |
| Fp. | 90°C |

### Beispiel III-6

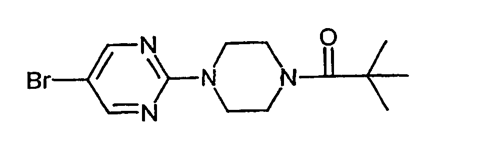

5-Brom-2-(1-piperazinyl)pyrimidin (1.43g, 5.90 mmol) und Triethylamin (1 ml) werden in Essigsäureethylester (30 ml) suspendiert. Man tropft bei 10°C Pivalinsäurechlorid (0.8 ml, 6.5 mmol) zu und rührt das Reaktionsgemisch bei Raumtemperatur für 16 Stunden nach und versetzt dann nacheinander mit Essigsäureethylester und Wasser. Die organische Phase wird mit gesättigter wässriger Natriumchloridlösung gewaschen, über Natriumsulfat getrocknet, filtriert und eingeengt.

Man erhält 1.47 g (76 % d. Th.) an 1-(tert-Butylcarbonyl)-4-(5-brom-2-pyrimidinyl)-piperazin.

| | |
|---|---|
| HPLC: | Log P (pH 2.3) = 2.86 (Reinheit: 99 %) |
| Fp. | 166-172°C |

### Beispiel III-7

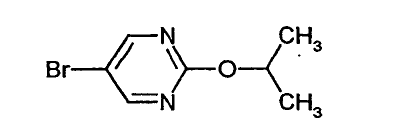

Zu vorgelegtem Isopropanol (25 ml) gibt man portionsweise unter Argonatmosphäre Natriumhydrid (0.50 g, 60 %ig, 16.5 mmol) zu und rührt das Reaktionsgemisch für 30 Minuten bei 60°C nach. Anschließend gibt man 5-Brom-2-chlorpyrimidin (2.90 g, 15.0 mmol) zu und rührt das Reaktionsgemisch unter Rückfluss für 16 Stunden nach. Nach Abkühlen wird das Reaktionsgemisch in Wasser (75 ml) eingerührt und anschließend mit Essigsäureethylester (2 × 75 ml) extrahiert. Die organische Phase wird über Natriumsulfat getrocknet, filtriert und eingeengt. Das Rohprodukt wird chromatographisch an Kieselgel (Laufmittel: n-Hexan/Essigsäureethylester 9:1, v/v) aufgereinigt.

Man erhält 2.10 g (65 % d. Th.) an 5-Brom-2-pyrimidinylisopropylether.

| | |
|---|---|
| HPLC | Log P (pH 2.3) = 2.39 (Reinheit: 99 %) |

### Beispiel III-8

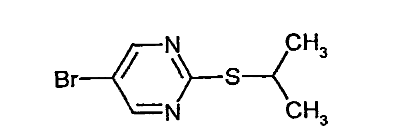

Natrium-2-propanthiolat (1.62 g, 16.5 mmol) wird in DMF (40 ml) vorgelegt. Bei Raumtemperatur gibt man 5-Brom-2-chlorpyrimidin (2.90 g, 15.0 mmol) zu und rührt das Reaktionsgemisch bei Raumtemperatur für 16 Stunden nach. Anschließend wird das Reaktionsgemisch in Wasser (100 ml) eingerührt und mit Essigsäureethylester (2 × 75 ml) extrahiert. Die organische Phase wird mit gesättigter Natriumchloridlösung gewaschen, über Natriumsulfat getrocknet, filtriert und eingeengt. Das Rohprodukt wird chromatographisch an Kieselgel (Laufmittel: n-Hexan/ Essigsäureethylester 20:1, v/v) aufgereinigt.

Man erhält 0.40 g (11 % d. Th.) an 5-Brom-2-pyrimidinylisopropylsulfid.

| | |
|---|---|
| HPLC | Log P (pH 2.3) = 3.34 (Reinheit: 100 %) |

### Beispiel III-9

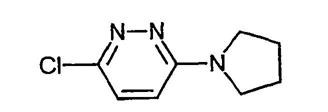

3,6-Dichlorpyridazin (3.35 g, 22.5 mmol), Kaliumcarbonat (3.45 g, 25.0 mmol), Pyrrolidin (1.78 g, 25.0 mmol) und Toluol (25 ml) werden in einem Autoklav 20 Stunden auf 110°C (Badtemperatur) erhitzt. Danach wird das Toluol unter vermindertem Druck entfemt, das Rohprodukt mit Wasser versetzt und das Produkt schließlich abgesaugt.

Man erhält 3.57 g (86 % d. Th.) an 3-Chlor-6-(1-pyrrolidinyl)pyridazin.

| | |
|---|---|
| Fp. | 129-131°C |

### Beispiel III-10

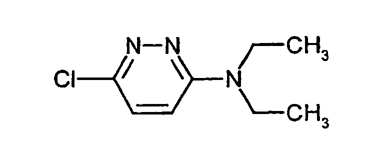

3,6-Dichlorpyridazin (3.35 g, 22.5 mmol), Kaliumcarbonat (3.45 g, 25.0 mmol), N,N-Diethylamin (1.83 g, 25.0 mmol) und Toluol (25 ml) werden in einem Autoklav 20 Stunden auf 110°C (Badtemperatur) erhitzt. Das Reaktionsgemisch wird mit Wasser (50 ml) und Essigsäureethylester (25 ml) versetzt und die Phasen werden getrennt. Die wässrige Phase wird mit Essigsäureethylester (25 ml) nochmals extrahiert, und die kombinierten organischen Phasen werden über Natriumsulfat getrocknet und filtriert. Zum Filtrat wird Florisil (10 g) zugegeben und das Lösungsmittel eingeengt. Das Rohprodukt wird chromatographisch an Kieselgel (Laufmittel: n-Hexan/Essigsäureethylester 3:1, v/v) aufgereinigt.

Man erhält 1.25 g (30 % d. Th.) an N-(6-Chlor-3-pyridazinyl)-N,N-diethylamin.

| | |
|---|---|
| HPLC: | Log P (pH 2.3) = 0.51 (Reinheit: 97 %) |
| Fp. | 42-46°C |

### Beispiel III-11

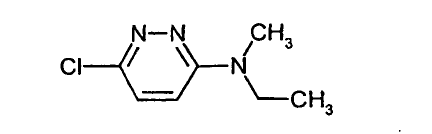

3,6-Dichlorpyridazin (3.35 g, 22.5 mmol), Kaliumcarbonat (3.45 g, 25.0 mmol), N-Methyl-N-ethylamin (1.48 g, 25.0 mmol) und Toluol (25 ml) werden in einem Autoklav 20 Stunden auf 110°C (Badtemperatur) erhitzt. Das Reaktionsgemisch wird mit Wasser (50 ml) und Essigsäureethylester (50 ml) versetzt und die Phasen werden getrennt. Die wässrige Phase wird mit Essigsäureethylester (25 ml) nochmals extrahiert, und die kombinierten organischen Phasen werden über Natriumsulfat getrocknet und filtriert. Zum Filtrat wird Florisil (10 g) zugegeben und das Lösungsmittel eingeengt. Das Rohprodukt wird chromatographisch an Kieselgel (Laufmittel: n-Hexan/Essigsäureethylester 4:1, v/v) aufgereinigt.

Man erhält 2.50 g (65 % d. Th.) an N-(6-Chlor-3-pyridazinyl)-N-ethyl-N-methylamin.

| | |
|---|---|
| Fp. | 61-63°C |

### Beispiel III-12

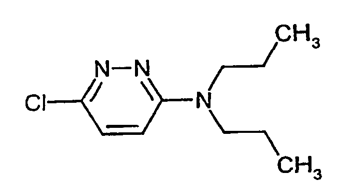

N,N-Dipropylamin (2.53 g, 25.0 mmol) und Kaliumcarbonat (3.45 g, 25.0 mmol) werden in DMF (50 ml) suspendiert und für 30 Minuten nachgerührt. 3,6-Dichlorpyridazin (3.35 g, 22.5 mmol) wird zugegeben und das Reaktionsgemisch für 16 Stunden bei 110°C nachgerührt. DMF wird unter vermindertem Druck entfernt und der Rückstand mit Wasser (50 ml) und Essigsäureethylester (50 ml) versetzt. Die organische Phase wird über Natriumsulfat getrocknet und filtriert. Zum Filtrat gibt man Florisil (10 g) und engt das Lösungsmittel ein. Das Rohprodukt wird chromatographisch an Kieselgel (Laufmittel: n-Hexan/Essigsäureethylester 9:1→4:1, jeweils v/v) aufgereinigt.

Man erhält 1.10 g (30 % d. Th.) an N-(6-Chlor-3-pyridazinyl)-N,N-dipropylamin.

| | |
|---|---|
| HPLC: | Log P (pH 2.3) = 1.82 (Reinheit: 99 %) |
| Fp. | 64-66°C |

### Beispiel III-13

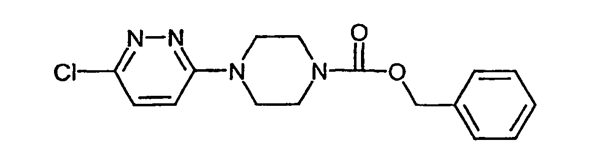

Piperazin-N-carbonsäurebenzylester (2.20 g, 0.01 mol) wird in Acetonitril (50 ml) vorgelegt. Kaliumcarbonat (1.38g, 0.01 mol) wird zugegeben und das Reaktionsgemisch 30 Minuten bei Raumtemperatur nachgerührt. 3,6-Dichlorpyridazin (1.49 g, 0.01 mol) wird zugegeben und das Reaktionsgemisch wird für 16 Stunden bei 100°C nachgerührt. Nach Abkühlen auf Raumtemperatur wird das Reaktionsgemisch in Wasser (100 ml) eingerührt. Das Produkt wird abgesaugt.

Man erhält 1.95 g (58 % d. Th.) an Benzyl 4-(6-chlor-3-pyridazinyl)-1-piperazin-carboxylat.

| | |
|---|---|
| HPLC: | Log P (pH 2.3) = 2.36 (Reinheit: 95 %) |
| Fp. | 124-125°C |

### Beispiel III-14

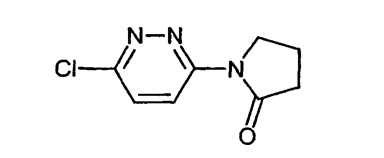

Natriumhydrid (0.80 g, 60 %ig, 0.02 mol) wird in DMF (40 ml) suspendiert. Unter einem Argonstrom wird 2-Pyrrolidon (1.70 g, 0.02 mol) bei 10°C zugetropft. Man lässt das Reaktionsgemisch auf Raumtemperatur kommen, gibt 3,6-Dichlorpyridazin (2.23 g, 0.015 mol) zu und rührt das Reaktionsgemisch für 16 Stunden bei Raumtemperatur nach. Das Reaktionsgemisch wird anschließend in Wasser (300 ml) eingerührt und mit Essigsäureethylester (200 ml) extrahiert. Die organische Phase wird mit gesättigter wässriger Natriumchloridlösung gewaschen, über Natriumsulfat getrocknet, filtriert und eingeengt. Das Rohprodukt wird chromatographisch an Kieselgel (Laufmittel: n-Hexan/Essigsäureethylester 1:1→3:7, jeweils v/v) aufgereinigt.

Man erhält 0.68 g (17 % d. Th.) an 1-(6-Chlor-3-pyridazinyl)-2-pyrrolidinon.

| | |
|---|---|
| HPLC | Log P (pH 2.3) = 1.09 (Reinheit: 75 %) |

### Beispiel III-15

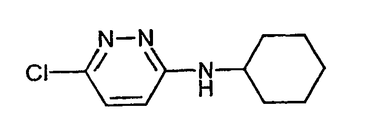

3,6-Dichlorpyridazin (11.20 g, 0.075 mol), Cyclohexylamin (25.70 ml, 0.225 mol) und Wasser (37.5 ml) werden vorgelegt. Bei Raumtemperatur wird konzentrierte Salzsäure (1.5 ml, 37 %ig) zugegeben und das Reaktionsgemisch 20 Stunden bei 100°C (Badtemperatur) nachgerührt. Nach Abkühlen wird gesättigte wässrige Natriumhydrogencarbonatlösung (30 ml) zugegeben. Der Niederschlag wird abgesaugt und mehrfach mit Wasser gewaschen.

Man erhält 12.30 g (78 % d. Th.) an N-(6-Chlor-3-pyridazinyl)-N-cyclohexylamin.

| | |
|---|---|
| HPLC: | Log P (pH 2.3) = 0.95 (Reinheit: 99 %) |
| Fp. | 161-163°C |

### Beispiel III-16

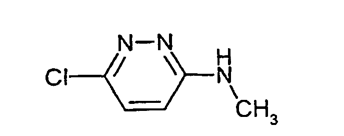

3,6-Dichlorpyridazin (25.0 g, 0.17 mol), Methylamin (39.0 g, 40 %ige Lösung in Wasser, 0.50 mol), Wasser (50 ml) und konzentrierte Salzsäure (3.4 ml, 37 %ig) werden in einem Autoklav für 20 Stunden bei 100°C (Badtemperatur) erhitzt. Nach Abkühlen wird der Niederschlag abgesaugt und mehrfach mit gesättigter wässriger Natriumhydrogencarbonatlösung gewaschen.

Man erhält 20.95 g (78 % d. Th.) an N-(6-Chlor-3-pyridazinyl)-N-methylamin.

| | |
|---|---|
| Fp. | 194-196°C |
| GC/MS: | Index 1472 (Reinheit: 100 %); M⁺ = 143/145 |

### Beispiel III-17

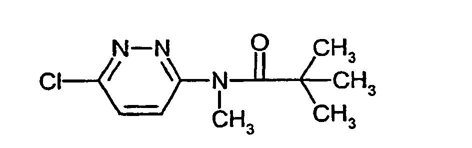

N-(6-Chlor-3-pyridazinyl)-N-methylamin (1.08 g, 0.0075 mol) und Triethylamin (1.4 ml, 0.01 mol) werden in Essigsäureethylester (50 ml) vorgelegt. Bei 60°C wird Pivalinsäurechlorid (1.23 ml, 0.01 mol) zugetropft und das Reaktionsgemisch für 16 Stunden bei 60°C nachgerührt. Nach Abkühlen wird das Reaktionsgemisch mit Wasser gewaschen, die organische Phase über Natriumsulfat getrocknet, filtriert und eingeengt.

Man erhält 1.21 g (71% d. Th.) an N-(6-Chlor-3-pyridazinyl)-N-methyl-2,2-trimethylpropanamid.

| | |
|---|---|
| HPLC: | Log P (pH 2.3) = 1.69 (Reinheit: 95 %) |
| Fp. | 84-87°C |

### Beispiel III-18

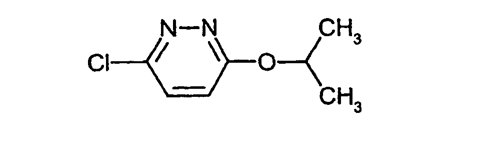

Zu vorgelegtem Isopropanol (60 ml) wird unter einem Argonstrom bei Raumtemperatur Natriumhydrid (1.0 g, 80 %ig, 0.033 mol) portionsweise zugegeben und anschließend für 30 Minuten bei 60°C nachgerührt. Bei Raumtemperatur wird 3,6-Dichlorpyridazin (4.50 g, 0.03 mol) zugegeben und das Reaktionsgemisch 48 Stunden unter Rückfluss nachgerührt. Das Reaktionsgemisch wird in Wasser (100 ml) eingerührt und mit Essigsäureethylester (2 × 100 ml) extrahiert. Die organische Phase wird über Natriumsulfat getrocknet, filtriert und eingeengt. Das Rohprodukt wird chromatographisch an Kieselgel (Laufmittel: n-Hexan/Essigsäureethylester 9:1, v/v) aufgereinigt.

Man erhält 1.85 g (36 % d. Th.) an 3-Chlor-6-isopropoxypyridazin.

| | |
|---|---|
| HPLC: | Log P (pH 2.3) = 1.94 (Reinheit: 98 %) |
| Fp. | 83-85°C |

### Beispiel III-19

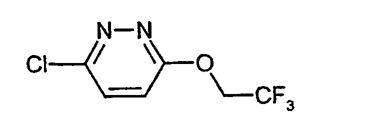

Trifluorethanol (25.0 g, 0.25 mol) wird in Acetonitril (200 ml) vorgelegt. Kaliumcarbonat (6.90 g, 0.05 mol) wird zugegeben und das Reaktionsgemisch 1 Stunde bei Raumtemperatur nachgerührt. Nach Zugabe von 3,6-Dichlorpyridazin (11.20 g, 0.075 mol) wird das Reaktionsgemisch 16 Stunden unter Rückfluss nachgerührt. Das Reaktionsgemisch wird in Wasser (500 ml) eingerührt und mit Essigsäureethylester (2 × 300 ml) extrahiert. Die organische Phase wird über Natriumsulfat getrocknet, filtriert und eingeengt.

Man erhält 12.38 g (68 % d. Th.) an 3-Chlor-6-(2,2,2-trifluorethoxy)pyridazin.

| | |
|---|---|
| HPLC | Log P (pH 2.3) = 2.05 (Reinheit: 88 %) |

### Beispiel III-20

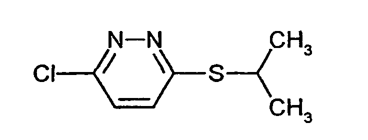

Natrium-2-propanthiolat (1.62 g, 16.5 mmol) wird in DMF (30 ml) vorgelegt. Bei Raumtemperatur wird eine Lösung aus 3,6-Dichlorpyridazin (2.20 g, 15.0 mmol) in DMF (10 ml) zugegeben. Das Reaktionsgemisch wird bei Raumtemperatur für 16 Stunden nachgerührt, in Wasser (100 ml) eingerührt und anschließend mit Essigsäureethylester (2 × 75 ml) extrahiert. Die organische Phase wird mit gesättigter Natriumchloridlösung (2 × 75 ml) gewaschen, über Natriumsulfat getrocknet, filtriert und eingeengt. Das Rohprodukt wird chromatographisch an Kieselgel (Laufmittel: n-Hexan/Essigsäureethylester 9:1, v/v) aufgereinigt.

Man erhält 1.40 g (50 % d. Th.) an 6-Chlor-3-pyridazinylisopropylsulfid.

| | |
|---|---|
| HPLC: | Log P (pH 2.3) = 2.34 (Reinheit: 100 %) |
| Fp. | 90-91°C |

### Beispiel III-21

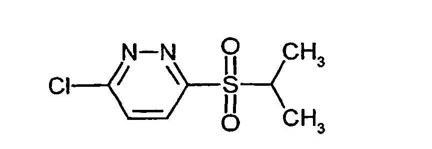

6-Chlor-3-pyridazinylisopropylsulfid (1.90g, 0.01 mol) wird in Dichlormethan (100 ml) vorgelegt. 3-Chlorperoxybenzoesäure (5.06 g, 0.022 mol) wird bei Raumtemperatur portionsweise zugegeben. Das Reaktionsgemisch wird bei Raumtemperatur für 16 Stunden nachgerührt, der Niederschlag abgesaugt und verworfen. Das Filtrat wird nacheinander mit wässriger Natriumdithonitlösung, gesättigter wässriger Natriumhydrogencarbonatlösung (2 × 50 ml) und Wasser (1 × 50 ml) gewaschen, über Natriumsulfat getrocknet, filtriert und eingeengt.

Man erhält 1.93 g (76 % d. Th.) an 6-Chlor-3-pyridazinylisopropylsulfon.

| | |
|---|---|
| HPLC: | Log P (pH 2.3) = 1.20 (Reinheit: 88 %) |
| Fp. | 122-124°C |

### Beispiel III-22

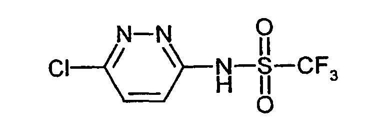

3-Amino-6-chlorpyridazin (3.20 g, 0.025 mmol) wird in Dichlormethan (100 ml) suspendiert. Bei Raumtemperatur wird nacheinander Triethylamin (4.20 ml, 0.025 mol) und Trifluormethansulfonsäureanhydrid (4.20 ml, 0.025 mol) zugetropft. Das Reaktionsgemisch wird bei Raumtemperatur eine Stunde nachgerührt, nacheinander mit gesättigter wässriger Natriumhydrogencarbonatlösung (3 × 50 ml), gesättigter wässriger Natriumchloridlösung (1 × 50 ml) gewaschen, über Natriumsulfat getrocknet, filtriert und eingeengt. Das Rohprodukt wird chromatographisch an Kieselgel (Laufmittel: n-Hexan/Essigsäureethylester 3:1→0:1, jeweils v/v) aufgereinigt.

Man erhält 2.51 g (38 % d. Th.) an N-(6-Chlor-3-pyridazinyl)(trifluor)methan-sulfonamid.

| | |
|---|---|
| HPLC | Log P (pH 2.3) = 1.56 (Reinheit: 99 %) |

### Beispiel III-23

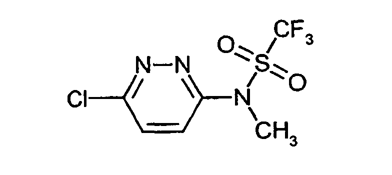

N-(6-Chlor-3-pyridazinyl)(trifluor)methansulfonamid (1.20 g, 4.6 mmol) wird in Acetonitril (40 ml) vorgelegt. Man gibt Kaliumcarbonat (0.95 g, 6.7 mmol) bei Raumtemperatur zu und rührt 30 Minuten nach. Anschließend wird bei Raumtemperatur Iodmethan (0.42 ml, 6.7 mmol) zugetropft und das Reaktionsgemisch bei 40°C für 16 Stunden nachgerührt. Das Reaktionsgemisch wird in Wasser (50 ml) eingerührt, der Niederschlag abgesaugt und mit Essigsäureethylester (2 × 50 ml) gewaschen. Die kombinierten organischen Phasen werden mit gesättigter wässriger Natriumchloridlösung (1 × 50 ml) gewaschen, über Natriumsulfat getrocknet, filtriert und eingeengt. Das Rohprodukt wird chromatographisch an Kieselgel (Laufmittel: n-Hexan/Essigsäureethylester 4:1, v/v) aufgereinigt.

Man erhält 0.68 g (54 % d. Th.) an N-(6-Chlor-3-pyridazinyl)(trifluor)-N-methyl-methansulfonamid.

| | |
|---|---|
| HPLC | Log P (pH 2.3) = 2.18 (Reinheit: 100 %) |

Die Bestimmung der angegebenen logP-Werte erfolgte gemäß EEC-Directive 79/831 Amex V.A8 durch HPLC (High Performance Liquid Chromatography) an einer Phasenumkehrsäule (C 18). Temperatur: 43°C.

Eluenten für die Bestimmung im sauren Bereich: 0,1 % wässrige Phosphorsäure, Acetonitril; linearer Gradient von 10 % Acetonitril bis 90 % Acetonitril.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A

### Heliothis virescens-Test

| | |
|---|---|
| Lösungsmittel | 30 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Sojatriebe (Glycine max) werden durch Tauchen in die Wirkstoffzubereitung der gewünschten Konzentration behandelt und mit Heliothis virescens-Raupen besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Wirkstoffe, Wirkstoffkonzentrationen und Versuchsergebnisse gehen aus der folgenden Tabelle hervor.

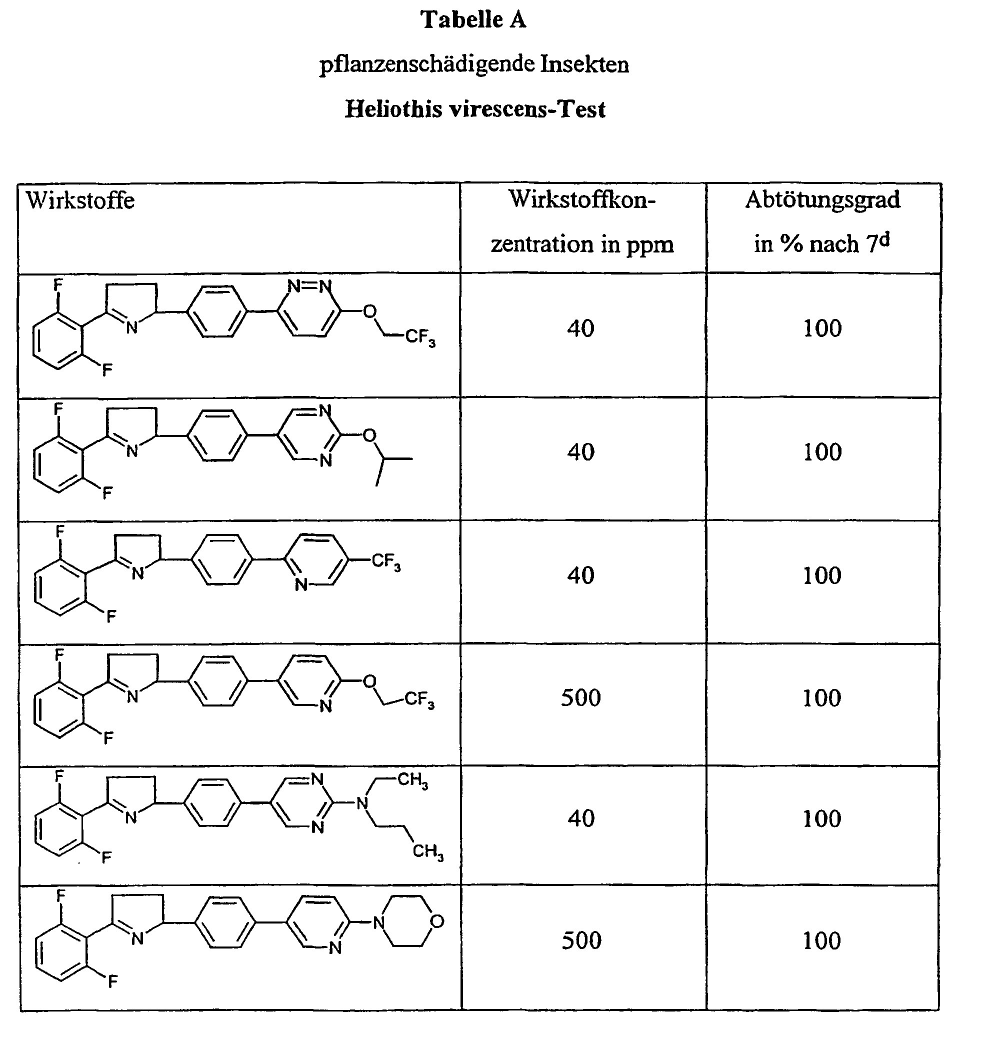

### Beispiel B

### Phaedon-Larven-Test

| | |
|---|---|
| Lösungsmittel | 30 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven des Meerrettichblattkäfers (Phaedon cochleariae) besetzt, solange die Blätter noch feucht sind.

Nach der gewünschten Zeit wird die Abtötung in % bestimmt. Dabei bedeutet 100%, dass alle Käferlarven abgetötet wurden; 0% bedeutet, dass keine Käferlarven abgetötet wurden.

Wirkstoffe, Wirkstoffkonzentrationen und Versuchsergebnisse gehen aus der folgenden Tabelle hervor.

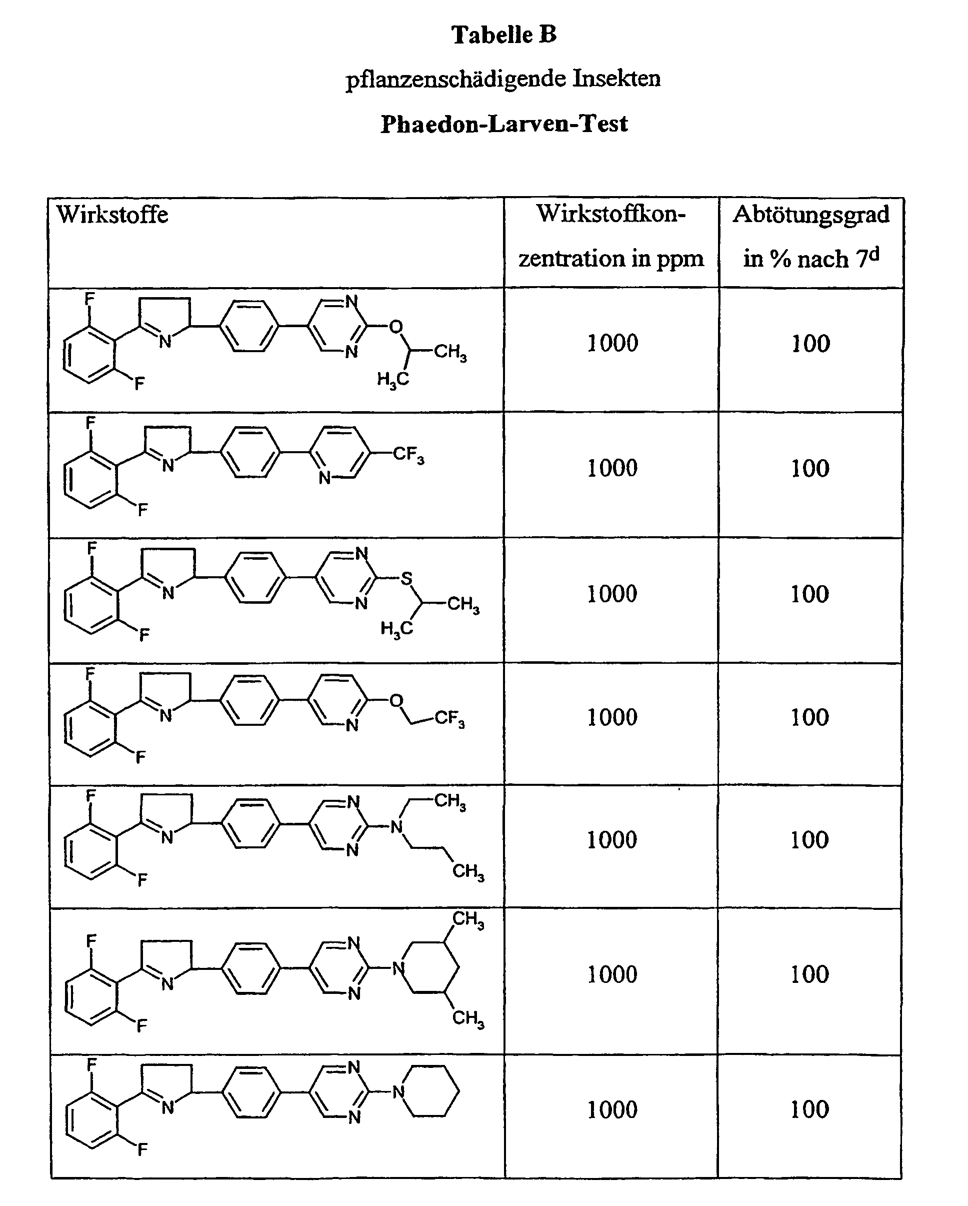

### Beispiel C

### Spodoptera exigua-Test

| | |
|---|---|
| Lösungsmittel | 30 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera exigua) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Wirkstoffe, Wirkstoffkonzentrationen und Versuchsergebnisse gehen aus der folgenden Tabelle hervor.

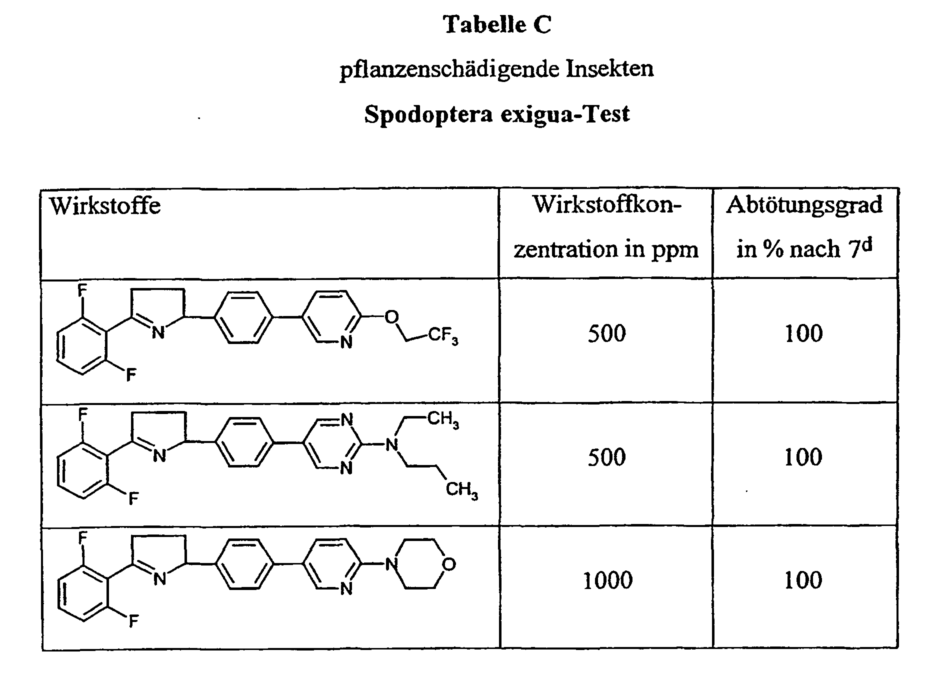

### Beispiel D

### Spodoptera frugiperda-Test

| | |
|---|---|
| Lösungsmittel | 30 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Wirkstoffe, Wirkstoffkonzentrationen und Versuchsergebnisse gehen aus der folgenden Tabelle hervor.

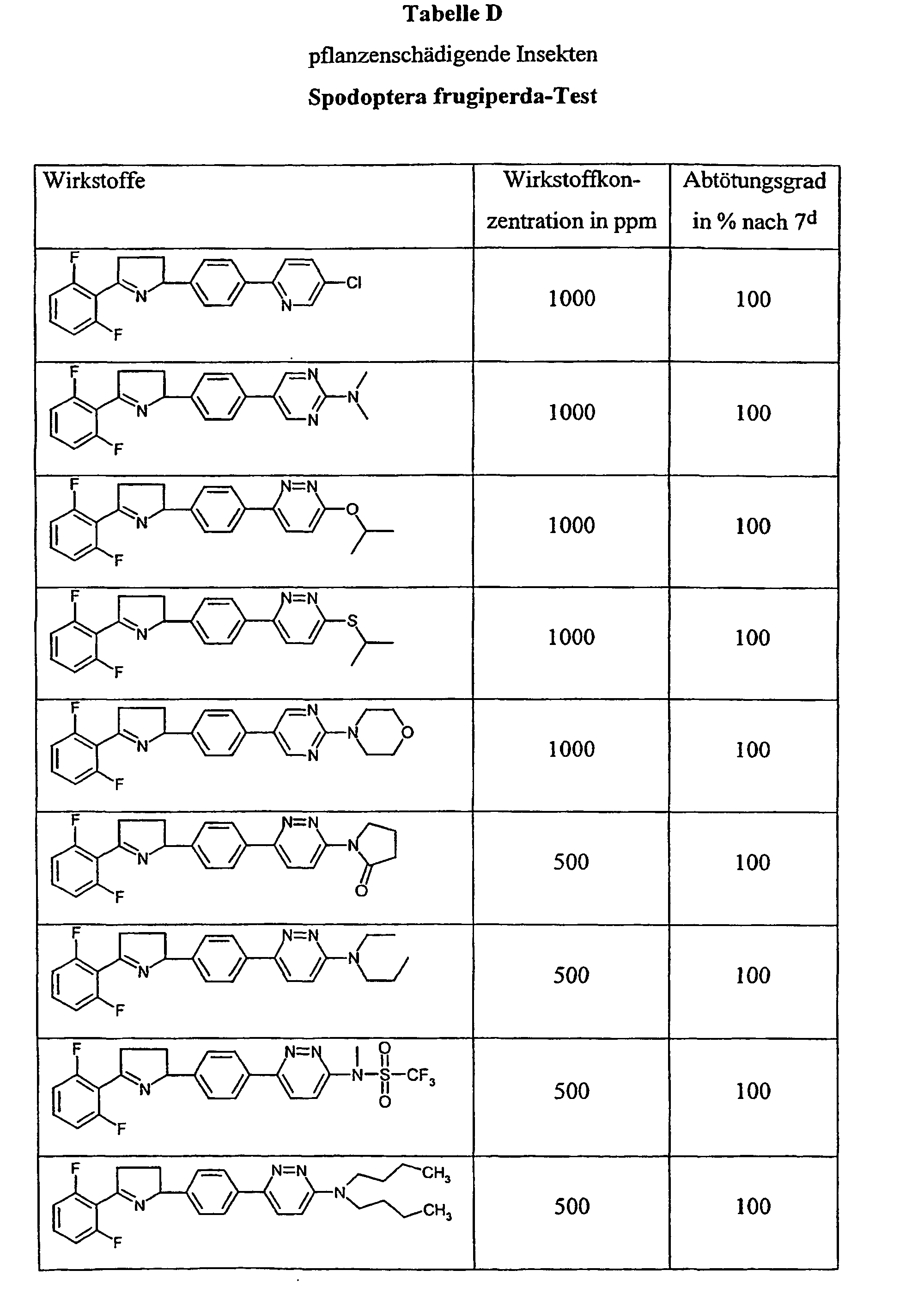

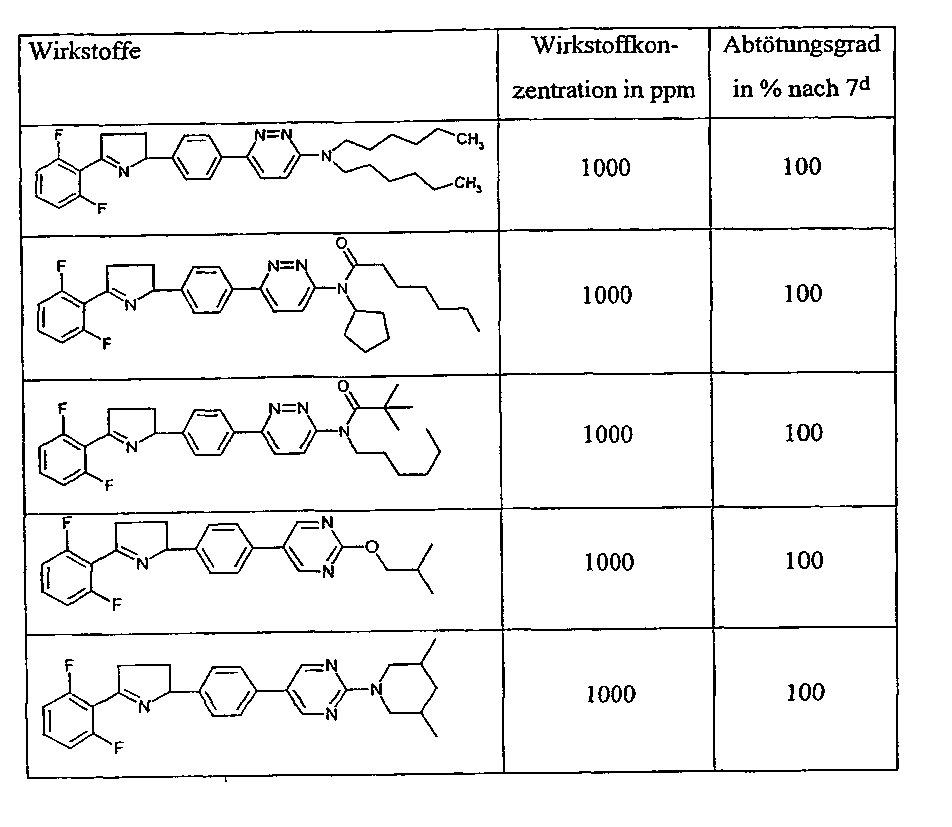

### Beispiel E

### Tetranychus-Test (OP-resistent/Tauchbehandlung)

| | |
|---|---|
| Lösungsmittel | 30 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100%, dass alle Spinnmilben abgetötet wurden; 0% bedeutet, dass keine Spinnmilben abgetötet wurden.

Wirkstoffe, Wirkstoffkonzentrationen und Versuchsergebnisse gehen aus der folgenden Tabelle hervor.

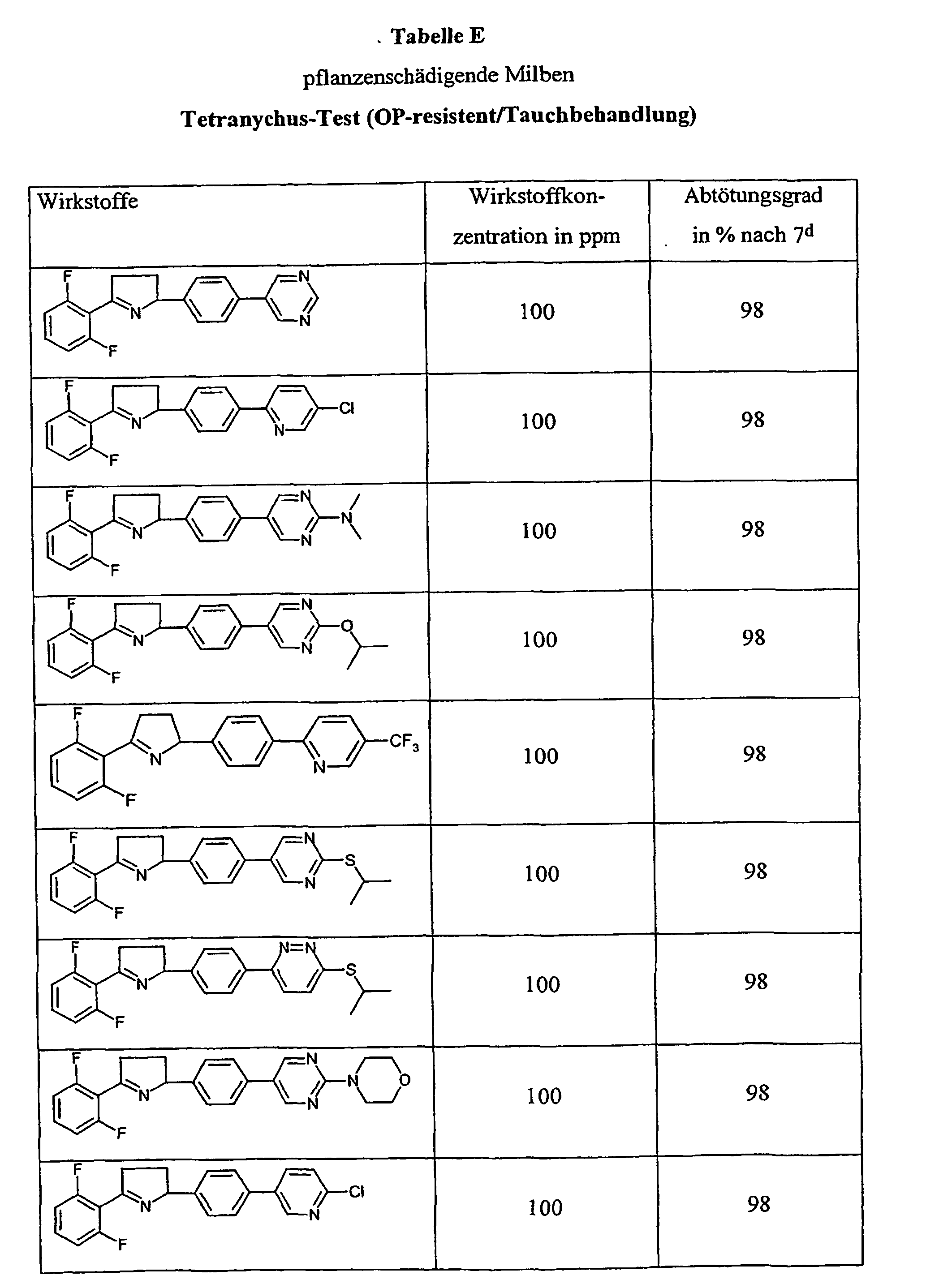

### Beispiel F

### Diabrotica balteata - Test (Larven im Boden)

Grenzkonzentrations-Test / Bodeninsekten - Behandlung transgener Pflanzen

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 1 Töpfe und lässt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20% Wirkung).

### Beispiel G

### Heliothis virescens - Test (Behandlung transgener Pflanzen)

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

## Patentansprüche

1. Δ¹-Pyrroline der Formel (I) 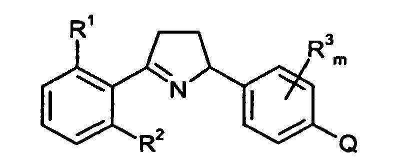 in welcher
R¹ für Halogen oder Methyl steht,
R² für Wasserstoff oder Halogen steht,
R³ für Halogen, für jeweils gegebenenfalls durch Halogen substituiertes Alkyl, Alkoxy oder Alkylthio steht,
m für 0, 1, 2, 3 oder 4 steht,
Q für eine der folgenden Gruppierungen 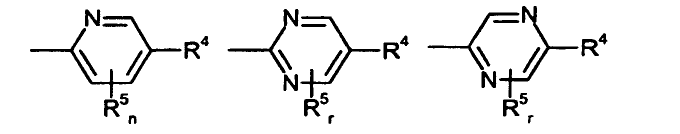 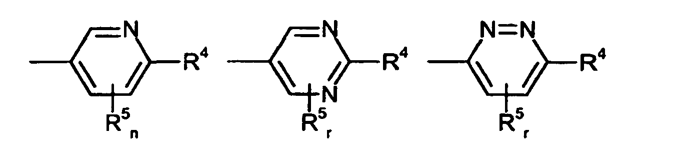 steht,
R⁴ für Wasserstoff, Halogen, Cyano, Formyl, Nitro, Trialkylsilyl; für jeweils gegebenenfalls einfach oder mehrfach, gleich oder verschieden durch Halogen, Cyano und/oder -NR⁷R⁸ substituiertes Alkyl, Alkenyl, Alkoxy, Alkenyloxy; für Pentafluorthio, -S(O)ₚR⁶, -NR⁷R⁸, -COR⁶, -CO₂R⁶, -CONR⁹R¹⁰, -N(R¹¹)COR¹² oder -C(R¹³)=N-OR¹⁴; oder für jeweils gegebenenfalls einfach oder mehrfach, gleich oder verschieden durch Halogen, Cyano, Nitro, Alkyl, Halogenalkyl, Alkenyl, Halogenalkenyl, Alkoxy, Halogenalkoxy, Alkylthio und/oder Halogenalkylthio substituiertes Cycloalkyl, Cycloalkylalkyl, Aryl, Arylalkyl, gesättigtes oder ungesättigtes, 5- bis 10-gliedriges Heterocyclyl oder Heterocyclylalkyl steht,
R⁴ außerdem für -CH=NOH, Formyl; für jeweils gegebenenfalls einfach oder mehrfach, gleich oder verschieden durch Halogen und/oder Alkyl substituiertes Cycloalkyloxy oder Cycloalkylalkoxy steht,
R⁵ für Halogen, Cyano, Formyl, Nitro, Trialkylsilyl; für jeweils gegebenenfalls einfach oder mehrfach, gleich oder verschieden durch Halogen, Cyano und/oder -NR⁷R⁸ substituiertes Alkyl, Alkenyl, Alkoxy, Alkenyloxy; für Pentafluorthio, -S(O)ₚR⁶, -NR⁷R⁸, -COR⁶, -CO₂R⁶, -CONR⁹R¹⁰, N(R¹¹)COR¹² oder -C(R¹³)=N-OR¹⁴; oder für jeweils gegebenenfalls einfach oder mehrfach, gleich oder verschieden durch Halogen, Alkyl, Halogenalkyl, Alkenyl, Halogenalkenyl, Alkoxy, Halogenalkoxy, Alkylthio und/oder Halogenalkylthio substituiertes Cycloalkyl, Cycloalkylalkyl, Aryl, Arylalkyl, gesättigtes oder ungesättigtes, 5- bis 10-gliedriges Heterocyclyl oder Heterocyclylalkyl steht,
p für 0, 1 oder 2 steht,
n für 0, 1, 2 oder 3 steht, wobei Substituenten R⁵ gleich oder verschieden sein können, wenn n für 2 oder 3 steht,
r für 0, 1 oder 2 steht, wobei Substituenten R⁵ gleich oder verschieden sein können, wenn r für 2 steht,
R⁶ für gegebenenfalls einfach oder mehrfach, gleich oder verschieden durch Halogen und/oder -NR⁷R⁸ substituiertes Alkyl, für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Aryl oder Arylalkyl steht,
R⁶ außerdem für einfach oder mehrfach, gleich oder verschieden substituiertes Alkyl steht, wobei die Substituenten zusätzlich aus Alkoxy, Alkylthio, Halogenalkoxy und/oder Halogenalkylthio ausgewählt sein können,
R⁷ und R⁸ unabhängig voneinander für Wasserstoff, -SO₂R⁶, -COR⁶, -CO₂R⁶, für jeweils gegebenenfalls einfach oder mehrfach, gleich oder verschieden durch Halogen, Alkylcarbonyl, Alkylcarbonyloxy, Alkylamino, Dialkylamino, Alkoxy, Halogenalkoxy, Alkylthio und/oder Halogenalkylthio substituiertes Alkyl oder Alkenyl; für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Cycloalkylalkyl, Aryl, Arylalkyl, gesättigtes oder ungesättigtes, 5- bis 10-gliedriges Heterocyclyl oder Heterocyclylalkyl stehen,
R⁷ und R⁸ außerdem gemeinsam für gegebenenfalls einfach oder mehrfach, gleich oder verschieden durch Halogen, Cyano, Alkoxy, Halogenalkoxy, Alkylthio und/oder Halogenalkylthio substituiertes Alkenylen oder für gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Alkylen, wobei die Alkylenkette jeweils durch -O-, -S- oder -NR¹⁵- unterbrochen sein kann, stehen,
R⁷ und R⁸ außerdem gemeinsam für einfach oder mehrfach, gleich oder verschieden substituiertes Alkylen, wobei die Substituenten zusätzlich aus Alkoxycarbonyl und/oder Oxyalkylenoxy ausgewählt sein können, stehen,
R⁷ und R⁸ außerdem gemeinsam für Alkylen, wobei die Alkylenkette entweder durch C=o oder C=NO-Alkyl unterbrochen ist, stehen,
R⁹ und R¹⁰ unabhängig voneinander für Wasserstoff, -SO₂R⁶, für jeweils gegebenenfalls einfach oder mehrfach, gleich oder verschieden durch Halogen, Alkylamino, Dialkylamino, Alkoxy und/oder Alkylthio substituiertes Alkyl oder Alkenyl; für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Cycloalkylalkyl, Aryl, Arylalkyl, gesättigtes oder ungesättigtes, 5- bis 10-gliedriges Heterocyclyl oder Heterocyclylalkyl stehen,
R⁹ und R¹⁰ außerdem gemeinsam für gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Alkylen, wobei die Alkylenkette jeweils durch -O-, -S- oder -NR¹⁵- unterbrochen sein kann, stehen,
R¹¹ und R¹² unabhängig voneinander für Wasserstoff, für gegebenenfalls einfach oder mehrfach, gleich oder verschieden durch Halogen, Cyano, Alkoxy und/oder Alkylthio substituiertes Alkyl, für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Cycloalkylalkyl, Aryl oder Arylalkyl stehen,
R¹¹ und R¹² außerdem gemeinsam für jeweils gegebenenfalls einfach oder mehrfach, gleich oder verschieden durch Halogen, Alkyl, Halogenalkyl, Alkoxy, Halogenalkoxy, Alkylthio und/oder Halogenalkylthio substituiertes Alkylen oder Alkenylen stehen,
R¹³ und R¹⁴ unabhängig voneinander für Wasserstoff, für jeweils gegebenenfalls einfach oder mehrfach durch Halogen substituiertes Alkyl oder Alkenyl stehen,
R¹⁵ für Wasserstoff, -SO₂R⁶, -COR⁶ oder -CO₂R⁶; für jeweils gegebenenfalls einfach oder mehrfach, gleich oder verschieden durch Halogen, Cyano, Alkylamino, Dialkylamino, Alkoxy, Halogenalkoxy, Alkylthio und/oder Halogenalkylthio substituiertes Alkyl oder Alkenyl; für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Cycloalkylalkyl, Aryl, Arylalkyl, gesättigtes oder ungesättigtes, 5- bis 10-gliedriges Heterocyclyl oder Heterocyclylalkyl steht.

2. Δ¹-Pyrroline der Formel (I) gemäß Anspruch 1, in welcher
R⁴ für Wasserstoff, Halogen, Cyano, Formyl, Nitro, Trialkylsilyl; für jeweils gegebenenfalls einfach oder mehrfach, gleich oder verschieden durch Halogen, Cyano und/oder -NR⁷R⁸ substituiertes Alkyl, Alkenyl, Alkoxy, Alkenyloxy; für Pentafluorthio, -S(O)ₚR⁶, -NR⁷R⁸, -COR⁶, -CO₂R⁶, -CONR⁹R¹⁰, -N(R¹¹)COR¹² oder -C(R¹³)=N-OR¹⁴; oder für jeweils gegebenenfalls einfach oder mehrfach, gleich oder verschieden durch Halogen, Cyano, Nitro, Alkyl, Halogenalkyl, Alkenyl, Halogenalkenyl, Alkoxy, Halogenalkoxy, Alkylthio und/oder Halogenalkylthio substituiertes Cycloalkyl, Cycloalkylalkyl, Aryl, Arylalkyl, gesättigtes oder ungesättigtes, 5- bis 10-gliedriges Heterocyclyl oder Heterocyclylalkyl steht,
R⁶ für gegebenenfalls einfach oder mehrfach, gleich oder verschieden durch Halogen und/oder -NR⁷R⁸ substituiertes Alkyl, für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Aryl oder Arylalkyl stehen,
R⁷ und R⁸ unabhängig voneinander für Wasserstoff, -SO₂R⁶, -COR⁶, -CO₂R⁶, für jeweils gegebenenfalls einfach oder mehrfach, gleich oder verschieden durch Halogen, Alkylcarbonyl, Alkylcarbonyloxy, Alkylamino, Dialkylamino, Alkoxy, Halogenalkoxy, Alkylthio und/oder Halogenalkylthio substituiertes Alkyl oder Alkenyl; für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Cycloalkylalkyl, Aryl, Arylalkyl, gesättigtes oder ungesättigtes, 5- bis 10-gliedriges Heterocyclyl oder Heterocyclylalkyl stehen,
R⁷ und R⁸ außerdem gemeinsam für gegebenenfalls einfach oder mehrfach, gleich oder verschieden durch Halogen, Cyano, Alkoxy, Halogenalkoxy, Alkylthio und/oder Halogenalkylthio substituiertes Alkenylen oder für gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Alkylen, wobei die Alkylenkette jeweils durch -O-, -S- oder -NR¹⁵- unterbrochen sein kann, stehen
und R¹, R², R³, m, Q, R⁵, p, n, r, R⁹, R¹⁰, R¹¹, R¹², R¹³, R¹⁴ und R¹⁵ die in Anspruch 1 angegebenen Bedeutungen haben.

3. Δ¹-Pyrroline der Formel (I) gemäß Anspruch 1, in welcher
R¹ für Fluor, Chlor, Brom oder Methyl steht,
R² für Wasserstoff, Fluor, Chlor oder Brom steht,
R³ für Fluor, Chlor, Brom, für jeweils gegebenenfalls einfach bis achtfach durch Halogen substituiertes C₁-C₆-Alkyl, C₁-C₆-Alkoxy oder C₁-C₆-Alkylthio steht,
m für 0, 1, 2 oder 3 steht,
Q für eine der folgenden Gruppierungen 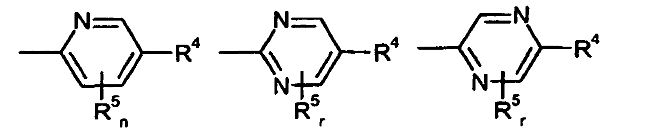 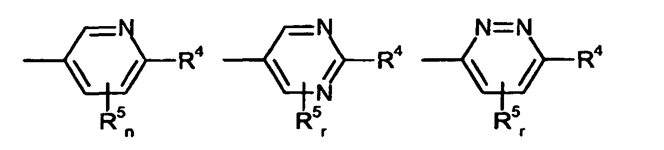 steht,
R⁴ für Wasserstoff, Fluor, Chlor, Brom, Cyano, Formyl, Nitro, Tri-(C₁-C₆-alkyl)silyl; für jeweils gegebenenfalls einfach oder mehrfach, gleich oder verschieden durch Halogen, Cyano, -NR⁷R⁸ substituiertes C₁-C₂₀-Alkyl, C₂-C₂₀-Alkenyl, C₁-C₂₀-Alkoxy, C₂-C₂₀-Alkenyloxy; für Pentafluorthio, -S(O)ₚR⁶, -NR⁷R⁸, -COR⁶, -CO₂R⁶, -CONR⁹R¹⁰, -N(R¹¹)COR¹² oder -C(R¹³)=N-OR¹⁴; oder für jeweils gegebenenfalls einfach bis vierfach, gleich oder verschieden, durch Halogen, Cyano, Nitro, C₁-C₆-Alkyl, C₁-C₆-Halogenalkyl, C₂-C₆-Alkenyl, C₂-C₆-Halogenalkenyl, C₁-C₆-Alkoxy, C₁-C₆-Halogenalkoxy, C₁-C₆-Alkylthio, C₁-C₆-Halogenalkylthio substituiertes C₃-C₁₂-Cycloalkyl, C₃-C₇-Cycloalkyl-C₁-C₄-alkyl, Aryl, Aryl-C₁-C₄-alkyl, gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidino, Pyrrolidinyl, Oxazolyl, Benzoxazolyl, Isoacazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Piperidino, Morpholinyl, Thiomorpholinyl, Morpholino, Thiomorpholino, Triazinyl, Triazolyl, Chinolinyl oder Isochinolinyl) steht,
R⁴ außerdem für -CH=NOH, Formyl; für jeweils gegebenenfalls einfach bis vierfach, gleich oder verschieden durch Fluor, Chlor, Brom und/oder C₁-C₂-Alkyl substituiertes C₃-C₆-Cycloalkyloxy oder C₃-C₆-Cycloalkyl-C₁-C₄-alkoxy steht,
R⁵ für Fluor, Chlor, Brom, Cyano, Formyl, Nitro, Tri-(C₁-C₆-alkyl)silyl; für jeweils gegebenenfalls einfach bis dreizehnfach durch Halogen substituiertes C₁-C₆-Alkyl, C₂-C₆-Alkenyl, C₁-C₆-Alkoxy, C₂-C₆-Alkenyloxy; für Pentafluorthio, -S(O)ₚR⁶, -NR⁷R⁸, -COR⁶, -CO₂R⁶, -CONR⁹R¹⁰ oder -N(R¹¹)COR¹² steht,
p für 0, 1 oder 2 steht,
n für 0, 1, 2 oder 3 steht, wobei Substituenten R⁵ gleich oder verschieden sein können, wenn n für 2 oder 3 steht,
r für 0, 1 oder 2 steht, wobei Substituenten R⁵ gleich oder verschieden sein können, wenn r für 2 steht,
R⁶ für gegebenenfalls einfach oder mehrfach, gleich oder verschieden durch Halogen oder -NR⁷R⁸ substituiertes C₁-C₂₀-Alkyl, für jeweils gegebenenfalls einfach bis achtfach, gleich oder verschieden durch Halogen, Cyano, C₁-C₆Alkyl, C₁-C₆Halogen-alkyl, C₁-C₆-Alkoxy, C₁-C₆-Halogenalkoxy, C₁-C₆-Alkylthio, C₁-C₆-Halogenalkylthio substituiertes C₃-C₆-Cycloalkyl, Aryl oder Aryl-C₁-C₄-alkyl steht,
R⁶ außerdem für einfach oder mehrfach, gleich oder verschieden substituiertes C₁-C₂₀-Alkyl steht, wobei die Substituenten zusätzlich aus C₁-C₆-Alkoxy, C₁-C₆-Alkylthio, C₁-C₆-Halogenalkoxy und/oder C₁-C₆-Halogenalkylthio ausgewählt sein können,
R⁷ und R⁸ unabhängig voneinander für Wasserstoff, -SO₂R⁶, -COR⁶, -CO₂R⁶, für jeweils gegebenenfalls einfach oder mehrfach, gleich oder verschieden durch Halogen, C₁-C₆-Alkylcarbonyl, C₁-C₆-Alkylcarbonyloxy, C₁-C₆-Alkylamino, Di-(C₁-C₆-alkyl)amino, C₁-C₆-Alkoxy, C₁-C₆-Halogenalkoxy, C₁-C₆-Alkylthio, C₁-C₆-Halogenalkylthio substituiertes C₁-C₂₀-Alkyl oder C₂-C₂₀-Alkenyl; für jeweils gegebenenfalls einfach bis vierfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₁₂-Cycloalkyl, C₃-C₇-Cycloalkyl-C₁-C₄-alkyl, Aryl, Aryl-C₁-C₄-alkyl, gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl) stehen,
R⁷ und R⁸ außerdem unabhängig voneinander für jeweils gegebenenfalls einfach bis vierfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Pyridinylmethyl, Pyridinylethyl, Pyrimidinylmethyl, Pyrimidinylethyl, Pyridazylmethyl, Pyridazylethyl, Pyrazinylmethyl oder Pyrazinylethyl) stehen,
R⁷ und R⁸ außerdem gemeinsam für gegebenenfalls einfach oder mehrfach, gleich oder verschieden durch Halogen, Cyano, C₁-C₆-Alkoxy, C₁-C₆-Halogenalkoxy, C₁-C₆-Alkylthio, C₁-C₆-Halogenalkylthio substituiertes C₂-C₁₂-Alkenylen oder für gegebenenfalls im Alkylenteil einfach oder mehrfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₁₂-Alkylen, wobei die Alkylenkette jeweils durch -O-, -S- oder -NR¹⁵- unterbrochen sein kann, stehen,
R⁷ und R⁸ außerdem gemeinsam für gegebenenfalls einfach oder mehrfach, gleich oder verschieden substituiertes C₃-C₁₂-Alkylen stehen, wobei die Substituenten zusätzlich aus C₁-C₄-Alkoxycarbonyl und/oder Oxy-(C₁-C4-alkylen)-oxy ausgewählt sein können,
R⁷ und R⁸ außerdem gemeinsam für C₃-C₈-Alkylen stehen, wobei die Alkylenkette entweder durch C=O oder C=NO-(C₁-C₆-Alkyl) unterbrochen ist,
R⁹ und R¹⁰ unabhängig voneinander für Wasserstoff, -SO₂R⁶, für jeweils gegebenenfalls einfach bis dreizehnfach, gleich oder verschieden durch Halogen, C₁-C₆-Alkylamino, Di-(C₁-C₆-alkyl)amino, C₁-C₆-Alkoxy, C₁-C₆-Halogenalkoxy, C₁-C₆-Alkylthio, C₁-C₆-Halogenalkylthio substituiertes C₁-C₆-Alkyl oder C₂-C₆-Alkenyl; für jeweils gegebenenfalls einfach bis vierfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₇-Cycloalkyl, C₃-C₇-Cycloalkyl-C₁-C₄-alkyl, Aryl, Aryl-C₁-C₄-alkyl, gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl) stehen,
R⁹ und R¹⁰ außerdem gemeinsam für jeweils gegebenenfalls im Alkylenteil einfach bis vierfach, gleich oder verschieden durch Halogen, Cyano, C₁-C₆-Alkyl, C₁-C6-Halogenalkyl, C₁-C₆-Alkoxy, C₁-C₆-Halogenalkoxy, C₁-C₆-Alkylthio, C₁-C₆-Halogenalkylthio substituiertes C₃-C₆-Alkylen, -(CH₂)₂-O-(CH₂)₂-, -(CH₂)₂-S-(CH₂)₂- oder -(CH₂)₂-N(R¹⁵)-(CH₂)₂- stehen,
R¹¹ und R¹² unabhängig voneinander für Wasserstoff, für gegebenenfalls einfach bis dreizehnfach, gleich oder verschieden durch Halogen, Cyano, C₁-C₆-Alkoxy, C₁-C₆-Alkylthio substituiertes C₁-C₆-Alkyl, für jeweils gegebenenfalls einfach bis achtfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₇-Cycloalkyl, C₃-C₇-Cycloalkyl-C₁-C₄-alkyl, Aryl oder Aryl-C₁-C₄-alkyl stehen,
R¹¹ und R¹² außerdem gemeinsam für jeweils gegebenenfalls einfach bis achtfach, gleich oder verschieden, durch Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₁₀-Alkylen oder C₃-C₁₀-Alkenylen stehen,
R¹³ und R¹⁴ unabhängig voneinander für Wasserstoff, für jeweils gegebenenfalls einfach oder mehrfach durch Halogen substituiertes C₁-C₆-Alkyl oder C₂-C₆-Alkenyl stehen,
R¹⁵ für Wasserstoff, -SO₂R⁶, -COR⁶ oder -CO₂R⁶; für jeweils gegebenenfalls einfach oder mehrfach, gleich oder verschieden durch Halogen, Cyano, C₁-C₆-Alkylamino, Di-(C₁-C₆-alkyl)amino, C₁-C₆-Alkoxy, C₁-C₆-Halogenalkoxy, C₁-C₆-Alkylthio, C₁-C₆-Halogenalkylthio substituiertes C₁-C₂₀-Alkyl oder C₂-C₂₀-Alkenyl; für jeweils gegebenenfalls einfach bis vierfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₁₂-Cycloalkyl, C₃-C₇-Cycloalkyl-C₁-C₄-alkyl, Aryl, Aryl-C₁-C₄-alkyl, gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl) steht.

4. Δ¹-Pyrroline der Formel (I) gemäß Anspruch 1, in welcher
R⁴ für Wasserstoff, Fluor, Chlor, Brom, Cyano, Formyl, Nitro, Tri-(C₁-C₆-alkyl)silyl; für jeweils gegebenenfalls einfach oder mehrfach, gleich oder verschieden durch Halogen, Cyano, -NR⁷R⁸ substituiertes C₁-C₂₀-Alkyl, C₂-C₂₀-Alkenyl, C₁-C₂₀-Alkoxy, C₂-C₂₀-Alkenyloxy; für Pentafluorthio, -S(O)ₚR⁶, -NR⁷R⁸, -COR⁶, -CO₂R⁶, -CONR⁹R¹⁰, -N(R¹¹)COR¹² oder -C(R¹³)=N-OR¹⁴; oder für jeweils gegebenenfalls einfach bis vierfach, gleich oder verschieden, durch Halogen, Cyano, Nitro, C₁-C₆-Alkyl, C₁-C₆-Halogenalkyl, C₂-C₆-Alkenyl, C₂-C₆-Halogenalkenyl, C₁-C₆-Alkoxy, C₁-C₆-Halogenalkoxy, C₁-C₆-Alkylthio, C₁-C₆-Halogenalkylthio substituiertes C₃-C₁₂-Cycloalkyl, C₃-C₇-Cyclo-C₁-C₄-alkyl, Aryl, Aryl-C₁-C₄-alkyl, gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidino, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Piperidino, Morpholinyl, Thiomorpholinyl, Morpholino, Thiomorpholino, Triazinyl, Triazolyl, Chinolinyl oder Isochinolinyl) steht,
R⁶ für gegebenenfalls einfach oder mehrfach, gleich oder verschieden durch Halogen oder -NR⁷R⁸ substituiertes C₁-C₂₀-Alkyl, für jeweils gegebenenfalls einfach bis achtfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C6-Halogenalkylthio substituiertes C₃-C₆-Cycloalkyl, Aryl oder Aryl-C₁-C₄-alkyl steht,
R⁷ und R⁸ unabhängig voneinander für Wasserstoff, -SO₂R⁶, -COR⁶, -CO₂R⁶, für jeweils gegebenenfalls einfach oder mehrfach, gleich oder verschieden durch Halogen, C₁-C₆-Alkylcarbonyl, C₁-C₆-Alkylcarbonyloxy, C₁-C₆-Alkylamino, Di-(C₁-C₆-alkyl)amino, C₁-C₆-Alkoxy, C₁-C₆-Halogenalkoxy, C₁-C₆-Alkylthio, C₁-C₆-Halogenalkylthio substituiertes C₁-C₂₀-Alkyl oder C₂-C₂₀-Alkenyl; für jeweils gegebenenfalls einfach bis vierfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₁₂-Cycloalkyl, C₃-C₇-Cycloalkyl-C₁-C₄-alkyl, Aryl, Aryl-C₁-C₄-alkyl, gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl) stehen,
R⁷ und R⁸ außerdem gemeinsam für gegebenenfalls einfach oder mehrfach, gleich oder verschieden durch Halogen, Cyano, C₁-C₆-Alkoxy, C₁-C₆-Halogenalkoxy, C₁-C₆-Alkylthio, C₁-C₆-Halogenalkylthio substituiertes C₂-C₁₂-Alkenylen oder für gegebenenfalls im Alkylenteil einfach oder mehrfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₁₂-Alkylen, wobei die Alkylenkette jeweils durch -O-, -S- oder -NR¹⁵- unterbrochen sein kann, stehen,
und R¹, R², R³, m, Q, R⁵, p, n, r, R⁹, R¹⁰, R¹¹, R¹², R¹³, R¹⁴ und R¹⁵ die in Anspruch 3 angegebenen Beutungen haben.

5. Δ¹-Pyrroline der Formel (I) gemäß Anspruch 1, in welcher
R¹ für Fluor, Chlor oder Methyl steht,
R² für Wasserstoff, Fluor oder Chlor steht,
R³ für Fluor, Chlor, C₁-C₄-Alkyl, C₁-C₄-Alkoxy, C₁-C₄-Alkylthio; C₁-C₄-Halogenalkyl, C₁-C₄-Halogenalkoxy, C₁-C₄-Halogenalkylthio mit jeweils 1 bis 9 Fluor, Chlor und/oder Bromatomen steht,
m für 0, 1 oder 2 steht,
Q für eine der folgenden Gruppierungen 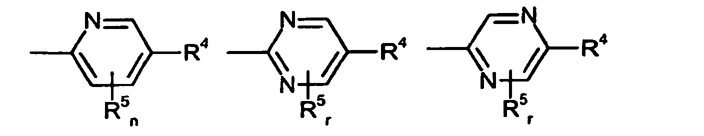 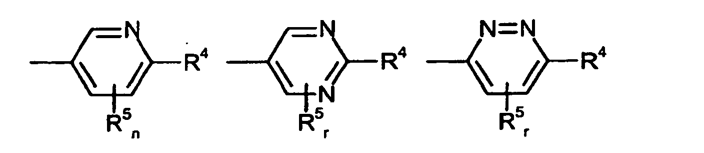 steht,
R⁴ für Wasserstoff, Fluor, Chlor, Brom, Cyano, Formyl, Nitro, Tri-(C₁-C₄-alkyl)silyl; für jeweils gegebenenfalls einfach oder mehrfach, gleich oder verschieden durch Fluor, Chlor, Brom, Cyano, -NR⁷R⁸ substituiertes C₁-C₁₆-Alkyl, C₂-C₁₆-Alkenyl, C₁-C₁₆-Alkoxy, C₂-C₁₆-Alkenyloxy; für -S(O)ₚR⁶, -NR⁷R⁸, -COR⁶, -CO₂R⁶, -CONR⁹R¹⁰ oder -N(R¹¹)COR¹²; oder für jeweils gegebenenfalls einfach bis dreifach, gleich oder verschieden, durch Fluor, Chlor, Brom, Cyano, C₁-C₄-Alkyl, C₁-C₄-Halogenalkyl, C₂-C₆-Alkenyl, C₂-C₆-Halogenalkenyl, C₁-C₄-Alkoxy, C₁-C₄-Halogenalkoxy, C₁-C₄-Alkylthio, C₁-C₄-Halogenalkylthio substituiertes C₃-C₁₀-Cycloalkyl, C₃-C₆-Cycloalkyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidino, Pyrrolidinyl, Oxazolyl, Benzoxazolyl, Isoxazolyl, Imidazolyl, Pyazolyl, Thiazolyl, Benzothiazolyl, Thiazolidinyl, Pyridinyl, Pyrimidinyl, Pyridazyl, Pyrazinyl, Piperidinyl, Piperidino, Morpholinyl, Thiomorpholinyl, Morpholino, Thiomorpholino, Triazinyl, Triazolyl, Chinolinyl oder Isochinolinyl steht,
R⁴ außerdem für -CH=NOH, Formyl; für jeweils gegebenenfalls einfach bis dreifach, gleich oder verschieden durch Fluor, Chlor und/oder Methyl substituiertes Cyclopropyloxy, Cyclobutyloxy, Cyclopentyloxy, Cyclohexyloxy, Cyclopropylmethoxy, Cyclobutylmethoxy, Cyclopentylmethoxy oder Cyclohexylmethoxy steht,
R⁵ für Fluor, Chlor, Trimethylsilyl, für jeweils gegebenenfalls einfach bis neunfach, gleich oder verschieden durch Fluor, Chlor und/oder Brom substituiertes C₁-C₄-Alkyl, C₂-C₆-Alkenyl, C₁-C₄-Alkoxy, C₂-C₆-Alkenyloxy, -S(O)ₚR⁶, -NR⁷R⁸, -COR⁶, -CO₂R⁶ oder -CONR⁹R¹⁰ steht,
p für 0, 1 oder 2 steht,
n für 0, 1 oder 2 steht, wobei Substituenten R⁵ gleich oder verschieden sein können, wenn n für 2 steht,
r für 0, 1 oder 2 steht, wobei Substituenten R⁵ gleich oder verschieden sein können, wenn r für 2 steht,
R⁶ für gegebenenfalls einfach oder mehrfach, gleich oder verschieden durch Fluor, Chlor, Brom und/oder -NR⁷R⁸ substituiertes C₁-C₁₀-Alkyl, für jeweils gegebenenfalls einfach bis vierfach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes Cyclopropyl, Cyclopentyl, Cyclohexyl, Phenyl oder Benzyl steht,
R⁶ außerdem für einfach bis dreifach, gleich oder verschieden substituiertes C₁-C₁₀-Alkyl steht, wobei die Substituenten zusätzlich aus C₁-C₄-Alkoxy, C₁-C₄-Alkylthio, C₁-C₄-Halogenalkoxy und/oder C₁-C₄-Halogenalkylthio ausgewählt sein können,
R⁷ und R⁸ unabhängig voneinander für Wasserstoff, -SO₂R⁶, -COR⁶, -CO₂R⁶, für jeweils gegebenenfalls einfach oder mehrfach, gleich oder verschieden, durch Fluor, Chlor, Brom, C₁-C₄-Alkylcarbonyl, C₁-C₄-Alkylcarbonyloxy, C₁-C₄-Alkylamino, Di-(C₁-C₄-alkyl)amino, C₁-C₄-Alkoxy, C₁-C₄-Halogenalkoxy, C₁-C₄-Alkylthio und/oder C₁-C₄-Halogenalkylthio substituiertes C₁-C₁₆-Alkyl oder C₂-C₁₆-Alkenyl; für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes C₃-C₁₀-Cycloalkyl, C₃-C₆-Cycloalkyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl stehen,
R⁷ und R⁸ außerdem unabhängig voneinander für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes Pyridinylmethyl, Pyridinylethyl, Pyrimidinylmethyl, Pyrimidinylethyl, Pyridazylmethyl, Pyridazylethyl, Pyrazinylmethyl oder Pyrazinylethyl stehen,
R⁷ und R⁸ außerdem gemeinsam für gegebenenfalls einfach oder mehrfach, gleich oder verschieden durch Fluor, Chlor, Brom, C₁-C₄-Alkoxy, C₁-C₄-Halogenalkoxy, C₁-C₄-Alkylthio und/oder C₁-C₄-Halogenalkylthio substituiertes C₂-C₁₀-Alkenylen oder für gegebenenfalls im Alkylenteil einfach oder mehrfach, gleich oder verschieden durch Fluor, Chlor, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₁₀-Alkylen, wobei die Alkylenkette jeweils durch -O-, -S- oder -NR¹⁵- unterbrochen sein kann, stehen,
R⁷ und R⁸ außerdem gemeinsam für einfach bis dreifach, gleich oder verschieden substituiertes C₃-C₁₀-Alkylen stehen, wobei die Substituenten zusätzlich aus n-Propoxycarbonyl, i-Propoxycarbonyl, Ethoxycarbonyl, Methoxycarbonyl, Oxypropylenoxy, Oxyethylenoxy und/oder Oxymethylenoxy ausgewählt sein können,
R⁷ und R⁸ außerdem gemeinsam für C₃-C₆-Alkylen stehen, wobei die Alkylenkette entweder durch C=O oder C=NO-(C₁-C₄-Alkyl) unterbrochen ist,
R⁹ und R¹⁰ unabhängig voneinander für Wasserstoff, -SO₂R⁶, für jeweils gegebenenfalls einfach bis neunfach, gleich oder verschieden durch Fluor, Chlor, Brom, C₁-C₄-Alkylamino, Di-(C₁-C₄-alkyl)amino, C₁-C₄-Alkoxy, C₁-C₄-Halogenalkoxy, C₁-C₄-Alkylthio und/oder C₁-C₄-Halogenalkylthio substituiertes C₁-C₄-Alkyl oder C₂-C₆-Alkenyl; für jeweils gegebenenfalls einfach bis dreifach, gleich oder verschieden durch Fluor, Chlor, Brom, C₁-C₄-Alkyl, Cyano, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes C₃-C₆-Cycloalkyl, C₃-C₆-Cycloalkyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl stehen,
R⁹ und R¹⁰ außerdem gemeinsam für jeweils gegebenenfalls im Alkylenteil einfach bis vierfach, gleich oder verschieden durch Fluor, Chlor, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₄-C₅-Alkylen, -(CH₂)₂-O-(CH₂)₂-, -(CH₂)₂-S-(CH₂)₂-, -(CH₂)₂-N(R¹⁵)-(CH₂)₂- stehen,
R¹¹ und R¹² unabhängig voneinander für Wasserstoff, für gegebenenfalls einfach bis neunfach, gleich oder verschieden durch Fluor, Chlor, Brom, C₁-C₄-Alkoxy, C₁-C₄-Alkylthio, substituiertes C₁-C₆-Alkyl, für gegebenenfalls einfach bis vierfach, gleich oder verschieden durch Fluor, Chlor, Brom, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₆-Cycloalkyl, C₃-C₆-Cycloalkyl-C₁-C₄-alkyl, Phenyl, Benzyl oder Phenylethyl stehen,
R¹¹ und R¹² außerdem gemeinsam für jeweils gegebenenfalls einfach bis vierfach, gleich oder verschieden durch Fluor, Chlor, Brom, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₈-Alkylen oder C₃-C₈-Alkenylen stehen,
R¹⁵ für Wasserstoff, -SO₂R⁶, für -COR⁶ oder -CO₂R⁶; für gegebenenfalls einfach oder mehrfach, gleich oder verschieden durch Fluor, Chlor, Brom, Cyano, Methylamino, Ethylamino, Di-(C₁-C₆-alkyl)amino, C₁-C₄-Alkoxy, C₁-C₄-Halogenalkoxy, C₁-C₄-Alkylthio, C₁-C₄-Halogenalkylthio substituiertes C₁-C₁₆-Alkyl oder C₂-C₁₆-Alkenyl; für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₁₀-Cycloalkyl, C₃-C₆-Cycloalkyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl steht.

6. Δ¹-Pyrroline der Formel (I) gemäß Anspruch 1, in welcher
R⁴ für Wasserstoff, Fluor, Chlor, Brom, Cyano, Formyl, Nitro, Tri-(C₁-C₄-alkyl)silyl; für jeweils gegebenenfalls einfach oder mehrfach, gleich oder verschieden durch Fluor, Chlor, Brom, Cyano, -NR⁷R⁸ substituiertes C₁-C₁₆-Alkyl, C₂-C₁₆-Alkenyl, C₁-C₁₆-Alkoxy, C₂-C₁₆-Alkenyloxy; für -S(O)ₚR⁶, -NR⁷R⁸, -COR⁶, -CO₂R⁶, -CONR⁹R¹⁰ oder -N(R¹¹)COR¹²; oder für jeweils gegebenenfalls einfach bis dreifach, gleich oder verschieden, durch Fluor, Chlor, Brom, Cyano, C₁-C₄-Alkyl, C₁-C₄-Halogenalkyl, C₂-C₆-Allcenyl, C₂-C₆-Halogenalkenyl, C₁-C₄-Alkoxy, C₁-C₄-Halogenalkoxy, C₁-C₄-Alkylthio, C₁-C₄-Halogenalkylthio substituiertes C₃-C₁₀-Cycloalkyl, C₃-C₆-Cycloalkyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidino, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Piperidino, Morpholinyl, Thiomorpholinyl, Morpholino, Thiomorpholino, Triazinyl, Triazolyl, Chinolinyl oder Isochinolinyl steht,
R⁶ für gegebenenfalls einfach oder mehrfach, gleich oder verschieden durch Fluor, Chlor, Brom und/oder -NR⁷R⁸ substituiertes C₁-C₁₀-Alkyl, für jeweils gegebenenfalls einfach bis vierfach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes Cyclopropyl, Cyclopentyl, Cyclohexyl, Phenyl oder Benzyl steht,
R⁷ und R⁸ unabhängig voneinander für Wasserstoff, -SO₂R⁶, -COR⁶, -CO₂R⁶, für jeweils gegebenenfalls einfach oder mehrfach, gleich oder verschieden, durch Fluor, Chlor, Brom, C₁-C₄-Alkylcarbonyl, C₁-C₄-Alkylcarbonyloxy, C₁-C₄-Alkylamino, Di-(C₁-C₄-alkyl)amino, C₁-C₄-Alkoxy, C₁-C₄-Halogenalkoxy, C₁-C₄-Alkylthio und/oder C₁-C₄-Halogenalkylthio substituiertes C₁-C₁₆-Alkyl oder C₂-C₁₆-Alkenyl; für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes C₃-C₁₀-Cycloalkyl, C₃-C₆-Cycloalkyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl stehen,
R⁷ und R⁸ außerdem gemeinsam für gegebenenfalls einfach oder mehrfach, gleich oder verschieden durch Fluor, Chlor, Brom, C₁-C₄-Alkoxy, C₁-C₄-Halogenalkoxy, C₁-C₄-Alkylthio und/oder C₁-C₄-Halogenalkylthio substituiertes C₂-C₁₀-Alkenylen oder für gegebenenfalls im Alkylenteil einfach oder mehrfach, gleich oder verschieden durch Fluor, Chlor, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₁₀-Alkylen, wobei die Alkylenkette jeweils durch -O-, -S- oder -NR¹⁵- unterbrochen sein kann, stehen
und R¹, R², R³, m, Q, R⁵, p, n, r, R⁹, R¹⁰, R¹¹, R¹², R¹³, R¹⁴ und R¹⁵ die in Anspruch 5 angegebenen Bedeutungen haben.

7. Δ¹-Pyrroline der Formel (I) gemäß Anspruch 1, in welcher
R¹ für Fluor oder Chlor steht,
R² für Wasserstoff oder Fluor steht,
R³ für Fluor, Chlor, Methyl, Ethyl, n-Propyl, i-Propyl, n-Butyl, i-Butyl, s-Butyl, t-Butyl, Trifluormethyl, Trifluorethyl, Methoxy, Ethoxy, n-Propoxy, i-Propoxy, n-Butoxy, i-Butoxy, s-Butoxy, t-Butoxy, Trifluormethoxy, Trifluorethoxy, Methylthio, Ethylthio, n-Propylthio, i-Propylthio, n-Butylthio, i-Butylthio, s-Butylthio, t-Butylthio, Trifluormethylthio oder Trifluorethylthio steht,
m für 0 oder 1 steht,
Q für eine der folgenden Gruppierungen 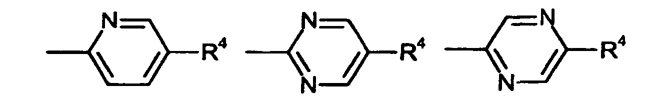 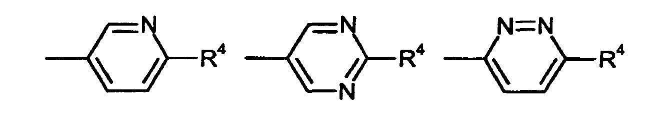 steht,
R⁴ für Wasserstoff, Fluor, Chlor, Brom, Cyano, Formyl, für jeweils gegebenenfalls einfach oder mehrfach, gleich oder verschieden durch Fluor, Chlor, Brom, Cyano, -NR⁷R⁸ substituiertes C₁-C₁₆-Alkyl, C₂-C₁₆-Alkenyl, C₁-C₁₆-Alkoxy, C₂-C₁₆-Alkenyloxy; für -S(O)ₚR⁶, -NR⁷R⁸, -COR⁶, -CO₂R⁶, -CONR⁹R¹⁰, -N(R¹¹)COR¹²; oder für jeweils gegebenenfalls einfach bis dreifach, gleich oder verschieden, durch Fluor, Chlor, Brom, Cyano, Methyl, Ethyl, n-Propyl, i-Propyl, -CF₃, -CEF₂, -CClF₂, -CF₂CHFCl, -CF₂CH₂F, -CF₂CCl₃, -CH₂CF₃, -CF₂CHFCF₃, -CH₂CF₂H, -CH₂CF₂CF₃, -CF₂CF₂H, -CF₂CEFCF₃, Vinyl, Allyl, 1-Propenyl, Butenyl, -CF=CHF, -CF=CH₂, -CF=CCl₂, -CH=CF₂, -CF₂CF=CF₂, -CH=CFH, -CH₂CF=CF₂, -CF=CF₂, -CF₂CH=CF₂, Methoxy, Ethoxy, n-Propoxy, i-Propoxy, n-Butoxy, i-Butoxy, s-Butoxy, t-Butoxy, Trifluormethoxy, Difluormethoxy, Chlordifluormethoxy, Trifluorethoxy, Methylthio, Ethylthio, n-Propylthio, i-Propylthio, n-Butylthio, i-Butylthio, s-Butylthio, t-Butylthio, Trifluormethylthio, Difluormethylthio, Chlordifluormethylthio, Trifluorethylthio substituiertes Cyclopropyl, Cyclopentyl, Cyclohexyl, Cyclopropylmethyl, Cyclopentylmethyl, Cyclohexylmethyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidino, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Piperidino, Morpholinyl, Thiomorpholinyl, Morpholino, Thiomorpholino, Triazinyl, Triazolyl, Chinolinyl oder Isochinolinyl steht,
R⁴ außerdem für -CH=NOH, Formyl, Cyclopropyloxy, Cyclobutyloxy, Cyclopentyloxy, Cyclohexyloxy, Cyclopropylmethoxy, Cyclobutylmethoxy, Cyclopentylmethoxy oder Cyclohexylmethoxy steht,
p für 0, 1 oder 2 steht,
R⁶ für Methyl, Ethyl, n-Propyl, i-Propyl, n-Butyl, i-Butyl, s-Butyl, t-Butyl, Pentyl, Hexyl, -CF₃, -CHF₂, -CCl₃, -CCl₂F, Dimethylaminomethyl, Dimethylaminoethyl, Diethylaminomethyl, Diethylaminoethyl, Cyclopropyl, Cyclopentyl, Cyclohexyl, Phenyl oder Benzyl steht,
R⁶ außerdem für Methoxymethyl, Trifluormethoxymethyl, Methylthiomethyl oder Trifluormethylthiomethyl steht,
R⁷ und R⁸ unabhängig voneinander für Wasserstoff, -SO₂R⁶, -COR⁶, -CO₂R⁶, für jeweils gegebenenfalls einfach oder mehrfach, gleich oder verschieden, durch Fluor, Chlor, Brom, C₁-C₄-Alkylcarbonyl, C₁-C₄-Alkylcarbonyloxy, C₁-C₄-Alkylamino, Di-(C₁-C₄-alkyl)-amino, C₁-C₄-Alkoxy, C₁-C₄-Halogenalkoxy, C₁-C₄-Alkylthio und/oder C₁-C₄-Halogenalkylthio substituiertes C₁-C₁₆-Alkyl, C₂-C₁₆-Alkenyl; für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes C₃-C₈-Cycloalkyl, Cyclopropylmethyl, Cyclopentylmethyl, Cyclohexylmethyl, Cyclopropylethyl, Cyclopentylethyl, Cyclohexylethyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl stehen,
R⁷ und R⁸ außerdem unabhängig voneinander für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes Pyridinylmethyl, Pyrimidinylmethyl, Pyridazylmethyl oder Pyrazinylmethyl stehen,
R⁷ und R⁸ außerdem gemeinsam für gegebenenfalls einfach oder mehrfach, gleich oder verschieden durch Fluor, Chlor, Brom, C₁-C₄-Alkoxy, C₁-C₄-Halogenalkoxy, C₁-C₄-Alkylthio und/oder C₁-C₄-Halogenalkylthio substituiertes C₂-C₈-Alkenylen oder für gegebenenfalls im Alkylenteil einfach oder mehrfach, gleich oder verschieden durch Fluor, Chlor, Cyano, Methyl, Ethyl, n-Propyl, i-Propyl, n-Butyl, i-Butyl, s-Butyl, t-Butyl, C₁-C₄-Halogenalkyl, Methoxy, Ethoxy, n-Propoxy, i-Propoxy, n-Butoxy, i-Butoxy, s-Butoxy, t-Butoxy, C₁-C₄-Halogenalkoxy, Methylthio, Ethylthio, n-Propylthio, i-Propylthio, n-Butylthio, i-Butylthio, s-Butylthio, t-Butylthio, C₁-C₄-Halogenalkylthio substituiertes C₃-C₈-Alkylen, wobei die Alkylenkette jeweils durch -O-, -S- oder -NR¹⁵- unterbrochen sein kann, stehen,
R⁷ und R⁸ außerdem gemeinsam für einfach oder zweifach, gleich oder verschieden substituiertes C₃-C₈-Alkylen stehen, wobei die Substituenten zusätzlich aus Ethoxycarbonyl, Methoxycarbonyl und/oder Oxyethylenoxy ausgewählt sein können,
R⁷ und R⁸ außerdem gemeinsam für -CH₂-CH₂-C(=O)-CH₂-CH₂-, -CH₂-CH₂-C(=NO-Me)-CH₂-CH₂-, -CH₂-CH₂-C(=NO-Et)-CH₂-CH₂- oder -CH₂-CH₂-C(=NO-iPr)-CH₂-CH₂- stehen,
R⁹ und R¹⁰ unabhängig voneinander für Wasserstoff, -SO₂CF₃, Methyl, Ethyl, n-Propyl, i-Propyl, n-Butyl, i-Butyl, s-Butyl, t-Butyl, Pentyl, Hexyl, -CF₃, -CH₂CF₃, -(CF₂)₃CF₃, Cyclopropyl, Cyclopentyl, Cyclohexyl, Methoxymethyl, Methoxyethyl, oder für jeweils gegebenenfalls durch Fluor, Chlor, Brom, Methyl, Trifluormethyl, Methoxy, Trifluormethoxy substituiertes Phenyl oder Benzyl stehen,
R⁹ und R¹⁰ außerdem gemeinsam für -(CH₂)₄-, -(CH₂)₅-, -(CH₂)₆-, -CH₂-CH(CH₃)-CH₂-CH(CH₃)-CH₂-, -(CH₂)₂-O-(CH₂)₂-, -(CH₂)₂-S-(CH₂)₂- oder -(CH₂)₂-N(R¹⁵)-(CH₂)₂- stehen,
R¹¹ und R¹² unabhängig voneinander für Methyl, Ethyl, n-Propyl, i-Propyl, n-Butyl, i-Butyl, s-Butyl, t-Butyl, n-Hexyl, Trifluormethyl, Trifluorethyl, Cyclopropyl, Cyclopentyl, Cyclohexyl oder für jeweils gegebenenfalls durch Fluor, Chlor, Brom, Methyl, Trifluormethyl, Methoxy, Trifluormethoxy substituiertes Phenyl oder Benzyl stehen,
R¹¹ und R¹² außerdem gemeinsam für jeweils gegebenenfalls durch Fluor, Chlor, Brom, Methyl, Ethyl, Methoxy, Ethoxy, Methylthio, Ethylthio, Trifluormethyl, Trifluormethoxy, Trifluormethylthio substituiertes -(CH₂)₃-, -(CH₂)₄-, -(CH₂)₅-, -(CH₂)₆- stehen,
R¹⁵ für Wasserstoff, -SO₂R⁶, für -COR⁶ oder -CO₂R⁶; für jeweils gegebenenfalls einfach oder mehrfach, gleich oder verschieden durch Fluor, Chlor, Brom, Cyano, Methylamino, Ethylamino, Di-(C₁-C₆-alkyl)amino, C₁-C₄-Alkoxy, C₁-C₄-Halogenalkoxy, C₁-C₄-Alkylthio, C₁-C₄-Halogenalkylthio substituiertes C₁-C₁₆-Alkyl, C₂-C₁₆-Alkenyl; für jeweils gegebenenfalls einfach bis dreifach, gleich oder verschieden durch Fluor, Chlor, Brom, Cyano, Methyl, Ethyl, n-Propyl, i-Propyl, n-Butyl, i-Butyl, s-Butyl, t-Butyl, C₁-C₄-Halogenalkyl, Methoxy, Ethoxy, n-Propoxy, i-Propoxy, n-Butoxy, i-Butoxy, s-Butoxy, t-Butoxy, C₁-C₄-Halogenalkoxy, Methylthio, Ethylthio, n-Propylthio, i-Propylthio, n-Butylthio, i-Butylthio, s-Butylthio, t-Butylthio, C₁-C₄-Halogen-alkylthio substituiertes C₃-C₈-Cycloalkyl, Cyclopropylmethyl, Cyclopentylmethyl, Cyclohexylmethyl, Cyclopropylethyl, Cyclopentylethyl, Cyclohexylethyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl steht.

8. Δ¹-Pyrroline der Formel (I) gemäß Anspruch 1, in welcher
R⁴ für Wasserstoff, Fluor, Chlor, Brom, Cyano, Formyl, für jeweils gegebenenfalls einfach oder mehrfach, gleich oder verschieden durch Fluor, Chlor, Brom, Cyano, -NR⁷R⁸ substituiertes C₁-C₁₆-Alkyl, C₂-C₁₆-Alkenyl, C₁-C₁₆-Alkoxy, C₂-C₁₆-Alkenyloxy; für -S(O)ₚR⁶, -NR⁷R⁸, -COR⁶, -CO₂R⁶, -CONR⁹R¹⁰, -N(R¹¹)COR¹²; oder für jeweils gegebenenfalls einfach bis dreifach, gleich oder verschieden, durch Fluor, Chlor, Brom, Cyano, Methyl, Ethyl, n-Propyl, i-Propyl, -CF₃, -CHF₂, -CClF₂, -CF₂CHFCl, -CF₂CH₂F, -CF₂CCl₃, -CH₂CF₃, -CF₂CHFCF₃, -CH₂CF₂H, -CH₂CF₂CF₃, -CF₂CF₂H, -CF₂CHFCF₃, Vinyl, Allyl, 1-Propenyl, Butenyl, -CF=CHF, -CF=CH₂, -CF=CCl₂, -CH=CF₂, -CF₂CF=CF₂, -CH=CFH, -CH₂CF=CF₂, -CF=CF₂, -CF₂CH=CF₂, Methoxy, Ethoxy, n-Propoxy, i-Propoxy, n-Butoxy, i-Butoxy, s-Butoxy, t-Butoxy, Trifluormethoxy, Difluormethoxy, Chlordifluormethoxy, Trifluorethoxy, Methylthio, Ethylthio, n-Propylthio, i-Propylthio, n-Butylthio, i-Butylthio, s-Butylthio, t-Butylthio, Trifluormethylthio, Difluormethylthio, Chlordifluormethylthio, Trifluorethylthio substituiertes Cyclopropyl, Cyclopentyl, Cyclohexyl, Cyclopropylmethyl, Cyclopentylmethyl, Cyclohexylmethyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidino, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Piperidino, Morpholinyl, Thiomorpholinyl, Morpholino, Thiomorpholino, Triazinyl, Triazolyl, Chinolinyl oder Isochinolinyl steht,
R⁶ für Methyl, Ethyl, n-Propyl, i-Propyl, n-Butyl, i-Butyl, s-Butyl, t-Butyl, Pentyl, Hexyl, -CF₃, -CHF₂, -CCl₃, -CCl₂F, Dimethylaminomethyl, Dimethylaminoethyl, Diethylaminomethyl, Diethylaminoethyl, Cyclopropyl, Cyclopentyl, Cyclohexyl, Phenyl oder Benzyl steht,
R⁷ und R³ unabhängig voneinander für Wasserstoff, -SO₂R⁶, -COR⁶, -CO₂R⁶, für jeweils gegebenenfalls einfach oder mehrfach, gleich oder verschieden, durch Fluor, Chlor, Brom, C₁-C₄-Alkylcarbonyl, C₁-C₄-Alkylcarbonyloxy, C₁-C₄-Alkylamino, Di-(C₁-C₄-alkyl)amino, C₁-C₄-Alkoxy, C₁-C₄-Halogenalkoxy, C₁-C₄-Alkylthio und/oder C₁-C₄-Halogenalkylthio substituiertes C₁-C₁₆-Alkyl, C₂-C₁₆-Alkenyl; für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio substituiertes C₃-C₈-Cycloalkyl, Cyclopropylmethyl, Cyclopentylmethyl, Cyclohexylmethyl, Cyclopropylethyl, Cyclopentylethyl, Cyclohexylethyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Henzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl stehen,
R⁷ und R⁸ außerdem gemeinsam für gegebenenfalls einfach oder mehrfach, gleich oder verschieden durch Fluor, Chlor, Brom, C₁-C₄-Alkoxy, C₁-C₄-Halogenalkoxy, C₁-C₄-Alkylthio und/oder C₁-C₄-Halogenalkylthio substituiertes C₂-C₈-Alkenylen oder für gegebenenfalls im Alkylenteil einfach oder mehrfach, gleich oder verschieden durch Fluor, Chlor, Cyano, Methyl, Ethyl, n-Propyl, i-Propyl, n-Butyl, i-Butyl, s-Butyl, t-Butyl, C₁-C₄-Halogenalkyl, Methoxy, Ethoxy, n-Propoxy, i-Propoxy, n-Butoxy, i-Butoxy, s-Butoxy, t-Butoxy, C₁-C₄-Halogenalkoxy, Methylthio, Ethylthio, n-Propylthio, i-Propylthio, n-Butylthio, i-Butylthio, s-Butylthio, t-Butylthio, C₁-C₄-Halogenalkylthio substituiertes C₃-C₈-Alkylen, wobei die Alkylenkette jeweils durch -O-, -S- oder -NR¹⁵- unterbrochen sein kann, stehen,
und R¹, R², R³, m, Q, p, R⁹, R¹⁰, R¹¹, R¹² und R¹⁵ die in Anspruch 7 angegebenen Bedeutungen haben.

9. Δ¹-Pyrroline der Formel (I) gemäß einem der Ansprüche 1 bis 8, in welcher R¹ und R² jeweils für Fluor stehen.

10. Δ¹-Pyrroline der Formel (I) gemäß einem der Ansprüche 1 bis 9, in welcher Q für 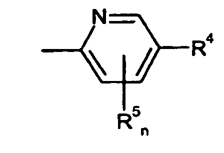 steht.

11. Δ¹-Pyrroline der Formel (I) gemäß einem der Ansprüche 1 bis 9, in welcher Q für 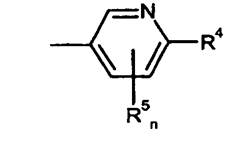 steht.

12. Δ¹-Pyrroline der Formel (I) gemäß einem der Ansprüche 1 bis 9, in welcher Q für 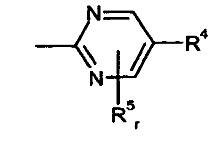 steht.

13. Δ¹-Pyrroline der Formel (I) gemäß einem der Ansprüche 1 bis 9, in welcher Q für 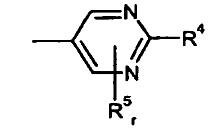 steht.

14. Δ¹-Pyrroline der Formel (I) gemäß einem der Ansprüche 1 bis 9, in welcher Q für 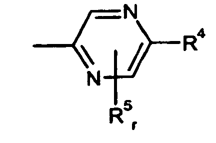 steht.

15. Δ¹-Pyrroline der Formel (I) gemäß einem der Ansprüche 1 bis 9, in welcher Q für 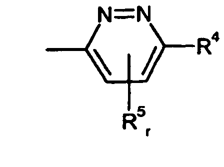 steht.

16. Verbindungen der Formeln (I-a) bis (I-e) 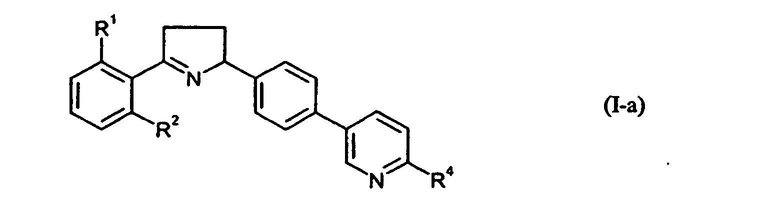 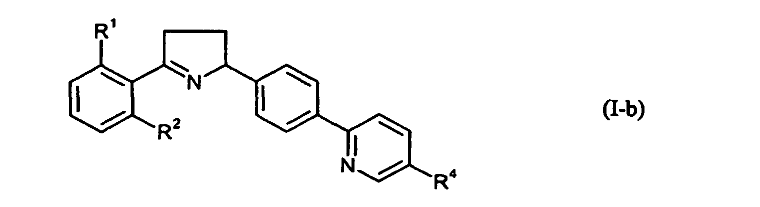 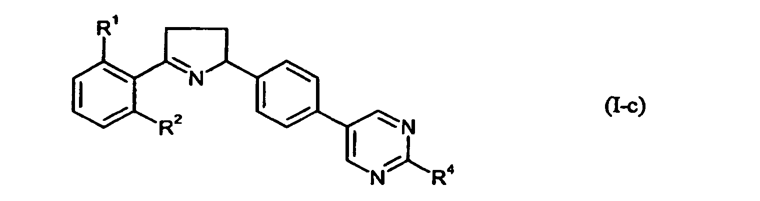 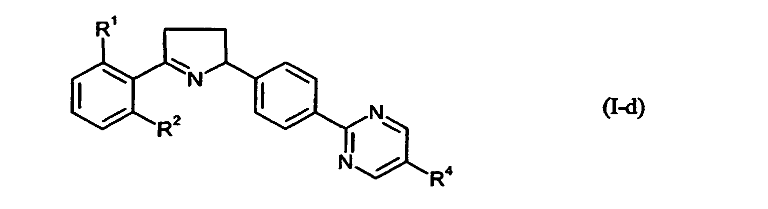 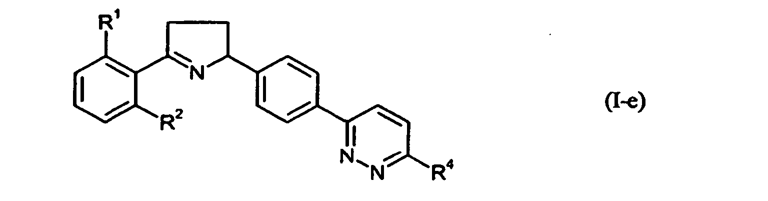 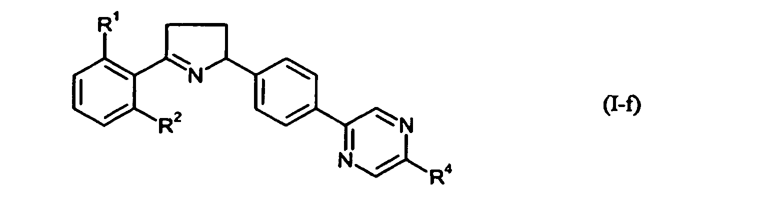 in welchen jeweils
R¹ für Fluor oder Chlor steht,
R² für Wasserstoff oder Fluor steht und
R⁴ die in einem der Ansprüche 1 bis 8 angegebenen Bedeutungen hat.

17. (R)-konfigurierte Verbindungen der Formeln (I-g) bis (I-l) 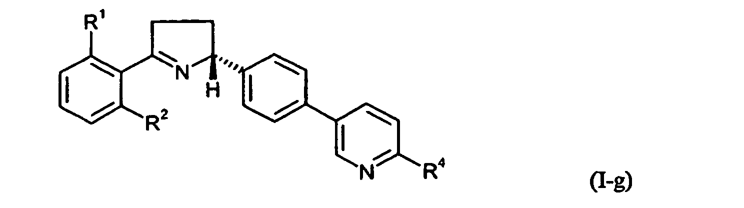 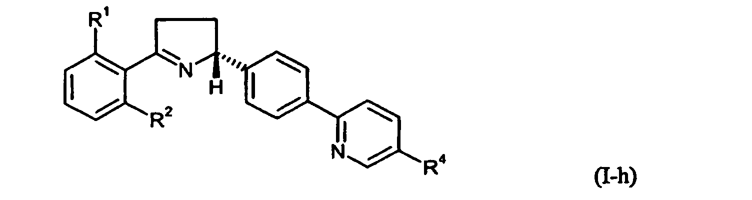 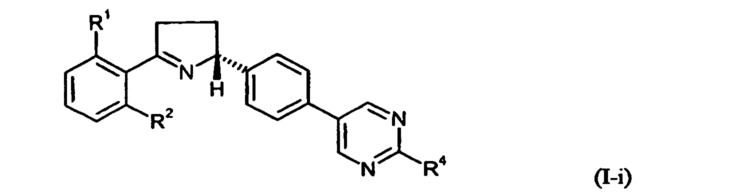 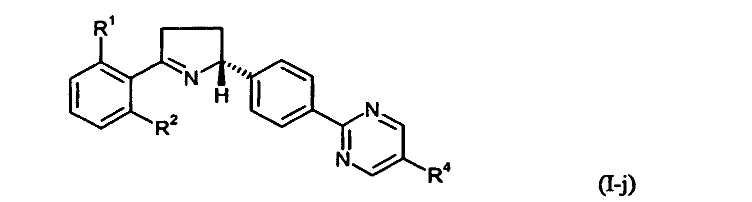 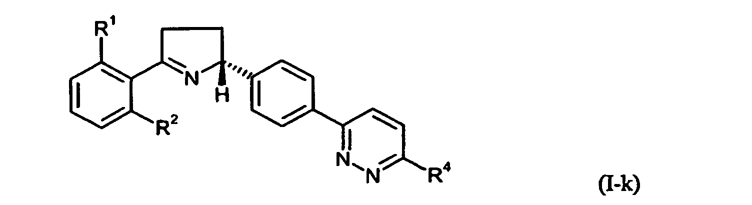 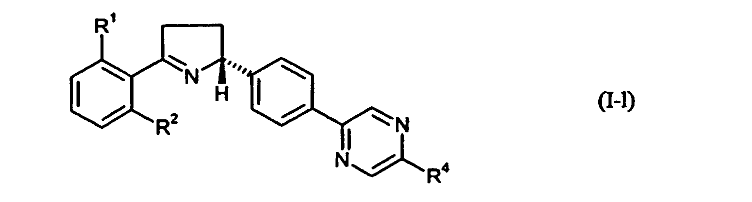 in welchen jeweils
R¹ für Fluor oder Chlor steht,
R² für Wasserstoff oder Fluor steht und
R⁴ die in einem der Ansprüche 1 bis 8 angegebenen Bedeutungen hat.

18. Verfahren zur Herstellung von Verbindungen der Formel (I) gemäß Anspruch 1, **dadurch gekennzeichnet, dass** man
A) Δ¹-Pyrroline der Formel (II) 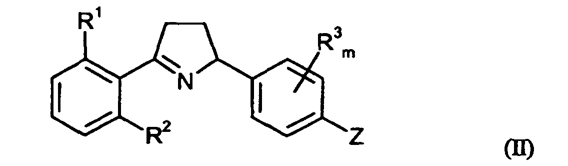 in welcher
R¹, R², R³ und m die in Anspruch 1 angegebenen Bedeutungen haben, und
Z für Chlor, Brom, Iod, -OSO₂CF₃ oder -OSO₂(CF₂)₃CF₃ steht,
mit Heterocyclen der Formel (III)
Q-X (III)
in welcher
Q die in Anspruch 1 angegebene Bedeutung hat, und
X für Chlor, Brom, Iod, -OSO₂CF₃ oder -OSO₂(CF₂)₃CF₃ steht,
in Gegenwart eines Katalysators, in Gegenwart eines Diboronsäureesters und gegebenenfalls in Gegenwart eines Säurebindemittels und gegebenenfalls in Gegenwart eines Verdünnungsmittels in einer Tandem-Reaktion umsetzt,
oder
B) Δ¹-Pyrroline der Formel (IV) 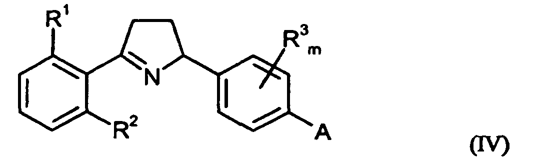 in welcher
R¹, R², R³ und m die in Anspruch 1 angegebenen Bedeutungen haben, und
A für -B(OH)₂, (4,4,5,5-Tetramethyl-1,3,2-dioxaborolan)-2-yl, (5,5-Dimethyl-1,3,2-dioxaborinan)-2-yl, (4,4,6-Trimethyl-1,3,2-dioxaborinan)-2-yl oder 1,3,2-Benzodioxaborol-2-yl steht,
mit Heterocyclen der Formel (III)
Q-X (III)
in welcher Q und X die in Anspruch 1 angegebenen Bedeutungen haben, in Gegenwart eines Katalysators, gegebenenfalls in Gegenwart eines Säurebindemittels und gegebenenfalls in Gegenwart eines Verdünnungsmittels umsetzt,
oder
C) Δ¹-Pyrroline der Formel (II) 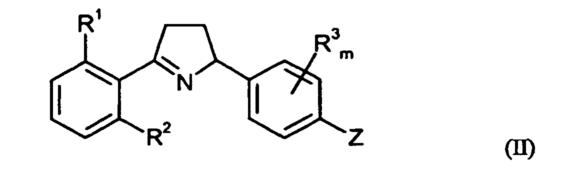 in welcher R¹, R², R³, m und Z die in Anspruch 1 angegebenen Bedeutungen haben, mit Boronsäure-Derivaten der Formel (V)
Q-A (V)
in welcher Q und A die in Anspruch 1 angegebenen Bedeutungen haben, in Gegenwart eines Katalysators, gegebenenfalls in Gegenwart eines Säurebindemittels und gegebenenfalls in Gegenwart eines Verdünnungsmittels umsetzt,
oder
D) Δ¹-Pyrroline der Formel (II-a) 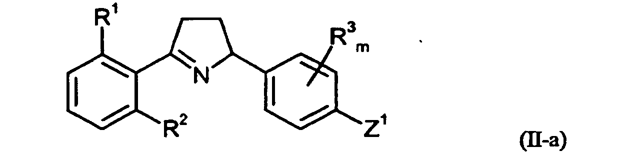 in welcher
R¹, R², R³, m die in Anspruch 1 angegebenen Bedeutungen haben, und
Z¹ für Brom oder Iod steht,
mit organometallischen Verbindungen der Formel (VI)
Q-M (VI)
in welcher
Q die in Anspruch 1 angegebenen Bedeutungen hat, und
M für ZnCl, Sn(Me)₃ oder Sn(n-Bu)₃ steht,
in Gegenwart eines Katalysators, gegebenenfalls in Gegenwart eines Säurebindemittels und gegebenenfalls in Gegenwart eines Verdünnungsmittels umsetzt.

19. Schädlingsbekämpfungsmittel, **gekennzeichnet durch** einen Gehalt an mindestens einer Verbindung der Formel (I) gemäß Anspruch 1 neben Streckmitteln und/oder oberflächenaktiven Stoffen.

20. Verwendung von Verbindungen der Formel (I) gemäß Anspruch 1 zur Bekämpfung von Schädlingen.

21. Verfahren zur Bekämpfung von Schädlingen, **dadurch gekennzeichnet, dass** man Verbindungen der Formel (I) gemäß Anspruch 1 auf Schädlinge und/oder ihren Lebensraum einwirken lässt.

22. Verfahren zur Herstellung von Schädlingsbekämpfungsmitteln, **dadurch gekennzeichnet, dass** man Verbindungen der Formel (I) gemäß Anspruch 1 mit Streckmitteln und/oder oberflächenaktiven Stoffen vermischt.

## Claims

1. Δ¹-Pyrrolines of the formula (I) 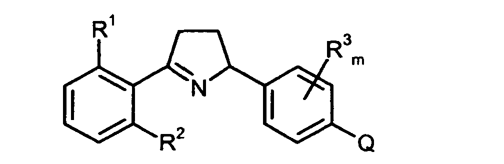 in which
R¹ represents halogen or methyl,
R² represents hydrogen or halogen,
R³ represents halogen, represents in each case optionally halogen-substituted alkyl, alkoxy or alkylthio,
m represents 0, 1, 2, 3 or 4,
Q represents one of the groupings below 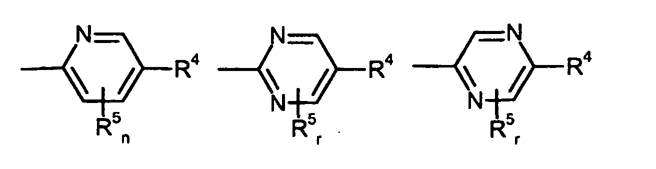 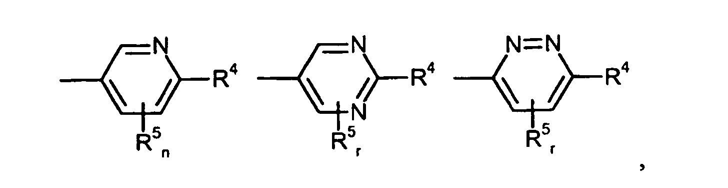
R⁴ represents hydrogen, halogen, cyano, formyl, nitro, trialkylsilyl; represents alkyl, alkenyl, alkoxy or alkenyloxy, each of which is optionally mono- or polysubstituted by identical or different substituents from the group consisting of halogen, cyano and -NR⁷R⁸; represents pentafluorothio, -S(O)ₚR⁶, -NR⁷R⁸, -COR⁶, -CO₂R⁶, -CONR⁹R¹⁰, -N(R¹¹)COR¹² or -C(R¹³)=N-OR¹⁴; or represents cycloalkyl, cycloalkylalkyl, aryl, arylalkyl, saturated or unsaturated, 5- to 10-membered heterocyclyl or heterocyclylalkyl, each of which is optionally mono- or polysubstituted by identical or different substituents from the group consisting of halogen, cyano, nitro, alkyl, halogenoalkyl, alkenyl, halogenoalkenyl, alkoxy, halogenoalkoxy, alkylthio and halogenoalkylthio,
R⁴ furthermore represents -CH=NOH, formyl; represents cycloalkyloxy or cycloalkylalkoxy, each of which is optionally mono- or polysubstituted by identical or different substituents from the group consisting of halogen and alkyl,
R⁵ represents halogen, cyano, formyl, nitro, trialkylsilyl; represents alkyl, alkenyl, alkoxy or alkenyloxy, each of which is optionally mono- or polysubstituted by identical or different substituents from the group consisting of halogen, cyano and -NR⁷R⁸; represents pentafluorothio, -S(O)ₚR⁶, -NR⁷R⁸, -COR⁶, -CO₂R⁶, -CONR⁹R¹⁰, -N(R¹¹)COR¹² or -C(R¹³)=N-OR¹⁴; or represents cycloalkyl, cycloalkylalkyl, aryl, arylalkyl, saturated or unsaturated, 5- to 10-membered heterocyclyl or heterocyclylalkyl, each of which is optionally mono- or polysubstituted by identical or different substituents from the group consisting of halogen, alkyl, halogenoalkyl, alkenyl, halogenoalkenyl, alkoxy, halogenoalkoxy, alkylthio and halogenoalkylthio,
p represents 0, 1 or 2,
n represents 0, 1, 2 or 3, where the substituents R⁵ may be identical or different if n represents 2 or 3,
r represents 0, 1 or 2, where the substituents R⁵ may be identical or different if r represents 2,
R⁶ represents alkyl which is optionally mono- or polysubstituted by identical or different substituents from the group consisting of halogen and -NR⁷R⁸, represents cycloalkyl, aryl or arylalkyl, each of which is optionally mono- or polysubstituted by identical or different substituents from the group consisting of halogen, cyano, alkyl, halogenoalkyl, alkoxy, halogenoalkoxy, alkylthio and halogenoalkylthio,
R⁶ furthermore represents alkyl which is mono- or polysubstituted by identical or different substituents, where the substituents may additionally be selected from the group consisting of alkoxy, alkylthio, halogenoalkoxy and halogenoalkylthio,
R⁷ and R⁸ independently of one another represent hydrogen, -SO₂R⁶, -COR⁶, -CO₂R⁶, represent alkyl or alkenyl, each of which is optionally mono- or polysubstituted by identical or different substituents from the group consisting of halogen, alkylcarbonyl, alkylcarbonyloxy, alkylamino, dialkylamino, alkoxy, halogenoalkoxy, alkylthio and halogenoalkylthio; represents cycloalkyl, cycloalkylalkyl, aryl, arylalkyl, saturated or unsaturated 5- to 10-membered heterocyclyl or heterocyclylalkyl, each of which is optionally mono- or polysubstituted by identical or different substituents from the group consisting of halogen, cyano, alkyl, halogenoalkyl, alkoxy, halogenoalkoxy, alkylthio and halogenoalkylthio,
R⁷ andR⁸ furthermore together represent alkenylene which is optionally mono- or polysubstituted by identical or different substituents from the group consisting of halogen, cyano, alkoxy, halogenoalkoxy, alkylthio and halogenoalkylthio or represent alkylene which is optionally mono- or polysubstituted by identical or different substituents from the group consisting of halogen, cyano, alkyl, halogenoalkyl, alkoxy, halogenoalkoxy, alkylthio and halogenoalkylthio, where the alkylene chain may in each case be interrupted by -O-, -S- or -NR¹⁵-,
R⁷ and R⁸ furthermore together represent alkylene which is mono- or polysubstituted by identical or different substituents, where the substituents may additionally be selected from the group consisting of alkoxycarbonyl and oxyalkyleneoxy,
R⁷ and R⁸ furthermore together represent alkylene, where the alkylene chain is interrupted either by C=O or by C=NO-alkyl,
R⁹ and R¹⁰ independently of one another represent hydrogen, -SO₂R⁶, represent alkyl or alkenyl, each of which is optionally mono- or polysubstituted by identical or different substituents from the group consisting of halogen, alkylamino, dialkylamino, alkoxy and alkylthio; represent cycloalkyl, cycloalkylalkyl, aryl, arylalkyl, saturated or unsaturated 5- to 10-membered heterocyclyl or heterocyclylalkyl, each of which is optionally mono- or polysubstituted by identical or different substituents from the group consisting of halogen, cyano, alkyl, halogenoalkyl, alkoxy, halogenoalkoxy, alkylthio and halogenoalkylthio,
R⁹ and R¹⁰ furthermore together represent alkylene which is optionally mono- or polysubstituted by identical or different substituents from the group consisting of halogen, cyano, alkyl, halogenoalkyl, alkoxy, halogenoalkoxy, alkylthio and halogenoalkylthio, where the alkylene chain may in each case be interrupted by -O-, -S- or -NR¹⁵-,
R¹¹ and R¹² independently of one another represent hydrogen, represent alkyl which is optionally mono- or polysubstituted by identical or different substitutents from the group consisting of halogen, cyano, alkoxy and alkylthio, represent cycloalkyl, cycloalkylalkyl, aryl or arylalkyl, each of which is optionally mono- or polysubstituted by identical or different substituents from the group consisting of halogen, cyano, alkyl, halogenoalkyl, alkoxy, halogenoalkoxy, alkylthio and halogenoalkylthio,
R¹¹ and R¹² furthermore together represent alkylene or alkenylene, each of which is optionally mono- or polysubstituted by identical or different substituents from the group consisting of halogen, alkyl, halogenoalkyl, alkoxy, halogenoalkoxy, alkylthio and halogenoalkylthio,
R¹³ and R¹⁴ independently of one another represent hydrogen, represent alkyl or alkenyl, each of which is optionally mono- or polysubstituted by halogen,
R¹⁵ represents hydrogen, -SO₂R⁶, -COR⁶ or -CO₂R⁶; represents alkyl or alkenyl, each of which is optionally mono- or polysubstituted by identical or different substituents from the group consisting of halogen, cyano, alkylamino, dialkylamino, alkoxy, halogenoalkoxy, alkylthio and halogenoalkylthio; represents cycloalkyl, cycloalkylalkyl, aryl, arylalkyl, saturated or unsaturated 5- to 10-membered heterocyclyl or heterocyclylalkyl, each of which is optionally mono- or polysubstituted by identical or different substituents from the group consisting of halogen, cyano, alkyl, halogenoalkyl, alkoxy, halogenoalkoxy, alkylthio and halogenoalkylthio.

2. Δ¹-Pyrrolines of the formula (I) according to Claim 1, in which
R⁴ represents hydrogen, halogen, cyano, formyl, nitro, trialkylsilyl; represents alkyl, alkenyl, alkoxy or alkenyloxy, each of which is optionally mono- or polysubstituted by identical or different substituents from the group consisting of halogen, cyano and -NR⁷R⁸; represents pentafluorothio, -S(O)ₚR⁶, -NR⁷R⁸, -COR⁶, -CO₂R⁶, -CONR⁹R¹⁰, -N(R¹¹)COR¹² or -C(R¹³)=N-OR¹⁴; or represents cycloalkyl, cycloalkylalkyl, aryl, arylalkyl, saturated or unsaturated 5- to 10-membered heterocyclyl or heterocyclylalky, each of which is optionally mono- or polysubstituted by identical or different substituents from the group consisting of halogen, cyano, nitro, alkyl, halogenoalkyl, alkenyl, halogenoalkenyl, alkoxy, halogenoalkoxy, alkylthio and halogenoalkylthio,
R⁶ represents alkyl which is optionally mono- or polysubstituted by identical or different substituents from the group consisting of halogen and -NR⁷R⁸, represents cycloalkyl, aryl or arylalkyl, each of which is optionally mono- or polysubstituted by identical or different substituents from the group consisting of halogen, cyano, alkyl, halogenoalkyl, alkoxy, halogenoalkoxy, alkylthio and halogenoalkylthio,
R⁷ and R⁸ independently of one another represent hydrogen, -SO₂R⁶, -COR⁶, -CO₂R⁶, represent alkyl or alkenyl, each of which is optionally mono- or polysubstituted by identical or different substituents from the group consisting of halogen, alkylcarbonyl, alkylcarbonyloxy, alkylamino, dialkylamino, alkoxy, halogenoalkoxy, alkylthio and halogenoalkylthio; represent cycloalkyl, cycloalkylalkyl, aryl, arylalkyl, saturated or unsaturated 5- to 10-membered heterocyclyl or heterocyclylalkyl, each of which is optionally mono- or polysubstituted by identical or different substituents from the group consisting of halogen, cyano, alkyl, halogenoalkyl, alkoxy, halogenoalkoxy, alkylthio and halogenoalkylthio,
R⁷ and R⁸ furthermore together represent alkenylene which is optionally mono- or polysubstituted by identical or different substituents from the group consisting of halogen, cyano, alkoxy, halogenoalkoxy, alkylthio and halogenoalkylthio or represent alkylene which is optionally mono- or polysubstituted by identical or different substituents from the group consisting of halogen, cyano, alkyl, halogenoalkyl, alkoxy, halogenoalkoxy, alkylthio and halogenoalkylthio, where the alkylene chain may in each case be interrupted by -O-, -S- or -NR¹⁵-,
and R¹, R², R³, m, Q, R⁵, p, n, r, R⁹, R¹⁰, R¹¹, R¹², R¹³, R¹⁴ and R¹⁵ have the meanings given in Claim 1.

3. Δ¹-Pyrrolines of the formula (I) according to Claim 1, in which
R¹ represents fluorine, chlorine, bromine or methyl,
R² represents hydrogen, fluorine, chlorine or bromine,
R³ represents fluorine, chlorine, bromine, represents C₁-C₆-alkyl, C₁-C₆-alkoxy or C₁-C₆-alkylthio, each of which is optionally mono- to octasubstituted by halogen,
m represents 0, 1, 2 or 3,
Q represents one of the groupings below 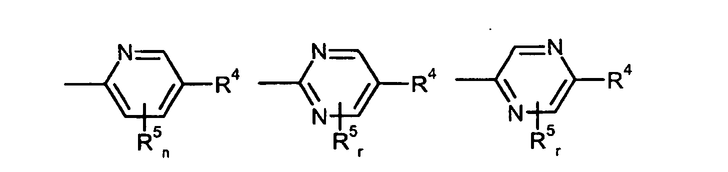 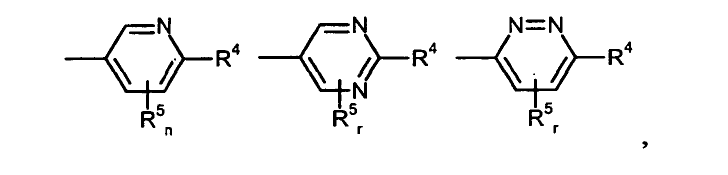
R⁴ represents hydrogen, fluorine, chlorine, bromine, cyano, formyl, nitro, tri-(C₁-C₆-alkyl)silyl; represents C₁-C₂₀-alkyl, C₂-C₂₀-alkenyl, C₁-C₂₀-alkoxy, C₂-C₂₀-alkenyloxy, each of which is optionally mono- or polysubstituted by identical or different substituents from the group consisting of halogen, cyano and -NR⁷R⁸; represents pentafluorothio, -S(O)ₚR⁶, -NR⁷R⁸, -COR⁶, -CO₂R⁶, -CONR⁹R¹⁰, -N(R¹¹)COR¹² or -C(R¹³)=N-OR¹⁴; or represents C₃-C₁₂-cycloalkyl, C₃-C₇-cycloalkyl-C₁-C₄-alkyl, aryl, aryl-C₁-C₄-alkyl, saturated or unsaturated, 5- to 10-membered heterocyclyl or heterocyclyl-C₁-C₄-alkyl having 1 to 4 heteroatoms, which comprise 0 to 4 nitrogen atoms, 0 to 2 nonadjacent oxygen atoms and/or 0 to 2 nonadjacent sulphur atoms (in particular tetrazolyl, furyl, furfuryl, benzofuryl, tetrahydrofuryl, thienyl, thenyl, benzothienyl, thiolanyl, pyrrolyl, indolyl, pyrrolinyl, pyrrolidino, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, piperidino, morpholinyl, thiomorpholinyl, morpholino, thiomorpholino, triazinyl, triazolyl, quinolinyl or isoquinolinyl), each of which is optionally mono- to tetrasubstituted by identical or different substituents from the group consisting of halogen, cyano, nitro, C₁-C₆-alkyl, C₁-C₆-halogenoalkyl, C₂-C₆-alkenyl, C₂-C₆-halogenoalkenyl, C₁-C₆-alkoxy, C₁-C₆-halogenoalkoxy, C₁-C₆-alkylthio and C₁-C₆-halogenoalkylthio,
R⁴ furthermore represents -CH=NOH, formyl; represents C₃-C₆-cycloalkyloxy or C₃-C₆-cycloalkyl-C₁-C₄-alkoxy, each of which is optionally mono- to tetrasubstituted by identical or different substituents from the group consisting of fluorine, chlorine, bromine and C ₁-C₂-alkyl,
R⁵ represents fluorine, chlorine, bromine, cyano, formyl, nitro, tri-(C₁-C₆-alkyl)silyl; represents C₁-C₆-alkyl, C₂-C₆-alkenyl, C₁-C₆-alkoxy, C₂-C₆-alkenyloxy; each of which is optionally mono- to tridecasubstituted by halogen; represents pentafluorothio, -S(O)ₚR⁶, -NR⁷R⁸, -COR⁶, -CO₂R⁶, -CONR⁹R¹⁰ or -N(R¹¹)COR¹²,
p represents 0, 1 or 2,
n represents 0, 1, 2 or 3, where the substituents R⁵ may be identical or different if n represents 2 or 3,
r represents 0, 1 or 2, where the substituents R⁵ may be identical or different if r represents 2,
R⁶ represents C₁-C₂₀-alkyl which is optionally mono- or polysubstituted by identical or different substituents from the group consisting of halogen and -NR⁷R⁸, represents C₃-C₆-cycloalkyl, aryl or aryl-C₁-C₄-alkyl, each of which is optionally mono- to octasubstituted by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio,
R⁶ furthermore represents C₁-C₂₀-alkyl which is mono- or polysubstituted by identical or different substituents, where the substituents may additionally be selected from the group consisting of C₁-C₆-alkoxy, C₁-C₆-alkylthio, C₁-C₆-halogenoalkoxy and C₁-C₆-halogenoalkylthio,
R⁷ and R⁸ independently of one another represent hydrogen, -SO₂R⁶, -COR⁶, -CO₂R⁶, represent C₁-C₂₀-alkyl or C₂-C₂₀-alkenyl, each of which is optionally mono- or polysubstituted by identical or different substituents from the group consisting of halogen, C₁-C₆-alkylcarbonyl, C₁-C₆-alkylcarbonyloxy, C₁-C₆-alkylamino, di-(C₁-C₆-alkyl)amino, C₁-C₆-alkoxy, C₁-C₆-halogenoalkoxy, C₁-C₆-alkylthio and C₁-C₆-halogenoalkylthio; represent C₃-C₁₂-cycloalkyl, C₃-C₇-cycloalkyl-C₁-C₄-alkyl, aryl, aryl-C₁-C₄-alkyl, saturated or unsaturated 5- to 10-membered heterocyclyl or heterocyclyl-C₁-C₄-alkyl having 1 to 4 heteroatoms, which comprise 0 to 4 nitrogen atoms, 0 to 2 nonadjacent oxygen atoms and/or 0 to 2 nonadjacent sulphur atoms (in particular tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl), each of which is optionally mono- to tetrasubstituted by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio,
R⁷ and R⁸ furthermore independently of one another represent heterocyclyl-C₁-C₄-alkyl having 1 to 4 heteroatoms, which comprise 0 to 4 nitrogen atoms, 0 to 2 nonadjacent oxygen atoms and/or 0 to 2 nonadjacent sulphur atoms (in particular pyridinylmethyl, pyridinylethyl, pyrimidinylmethyl, pyrimidinylethyl, pyridazylmethyl, pyridazylethyl, pyrazinylmethyl or pyrazinylethyl), each of which is optionally mono- to tetrasubstituted by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio,
R⁷ and R⁸ furthermore together represent C₂-C₁₂-alkenylene which is optionally mono- or polysubstituted by identical or different substituents from the group consisting of halogen, cyano, C₁-C₆-alkoxy, C₁-C₆-halogenoalkoxy, C₁-C₆-alkylthio and C₁-C₆-halogenoalkylthio or represent C₃-C₁₂-alkylene which is optionally mono- or polysubstituted in the alkylene moiety by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio, where the alkylene chain may in each case be interrupted by -O-, -S- or -NR¹⁵-,
R⁷ and R⁸ furthermore together represent C₃-C₁₂-alkylene which is optionally mono- or polysubstituted by identical or different substituents, where the substituents may additionally be selected from the group consisting of C₁-C₄-alkoxycarbonyl and oxy-(C₁-C₄-alkylene)-oxy,
R⁷ and R⁸ furthermore together represent C₃-C₈-alkylene, where the alkylene chain is interrupted either by C=O or C=NO-(C₁-C₆-alkyl),
R⁹ and R¹⁰ independently of one another represent hydrogen, -SO₂R⁶, represent C₁-C₆-alkyl or C₂-C₆-alkenyl, each of which is optionally mono- to tridecasubstituted by identical or different substituents from the group consisting of halogen, C₁-C₆-alkylamino, di-(C₁-C₆-alkyl)amino, C₁-C₆-alkoxy, C₁-C₆-halogenoalkoxy, C₁-C₆-alkylthio and C₁-C₆-halogenoalkylthio; represent C₃-C₇-cycloalkyl, C₃-C₇-cycloalkyl-C₁-C₄-alkyl, aryl, aryl-C₁-C₄-alkyl, saturated or unsaturated 5- to 10-membered heterocyclyl or heterocyclyl-C₁-C₄-alkyl having 1 to 4 heteroatoms, which comprise 0 to 4 nitrogen atoms, 0 to 2 nonadjacent oxygen atoms and/or 0 to 2 nonadjacent sulphur atoms (in particular tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl), each of which is optionally mono- to tetrasubstituted by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio and C₁-C₆-halogeno-alkylthio,
R⁹ and R¹⁰ furthermore together represent C₃-C₆-alkylene, -(CH₂)₂-O-(CH₂)₂-, -(CH₂)₂-S-(CH₂)₂- or -(CH₂)₂-N(R¹⁵)-(CH₂)₂-, each of which is optionally mono- to tetrasubstituted in the alkylene moiety by identical or different substituents from the group consisting of halogen, cyano, C₁-C₆-alkyl, C₁-C₆-halogenoalkyl, C₁-C₆-alkoxy, C₁-C₆-hatogenoalkoxy, C₁-C₆-alkylthio and C₁-C₆-halogenoalkylthio,
R¹¹ and R¹² independently of one another represent hydrogen, represent C₁-C₆-alkyl which is optionally mono- to tridecasubstituted by identical or different substituents from the group consisting of halogen, cyano, C₁-C₆-alkoxy and C₁-C₆-alkylthio, represent C₃-C₇-cycloalkyl, C₃-C₇-cycloalkyl-C₁-C₄-alkyl, aryl or aryl-C₁-C₄-alkyl, each of which is optionally mono- to octasubstituted by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio,
R¹¹ and R¹² furthermore together represent C₃-C₁₀-alkylene or C₃-C₁₀-alkenylene, each of which is optionally mono- to octasubstituted by identical or different substituents from the group consisting of halogen, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio,
R¹³ and R¹⁴ independently of one another represent hydrogen, represent C₁-C₆-alkyl or C₂-C₆-alkenyl, each of which is optionally mono- or polysubstituted by halogen,
R¹⁵ represents hydrogen, -SO₂R⁶, -COR⁶ or -CO₂R⁶; represents C₁-C₂₀-alkyl or C₂-C₂₀-alkenyl, each of which is optionally mono- or polysubstituted by identical or different substituents from the group consisting of halogen, cyano, C₁-C₆-alkylamino, di-(C₁-C₆-alkyl)amino, C₁-C₆-alkoxy, C₁-C₆-halogenoalkoxy, C₁-C₆-alkylthio and C₁-C₆-halogenoalkylthio; represents C₃-C₁₂-cycloalkyl, C₃-C₇-cycloalkyl-C₁-C₄-alkyl, aryl, aryl-C₁-C₄-alkyl, saturated or unsaturated 5- to 10-membered heterocyclyl or heterocyclyl-C₁-C₄-alkyl having 1 to 4 heteroatoms, which comprise 0 to 4 nitrogen atoms, 0 to 2 nonadjacent oxygen atoms and/or 0 to 2 nonadjacent sulphur atoms (in particular tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl), each of which is optionally mono- to tetrasubstituted by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio.

4. Δ¹-Pyrrolines of the formula (I) according to Claim 1, in which
R⁴ represents hydrogen, fluorine, chlorine, bromine, cyano, formyl, nitro, tri-(C₁-C₆-alkyl)silyl; represents C₁-C₂₀-alkyl, C₂-C₂₀-alkenyl, C₁-C₂₀-alkoxy or C₂-C₂₀-alkenyloxy, each of which is optionally mono- or polysubstituted by identical or different substituents from the group consisting of halogen, cyano, -NR⁷R⁸, represents pentafluorothio, -S(O)ₚR⁶, -NR⁷R⁸, -COR⁶, -CO₂R⁶, -CONR⁹R¹⁰, -N(R¹¹)COR¹² or -C(R¹³)=N-OR¹⁴; or represents C₃-C₁₂-cycloalkyl, C₃-C₇-cyclo-C₁-C₄-alkyl, aryl, aryl-C₁-C₄-alkyl, saturated or unsaturated 5- to 10-membered heterocyclyl or heterocyclyl-C₁-C₄-alkyl having 1 to 4 heteroatoms, which comprise 0 to 4 nitrogen atoms, 0 to 2 nonadjacent oxygen atoms and/or 0 to 2 nonadjacent sulphur atoms (in particular tetrazolyl, furyl, furfuryl, benzofuryl, tetrahydrofuryl, thienyl, thenyl, benzothienyl, thiolanyl, pyrrolyl, indolyl, pyrrolinyl, pyrrolidino, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, piperidino, morpholinyl, thiomorpholinyl, morpholino, thiomorpholino, triazinyl, triazolyl, quinolinyl or isoquinolinyl), each of which is optionally mono- to tetrasubstituted by identical or different substituents from the group consisting of halogen, cyano, nitro, C₁-C₆-alkyl, C₁-C₆-halogenoalkyl, C₂-C₆-alkenyl, C₂-C₆-halogenoalkenyl, C₁-C₆-alkoxy, C₁-C₆-halogenoalkoxy, C₁-C₆-alkylthio and C₁-C₆-halogenoalkylthio,
R⁶ represents C₁-C₂₀-alkyl which is optionally mono- or polysubstituted by identical or different substituents from the group consisting of halogen and -NR⁷R⁸, represents C₃-C₆-cycloalkyl, aryl or aryl-C₁-C₄-alkyl, each of which is optionally mono- to octasubstituted by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio,
R⁷ and R⁸ independently of one another represent hydrogen, -SO₂R⁶, -COR⁶, -CO₂R⁶, represent C₁-C₂₀-alkyl or C₂-C₂₀-alkenyl, each of which is optionally mono- or polysubstituted by identical or different substituents from the group consisting of halogen, C₁-C₆-alkylcarbonyl, C₁-C₆-alkylcarbonyloxy, C₁-C₆-alkylamino, di-(C₁-C₆-alkyl)amino, C₁-C₆-alkoxy, C₁-C₆-halogenoalkoxy, C₁-C₆-alkylthio and C₁-C₆-halogenoalkylthio; represent C₃-C₁₂-cycloalkyl, C₃-C₇-cycloalkyl-C₁-C₄-alkyl, aryl, aryl-C₁-C₄-alkyl, saturated or unsaturated 5- to 10-membered heterocyclyl or heterocyclyl-C₁-C₄-alkyl having 1 to 4 heteroatoms, which comprise 0 to 4 nitrogen atoms, 0 to 2 nonadjacent oxygen atoms and/or 0 to 2 nonadjacent sulphur atoms (in particular tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl), each of which is optionally mono- to tetrasubstituted by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio,
R⁷ and R⁸ furthermore together represent C₂-C₁₂-alkenylene which is optionally mono- or polysubstituted by identical or different substituents from the group consisting of halogen, cyano, C₁-C₆-alkoxy, C₁-C₆-halogenoalkoxy, C₁-C₆-alkylthio and C₁-C₆-halogenoalkylthio or represent C₃-C₁₂-alkylene, which is optionally mono- or polysubstituted in the alkylene moiety by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio, where the alkylene chain may in each case be interrupted by -O-, -S- or -NR¹⁵-,
and R¹, R², R³, m, Q, R⁵, p, n, r, R⁹, R¹⁰, R¹¹, R¹², R¹³, R¹⁴ and R¹⁵ have the meanings given in Claim 3.

5. Δ¹-Pyrrolines of the formula (I) according to Claim 1, in which
R¹ represents fluorine, chlorine or methyl,
R² represents hydrogen, fluorine or chlorine,
R³ represents fluorine, chlorine, C₁-C₄-alkyl, C₁-C₄-alkoxy, C₁-C₄-alkylthio; C₁-C₄-halogenoalkyl, C₁-C₄-halogenoalkoxy, C₁-C₄-halogenoalkylthio having in each case 1 to 9 fluorine, chlorine and/or bromine atoms,
m represents 0, 1 or 2,
Q represents one of the groupings below 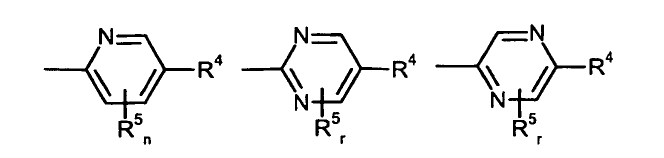 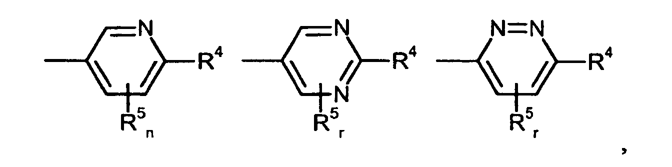
R⁴ represents hydrogen, fluorine, chlorine, bromine, cyano, formyl, nitro, tri-(C₁-C₄-alkyl)silyl; represents C₁-C₁₆-alkyl, C₂-C₁₆-alkenyl, C₁-C₁₆-alkoxy or C₂-C₁₆-alkenyloxy, each of which is optionally mono- or polysubstituted by identical or different substituents from the group consisting of fluorine, chlorine, bromine, cyano and -NR⁷R⁸; represents -S(O)ₚR⁶, -NR⁷R⁸, -COR⁶, -CO₂R⁶, -CONR⁹R¹⁰ or -N(R¹¹)COR¹²; or represents C₃-C₁₀-cycloalkyl, C₃-C₆-cycloalkyl-C₁-C₄-alkyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidino, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, piperidino, morpholinyl, thiomorpholinyl, morpholino, thiomorpholino, triazinyl, triazolyl, quinolinyl or isoquinolinyl, each of which is optionally mono- to trisubstituted by identical or different substituents from the group consisting of fluorine, chlorine, bromine, cyano, C₁-C₄-alkyl, C₁-C₄-halogenoalkyl, C₂-C₆-alkenyl, C₂-C₆-halogenoalkenyl, C₁-C₄-alkoxy, C₁-C₄-halogenoalkoxy, C₁-C₄-alkylthio and C₁-C₄-halogenoalkylthio,
R⁴ furthermore represents -CH=NOH, formyl; represents cyclopropyloxy, cyclobutyloxy, cyclopentyloxy, cyclohexyloxy, cyclopropylmethoxy, cyclobutylmethoxy, cyclopentylmethoxy or cyclohexylmethoxy, each of which is optionally mono- to trisubstituted by identical or different substituents from the group consisting of fluorine, chlorine and methyl,
R⁵ represents fluorine, chlorine, trimethylsilyl, represents C₁-C₄-alkyl, C₂-C₆-alkenyl, C₁-C₄-alkoxy or C₂-C₆-alkenyloxy, each of which is optionally mono- to nonasubstituted by identical or different substituents from the group consisting of fluorine, chlorine and bromine, represents -S(O)ₚR⁶, -NR⁷R⁸, -COR⁶, -CO₂R⁶ or -CONR⁹R¹⁰,
p represents 0, 1 or 2,
n represents 0, 1 or 2, where the substituents R⁵ can be identical or different if n represents 2,
r represents 0, 1 or 2, where the substituents R⁵ can be identical or different if r represents 2,
R⁶ represents C₁-C₁₀-alkyl which is optionally mono- or polysubstituted by identical or different substituents from the group consisting of fluorine, chlorine, bromine and -NR⁷R⁸, represents cyclopropyl, cyclopentyl, cyclohexyl, phenyl or benzyl, each of which is optionally mono- to tetrasubstituted by identical or different substituents from the group consisting of fluorine, chlorine, bromine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoallcylthio,
R⁶ furthermore represents C₁-C₁₀-alkyl which is mono- to trisubstituted by identical or different substituents, where the substituents may additionally be selected from the group consisting of C₁-C₄-alkoxy, C₁-C₄-alkylthio, C₁-C₄-halogenoalkoxy and C₁-C₄-halogenoalkylthio,
R⁷ and R⁸ independently of one another represent hydrogen, -SO₂R⁶, -COR⁶, -CO₂R⁶, represent C₁-C₁₆-alkyl or C₂-C₁₆-alkenyl, each of which is optionally mono- or polysubstituted by identical or different substituents from the group consisting of fluorine, chlorine, bromine, C₁-C₄-alkylcarbonyl, C₁-C₄-alkylcarbonyloxy, C₁-C₄-alkylamino, di-(C₁-C₄-alkyl)amino, C₁-C₄-alkoxy, C₁-C₄-halogenoalkoxy, C₁-C₄-alkylthio and C₁-C₄-halogenoalkylthio; represent C₃-C₁₀-cycloalkyl, C₃-C₆-cycloalkyl-C₁-C₄-alkyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl, each of which is optionally mono- to trisubstituted by identical or different substituents from the group consisting of fluorine, chlorine, bromine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
R⁷ and R⁸ furthermore independently of one another represent pyridinylmethyl, pyridinylethyl, pyrimidinylmethyl, pyrimidinylethyl, pyridazylmethyl, pyridazylethyl, pyrazinylmethyl or pyrazinylethyl, each of which is optionally mono- to trisubstituted by identical or different substituents from the group consisting of fluorine, chlorine, bromine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
R⁷ and R⁸ furthermore together represent C₂-C₁₀-alkenylene, which is optionally mono- or polysubstituted by identical or different substituents from the group consisting of fluorine, chlorine, bromine, C₁-C₄-alkoxy, C₁-C₄-halogenoalkoxy, C₁-C₄-alkylthio and C₁-C₄-halogenoalkylthio or represent C₃-C₁₀-alkylene, which is optionally mono- or polysubstituted in the alkylene moiety by identical or different substituents from the group consisting of fluorine, chlorine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio, where the alkylene chain may in each case be interrupted by -O-, -S- or -NR¹⁵-,
R⁷ and R⁸ furthermore together represent C₃-C₁₀-alkylene which is mono- to trisubstituted by identical or different substituents, where the substituents may additionally be selected from n-propoxycarbonyl, isopropoxycarbonyl, ethoxycarbonyl, methoxycarbonyl, oxypropyleneoxy, oxyethyleneoxy and oxymethyleneoxy,
R⁷ and R⁸ furthermore together represent C₃-C₆-alkylene, where the alkylene chain is interrupted either by C=O or by C=NO-(C₁-C₄-alkyl),
R⁹ and R¹⁰ independently of one another represent hydrogen, -SO₂R⁶, represent C₁-C₄-alkyl or C₂-C₆-alkenyl, each of which is optionally mono- to nonasubstituted by identical or different substituents from the group consisting of fluorine, chlorine, bromine, C₁-C₄-alkylamino, di-(C₁-C₄-alkyl)amino, C₁-C₄-alkoxy, C₁-C₄-halogenoalkoxy, C₁-C₄-alkylthio and C₁-C₄-halogenoalkylthio; represent C₃-C₆-cycloalkyl, C₃-C₆-cycloalkyl-C₁-C₄-alkyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl, each of which is optionally mono- to trisubstituted by identical or different substituents from the group consisting of fluorine, chlorine, bromine, C₁-C₄-alkyl, cyano, C₁-C₄-halogenoalkyl, C₁-C₄-alkoxy, C₁-C₄-halogenoalkoxy, C₁-C₄-alkylthio and C₁-C₄-halogenoalkylthio,
R⁹ and R¹⁰ furthermore together represent C₄-C₅-alkylene, -(CH₂)₂-O-(CH₂)₂-, -(CH₂)₂-S-(CH₂)₂-, -(CH₂)₂-N(R¹⁵)-(CH₂)₂-, each of which is optionally mono- to tetrasubstituted in the alkylene moiety by identical or different substituents from the group consisting of fluorine, chlorine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
R¹¹ and R¹² independently of one another represent hydrogen, represent C₁-C₆-alkyl which is optionally mono- to nonasubstituted by identical or different substituents from the group consisting of fluorine, chlorine, bromine, C₁-C₄-alkoxy and C₁-C₄-alkylthio, represent C₃-C₆-cycloalkyl, C₃-C₆-cycloalkyl-C₁-C₄-alkyl, phenyl, benzyl or phenylethyl, each of which is optionally mono- to tetrasubstituted by identical or different substituents from the group consisting of fluorine, chlorine, bromine, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
R¹¹ and R¹² furthermore together represent C₃-C₈-alkylene or C₃-C₈-alkenylene, each of which is optionally mono- to tetrasubstituted by identical or different substituents from the group consisting of fluorine, chlorine, bromine, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
R¹⁵ represents hydrogen, -SO₂R⁶, represents -COR⁶ or -CO₂R⁶; represents C₁-C₁₆-alkyl or C₂-C₁₆-alkenyl, each of which is optionally mono- or polysubstituted by identical or different substituents from the group consisting of fluorine, chlorine, bromine, cyano, methylamino, ethylamino, di-(C₁-C₆-alkyl)amino, C₁-C₄-alkoxy, C₁-C₄-halogenoalkoxy, C₁-C₄-alkylthio and C₁-C₄-halogenoalkylthio; represents C₃-C₁₀-cycloalkyl, C₃-C₆-cycloalkyl-C₁-C₄-alkyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl, each of which is optionally mono- to trisubstituted by identical or different substituents from the group consisting of fluorine, chlorine, bromine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio.

6. Δ¹-Pyrrolines of the formula (I) according to Claim 1, in which
R⁴ represents hydrogen, fluorine, chlorine, bromine, cyano, formyl, nitro, tri-(C₁-C₄-alkyl)silyl; represents C₁-C₁₆-alkyl, C₂-C₁₆-alkenyl, C₁-C₁₆-alkoxy, C₂-C₁₆-alkenyloxy, each of which is optionally mono- or polysubstituted by identical or different substituents from the group consisting of fluorine, chlorine, bromine, cyano and -NR⁷R⁸; represents -S(O)ₚR⁶, -NR⁷R⁸, -COR⁶, -CO₂R⁶, -CONR⁹R¹⁰ or -N(R¹¹)COR¹²; or represents C₃-C₁₀-cycloalkyl, C₃-C₆-cycloalkyl-C₁-C₄-alkyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidino, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, piperidino, morpholinyl, thiomorpholinyl, morpholino, thiomorpholino, triazinyl, triazolyl, quinolinyl or isoquinolinyl, each of which is optionally mono- to trisubstituted by identical or different substituents from the group consisting of fluorine, chlorine, bromine, cyano, C₁-C₄-alkyl, C₁-C₄-halogenoalkyl, C₂-C₆-alkenyl, C₂-C₆-halogenoalkenyl, C₁-C₄-alkoxy, C₁-C₄-halogenoalkoxy, C₁-C₄-alkylthio and C₁-C₄-halogenoalkylthio,
R⁶ represents C₁-C₁₀-alkyl which is optionally mono- or polysubstituted by identical or different substituents from the group consisting of fluorine, chlorine, bromine and -NR⁷R⁸, represents cyclopropyl, cyclopentyl, cyclohexyl, phenyl or benzyl, each of which is optionally mono- to tetrasubstituted by identical or different substituents from the group consisting of fluorine, chlorine, bromine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
R⁷ and R⁸ independently of one another represent hydrogen, -SO₂R⁶, -COR⁶, -CO₂R⁶, represent C₁-C₁₆-alkyl or C₂-C₁₆-alkenyl, each of which is optionally mono- or polysubstituted by identical or different substituents from the group consisting of fluorine, chlorine, bromine, C₁-C₄-alkylcarbonyl, C₁-C₄-alkylcarbonyloxy, C₁-C₄-alkylamino, di-(C₁-C₄-alkyl)amino, C₁-C₄-alkoxy, C₁-C₄-halogenoalkoxy, C₁-C₄-alkylthio and C₁-C₄-halogenoalkylthio; represent C₃-C₁₀-cycloalkyl, C₃-C₆-cycloalkyl-C₁-C₄-alkyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl, each of which is optionally mono- to trisubstituted by identical or different substituents from the group consisting of fluorine, chlorine, bromine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
R⁷ and R⁸ furthermore together represent C₂-C₁₀-alkenylene, which is optionally mono- or polysubstituted by identical or different substituents from the group consisting of fluorine, chlorine, bromine, C₁-C₄-alkoxy, C₁-C₄-halogenoalkoxy, C₁-C₄-alkylthio and C₁-C₄-halogenoalkylthio, or represent C₃-C₁₀-alkylene, which is optionally mono- or polysubstituted in the alkylene moiety by identical or different substituents from the group consisting of fluorine, chlorine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio, where the alkylene chain may in each case be interrupted by -O-, -S- or -NR¹⁵-,
and R¹, R², R3, m, Q, R⁵, p, n, r, R⁹, R¹⁰, R¹¹, R¹², R¹³, R¹⁴ and R¹⁵ have the meanings given in Claim 5.

7. Δ¹-Pyrrolines of the formula (I) according to Claim 1, in which
R¹ represents fluorine or chlorine,
R² represents hydrogen or fluorine,
R³ represents fluorine, chlorine, methyl, ethyl, n-propyl, isopropyl, n-butyl, isobutyl, sec-butyl, tert-butyl, trifluoromethyl, trifluoroethyl, methoxy, ethoxy, n-propoxy, isopropoxy, n-butoxy, isobutoxy, sec-butoxy, tert-butoxy, trifluoromethoxy, trifluoroethoxy, methylthio, ethylthio, n-propylthio, isopropylthio, n-butylthio, isobutylthio, sec-butylthio, tert-butylthio, trifluoromethylthio or trifluoroethylthio,
m represents 0 or 1,
Q represents one of the groupings below 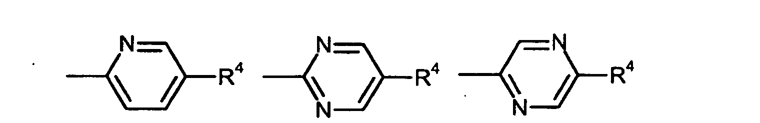 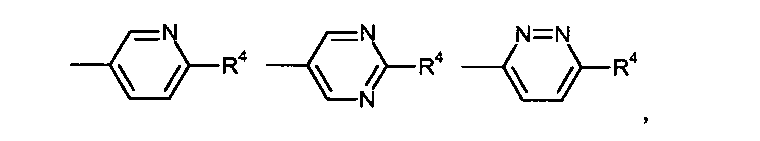
R⁴ represents hydrogen, fluorine, chlorine, bromine, cyano, formyl, represents C₁-C₁₆-alkyl, C₂-C₁₆-alkenyl, C₁-C₁₆-alkoxy, C₂-C₁₆-alkenyloxy, each of which is optionally mono- or polysubstituted by identical or different substituents from the group consisting of fluorine, chlorine, bromine, cyano and -NR⁷R⁸; represents -S(O)ₚR⁶, -NR⁷R⁸, -COR⁶, -CO₂R⁶, -CONR⁹R¹⁰, -N(R¹¹)COR¹²; or represents cyclopropyl, cyclopentyl, cyclohexyl, cyclopropylmethyl, cyclopentylmethyl, cyclohexylmethyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidino, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, piperidino, morpholinyl, thiomorpholinyl, morpholino, thiomorpholino, triazinyl, triazolyl, quinolinyl or isoquinolinyl, each of which is optionally mono- to trisubstituted by identical or different substituents from the group consisting of fluorine, chlorine, bromine, cyano, methyl, ethyl, n-propyl, isopropyl, -CF₃, -CHF₂, -CCIF₂, -CF₂CHFCl, -CF₂CH₂F, -CF₂CCl₃, -CH₂CF₃, -CF₂CHFCF₃, -CH₂CF₂H, -CH₂CF₂CF₃, -CF₂CF₂H, -CF₂CHFCF₃, vinyl, allyl, 1-propenyl, butenyl, -CF=CHF, -CF=CH₂, -CF=CCl₂, -CH=CF₂, -CF₂CF=CF₂, -CH=CFH, -CH₂CF=CF₂, -CF=CF₂, -CF₂CH=CF₂, methoxy, ethoxy, n-propoxy, isopropoxy, n-butoxy, isobutoxy, sec-butoxy, tert-butoxy, trifluoromethoxy, difluoromethoxy, chlorodifluoromethoxy, trifluoroethoxy, methylthio, ethylthio, n-propylthio, isopropylthio, n-butylthio, isobutylthio, sec-butylthio, tert-butylthio, trifluoromethylthio, difluoromethylthio, chlorodifluoromethylthio and trifluoroethylthio,
R⁴ furthermore represents -CH=NOH, formyl, cyclopropyloxy, cyclobutyloxy, cyclopentyloxy, cyclohexyloxy, cyclopropylmethoxy, cyclobutylmethoxy, cyclopentylmethoxy or cyclohexylmethoxy,
p represents 0, 1 or 2,
R⁶ represents methyl, ethyl, n-propyl, isopropyl, n-butyl, isobutyl, sec-butyl, tert-butyl, pentyl, hexyl, -CF₃, -CHF₂, -CCl₃, -CCl₂F, dimethylaminomethyl, dimethylaminoethyl, diethylaminomethyl, diethylaminoethyl, cyclopropyl, cyclopentyl, cyclohexyl, phenyl or benzyl,
R⁶ furthermore represents methoxymethyl, trifluoromethoxymethyl, methylthiomethyl or trifluoromethylthiomethyl,
R⁷ and R⁸ independently of one another represent hydrogen, -SO₂R⁶, -COR⁶, -CO₂R⁶, represent C₁-C₁₆-alkyl or C₂-C₁₆-alkenyl, each of which is optionally mono- or polysubstituted by identical or different substituents from the group consisting of fluorine, chlorine, bromine, C₁-C₄-alkylcarbonyl, C₁-C₄-alkylcarbonyloxy, C₁-C₄-alkylamino, di-(C₁-C₄-alkyl)amino, C₁-C₄-alkoxy, C₁-C₄-halogenoalkoxy, C₁-C₄-alkylthio or C₁-C₄-halogenoalkylthio; represent C₃-C₈-cycloalkyl, cyclopropylmethyl, cyclopentylmethyl, cyclohexylmethyl, cyclopropylethyl, cyclopentylethyl, cyclohexylethyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl, each of which is optionally mono- to trisubstituted by identical or different substituents from the group consisting of fluorine, chlorine, bromine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
R⁷ and R⁸ furthermore independently of one another represent pyridinylmethyl, pyrimidinylmethyl, pyridazylmethyl or pyrazinylmethyl, each of which is optionally mono- to trisubstituted by identical or different substituents from the group consisting of fluorine, chlorine, bromine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
R⁷ and R⁸ furthermore together represent C₂-C₈-alkenylene, which is optionally mono- or polysubstituted by identical or different substituents from the group consisting of fluorine, chlorine, bromine, C₁-C₄-alkoxy, C₁-C₄-halogenoalkoxy, C₁-C₄-alkylthio and C₁-C₄-halogenoalkylthio or represent C₃-C₈-alkylene, which is optionally mono- or polysubstituted in the alkylene moiety by identical or different substituents from the group consisting of fluorine, chlorine, cyano, methyl, ethyl, n-propyl, isopropyl, n-butyl, isobutyl, sec-butyl, tert-butyl, C₁-C₄-halogenoalkyl, methoxy, ethoxy, n-propoxy, isopropoxy, n-butoxy, isobutoxy, sec-butoxy, tert-butoxy, C₁-C₄-halogenoalkoxy, methylthio, ethylthio, n-propylthio, isopropylthio, n-butylthio, isobutylthio, sec-butylthio, tert-butylthio and C₁-C₄-halogenoalkylthio, where the alkylene chain may in each case be interrupted by -O-, -S- or -NR¹⁵-,
R⁷ and R⁸ furthermore together represent C₃-C₈-alkylene which is mono- or disubstituted by identical or different substituents, where the substituents may additionally be selected from the group consisting of ethoxycarbonyl, methoxycarbonyl and oxyethyleneoxy,
R⁷ and R⁸ furthermore together represent -CH₂-CH₂-C(=O)-CH₂-CH₂-, -CH₂-CH₂-C(=NO-Me)-CH₂-CH₂-, -CH₂-CH₂-C(=NO-Et)-CH₂-CH₂- or -CH₂-CH₂-C(=NO-iPr)-CH₂-CH₂-,
R⁹ and R¹⁰ independently of one another represent hydrogen, -SO₂CF₃, methyl, ethyl, n-propyl, isopropyl, n-butyl, isobutyl, sec-butyl, tert-butyl, pentyl, hexyl, -CF₃, -CH₂CF₃, -(CF₂)₃CF₃, cyclopropyl, cyclopentyl, cyclohexyl, methoxymethyl, methoxyethyl, or represent phenyl or benzyl, each of which is optionally substituted by fluorine, chlorine, bromine, methyl, trifluoromethyl, methoxy or trifluoromethoxy,
R⁹ and R¹⁰ furthermore together represent -(CH₂)₄-, -(CH₂)₅-, -(CH₂)₆-, -CH₂-CH(CH₃)-CH₂-CH(CH₃)-CH₂-, -(CH₂)₂-O-(CH₂)₂-, -(CH₂)₂-S-(CH₂)₂- or -(CH₂)₂-N(R¹⁵)-(CH₂)₂-,
R¹¹ and R¹² independently of one another represent methyl, ethyl, n-propyl, isopropyl, n-butyl, isobutyl, sec-butyl, tert-butyl, n-hexyl, trifluoromethyl, trifluoroethyl, cyclopropyl, cyclopentyl, cyclohexyl, or represent phenyl or benzyl, each of which is optionally substituted by fluorine, chlorine, bromine, methyl, trifluoromethyl, methoxy or trifluoromethoxy,
R¹¹ and R¹² furthermore together represent -(CH₂)₃-, -(CH₂)₄-, -(CH₂)₅- or -(CH₂)₆-, each of which is optionally substituted by fluorine, chlorine, bromine, methyl, ethyl, methoxy, ethoxy, methylthio, ethylthio, trifluoromethyl, trifluoromethoxy or trifluoromethylthio,
R¹⁵ represents hydrogen, -SO₂R⁶, represents -COR⁶ or -CO₂R⁶; represents C₁-C₁₆-alkyl, C₂-C₁₆-alkenyl, each of which is optionally mono- or polysubstituted by identical or different substituents from the group consisting of fluorine, chlorine, bromine, cyano, methylamino, ethylamino, di-(C₁-C₆-alkyl)amino, C₁-C₄-alkoxy, C₁-C₄-halogenoalkoxy, C₁-C₄-alkylthio and C₁-C₄-halogenoalkylthio; represents C₃-C₈-cycloalkyl, cyclopropylmethyl, cyclopentylmethyl, cyclohexylmethyl, cyclopropylethyl, cyclopentylethyl, cyclohexylethyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benz-oxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl, each of which is optionally mono- to trisubstituted by identical or different substituents from the group consisting of fluorine, chlorine, bromine, cyano, methyl, ethyl, n-propyl, isopropyl, n-butyl, isobutyl, sec-butyl, tert-butyl, C₁-C₄-halogenoalkyl, methoxy, ethoxy, n-propoxy, isopropoxy, n-butoxy, isobutoxy, sec-butoxy, tert-butoxy, C₁-C₄-halogenoalkoxy, methylthio, ethylthio, n-propylthio, isopropylthio, n-butylthio, isobutylthio, sec-butylthio, tert-butylthio and C₁-C₄-halogenoalkylthio.

8. Δ¹-Pyrrolines of the formula (I) according to Claim 1, in which
R⁴ represents hydrogen, fluorine, chlorine, bromine, cyano, formyl, represents C₁-C₁₆-alkyl, C₂-C₁₆-alkenyl, C₁-C₁₆-alkoxy, C₂-C₁₆-alkenyloxy, each of which is optionally mono- or polysubstituted by identical or different substituents from the group consisting of fluorine, chlorine, bromine, cyano and -NR⁷R⁸; represents -S(O)ₚR⁶, -NR⁷R⁸, -COR⁶, -CO₂R⁶, -CONR⁹R¹⁰, -N(R¹¹)COR¹²; or represents cyclopropyl, cyclopentyl, cyclohexyl, cyclopropylmethyl, cyclopentylmethyl, cyclohexylmethyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidino, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, piperidino, morpholinyl, thiomorpholinyl, morpholino, thiomorpholino, triazinyl, triazolyl, quinolinyl or isoquinolinyl, each of which is optionally mono- to trisubstituted by identical or different substituents from the group consisting of fluorine, chlorine, bromine, cyano, methyl, ethyl, n-propyl, isopropyl, -CF₃, -CHF₂, -CCIF₂, -CF₂CHFCl, -CF₂CH₂F, -CF₂CCl₃, -CH₂CF₃, -CF₂CHFCF₃, -CH₂CF₂H, -CH₂CF₂CF₃, -CF₂CF₂H, -CF₂CHFCF₃, vinyl, allyl, 1-propenyl, butenyl, -CF=CHF, -CF=CH₂, -CF=CCl₂, -CH=CF₂, -CF₂CF=CF₂, -CH=CFH, -CH₂CF=CF₂, -CF=CF₂, -CF₂CH=CF₂, methoxy, ethoxy, n-propoxy, isopropoxy, n-butoxy, isobutoxy, sec-butoxy, tert-butoxy, trifluoromethoxy, difluoromethoxy, chlorodifluoromethoxy, trifluoroethoxy, methylthio, ethylthio, n-propylthio, isopropylthio, n-butylthio, isobutylthio, sec-butylthio, tert-butylthio, trifluoromethylthio, difluoromethylthio, chlorodifluoromethylthio and trifluoroethylthio,
R⁶ represents methyl, ethyl, n-propyl, isopropyl, n-butyl, isobutyl, sec-butyl, tert-butyl, pentyl, hexyl, -CF₃, -CHF₂, -CCl₃, -CCl₂F, dimethylaminomethyl, dimethylaminoethyl, diethylaminomethyl, diethylaminoethyl, cyclopropyl, cyclopentyl, cyclohexyl, phenyl or benzyl,
R⁷ and R⁸ independently of one another represent hydrogen, -SO₂R⁶, -COR⁶, -CO₂R⁶, represent C₁-C₁₆-alkyl, C₂-C₁₆-alkenyl, each of which is optionally mono- or polysubstituted by identical or different substituents from the group consisting of fluorine, chlorine, bromine, C₁-C₄-alkylcarbonyl, C₁-C₄-alkylcarbonyloxy, C₁-C₄-alkylamino, di-(C₁-C₄-alkyl)amino, C₁-C₄-alkoxy, C₁-C₄-halogenoalkoxy, C₁-C₄-alkylthio and C₁-C₄-halogenoalkylthio; represent C₃-C₈-cycloalkyl, cyclopropylmethyl, cyclopentylmethyl, cyclohexylmethyl, cyclopropylethyl, cyclopentylethyl, cyclohexylethyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl, each of which is optionally mono- to trisubstituted by identical or different substituents from the group consisting of fluorine, chlorine, bromine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
R⁷ and R⁸ furthermore together represent C₂-C₈-alkenylene which is optionally mono- or polysubstituted by identical or different substituents from the group consisting of fluorine, chlorine, bromine, C₁-C₄-alkoxy, C₁-C₄-halogenoalkoxy, C₁-C₄-alkylthio and C₁-C₄-halogenoalkylthio, or represent C₃-C₈-alkylene which is optionally mono- or polysubstituted in the alkylene moiety by identical or different substituents from the group consisting of fluorine, chlorine, cyano, methyl, ethyl, n-propyl, isopropyl, n-butyl, isobutyl, sec-butyl, tert-butyl, C₁-C₄-halogenoalkyl, methoxy, ethoxy, n-propoxy, isopropoxy, n-butoxy, isobutoxy, sec-butoxy, tert-butoxy, C₁-C₄-halogenoalkoxy, methylthio, ethylthio, n-propylthio, isopropylthio, n-butylthio, isobutylthio, sec-butylthio, tert-butylthio and C₁-C₄-halogenoalkylthio, where the alkylene chain may in each case be interrupted by -O-, -S- or -NR¹⁵-,
and R¹, R², R³, m, Q, p, R⁹, R¹⁰, R¹¹, R¹² and R¹⁵ have the meanings given in Claim 7.

9. Δ¹-Pyrrolines of the formula (I) according to any of Claims 1 to 8, in which R¹ and R² each represent fluorine.

10. Δ¹-Pyrrolines of the formula (I) according to any of Claims 1 to 9, in which Q represents

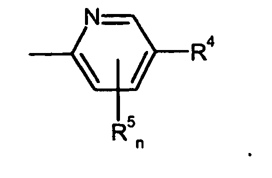

11. Δ¹-Pyrrolines of the formula (I) according to any of Claims 1 to 9, in which Q represents

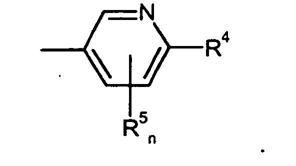

12. Δ¹-Pyrrolines of the formula (I) according to any of Claims 1 to 9, in which Q represents

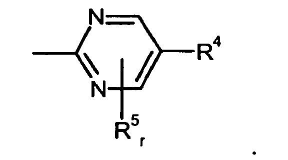

13. Δ¹-Pyrrolines of the formula (I) according to any of Claims 1 to 9, in which Q represents

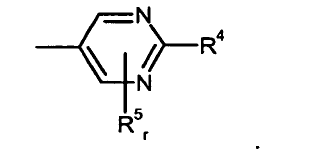

14. Δ¹-Pyrrolines of the formula (I) according to any of Claims 1 to 9, in which Q represents

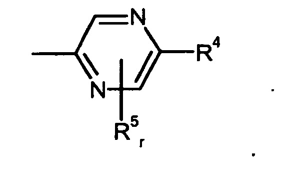

15. Δ¹-Pyrrolines of the formula (I) according to any of Claims 1 to 9, in which Q represents

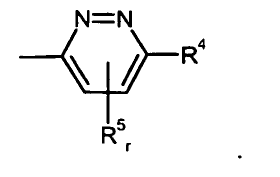

16. Compounds of the formulae (I-a) to (I-f) 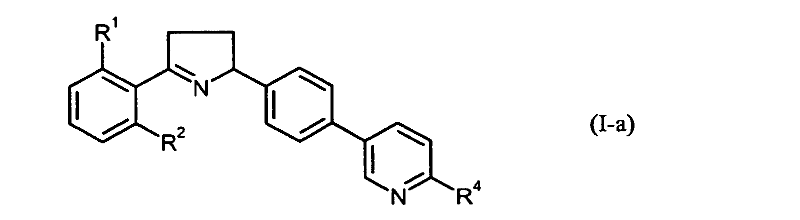 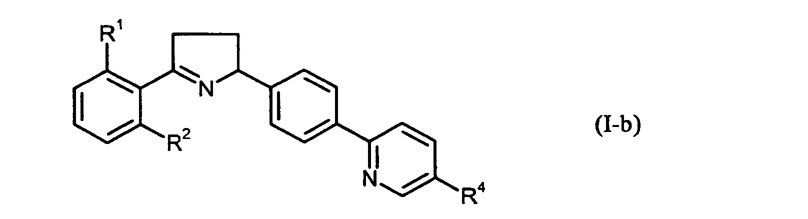 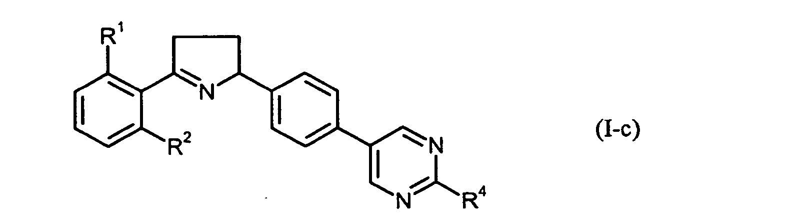 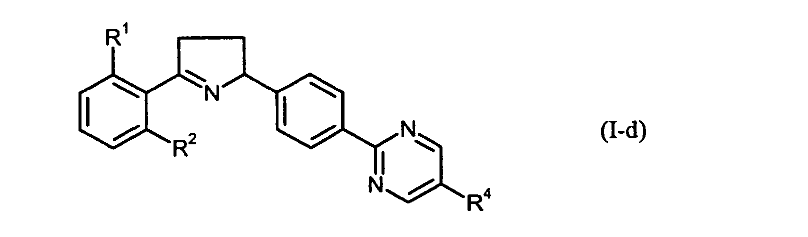 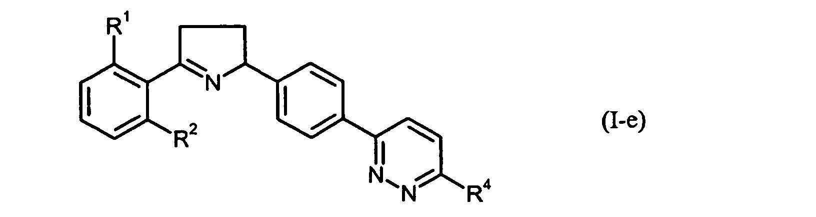 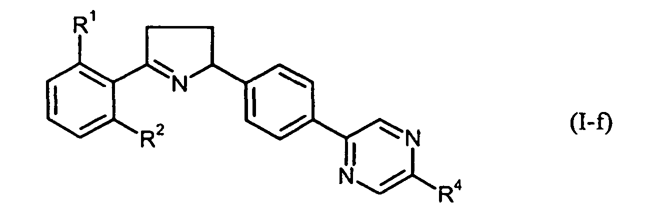 in which in each case
R¹ represents fluorine or chlorine,
R² represents hydrogen or fluorine and
R⁴ has the meanings given in any of Claims 1 to 8.

17. (R)-Configured compounds of the formulae (I-g) to (I-1) 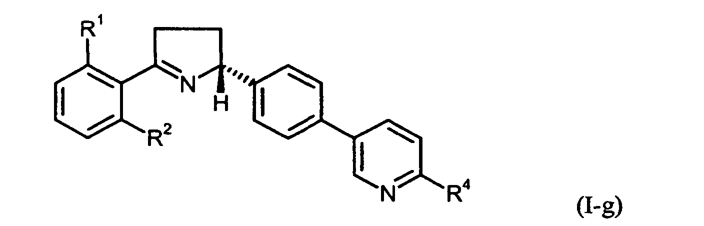 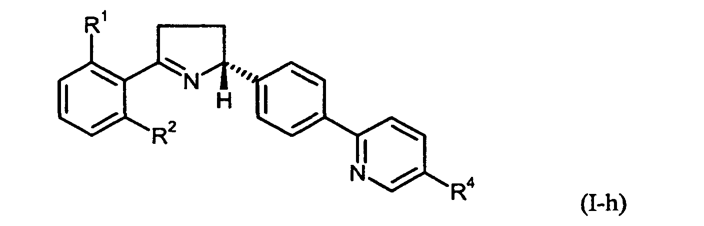 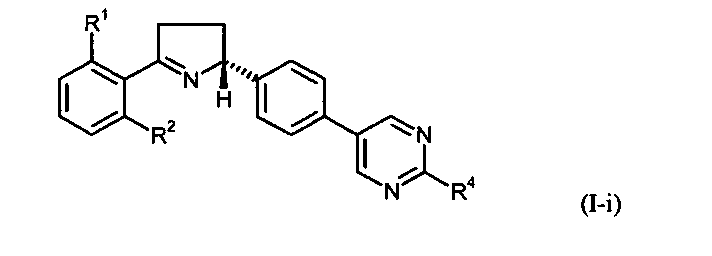 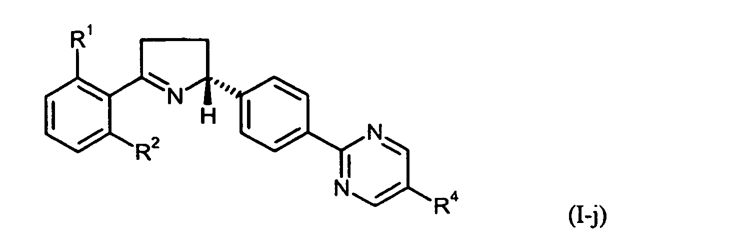 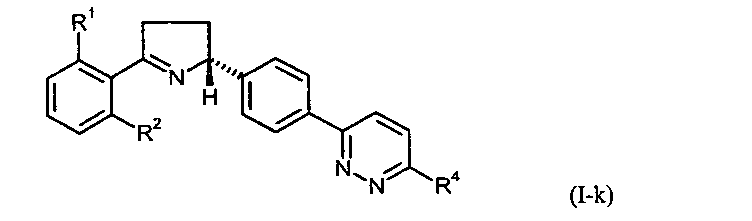 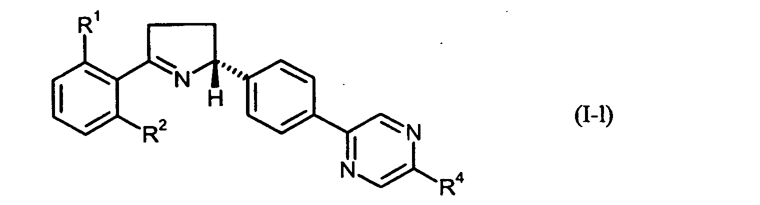 in which in each case
R¹ represents fluorine or chlorine,
R² represents hydrogen or fluorine and
R⁴ has the meanings given in any of Claims 1 to 8.

18. Process for preparing compounds of the formula (I) according to Claim 1, **characterized in that**
A) Δ¹-pyrrolines of the formula (II) 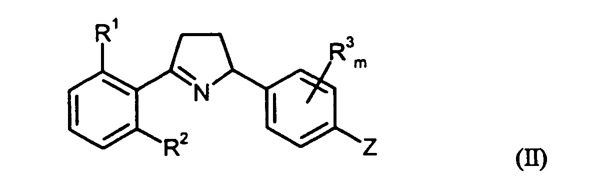 in which
R¹, R², R³ and m have the meanings given in Claim 1 and
Z represents chlorine, bromine, iodine, -OSO₂CF₃ or -OSO₂(CF₂)₃CF₃,
are reacted in a tandem reaction with heterocycles of the formula (III)
Q-X (III)
in which
Q has the meaning given in Claim 1 and
X represents chlorine, bromine, iodine, -OSO₂CF₃ or -OSO₂(CF₂)₃CF₃,
in the presence of a catalyst, in the presence of a diboronic acid ester and, if appropriate, in the presence of an acid binder and, if appropriate, in the presence of a diluent,
or
B) Δ¹-pyrrolines of the formula (IV) 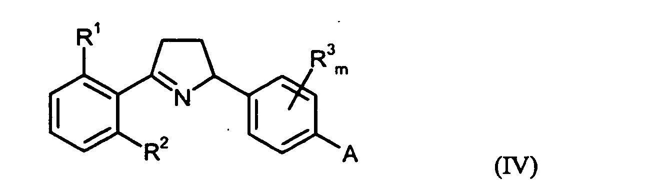 in which
R¹, R², R³ and m have the meanings given in Claim 1 and
A represents -B(OH)₂, (4,4,5,5-tetramethyl-1,3,2-dioxaborolan)-2-yl, (5,5-dimethyl-1,3,2-dioxaborinan)-2-yl, (4,4,6-trimethyl-1,3,2-dioxaborinan)-2-yl or 1,3,2-benzodioxaborol-2-yl
are reacted with heterocycles of the formula (III)
Q-X (III)
in which
Q and X have the meanings given in Claim 1,
in the presence of a catalyst, if appropriate in the presence of an acid binder and, if appropriate, in the presence of a diluent,
or
C) Δ¹-pyrrolines of the formula (II) 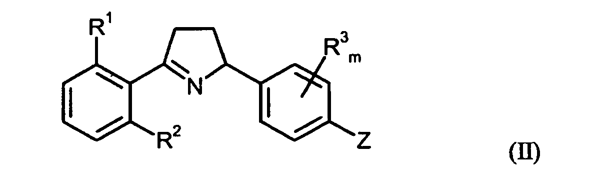 in which
R¹, R², R³, m and Z have the meanings given in Claim 1
are reacted with boronic acid derivatives of the formula (V)
Q-A (V)
in which
Q and A have the meanings given in Claim 1,
in the presence of a catalyst, if appropriate in the presence of an acid binder and, if appropriate, in th epresence of a diluent,
or
D) Δ¹-pyrrolines of the formula (II-a) 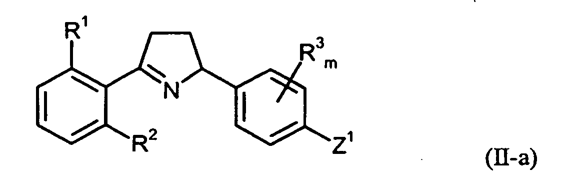
in which
R¹, R², R³ and m have the meanings given in Claim 1 and
Z¹ represents bromine or iodine
are reacted with organometallic compounds of the formula (VI)
Q-M (VI)
in which
Q has the meanings given in Claim 1 and
M represents ZnCl, Sn(Me)₃ or Sn(n-Bu)₃,
in the presence of a catalyst, if appropriate in the presence of an acid binder and, if appropriate, in the presence of a diluent.

19. Pesticides, **characterized in that** they comprise at least one compound of the formula (I) according to Claim 1, in addition to extenders and surfactants.

20. Use of compounds of the formula (I) according to Claim 1 for controlling pests.

21. Method for controlling pests, **characterized in that** compounds of the formula (I) according to Claim 1 are allowed to act on pests and/or their habitat.

22. Process for preparing pesticides, **characterized in that** compounds of the formula (I) according to Claim 1 are mixed with extenders and/or surfactants.

## Revendications

1. Δ¹-pyrrolines de formule (I) 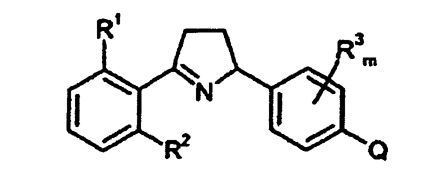 dans laquelle
R¹ est un halogène ou un reste méthyle,
R² est l'hydrogène ou un halogène,
R³ est un halogène, un reste alkyle, alkoxy ou alkylthio, chacun éventuellement substitué par un halogène,
m a la valeur 0, 1, 2, 3 ou 4,
Q représente l'un des groupements suivants 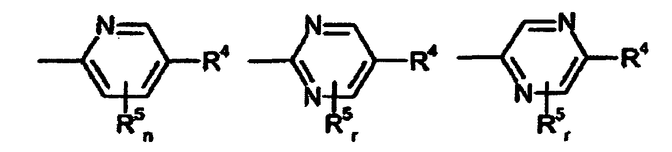 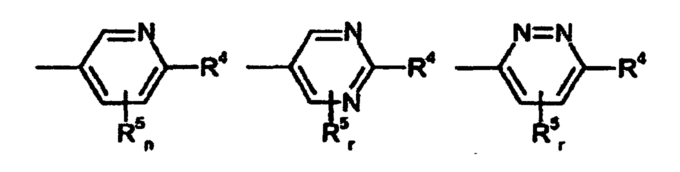
R⁴ représente l'hydrogène, un halogène, un groupe cyano, formyle, nitro, un reste trialkylsilyle ; un reste alkyle, alcényle, alkoxy, alcényloxy, chacun éventuellement substitué une ou plusieurs fois identiques ou différentes par un radical halogéno, cyano, et/ou -NR⁷R⁸ ; un reste pentafluorothio, -S(O)ₚR⁶, -NR⁷R⁸, -COR⁶, -CO₂R⁶, -CONR⁹R¹⁰, -N(R¹¹)COR¹² ou -C(R¹³)=N-OR¹⁴ ;
ou un reste cycloalkyle, cycloalkylalkyle, aryle, arylalkyle, hétérocyclyle pentagonal à décagonal saturé ou non saturé ou hétérocyclylalkyle, chacun éventuellement substitué une ou plusieurs fois identiques ou différentes par un radical halogéno, cyano, nitro, alkyle, halogénalkyle, alcényle, halogénalcényle, alkoxy, halogénalkoxy, alkylthio et/ou halogénalkyl-thio,
R⁴ représente en outre un groupe -CH=NOH, formyle ; un reste cycloalkyloxy ou cycloalkylalkoxy, chacun éventuellement substitué une ou plusieurs fois identiques ou différentes par un radical halogéno et/ou alkyle,
R⁵ représente un halogène, un groupe cyano, formyle, nitro, un reste trialkylsilyle ; un reste alkyle, alcényle, alkoxy, alcényloxy, chacun éventuellement substitué une ou plusieurs fois identiques ou différentes par un radical halogéno, cyano, et/ou -NR⁷R⁸ ; un reste pentafluorothio, -S(O)ₚR⁶, -NR⁷R⁸, -COR⁶, -CO₂R⁶, -CONR⁹R¹⁰, -N(R¹¹)COR¹² ou -C(R¹³)=N-OR¹⁴ ; ou un reste cycloalkyle, cycloalkylalkyle, aryle, arylalkyle, hétérocyclyle pentagonal à décagonal saturé ou non saturé ou hétérocyclylalkyle, chacun éventuellement substitué une ou plusieurs fois identiques ou différentes par un radical halogéno, alkyle, halogénalkyle, alcényle, halogénalcényle, alkoxy, halogénalkoxy, alkylthio et/ou halogénalkylthio,
p a la valeur 0, 1 ou 2,
n a la valeur 0, 1, 2 ou 3, les substituants R⁵ pouvant être identiques ou différents lorsque n a la valeur 2 ou 3,
r a la valeur 0, 1 ou 2, les substituants R⁵ pouvant être identiques ou différents lorsque r est égal à 2,
R⁶ représente un reste alkyle éventuellement substitué une ou plusieurs fois identiques ou différentes par un radical halogéno et/ou -NR⁷R⁸, un reste cycloalkyle, aryle ou arylalkyle, chacun éventuellement substitué une ou plusieurs fois identiques ou différentes par un radical halogéno, cyano, alkyle, halogénalkyle, alkoxy, halogénalkoxy, alkylthio et/ou halogénalkylthio,
R⁶ représente en outre un reste alkyle substitué une ou plusieurs fois identiques ou différentes, les substituants pouvant en outre être choisis entre alkoxy, alkylthio, halogénalkoxy et/ou halogénalkylthio,
R⁷ et R⁸ représentent, indépendamment l'un de l'autre, l'hydrogène, un groupe -SO₂R⁶, -COR⁶, -CO₂R⁶, un reste alkyle ou alcényle chacun éventuellement substitué une ou plusieurs fois identiques ou différentes par un radical halogéno, alkylcarbonyle, alkylcarbonyloxy, alkylamino, dialkylamino, alkoxy, halogénalkoxy, alkylthio et/ou halogénalkylthio ; un reste cycloalkyle, cycloalkylalkyle, aryle, arylalkyle, hétérocyclyle pentagonal à décagonal saturé ou non saturé ou hétérocyclylalkyle chacun éventuellement substitué une ou plusieurs fois identiques ou différentes par un radical halogéno, cyano, alkyle, halogénalkyle, alkoxy, halogénalkoxy, alkylthio et/ou halogénalkylthio,
R⁷ et R⁸ représentent en outre conjointement un reste alcénylène éventuellement substitué une ou plusieurs fois identiques ou différentes par un radical halogéno, cyano, alkoxy, halogénalkoxy, alkylthio et/ou halogénalkylthio ou un reste alkylène éventuellement substitué une ou plusieurs fois identiques ou différentes par un radical halogéno, cyano, alkyle, halogénalkyle, alkoxy, halogénalkoxy, alkylthio, et/ou halogénalkylthio, la chaîne alkylénique pouvant être interrompue dans chaque cas par -O-, -S- ou -NR¹⁵-,
R⁷ et R⁸ représentent en outre conjointement un reste alkylène substitué une ou plusieurs fois identiques ou différentes, les substituants pouvant être choisis en outre entre alkoxycarbonyle et/ou oxyalkylénoxy,
R⁷ et R⁸ représentent en outre conjointement un reste alkylène, la chaîne alkylénique étant interrompue soit par C=O, soit par C=NO-alkyle,
R⁹ et R¹⁰ représentent indépendamment l'un de l'autre l'hydrogène, un groupe -SO₂R⁶, un reste alkyle ou alcényle, chacun éventuellement substitué une ou plusieurs fois identiques ou différentes par un radical halogéno, alkylamino, dialkylamino, alkoxy et/ou alkylthio ; un reste cycloalkyle, cycloalkylalkyle, aryle, arylalkyle, hétérocyclyle pentagonal à décagonal saturé ou non saturé ou hétérocyclylalkyle, chacun éventuellement substitué une ou plusieurs fois identiques ou différentes par un radical halogéno, cyano, alkyle, halogénalkyle, alkoxy, halogénalkoxy, alkythio et/ou halogénalkylthio,
R⁹ et R¹⁰ représentent en outre conjointement un reste alkylène éventuellement substitué une ou plusieurs fois identiques ou différentes par un radical halogéno, cyano, alkyle, halogénalkyle, alkoxy, halogénalkoxy, alkylthio et/ou halogénalkylthio, la chaîne alkylénique pouvant être interrompue dans chaque cas par -O-, -S- ou -NR¹⁵-,
R¹¹ et R¹² représentent indépendamment l'un de l'autre, l'hydrogène, un reste alkyle éventuellement substitué une ou plusieurs fois identiques ou différentes par un radical halogéno, cyano, alkoxy et/ou alkylthio, un reste cycloalkyle, cycloalkylalkyle, aryle ou arylalkyle, chacun éventuellement substitué une ou plusieurs fois identiques ou différentes par un radical halogéno, cyano, alkyle, halogénalkyle, alkoxy, halogénalkoxy, alkylthio et/ou halogénalkylthio,
R¹¹ et R¹² représentent en outre conjointement un reste alkylène ou alcénylène, chacun éventuellement substitué une ou plusieurs fois identiques ou différentes par un radical halogéno, alkyle, halogénalkyle, alkoxy, halogénalkoxy, alkylthio et/ou halogénalkylthio,
R¹³ et R¹⁴ représentent indépendamment l'un de l'autre, l'hydrogène, un reste alkyle ou alcényle, chacun éventuellement substitué une ou plusieurs fois par un halogène,
R¹⁵ représente l'hydrogène, un groupe -SO₂R⁶, -COR⁶ ou -CO₂R⁶ ; un reste alkyle ou alcényle, chacun éventuellement substitué une ou plusieurs fois identiques ou différentes par un radical halogéno, cyano, alkylamino, dialkylamino, alkoxy, halogénalkoxy, alkylthio et/ou halogénalkylthio ; un reste cycloalkyle, cycloalkylalkyle, aryle, arylalkyle, hétérocyclyle pentagonal à décagonal saturé ou non saturé ou hétérocyclylalkyle, chacun éventuellement substitué une ou plusieurs fois identiques ou différentes par un radical halogéno, cyano, alkyle, halogénalkyle, alkoxy, halogénalkoxy, alkylthio et/ou halogénalkylthio.

2. Δ¹-pyrrolines de formule (I) suivant la revendication 1, formule dans laquelle
R⁴ représente l'hydrogène, un halogène, un groupe cyano, formyle, nitro, un reste trialkylsilyle ; un reste alkyle, alcényle, alkoxy, alcényloxy, chacun éventuellement substitué une ou plusieurs fois identiques ou différentes par un radical halogéno, cyano, et/ou -NR⁷R⁸ ; un reste pentafluorothio, -S(O)ₚR⁶, -NR⁷R⁸, -COR⁶, -CO₂R⁶, -CONR⁹R¹⁰, -N(R¹¹)COR¹² ou -C(R¹³)=N-OR¹⁴ ; ou un reste cycloalkyle, cycloalkylalkyle, aryle, arylalkyle, hétérocyclyle pentagonal à décagonal saturé ou non saturé ou hétérocyclylalkyle, chacun éventuellement substitué une ou plusieurs fois identiques ou différentes par un radical halogéno, cyano, nitro, alkyle, halogénalkyle, alcényle, halogénalcényle, alkoxy, halogénalkoxy, alkylthio et/ou halogénalkylthio,
R⁶ est un reste alkyle éventuellement substitué une ou plusieurs fois identiques ou différentes par un radical halogéno et/ou -NR⁷R⁸, un reste cycloalkyle, aryle ou arylalkyle, chacun éventuellement substitué une ou plusieurs fois identiques ou différentes par un radical halogéno, cyano, alkyle, halogénalkyle, alkoxy, halogénalkoxy, alkylthio et/ou halogénalkylthio,
R⁷ et R⁸ représentent, indépendamment l'un de l'autre, l'hydrogène, un groupe -SO₂R⁶, -COR⁶, -CO₂R⁶, un reste alkyle ou alcényle chacun éventuellement substitué une ou plusieurs fois identiques ou différentes par un radical halogéno, alkylcarbonyle, alkylcarbonyloxy, alkylamino, dialkylamino, alkoxy, halogénalkoxy, alkylthio et/ou halogénalkylthio ; un reste cycloalkyle, cycloalkylalkyle, aryle, arylalkyle, hétérocyclyle pentagonal à décagonal saturé ou non saturé ou hétérocyclylalkyle chacun éventuellement substitué une ou plusieurs fois identiques ou différentes par un radical halogéno, cyano, alkyle, halogénalkyle, alkoxy, halogénalkoxy, alkylthio et/ou halogénalkylthio,
R⁷ et R⁸ représentent en outre conjointement un reste alcénylène éventuellement substitué une ou plusieurs fois identiques ou différentes par un radical halogéno, cyano, alkoxy, halogénalkoxy, alkylthio et/ou halogénalkylthio ou un reste alkylène éventuellement substitué une ou plusieurs fois identiques ou différentes par un radical halogéno, cyano, alkyle, halogénalkyle, alkoxy, halogénalkoxy, alkylthio, et/ou halogénalkylthio, la chaîne alkylénique pouvant être interrompue dans chaque cas par -O-, -S- ou -NR¹⁵-,
et R¹, R², R³, m, Q, R⁵, p, n, r, R⁹, R¹⁰, R¹¹, R¹², R¹³, R¹⁴ et R¹⁵ ont les définitions indiquées dans la revendication 1.

3. Δ¹-pyrrolines de formule (I) suivant la revendication 1, formule dans laquelle
R¹ représente le fluor, le chlore, le brome ou le reste méthyle,
R² représente l'hydrogène, le fluor, le chlore ou le brome,
R³ représente le fluor, le chlore, le brome, un reste alkyle en C₁ à C₆, alkoxy en C₁ à C₆ ou alkylthio en C₁ à C₆, chacun éventuellement substitué une à huit fois par un halogène,
m a la valeur 0, 1, 2 ou 3,
Q représente l'un des groupements 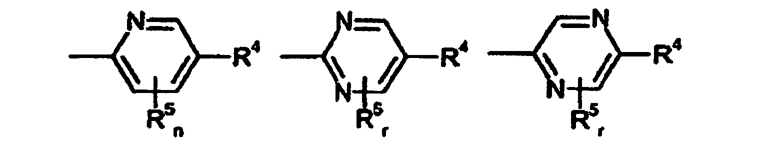 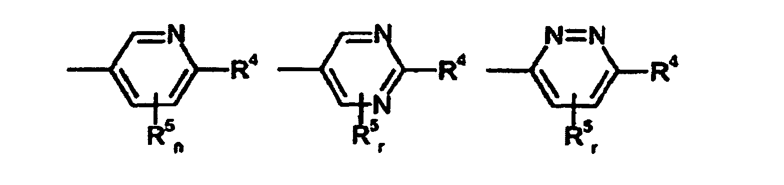
R⁴ représente l'hydrogène, le fluor, le chlore, le brome, un groupe cyano, formyle, nitro, un reste tri-(alkyle en C₁ à C₆)-silyle ; un reste alkyle en C₁ à C₂₀, alcényle en C₂ à C₂₀, alkoxy en C₁ à C₂₀, alcényloxy en C₂ à C₂₀, chacun éventuellement substitué une ou plusieurs fois identiques ou différentes par un radical halogéno, cyano, -NR⁷R⁸ ; un groupe pentafluorothio, -S(O)ₚR⁶, -NR⁷R⁸, -COR⁶, -CO₂R⁶, -CONR⁹R¹⁰, -N(R¹¹)COR¹² ou -C(R¹³)=N-OR¹⁴ ; ou un reste cycloalkyle en C₃ à C₁₂, (cycloalkyle en C₃ à C₇)-(alkyle en C₁ à C₄), aryle, aryl- (alkyle en C₁ à C₄), hétérocyclyle pentagonal à décagonal saturé ou non saturé ou hétérocyclyl-(alkyle en C₁ à C₄) avec 1 à 4 hétéroatomes, contenant 0 à 4 atomes d'azote, 0 à 2 atomes d'oxygène non contigus et/ou 0 à 2 atomes de soufre non contigus (en particulier tétrazolyle, furyle, furfuryle, benzofuryle, tétrahydrofuryle, thiényle, thényle, benzothiényle, thiolanyle, pyrrolyle, indolyle, pyrrolinyle, pyrrolidino, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, pipéridino, morpholinyle, thiomorpholinyle, morpholino, thiomorpholino, triazinyle, triazolyle, quinolinyle ou isoquinolinyle), chacun éventuellement substitué jusqu'à quatre fois identiques ou différentes par un radical halogéno, cyano, nitro, alkyle en C₁ à C₆, halogénalkyle en C₁ à C₆, alcényle en C₂ à C₆, halogénalcényle en C₂ à C₆, alkoxy en C₁ à C₆, halogénalkoxy en C₁ à C₆, alkylthio en C₁ à C₆, halogénalkylthio en C₁ à C₆,
R⁴ représente en outre un groupe -CH=NOH, formyle ; un reste cycloalkyloxy en C₃ à C₆, (cycloalkyle en C₃ à C₆)-(alkoxy en C₁ à C₄), chacun éventuellement substitué une à quatre fois identiques ou différentes par un radical fluoro, chloro, bromo et/ou alkyle en C₁ ou C₂,
R⁵ représente le fluor, le chlore, le brome, un groupe cyano, formyle, nitro, un reste tri-(alkyle en C₁ à C₆)-silyle ; un reste alkyle en C₁ à C₆, alcényle en C₂ à C₆, alkoxy en C₁ à C₆, alcényloxy en C₂ à C₆, chacun éventuellement substitué une à treize fois par un radical halogéno ; un groupe pentafluorothio, -S(O)ₚR⁶, -NR⁷R⁸, -COR⁶, -CO₂R⁶, -CONR⁹R¹⁰ ou -N(R¹¹)COR¹²,
p a la valeur 0, 1 ou 2,
n a la valeur 0, 1, 2 ou 3, les substituants R⁵ pouvant être identiques ou différents lorsque n a la valeur 2 ou 3,
r a la valeur 0, 1 ou 2, les substituants R⁵ pouvant être identiques ou différents lorsque r est égal à 2,
R⁶ est un reste alkyle en C₁ à C₂₀ éventuellement substitué une ou plusieurs fois identiques ou différentes par un radical halogéno ou -NR⁷R⁸, un reste cycloalkyle en C₃ à C₆, aryle ou aryl-(alkyle en C₁ à C₄), chacun éventuellement substitué une à huit fois identiques ou différentes par un radical halogéno, cyano, alkyle en C₁ à C₆, halogénalkyle en C₁ à C₆, alkoxy en C₁ à C₆, halogénalkoxy en C₁ à C₆, alkylthio en C₁ à C₆, halogénalkylthio en C₁ à C₆,
R⁶ représente en outre un reste alkyle en C₁ à C₂₀ substitué une ou plusieurs fois identiques ou différentes, les substituants pouvant en outre être choisis entre alkoxy en C₁ à C₆, alkylthio en C₁ à C₆, halogénalkoxy en C₁ à C₆ et/ou halogénalkylthio en C₁ à C₆,
R⁷ et R⁸ représentent, indépendamment l'un de l'autre, l'hydrogène, un groupe -SO₂R⁶, -COR⁶, -CO₂R⁶, un reste alkyle en C₁ à C₂₀ ou alcényle en C₂ à C₂₀, chacun éventuellement substitué une ou plusieurs fois identiques ou différentes par un radical halogéno, (alkyle en C₁ à C₆)-carbonyle, (alkyle en C₁ à C₆)-carbonyloxy, alkylamino en C₁ à C₆, di-(alkyle en C₁ à C₆)-amino, alkoxy en C₁ à C₆, halogénalkoxy en C₁ à C₆, alkylthio en C₁ à C₆, halogénalkylthio en C₁ à C₆ ; un reste cycloalkyle en C₃ à C₁₂, (cycloalkyle en C₃ à C₇)-(alkyle en C₁ à C₄), aryle, aryl-(alkyle en C₁ à C₄), hétérocyclyle pentagonal à décagonal saturé ou non saturé ou hétérocyclyl- (alkyle en C₁ à C₄) avec 1 à 4 hétéroatomes qui comprennent 0 à 4 atomes d'azote, 0 à 2 atomes d'oxygène non contigus et/ou 0 à 2 atomes de soufre non contigus (en particulier tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle), chacun éventuellement substitué une à quatre fois identiques ou différentes par un radical halogéno, cyano, alkyle en C₁ à C₆, halogénalkyle en C₁ à C₆, alkoxy en C₁ à C₆, halogénalkoxy en C₁ à C₆, alkylthio en C₁ à C₆, halogénalkylthio en C₁ à C₆,
R⁷ et R⁸ représentent en outre, indépendamment l'un de l'autre, un reste hétérocyclyl-(alkyle en C₁ à C₄) avec 1 à 4 hétéroatomes qui comprennent 0 à 4 atomes d'azote, 0 à 2 atomes d'oxygène non contigus et/ou 0 à 2 atomes de soufre non contigus (en particulier pyridinylméthyle, pyridinyléthyle, pyrimidinylméthyle, pyrimidinyléthyle, pyridazylméthyle, pyridazyléthyle, pyrazinylméthyle ou pyrazinyléthyle), chacun éventuellement substitué une à quatre fois identiques ou différentes par un radical halogéno, cyano, alkyle en C₁ à C₆, halogénalkyle en C₁ à C₆, alkoxy en C₁ à C₆, halogénalkoxy en C₁ à C₆, alkylthio en C₁ à C₆, halogénalkylthio en C₁ à C₆,
R⁷ et R⁸ représentent en outre conjointement un reste alcénylène en C₂ à C₁₂ éventuellement substitué une ou plusieurs fois identiques ou différentes par un radical halogéno, cyano, alkoxy en C₁ à C₆, halogénalkoxy en C₁ à C₆, alkylthio en C₁ à C₆, halogénalkylthio en C₁ à C₆, ou un reste alkylène en C₃ à C₁₂ éventuellement substitué dans la partie alkylène une ou plusieurs fois identiques ou différentes par un radical halogéno, cyano, alkyle en C₁ à C₆, halogénalkyle en C₁ à C₆, alkoxy en C₁ à C₆, halogénalkoxy en C₁ à C₆, alkylthio en C₁ à C₆, halogénalkylthio en C₁ à C₆, la chaîne alkylénique pouvant être interrompue dans chaque cas par -O-, -S- ou -NR¹⁵-,
R⁷ et R⁸ représentent en outre conjointement un reste alkylène en C₃ à C₁₂ éventuellement substitué une ou plusieurs fois identiques ou différentes, les substituants pouvant être choisis en outre entre (alkoxy en C₁ à C₄)-carbonyle et/ou oxy-(alkylène en C₁ à C₄)-oxy,
R⁷ et R⁸ représentent en outre conjointement un reste alkylène en C₃ à C₈, la chaîne alkylénique étant interrompue par C=O ou par C=NO-(alkyle en C₁ à C₆),
R⁹ et R¹⁰ représentent indépendamment l'un de l'autre, l'hydrogène, un groupe -SO₂R⁶, un reste alkyle en C₁ à C₆ ou alcényle en C₂ à C₆, chacun éventuellement substitué une à treize fois identiques ou différentes par un radical halogéno, alkylamino en C₁ à C₆, di-(alkyle en C₁ à C₆)-amino, alkoxy en C₁ à C₆, halogénalkoxy en C₁ à C₆, alkylthio en Ci à C₆, halogénalkylthio en C₁ à C₆ ; un reste cycloalkyle en C₃ à C₇, (cycloalkyle en C₃ à C₇)-(alkyle en C₁ à C₄), aryle, aryl-(alkyle en C₁ à C₄), hétérocyclyle pentagonal à décagonal saturé ou non saturé ou hétérocyclyl-(alkyle en C₁ à C₄) avec 1 à 4 hétéroatomes, qui comprennent 0 à 4 atomes d'azote, 0 à 2 atomes d'oxygène non contigus et/ou 0 à 2 atomes de soufre non contigus (en particulier tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle), chacun éventuellement substitué une à quatre fois identiques ou différentes par un radical halogéno, cyano, alkyle en C₁ à C₆, halogénalkyle en C₁ à C₆, alkoxy en C₁ à C₆, halogénalkoxy en C₁ à C₆, alkylthio en C₁ à C₆, halogénalkylthio en C₁ à C₆,
R⁹ et R¹⁰ représentent en outre conjointement un reste alkylène en C₃ à C₆, -(CH₂)₂-O-(CH₂)₂-, -(CH₂)₂-S-(CH₂)₂- ou - (CH₂)₂-N(R¹⁵)-(CH₂)₂-, chacun éventuellement substitué dans la partie alkylène une à quatre fois identiques ou différentes par un radical halogéno, cyano, alkyle en C₁ à C₆, halogénalkyle en C₁ à C₆, alkoxy en C₁ à C₆, halogénalkoxy en C₁ à C₆, alkylthio en C₁ à C₆, halogénalkylthio en C₁ à C₆,
R¹¹ et R¹² représentent indépendamment l'un de l'autre l'hydrogène, un reste alkyle en C₁ à C₆ éventuellement substitué une à treize fois identiques ou différentes par un radical halogéno, cyano, alkoxy en C₁ à C₆, alkylthio en C₁ à C₆, un reste cycloalkyle en C₃ à C₇, (cycloalkyle en C₃ à C₇)-(alkyle en C₁ à C₄), aryle ou aryl-(alkyle en C₁ à C₄), chacun éventuellement substitué une à huit fois identiques ou différentes par un radical halogéno, cyano, alkyle en C₁ à C₆, halogénalkyle en C₁ à C₆, alkoxy en C₁ à C₆, halogénalkoxy en C₁ à C₆, alkylthio en C₁ à C₆, halogénalkylthio en C₁ à C₆,
R¹¹ et R¹² représentent en outre conjointement un reste alkylène en C₃ à C₁₀ ou alcénylène en C₃ à C₁₀, chacun éventuellement substitué une à huit fois identiques ou différentes par un radical halogéno, alkyle en C₁ à C₆, halogénalkyle en C₁ à C₆, alkoxy en C₁ à C₆, halogénalkoxy en C₁ à C₆, alkylthio en C₁ à C₆, halogénalkylthio en C₁ à C₆,
R¹³ et R¹⁴ représentent indépendamment l'un de l'autre l'hydrogène, un reste alkyle en C₁ à C₆ ou alcényle en C₂ à C₆, chacun éventuellement substitué une ou plusieurs fois par un halogène,
R¹⁵ représente l'hydrogène, un groupe -SO₂R⁶, -COR⁶ ou -CO₂R⁶ ; un reste alkyle en C₁ à C₂₀ ou alcényle en C₂ à C₂₀, chacun éventuellement substitué une ou plusieurs fois identiques ou différentes par un radical halogéno, cyano, alkylamino en C₁ à C₆, di-(alkyle en C₁ à C₆)-amino, alkoxy en C₁ à C₆, halogénalkoxy en C₁ à C₆, alkylthio en C₁ à C₆, halogénalkylthio en C₁ à C₆, un reste cycloalkyle en C₃ à C₁₂, (cycloalkyle en C₃ à C₇)-(alkyle en C₁ à C₄), aryle, aryl-(alkyle en C₁ à C₄), hétérocyclyle pentagonal à décagonal saturé ou non saturé ou hétérocyclyl-(alkyle en C₁ à C₄) avec 1 à 4 hétéroatomes qui comprennent 0 à 4 atomes d'azote, 0 à 2 atomes d'oxygène non contigus et/ou 0 à 2 atomes de soufre non contigus (en particulier tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle), chacun éventuellement substitué une à quatre fois identiques ou différentes par un radical halogéno, cyano, alkyle en C₁ à C₆, halogénalkyle en C₁ à C₆, alkoxy en C₁ à C₆, halogénalkoxy en C₁ à C₆, alkylthio en C₁ à C₆, halogénalkylthio en C₁ à C₆.

4. Δ¹-pyrroline de formule (I) suivant la revendication 1, formule dans laquelle
R⁴ représente l'hydrogène, le fluor, le chlore, le brome, un groupe cyano, formyle, nitro, un reste tri-(alkyle en C₁ à C₆)-silyle ; un reste alkyle en C₁ à C₂₀, alcényle en C₂ à C₂₀, alkoxy en C₁ à C₂₀, alcényloxy en C₂ à C₂₀, chacun éventuellement substitué une ou plusieurs fois identiques ou différentes par un radical halogéno, cyano, -NR⁷R⁸ ; un groupe pentafluorothio, -S(O)ₚR⁶, -NR⁷R⁸, -COR⁶, -CO₂R⁶, -CONR⁹R¹⁰, -N(R¹¹)COR¹² ou -C(R¹³)=N-OR¹⁴ ; ou un reste cycloalkyle en C₃ à C₁₂, (cycloalkyle en C₃ à C₇)-alkyle en C₁ à C₄), aryle, aryl-(alkyle en C₁ à C₄), hétérocyclyle pentagonal à décagonal saturé ou non saturé ou hétérocyclyl-(alkyle en C₁ à C₄) avec 1 à 4 hétéroatomes, contenant 0 à 4 atomes d'azote, 0 à 2 atomes d'oxygène non contigus et/ou 0 à 2 atomes de soufre non contigus (en particulier tétrazolyle, furyle, furfuryle, benzofuryle, tétrahydrofuryle, thiényle, thényle, benzothiényle, thiolanyle, pyrrolyle, indolyle, pyrrolinyle, pyrrolidino, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, pipéridino, morpholinyle, thiomorpholinyle, morpholino, thiomorpholino, triazinyle, triazolyle, quinolinyle ou isoquinolinyle), chacun éventuellement substitué une à quatre fois identiques ou différentes par un radical halogéno, cyano, nitro, alkyle en C₁ à C₆, halogénalkyle en C₁ à C₆, alcényle en C₂ à C₆, halogénalcényle en C₂ à C₆, alkoxy en C₁ à C₆, halogénalkoxy en C₁ à C₆, alkylthio en C₁ à C₆, halogénalkylthio en C₁ à C₆,
R⁶ est un reste alkyle en C₁ à C₂₀ éventuellement substitué une ou plusieurs fois identiques ou différentes par un radical halogéno ou -NR⁷R⁸, un reste cycloalkyle en C₃ à C₆, aryle ou aryl-(alkyle en C₁ à C₄), chacun éventuellement substitué une à huit fois identiques ou différentes par un radical halogéno, cyano, alkyle en C₁ à C₆, halogénalkyle en C₁ à C₆, alkoxy en C₁ à C₆, halogénalkoxy en C₁ à C₆, alkylthio en C₁ à C₆, halogénalkylthio en C₁ à C₆,
R⁷ et R⁸ représentent indépendamment l'un de l'autre l'hydrogène, un groupe -SO₂R₆, -COR⁶, -CO₂R⁶, un reste alkyle en C₁ à C₂₀ ou alcényle en C₂ à C₂₀, chacun éventuellement substitué une ou plusieurs fois identiques ou différentes par un radical halogéno, alkyle en C₁ à C₆, (alkyle en C₁ à C₆)-carbonyloxy, alkylamino en C₁ à C₆, di-(alkyle en C₁ à C₆)-amino, alkoxy en C₁ à C₆, halogénalkoxy en C₁ à C₆, alkylthio en C₁ à C₆, halogénalkylthio en C₁ à C₆ ; un reste cycloalkyle en C₃ à C₁₂, (cycloalkyle en C₃ à C₇)-(alkyle en C₁ à C₄), aryle, aryl- (alkyle en C₁ à C₄), hétérocyclyle pentagonal à décagonal saturé ou non saturé ou hétérocyclyl- (alkyle en C₁ à C₄) avec 1 à 4 hétéroatomes qui comprennent 0 à 4 atomes d'azote, 0 à 2 atomes d'oxygène contigus et/ou 0 à 2 atomes de soufre contigus (en particulier tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle), chacun éventuellement substitué une à quatre fois identiques ou différentes par un radical halogéno, cyano, alkyle en C₁ à C₆, halogénalkyle en C₁ à C₆, alkoxy en C₁ à C₆, halogénalkoxy en C₁ à C₆, alkylthio en C₁ à C₆, halogénalkylthio en C₁ à C₆,
R⁷ et R⁸ représentent en outre conjointement un reste alcénylène en C₂ à C₁₂ éventuellement substitué une ou plusieurs fois identiques ou différentes par un radical halogéno, cyano, alkoxy en C₁ à C₆, halogénalkoxy en C₁ à C₆, alkylthio en C₁ à C₆, halogénalkylthio en C₁ à C₆, ou un reste alkylène en C₃ à C₁₂ éventuellement substitué dans la partie alkylène une ou plusieurs fois identiques ou différentes par un radical halogéno, cyano, alkyle en C₁ à C₆, halogénalkyle en C₁ à C₆, alkoxy en C₁ à C₆, halogénalkoxy en C₁ à C₆, alkylthio en C₁ à C₆, halogénalkylthio en C₁ à C₆, la chaîne alkylénique pouvant être interrompue dans chaque cas par -O-, -S- ou -NR¹⁵-,
et R¹, R², R³, m, Q, R⁵, p, n, r, R⁹, R¹⁰, R¹¹, R¹², R¹³, R¹⁴ et R¹⁵ ont les définitions indiquées dans la revendication 3.

5. Δ¹-pyrrolines de formule (I) suivant la revendication 1, formule dans laquelle
R¹ représente le fluor, le chlore ou un reste méthyle,
R² représente l'hydrogène, le fluor ou le chlore,
R³ représente le fluor, le chlore, un reste alkyle en C₁ à C₄, alkoxy en C₁ à C₄, alkylthio en C₁ à C₄ ; un reste halogénalkyle en C₁ à C₄, halogénalkoxy en C₁ à C₄, halogénalkylthio en C₁ à C₄ ayant dans chaque cas 1 à 9 atomes de fluor, de chlore et/ou de brome,
m a la valeur 0, 1 ou 2,
Q représente l'un des groupements suivants : 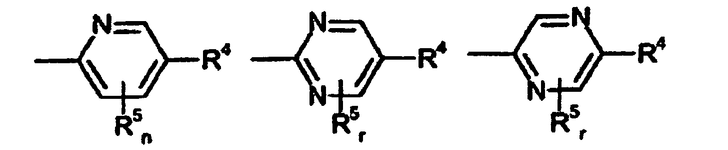 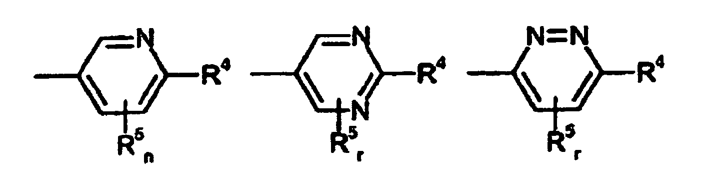
R⁴ représente l'hydrogène, le fluor, le chlore, le brome, un groupe cyano, formyle, nitro, un reste tri-(alkyle en C₁ à C₄)-silyle ; un reste alkyle en C₁ à C₁₆, alcényle en C₂ à C₁₆, alkoxy en C₁ à C₁₆, alcényloxy en C₂ à C₁₆, chacun éventuellement substitué une ou plusieurs fois identiques ou différentes par un radical fluoro, chloro, bromo, cyano, -NR⁷R⁸ ; un reste -S(O)ₚR⁶, -NR⁷R⁸, -COR⁶, -CO₂R⁶, -CONR⁹R¹⁰ ou -N(R¹¹)COR¹² ; ou un reste cycloalkyle en C₃ à C₁₀, (cycloalkyle en C₃ à C₆)-(alkyle en C₁ à C₄), phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidino, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, pipéridino, morpholinyle, thiomorpholinyle, morpholino, thiomorpholino, triazinyle, triazolyle, quinolinyle ou isoquinolinyle, chacun éventuellement substitué une à trois fois identiques ou différentes par un radical fluoro, chloro, bromo, cyano, alkyle en C₁ à C₄, halogénalkyle en C₁ à C₄, alcényle en C₂ à C₆, halogénalcényle en C₂ à C₆, alkoxy en C₁ à C₄, halogénalkoxy en C₁ à C₄, alkylthio en C₁ à C₄, halogénalkylthio en C₁ à C₄ ;
R⁴ représente en outre un groupe -CH=NOH, formyle ; un reste cyclopropyloxy, cyclobutyloxy, cyclopentyloxy, cyclohexyloxy, cyclopropylméthoxy, cyclobutylméthoxy, cyclopentylméthoxy ou cyclohexylméthoxy, chacun éventuellement substitué une à trois fois identiques ou différentes par un radical fluoro, chloro et/ou méthyle,
R⁵ représente le fluor, le chlore, un reste triméthylsilyle, un reste alkyle en C₁ à C₄, alcényle en C₂ à C₆, alkoxy en C₁ à C₄, alcényloxy en C₂ à C₆, -S(O)ₚR⁶, -NR⁷R⁸, -COR⁶, CO₂R⁶ ou -CONR⁹R¹⁰, chacun éventuellement substitué une à neuf fois identiques ou différentes par un radical fluoro, chloro et/ou bromo,
p a la valeur 0, 1 ou 2,
n a la valeur 0, 1 ou 2, les substituants R⁵ pouvant être identiques ou différents lorsque n est égal à 2,
r a la valeur 0, 1 ou 2, les substituants R⁵ pouvant être identiques ou différents lorsque r est égal à 2,
R⁶ représente un reste alkyle en C₁ à C₁₀ éventuellement substitué une ou plusieurs fois identiques ou différentes par un radical fluoro, chloro, bromo et/ou -NR⁷R⁸, un reste cyclopropyle, cyclopentyle, cyclohexyle, phényle ou benzyle, chacun éventuellement substitué une à quatre fois identiques ou différentes par un radical fluoro, chloro, bromo, cyano, alkyle en C₁ à C₄, halogénalkyle en C₁ à C₄, alkoxy en C₁ à C₄, halogénalkoxy en C₁ à C₄, alkylthio en C₁ à C₄ et/ou halogénalkylthio en C₁ à C₄,
R⁶ représente en outre un reste alkyle en C₁ à C₁₀ substitué une à trois fois identiques ou différentes, les substituants pouvant en outre être choisis entre alkoxy en C₁ à C₄, alkylthio en C₁ à C₄, halogénalkoxy en C₁ à C₄ et/ou halogénalkylthio en C₁ à C₄,
R⁷ et R⁸ représentent indépendamment l'un de l'autre l'hydrogène, un groupe -SO₂R₆, -COR⁶, -CO₂R⁶, un reste alkyle en C₁ à C₁₆ ou alcényle en C₂ à C₁₆, chacun éventuellement substitué une ou plusieurs fois identiques ou différentes par un radical fluoro, chloro, bromo, (alkyle en C₁ à C₄)-carbonyle, (alkyle en C₁ à C₄)-carbonyloxy, alkylamino en C₁ à C₄, di-(alkyle en C₁ à C₄)-amino, alkoxy en C₁ à C₄, halogénalkoxy en C₁ à C₄, alkylthio en C₁ à C₄ et/ou halogénalkylthio en C₁ à C₄ ; un reste cycloalkyle en C₃ à C₁₀, (cycloalkyle en C₃ à C₆)-(alkyle en C₁ à C₄), phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazinyle, pyrazinyle, pipéridinyle, morpholinyle, thiomorpholinyle, triazinyle, triazolyle, quinolinyle ou isoquinolinyle, chacun éventuellement substitué une à trois fois identiques ou différentes par un radical fluoro, chloro, bromo, cyano, alkyle en C₁ à C₄, halogénalkyle en C₁ à C₄, alkoxy en C₁ à C₄, halogénalkoxy en C₁ à C₄, alkylthio en C₁ à C₄, et/ou halogénalkylthio en C₁ à C₄,
R⁷ et R⁸ représentent en outre indépendamment l'un de l'autre un reste pyridinylméthyle, pyridinyléthyle, pyrimidinylméthyle, pyrimidinyléthyle, pyridazylméthyle, pyridazyléthyle, pyrazinylméthyle ou pyrazinyléthyle, chacun éventuellement substitué une à trois fois identiques ou différentes par un radical fluoro, chloro, bromo, cyano, alkyle en C₁ à C₄, halogénalkyle en C₁ à C₄, alkoxy en C₁ à C₄, halogénalkoxy en C₁ à C₄, alkylthio en C₁ à C₄ et/ou halogénalkylthio en C₁ à C₄,
R⁷ et R⁸ représentent en outre conjointement un reste alcénylène en C₂ à C₁₀ éventuellement substitué une ou plusieurs fois identiques ou différentes par un radical fluoro, chloro, bromo, alkoxy en C₁ à C₄, halogénalkoxy en C₁ à C₄, alkylthio en C₁ à C₄ et/ou halogénalkylthio en C₁ à C₄, ou un reste alkylène en C₃ à C₁₀ éventuellement substitué dans la partie alkylène une ou plusieurs fois identiques ou différentes par un radical fluoro, chloro, cyano, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄, la chaîne alkylénique pouvant être interrompue dans chaque cas par -O-, -S- ou -NR¹⁵-,
R⁷ et R⁸ représentent en outre conjointement un reste alkylène en C₃ à C₁₀ substitué une à trois fois identiques ou différentes, les substituants pouvant en outre être choisis entre n-propoxycarbonyle, isopropoxycarbonyle, éthoxycarbonyle, méthoxycarbonyle, oxypropylénoxy, oxyéthylénoxy et/ou oxyméthylénoxy,
R⁷ et R⁸ représentent en outre conjointement un reste alkylène en C₃ à C₆, la chaîne alkylénique étant interrompue soit par C=O soit par C=NO-(alkyle en C₁ à C₄),
R⁹ et R¹⁰ représentent indépendamment l'un de l'autre l'hydrogène, un groupe -SO₂R⁶, un reste alkyle en C₁ à C₄ ou alcényle en C₂ à C₆, chacun éventuellement substitué une à neuf fois identiques ou différentes par un radical fluoro, chloro, bromo, alkylamino en C₁ à C₄, di-(alkyle en C₁ à C₄)-amino, alkoxy en C₁ à C₄, halogénalkoxy en C₁ à C₄, alkylthio en C₁ à C₄, et/ou halogénalkylthio en C₁ à C₄ ; un reste cycloalkyle en C₃ à C₆, (cycloalkyle en C₃ à C₆)-(alkyle en C₁ à C₄), phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle, chacun éventuellement substitué une à trois fois identiques ou différentes par un radical fluoro, chloro, bromo, alkyle en C₁ à C₄, cyano, halogénalkyle en C₁ à C₄, alkoxy en C₁ à C₄, halogénalkoxy en C₁ à C₄, alkylthio en C₁ à C₄ et/ou halogénalkylthio en C₁ à C₄,
R⁹ et R¹⁰ représentent en outre conjointement un reste alkylène en C₄ ou C₅, - (CH₂)₂-O-(CH₂)₂-, - (CH₂)₂-S- (CH₂)₂-, -(CH₂)₂-N(R¹⁵)-(CH₂)₂-, chacun éventuellement substitué dans la partie alkylène une à quatre fois identiques ou différentes par un radical fluoro, chloro, cyano, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄,
R¹¹ et R¹² représentent indépendamment l'un de l'autre l'hydrogène, un reste alkyle en C₁ à C₆ éventuellement substitué une à neuf fois identiques ou différentes par un radical fluoro, chloro, bromo, alkoxy en C₁ à C₄, alkylthio en C₁ à C₄, un reste cycloalkyle en C₃ à C₆, (cycloalkyle en C₃ à C₆)-(alkyle en C₁ à C₄), phényle, benzyle ou phényléthyle, chacun éventuellement substitué une à quatre fois identiques ou différentes par un radical fluoro, chloro, bromo, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄,
R¹¹ et R¹² représentent en outre conjointement un reste alkylène en C₃ à C₈ ou alcénylène en C₃ à C₈, chacun éventuellement substitué une à quatre fois identiques ou différentes par un radical fluoro, chloro, bromo, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄,
R¹⁵ représente l'hydrogène, un groupe -SO₂R⁶, un groupe -COR⁶ ou -CO₂R⁶ ; un reste alkyle en C₁ à C₁₆ ou alcényle en C₂ à C₁₆, chacun éventuellement substitué une ou plusieurs fois identiques ou différentes par un radical fluoro, chloro, bromo, cyano, méthylamino, éthylamino, di-(alkyle en C₁ à C₆) -amino, alkoxy en C₁ à C₄, halogénalkoxy en C₁ à C₄, alkylthio en C₁ à C₄, halogénalkylthio en C₁ à C₄ ; un reste cycloalkyle en C₃ à C₁₀, (cycloalkyle en C₃ à C₆)-(alkyle en C₁ à C₄), phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle, chacun éventuellement substitué une à trois fois identiques ou différentes par un radical fluoro, chloro, bromo, cyano, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄.

6. Δ¹-pyrrolines de formule (I) suivant la revendication 1, formule dans laquelle
R⁴ représente l'hydrogène, le fluor, le chlore, le brome, un groupe cyano, formyle, nitro, un reste tri-(alkyle en C₁ à C₄)-silyle ; un reste alkyle en C₁ à C₁₆, alcényle en C₂ à C₁₆, alkoxy en C₁ à C₁₆, alcényloxy en C₂ à C₁₆, chacun éventuellement substitué une ou plusieurs fois identiques ou différentes par un radical fluoro, chloro, bromo, cyano, -NR⁷R⁸ ; un groupe -S(O)ₚR⁶, -NR⁷R⁸, -COR⁶, -CO₂R⁶, -CONR⁹R¹⁰ ou -N(R¹¹)COR¹² ; ou un reste cycloalkyle en C₃ à C₁₀, (cycloalkyle en C₃ à C₆)-(alkyle en C₁ à C₄), phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidino, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, pipéridino, morpholinyle, thiomorpholinyle, morpholino, thiomorpholino, triazinyle, triazolyle, quinolinyle ou isoquinolinyle, chacun éventuellement substitué une à trois fois identiques ou différentes par un radical fluoro, chloro, bromo, cyano, alkyle en C₁ à C₄, halogénalkyle en C₁ à C₄, alcényle en C₂ à C₆, halogénalcényle en C₂ à C₆, alkoxy en C₁ à C₄, halogénalkoxy en C₁ à C₄, alkylthio en C₁ à C₄, halogénalkylthio en C₁ à C₄,
R⁶ représente un reste alkyle en C₁ à C₁₀ éventuellement substitué une ou plusieurs fois identiques ou différentes par un radical fluoro, chloro, bromo et/ou -NR⁷R⁸, un reste cyclopropyle, cyclopentyle, cyclohexyle, phényle ou benzyle, chacun éventuellement substitué une à quatre fois identiques ou différentes par un radical fluoro, chloro, bromo, cyano, alkyle en C₁ à C₄, halogénalkyle en C₁ à C₄, alkoxy en C₁ à C₄, halogénalkoxy en C₁ à C₄, alkylthio en C₁ à C₄ et/ou halogénalkylthio en C₁ à C₄,
R⁷ et R⁸ représentent, indépendamment l'un de l'autre, l'hydrogène, un groupe -SO₂R₆, -COR⁶, -CO₂R⁶, un reste alkyle en C₁ à C₁₆ ou alcényle en C₂ à C₁₆, chacun éventuellement substitué une ou plusieurs fois, identiques ou différentes par un radical fluoro, chloro, bromo, (alkyle en C₁ à C₄)-carbonyle, (alkyle en C₁ à C₄)-carbonyloxy, alkylamino en C₁ à C₄, di-(alkyle en C₁ à C₄)-amino, alkoxy en C₁ à C₄, halogénalkoxy en C₁ à C₄, alkylthio en C₁ à C₄ et/ou halogénalkylthio en C₁ à C₄ ; un reste cycloalkyle en C₃ à C₁₀, (cycloalkyle en C₃ à C₆)-(alkyle en C₁ à C₄), phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle, chacun éventuellement substitué une à trois fois identiques ou différentes par un radical fluoro, chloro, bromo, cyano, alkyle en C₁ à C₄, halogénalkyle en C₁ à C₄, alkoxy en C₁ à C₄, halogénalkoxy en C₁ à C₄, alkylthio en C₁ à C₄ et/ou halogénalkylthio en C₁ à C₄,
R⁷ et R⁸ représentent en outre conjointement un reste alcénylène en C₂ à C₁₀ éventuellement substitué une ou plusieurs fois identiques ou différentes par un radical fluoro, chloro, bromo, alkoxy en C₁ à C₄, halogénalkoxy en C₁ à C₄, alkylthio en C₁ à C₄ et/ou halogénalkylthio en C₁ à C₄, ou un reste alkylène en C₃ à C₁₀ éventuellement substitué dans la partie alkylène une ou plusieurs fois identiques ou différentes par un radical fluoro, chloro, cyano, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄, la chaîne alkylénique pouvant être interrompue dans chaque cas par -O-, -S- ou -NR¹⁵-,
et R¹, R², R³, m, Q, R⁵, p, n, r, R⁹, R¹⁰, R¹¹, R¹², R¹³, R¹⁴ et R¹⁵ ont les définitions indiquées dans la revendication 5.

7. Δ¹-pyrrolines de formule (I) suivant la revendication 1, formule dans laquelle
R¹ représente le fluor ou le chlore,
R² représente l'hydrogène ou le fluor,
R³ représente le fluor, le chlore, un reste méthyle, éthyle, n-propyle, isopropyle, n-butyle, isobutyle, sec.-butyle, tertiobutyle, trifluorométhyle, trifluoréthyle, méthoxy, éthoxy, n-propoxy, isopropoxy, n-butoxy, isobutoxy, sec.-butoxy, tertiobutoxy, trifluorométhoxy, trifluoréthoxy, méthylthio, éthylthio, n-propylthio, isopropylthio, n-butylthio, isobutylthio, sec.-butylthio, tertiobutylthio, trifluorométhylthio ou trifluoréthylthio,
m a la valeur 0 ou 1,
Q représente l'un des groupements suivants 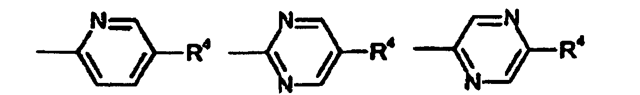 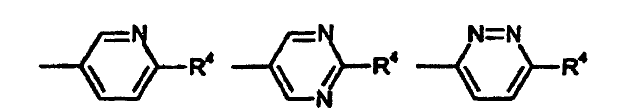
R⁴ représente l'hydrogène, le fluor, le chlore, le brome, un groupe cyano, formyle, un reste alkyle en C₁ à C₁₆, alcényle en C₂ à C₁₆, alkoxy en C₁ à C₁₆, alcényloxy en C₂ à C₁₆, chacun éventuellement substitué une ou plusieurs fois identiques ou différentes par un radical fluoro, chloro, bromo, cyano, -NR⁷R⁸ ; un groupe -S(O)ₚR⁶, -NR⁷R⁸, -COR⁶, -CO₂R⁶, -CONR⁹R¹⁰, -N(R¹¹)COR¹² ;
ou bien un reste cyclopropyle, cyclopentyle, cyclohexyle, cyclopropylméthyle, cyclopentylméthyle, cyclohexylméthyle, phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidino, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, pipéridino, morpholinyle, thiomorpholinyle, morpholino, thiomorpholino, triazinyle, triazolyle, quinolinyle ou isoquinolinyle, chacun éventuellement substitué une à trois fois identiques ou différentes par un radical fluoro, chloro, bromo, cyano, méthyle, éthyle, n-propyle, isopropyle, -CF₃, -CHF₂, -CClF₂, -CF₂CHFCl, -CF₂CH₂F, -CF₂CCl₃, -CH₂CF₃, -CF₂CHFCF₃, -CH₂CF₂H, -CH₂CF₂CF₃, -CF₂CF₂H, -CF₂CHFCF₃, vinyle, allyle, 1-propényle, butényle, -CF=CHF, -CF=CH₂, -CF=CCl₂, -CH=CF₂, -CF₂CF=CF₂, -CH=CFH, -CH₂CF=CF₂, -CF=CF₂, -CF₂CH=CF₂, méthoxy, éthoxy, n-propoxy, isopropoxy, n-butoxy, isobutoxy, sec.-butoxy, tertiobutoxy, trifluorométhoxy, difluorométhoxy, chlorodifluorométhoxy, trifluoréthoxy, méthylthio, éthylthio, n-propylthio, isopropylthio, n-butylthio, isobutylthio, sec.-butylthio, tertiobutylthio, trifluorométhylthio, difluorométhylthio, chlorodifluorométhylthio, trifluoréthylthio,
R⁴ représente en outre un groupe -CH=NOH, un reste formyle, cyclopropyloxy, cyclobutyloxy, cyclopentyloxy, cyclohexyloxy, cyclopropylméthoxy, cyclobutylméthoxy, cyclopentylméthoxy ou cyclohexylméthoxy,
p a la valeur 0, 1 ou 2,
R⁶ est un reste méthyle, éthyle, n-propyle, isopropyle, n-butyle, isobutyle, sec.-butyle, tertiobutyle, pentyle, hexyle, -CF₃, -CHF₂, -CCl₃, -CCl₂F, diméthylaminométhyle, diméthylaminoéthyle, diéthylaminométhyle, diéthylaminoéthyle, cyclopropyle, cyclopentyle, cyclohexyle, phényle ou benzyle,
R⁶ représente en outre un reste méthoxyméthyle, trifluorométhoxyméthyle, méthylthiométhyle ou trifluorométhylthiométhyle,
R⁷ et R⁸ représentent indépendamment l'un de l'autre l'hydrogène, un groupe -SO₂R⁶, -COR⁶, -CO₂R⁶, un reste alkyle en C₁ à C₁₆, alcényle en C₂ à C₁₆, chacun éventuellement substitué une ou plusieurs fois identiques ou différentes par un radical fluoro, chloro, bromo, (alkyle en C₁ à C₄)-carbonyle, (alkyle en C₁ à C₄)-carbonyloxy, alkylamino en C₁ à C₄, di-(alkyle en C₁ à C₄)-amino, alkoxy en C₁ à C₄, halogénalkoxy en C₁ à C₄, alkylthio en C₁ à C₄ et/ou halogénalkylthio en C₁ à C₄ ; un reste cycloalkyle en C₃ à C₈, cyclopropylméthyle, cyclopentylméthyle, cyclohexylméthyle, cyclopropyléthyle, cyclopentyléthyle, cyclohexyléthyle, phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle, chacun éventuellement substitué une à trois fois identiques ou différentes par un radical fluoro, chloro, bromo, cyano, alkyle en C₁ à C₄, halogénalkyle en C₁ à C₄, alkoxy en C₁ à C₄, halogénalkoxy en C₁ à C₄, alkylthio en C₁ à C₄ et/ou halogénalkylthio en C₁ à C₄,
R⁷ et R⁸ représentent en outre, indépendamment l'un de l'autre, un reste pyridinylméthyle, pyrimidinylméthyle, pyridazylméthyle ou pyrazinylméthyle, chacun éventuellement substitué une à trois fois identiques ou différentes par un radical fluoro, chloro, bromo, cyano, alkyle en C₁ à C₄, halogénalkyle en C₁ à C₄, alkoxy en C₁ à C₄, halogénalkoxy en C₁ à C₄, alkythio en C₁ à C₄ et/ou halogénalkylthio en C₁ à C₄,
R⁷ et R⁸ représentent en outre conjointement un reste alcénylène en C₂ à C₈, éventuellement substitué une ou plusieurs fois identiques ou différentes par un radical fluoro, chloro, bromo, alkoxy en C₁ à C₄, halogénalkoxy en C₁ à C₄, alkylthio en C₁ à C₄ et/ou halogénalkylthio en C₁ à C₄, ou un reste alkylène en C₃ à C₈ éventuellement substitué dans la partie alkylène une ou plusieurs fois identiques ou différentes par un radical fluoro, chloro, cyano, méthyle, éthyle, n-propyle, isopropyle, n-butyle, isobutyle, sec.-butyle, tertiobutyle, halogénalkyle en C₁ à C₄, méthoxy, éthoxy, n-propoxy, isopropoxy, n-butoxy, isobutoxy, sec.-butoxy, tertiobutoxy, halogénalkoxy en C₁ à C₄, méthylthio, éthylthio, n-propylthio, isopropylthio, n-butylthio, isobutylthio, sec.-butylthio, tertiobutylthio, halogénalkylthio en C₁ à C₄, la chaîne alkylénique pouvant être interrompue dans chaque cas par -O-, -S- ou -NR¹⁵-,
R⁷ et R⁸ représentent en outre conjointement un reste alkylène en C₃ à C₈ substitué une ou deux fois identiques ou différentes, les substituants pouvant être choisis en outre parmi les substituants éthoxycarbonyle, méthoxycarbonyle et/ou oxyéthylénoxy,
R⁷ et R⁸ représentent en outre conjointement un groupe -CH₂-CH₂-C(=O)-CH₂-CH₂-, -CH₂-CH₂C(=NO-Me)-CH₂-CH₂-, -CH₂-CH₂-C(=NO-Et)-CH₂-CH₂- ou -CH₂-CH₂-C(=NO-iPr)-CH₂-CH₂-,
R⁹ et R¹⁰ représentent indépendamment l'un de l'autre l'hydrogène, un groupe -SO₂CF₃, un reste méthyle, éthyle, n-propyle, isopropyle, n-butyle, isobutyle, sec.-butyle, tertiobutyle, pentyle, hexyle, -CF₃, -CH₂CF₃, - (CF₂)₃CF₃, cyclopropyle, cyclopentyle, cyclohexyle, méthoxyméthyle, méthoxyéthyle, ou un reste phényle ou benzyle chacun éventuellement substitué par un radical fluoro, chloro, bromo, méthyle, trifluorométhyle, méthoxy, trifluorométhoxy,
R⁹ et R¹⁰ représentent en outre conjointement un groupe -(CH₂)₄-, -(CH₂)₅-, -(CH₂)₆-, -CH₂CH(CH₃) -CH₂-CH(CH₃)-CH₂-, - (CH₂)₂-O-(CH₂)₂-, -(CH₂)₂-S-(CH₂)₂- ou - (CH₂)₂-N(R¹⁵)-(CH₂)₂-,
R¹¹ et R¹² représentent indépendamment l'un de l'autre un reste méthyle, éthyle, n-propyle, isopropyle, n-butyle, isobutyle, sec.-butyle, tertiobutyle, n-hexyle, trifluorométhyle, trifluoréthyle, cyclopropyle, cyclopentyle, cyclohexyle ou un reste phényle ou benzyle, chacun éventuellement substitué par un radical fluoro, chloro, bromo, méthyle, trifluorométhyle, méthoxy, trifluorométhoxy,
R¹¹ et R¹² représentent en outre conjointement un reste -(CH₂)₃-, -(CH₂)₄-, -(CH₂)₅-, -(CH₂)₆-, chacun éventuellement substitué par un radical fluoro, chloro, bromo, méthyle, éthyle, méthoxy, éthoxy, méthylthio, éthylthio, trifluorométhyle, trifluorométhoxy, trifluorométhylthio,
R¹⁵ représente l'hydrogène, un groupe -SO₂R⁶, un groupe -COR⁶ ou un groupe -CO₂R⁶ ; un reste alkyle en C₁ à C₁₆, alcényle en C₂ à C₁₆, chacun éventuellement substitué une ou plusieurs fois identiques ou différentes par un radical fluoro, chloro, bromo, cyano, méthylamino, éthylamino, di-(alkyle en C₁ à C₆)-amino, alkoxy en C₁ à C₄, halogénalkoxy en C₁ à C₄, alkylthio en C₁ à C₄, halogénalkylthio en C₁ à C₄ ; un reste cycloalkyle en C₃ à C₈, cyclopropylméthyle, cyclopentylméthyle, cyclohexylméthyle, cyclopropyléthyle, cyclopentyléthyle, cyclohexyléthyle, phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle, chacun éventuellement substitué une à trois fois identiques ou différentes par un radical fluoro, chloro, bromo, cyano, méthyle, éthyle, n-propyle, isopropyle, n-butyle, isobutyle, sec.-butyle, tertiobutyle, halogénalkyle en C₁ à C₄, méthoxy, éthoxy, n-propoxy, isopropoxy, n-butoxy, isobutoxy, sec.-butoxy, tertiobutoxy, halogénalkoxy en C₁ à C₄, méthylthio, éthylthio, n-propylthio, isopropylthio, n-butylthio, isobutylthio, sec.-butylthio, tertiobutylthio, halogénalkylthio en C₁ à C₄.

8. Δ¹-pyrrolines de formule (I) suivant la revendication 1, formule dans laquelle
R⁴ représente l'hydrogène, le fluor, le chlore, le brome, un groupe cyano, formyle, un reste alkyle en C₁ à C₁₆, alcényle en C₂ à C₁₆, alkoxy en C₁ à C₁₆, alcényloxy en C₂ à C₁₆, chacun éventuellement substitué une ou plusieurs fois identiques ou différentes par un radical fluoro, chloro, bromo, cyano, -NR⁷R⁸ ; un groupe -S(O)ₚR⁶, -NR⁷R⁸, -COR⁶, -CO₂R⁶, -CONR⁹R¹⁰, -N(R¹¹)COR¹² ; un reste cyclopropyle, cyclopentyle, cyclohexyle, cyclopropylméthyle, cyclopentylméthyle, cyclohexylméthyle, phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidino, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, pipéridino, morpholinyle, thiomorpholinyle, morpholino, thiomorpholino, triazinyle, triazolyle, quinolinyle ou isoquinolinyle, chacun éventuellement substitué une à trois fois identiques ou différentes par un radical fluoro, chloro, bromo, cyano, méthyle, éthyle, n-propyle, isopropyle, -CF₃, -CHF₂, -CClF₂, -CF₂CHFCl, -CF₂CH₂F, -CF₂CCl₃, -CH₂CF₃, -CF₂CHFCF₃, -CH₂CF₂H, -CH₂CF₂F₃, -CF₂CF₂H, -CF₂CHFCF₃, vinyle, allyle, 1-propényle, butényle, -CF=CHF, -CF=CH₂, -CF=CCl₂, -CH=CF₂, -CF₂CF=CF₂, -CH=CFH, -CH₂CF=CF₂, -CF=CF₂, -CF₂CH=CF₂, méthoxy, éthoxy, n-propoxy, isopropoxy, n-butoxy, isobutoxy, sec.-butoxy, tertiobutoxy, trifluorométhoxy, difluorométhoxy, chlorodifluorométhoxy, trifluoréthoxy, méthylthio, éthylthio, n-propylthio, isopropylthio, n-butylthio, isobutylthio, sec.-butylthio, tertiobutylthio, trifluorométhylthio, difluorométhylthio, chlorodifluorométhylthio, trifluoréthylthio,
R⁶ représente un reste méthyle, éthyle, n-propyle, isopropyle, n-butyle, isobutyle, sec.-butyle, tertiobutyle, pentyle, hexyle, -CF₃, -CHF₂, -CCl₃, -CCl₂F, diméthylaminométhyle, diméthylaminoéthyle, diéthylaminométhyle, diéthylaminoéthyle, cyclopropyle, cyclopentyle, cyclohexyle, phényle ou benzyle,
R⁷ et R⁸ représentent indépendamment l'un de l'autre l'hydrogène, un groupe -SO₂R⁶, -COR⁶, -CO₂R⁶, un reste alkyle en C₁ à C₁₆, alcényle en C₂ à C₁₆, chacun éventuellement substitué une ou plusieurs fois identiques ou différentes par un radical fluoro, chloro, bromo, (alkyle en C₁ à C₄)-carbonyle, (alkyle en C₁ à C₄)-carbonyloxy, alkylamino en C₁ à C₄, di-(alkyle en C₁ à C₄)-amino, alkoxy en C₁ à C₄, halogénalkoxy en C₁ à C₄, alkylthio en C₁ à C₄ et/ou halogénalkylthio en C₁ à C₄ ; un reste cycloalkyle en C₃ à C₈, cyclopropylméthyle, cyclopentylméthyle, cyclohexylméthyle, cyclopropyléthyle, cyclopentyléthyle, cyclohexyléthyle, phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle, chacun éventuellement substitué une à trois fois identiques ou différentes par un radical fluoro, chloro, bromo, cyano, alkyle en C₁ à C₄, halogénalkyle en C₁ à C₄, alkoxy en C₁ à C₄, halogénalkoxy en C₁ à C₄, alkylthio en C₁ à C₄ et/ou halogénalkylthio en C₁ à C₄,
R⁷ et R⁸ représentent en outre conjointement un reste alcénylène en C₂ à C₈, éventuellement substitué une ou plusieurs fois identiques ou différentes par un radical fluoro, chloro, bromo, alkoxy en C₁ à C₄, halogénalkoxy en C₁ à C₄, alkylthio en C₁ à C₄ et/ou halogénalkylthio en C₁ à C₄, ou un reste alkylène en C₃ à C₈ éventuellement substitué dans la partie alkylène une ou plusieurs fois identiques ou différentes par un radical fluoro, chloro, cyano, méthyle, éthyle, n-propyle, isopropyle, n-butyle, isobutyle, sec.-butyle, tertiobutyle, halogénalkyle en C₁ à C₄, méthoxy, éthoxy, n-propoxy, isopropoxy, n-butoxy, isobutoxy, sec.-butoxy, tertiobutoxy, halogénalkoxy en C₁ à C₄, méthylthio, éthylthio, n-propylthio, isopropylthio, n-butylthio, isobutylthio, sec.-butylthio, tertiobutylthio, halogénalkylthio en C₁ à C₄, la chaîne alkylénique pouvant être interrompue dans chaque cas par -O-, -S- ou -NR¹⁵-,
et R¹, R², R³, m, Q, p, R⁹, R¹⁰, R¹¹, R¹² et R¹⁵ ont les définitions indiquées dans la revendication 7.

9. Δ¹-pyrrolines de formule (I) suivant l'une des revendications 1 à 8, formule dans laquelle R¹ et R² représentent chacun le fluor.

10. Δ¹-pyrrolines de formule (I) suivant l'une des revendications 1 à 9, formule dans laquelle Q représente

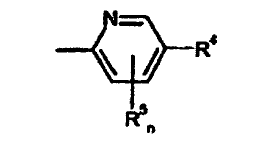

11. Δ¹-pyrrolines de formule (I) suivant l'une des revendications 1 à 9, formule dans laquelle Q représente

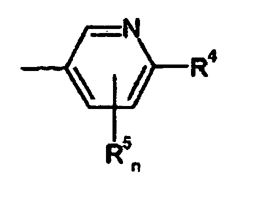

12. Δ¹-pyrrolines de formule (I) suivant l'une des revendications 1 à 9, formule dans laquelle Q représente

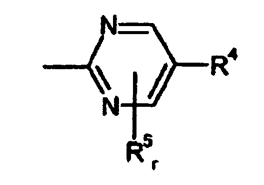

13. Δ¹-pyrrolines de formule (I) suivant l'une des revendications 1 à 9, formule dans laquelle Q représente

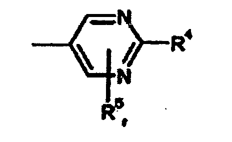

14. Δ¹-pyrrolines de formule (I) suivant l'une des revendications 1 à 9, formule dans laquelle Q représente

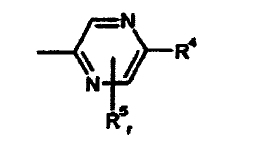

15. Δ¹-pyrrolines de formule (I) suivant l'une des revendications 1 à 9, formule dans laquelle Q représente

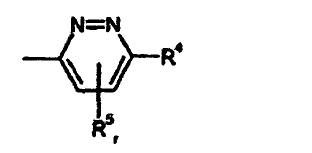

16. Composés de formules (I-a) à (I-e) 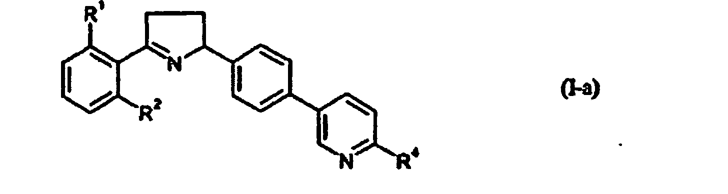 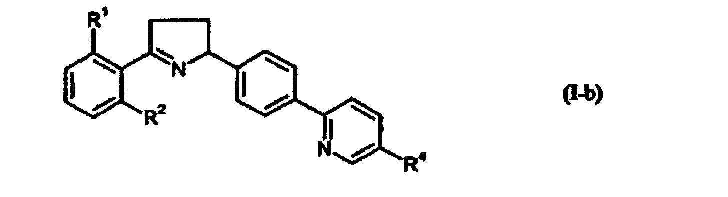 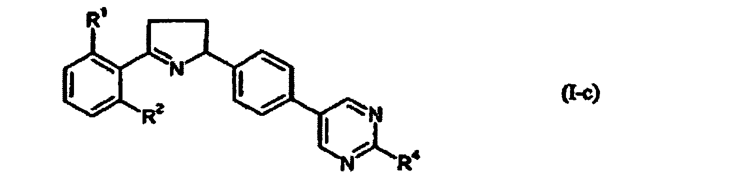 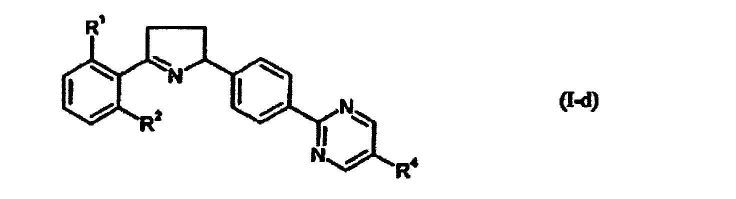 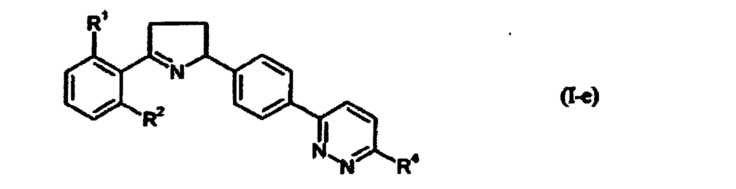 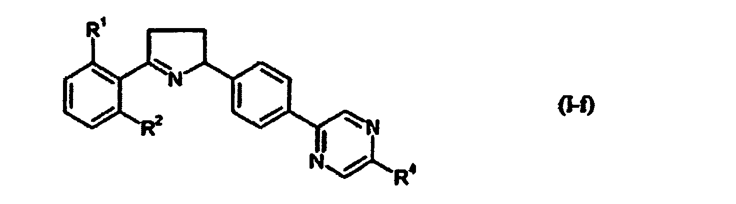 dans chacune desquelles
R¹ représente le fluor ou le chlore,
R² représente l'hydrogène ou le fluor et
R⁴ a les définitions indiquées dans l'une des revendications 1 à 8.

17. Composés à configuration (R), de formules (I-g) à (I-l) 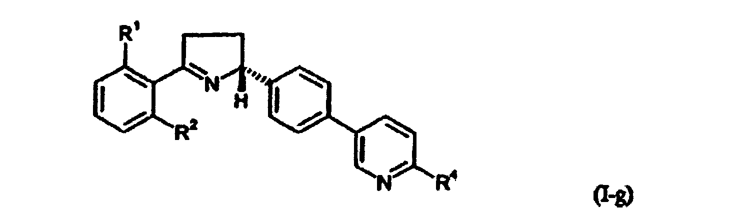 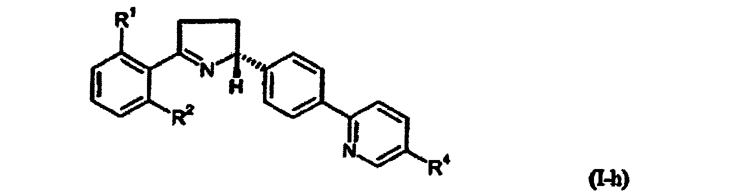 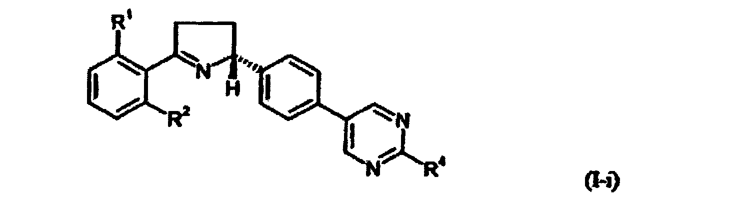 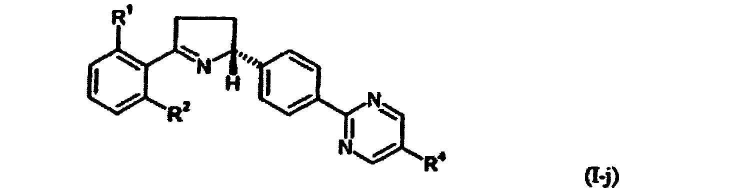 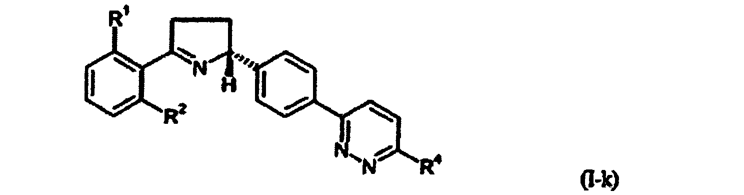 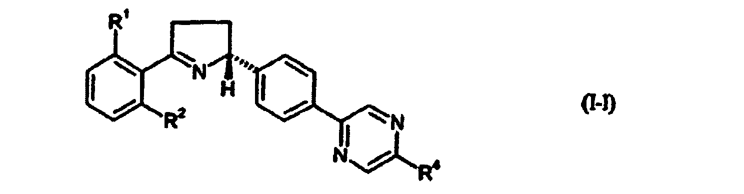 dans chacune desquelles
R¹ représente le fluor ou le chlore,
R² représente l'hydrogène ou le fluor et
R⁴ a les définitions indiquées dans l'une des revendications 1 à 8.

18. Procédé de production de composés de formule (I) suivant la revendication 1, **caractérisé en ce que**
A) on fait réagir des Δ¹-pyrrolines de formule (II) 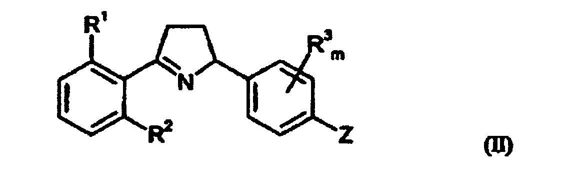 dans laquelle
R¹, R², R³ et m ont les définitions indiquées dans la revendication 1, et
Z représente le chlore, le brome, l'iode, un groupe -OSO₂CF₃ ou -OSO₂(CF₂)₃CF₃,
avec des hétérocycles de formule (III)
Q-X (III)
dans laquelle
Q a la définition indiquée dans la revendication 1, et
X représente le chlore, le brome, l'iode, un groupe -OSO₂CF₃ ou -OSO₂(CF₂)₃CF₃,
en présence d'un catalyseur, en présence d'un ester d'acide diboronique et, éventuellement, en présence d'un accepteur d'acide et, le cas échéant, en présence d'un diluant, dans une réaction en tandem,
ou bien
B) on fait réagir des Δ¹-pyrrolines de formule (IV) 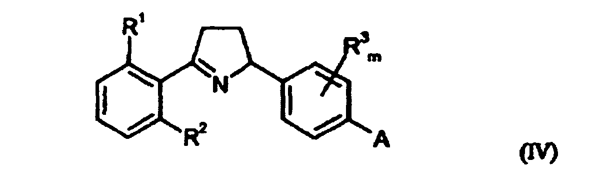 dans laquelle
R¹, R², R³ et m ont les définitions indiquées dans la revendication 1, et
A représente un groupe -B(OH)₂, (4,4,5,5-tétraméthyl-1,3,2-dioxaborolane)-2-yle, (5,5-diméthyl-1,3,2-dioxaborinane)-2-yle, (4,4,6-triméthyl-1,3,2-dioxaborinane)-2-yle ou 1,3,2-benzodioxaborol-2-yle,
avec des hétérocycles de formule (III)
Q-X (III)
dans laquelle Q et X ont les définitions indiquées dans la revendication 1,
en présence d'un catalyseur, éventuellement en présence d'un accepteur d'acide et, le cas échéant, en présence d'un diluant,
ou
C) on fait réagir des Δ¹-pyrrolines de formule (II) 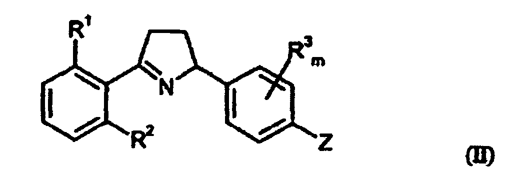 dans laquelle R¹, R², R³, m et Z ont les définitions indiquées dans la revendication 1
avec des dérivés d'acide boronique de formule (V)
Q-A (V)
dans laquelle Q et A ont les définitions indiquées dans la revendication 1,
en présence d'un catalyseur, éventuellement en présence d'un accepteur d'acide et, le cas échéant, en présence d'un diluant,
ou bien
D) on fait réagir des Δ¹-pyrrolines de formule (II-a) 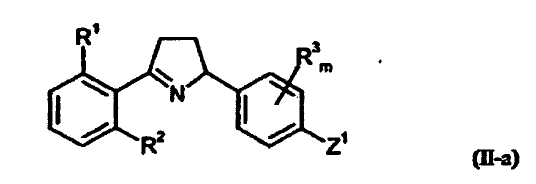 dans laquelle
R¹, R², R³, m ont les définitions indiquées dans la revendication 1, et
Z¹ représente le brome ou l'iode,
avec des composés organométalliques de formule (VI)
Q-M (VI)
dans laquelle
Q a les définitions indiquées dans la revendication 1, et
M représente ZnCl, Sn(Me)₃ ou Sn(n-Bu)₃,
en présence d'un catalyseur, éventuellement en présence d'un accepteur d'acide et, le cas échéant, en présence d'un diluant.

19. Compositions pesticides, **caractérisées par** une teneur en au moins un composé de formule (I) suivant la revendication 1, à côté de diluants et/ou de substances tensioactives.

20. Utilisation de composés de formule (I) suivant la revendication 1 pour combattre des parasites.

21. Procédé de lutte contre des parasites, **caractérisé en ce qu'**on fait agir des composés de formule (I) suivant la revendication 1 sur les parasites et/ou sur leur milieu.

22. Procédé de préparation de compositions pesticides, **caractérisé en ce qu'**on mélange des composés de formule (I) selon la revendication 1 avec des diluants et/ou des substances tensioactives.
